(19)

Europäisches
Patentamt
European
Patent Office
Office européen
des brevets

(11) **EP 2 405 022 A2**

(12) **EUROPEAN PATENT APPLICATION**

(43) Date of publication:
**11.01.2012 Bulletin 2012/02**

(51) Int Cl.:
*C12Q 1/68* (2006.01)   *G01N 33/50* (2006.01)

(21) Application number: **11176860.2**

(22) Date of filing: **08.07.2009**

(84) Designated Contracting States:
**AT BE BG CH CY CZ DE DK EE ES FI FR GB GR HR HU IE IS IT LI LT LU LV MC MK MT NL NO PL PT RO SE SI SK SM TR**
Designated Extension States:
**AL BA RS**

(30) Priority: **08.07.2008 US 134208 P**
**15.07.2008 US 135007 P**
**10.09.2008 US 191688 P**

(62) Document number(s) of the earlier application(s) in accordance with Art. 76 EPC:
**09790154.0 / 2 315 858**

(71) Applicant: **Genomic Health, Inc.**
**Redwood City, CA 94063 (US)**

(72) Inventors:
• **Davis-Bankaitis, Danute**
**Longmont, CO 80503 (US)**

• **Siconolfi, Lisa**
**Westminster, CO 80234 (US)**
• **Storm, Kathleen**
**Longmont, CO 80503 (US)**
• **Wassmann, Karl**
**Dover, MA 02030 (US)**

(74) Representative: **Ellis, Katherine Elizabeth Sleigh**
**Williams Powell**
**Staple Court**
**11 Staple Inn Buildings**
**London, WC1V 7QH (GB)**

Remarks:
This application was filed on 08-08-2011 as a divisional application to the application mentioned under INID code 62.

(54)   **Gene expression profiling for predicting the survivability of prostate cancer subjects**

(57)   A method is provided in various embodiments for determining a profile data set for predicting the survivability of a subject with prostate cancer based on a sample from the subject, wherein the sample provides a source of RNAs. The method includes using amplification under measurement conditions that are substantially repeatable for measuring the amount of RNA corresponding to at least SPARC. Alternatively, the method uses electrophoresis or immunohistochemistry for measuring the mount of protein corresponding to at least SPARC. The profile data set comprises the measure of each constituent.

Markov: c1(t) +2.07 ABL2 - 1.08 C1QA
ZIP:    c2   +2.05 ABL2 - 0.94 C1QA
Cox:    c3(t) +2.26 ABL2 - 1.31 C1QA

**Legend:**

| Line | Model Type |
|---|---|
| - - - - - - | Zero Inflated Poisson |
| ———— | Cox-Type |
| — · — · — | Markov Model |

Fig. 1

**EP 2 405 022 A2**

## Description

### REFERENCE TO RELATED APPLICATIONS

This application claims the benefit of U.S. Provisional Application No. 61/134,208 filed July 8, 2008, U.S. Provisional Application No. 61/135,007 filed July 15, 2008, and U.S. Provisional Application No. 61/191,688 filed September 10, 2008. The contents of each are hereby incorporated by reference their entireties.

### FIELD OF THE INVENTION

The present invention relates generally to the identification of biological markers of prostate cancer-diagnosed subjects capable of predicting primary end-points of prostate cancer progression. More specifically, the present invention relates to the use of gene expression data in the prediction of the survivability and/or survival time of prostate cancer-diagnosed subjects.

### BACKGROUND OF THE INVENTION

Prostate cancer is the most common cancer diagnosed among American men, with more than 234,000 new cases per year. As a man increases in age, his risk of developing prostate cancer increases exponentially. Under the age of 40, 1 in 1000 men will be diagnosed; between ages 40-59, 1 in 38 men will be diagnosed and between the ages of 60-69, 1 in 14 men will be diagnosed. More that 65% of all prostate cancers are diagnosed in men over 65 years of age. Beyond the significant human health concerns related to this dangerous and common form of cancer, its economic burden in the U.S. has been estimated at $8 billion dollars per year, with average annual costs per patient of approximately $12,000.

Prostate cancer is a heterogeneous disease, ranging from asymptomatic to a rapidly fatal metastatic malignancy.

Early prostate cancer usually causes no symptoms. However, the symptoms that do present are often similar to those of diseases such as benign prostatic hypertrophy. Such symptoms include frequent urination, increased urination at night, difficulty starting and maintaining a steady stream of urine, blood in the urine, and painful urination. Prostate cancer may also cause problems with sexual function, such as difficulty achieving erection or painful ejaculation.

Currently, there is no single diagnostic test capable of differentiating clinically aggressive from clinically benign disease, or capable of predicting the progression of localized prostate cancer and the likelihood of metastasis. Since individuals can have prostate cancer for several years and remain asymptomatic while the disease progresses and metastasizes, screenings are essential to detect prostate cancer at the earliest stage possible. Although early detection of prostate cancer is routinely achieved with physical examination and/or clinical tests such as serum prostate-specific antigen (PSA) test, this test is not definitive, since PSA levels can also be elevated due to prostate infection, enlargement, race and age effects. Generally, the higher the level of PSA, the more likely prostate cancer is present. However, a PSA level above the normal range (depending on the age of the patient) could be due to benign prostatic disease. In such instances, a diagnosis would be impossible to confirm without biopsying the prostate and assigning a Gleason Score. Additionally, regular screening of asymptomatic men remains controversial since the PSA screening methods currently available are associated with high false- positive rates, resulting in unnecessary biopsies, which can result in significant morbidity.

Additionally, there are currently no available prognostic tests capable of predicting the survival time of a prostate cancer patient. Previous studies have correlated survival time of the patient with the extent and spread of the prostatic carcinoma. For example, studies have shown that when the cancer is confined to the prostate gland, median survival in excess of 5 years can be anticipated. Patients with locally advanced cancer are not usually curable, and a substantial fraction will eventually die of their tumor, within a median of 1-3 years. Other factors affecting the prognosis of patients with prostate cancer that may be useful in making therapeutic decisions include histologic grade of the tumor, patient's age, other medical illnesses, and PSA levels. However, such studies and factors are guesses at best and are incapable guiding therapeutic decisions.

Information on any condition of a particular patient and a patient's response to therapeutic or nutritional agents has become an important issue in clinical medicine today not only from the aspect of efficiency of medical practice for the health care industry but for improved outcomes and benefits for the patients. The clinical course of prostate cancer disease can be unpredictable and the prognostic significance of the current diagnostic measures remains unclear. Thus there is the need for tests which can aid in the diagnosis, monitor the progression and treatment, as well as predict the survival time of patients with prostate cancer.

## SUMMARY OF THE INVENTION

The invention is in based in part upon the identification of gene and/or protein expression profiles (Precision Profiles™) associated with prostate cancer. These genes and/or proteins are referred to herein as prostate cancer survivability genes, prostate cancer survivability proteins or prostate cancer survivability constituents. More specifically, the invention is based upon the surprising discovery that detection of as few as one prostate cancer survivability gene and/or protein in a subject derived sample is capable of predicting the survivability and/or survival time of a patient suffering from prostate cancer with at least 75% accuracy. More particularly, the invention is based upon the surprising discovery that the methods provided by the invention are capable of predicting the survivability and/or survival time of a prostate cancer-diagnosed subject by assaying blood samples. Even more surprisingly, the predictive nature of the genes shown in the Precision Profile™ for Prostate Cancer Survivability (Table 1) or the Precision Protein Panel for Prostate Cancer Survivability (Table 20) is independent of any treatment of the prostate cancer diagnosed subject (*e.g.*, chemotherapy, hormone therapy, radiotherapy). The invention provides methods of evaluating the predicted survivability and/or survival time of a prostate cancer-diagnosed subject, based on a sample from the subject, the sample providing a source of RNAs, and determining a quantitative measure of the amount of at least one constituent of any constituent (*e.g.*, prostate cancer survivability gene) of Table 1 and arriving at a measure of each constituent. The invention also provides methods of evaluating the predicted survivability and/or survival time of a prostate cancer-diagnosed subject, based on a sample from the subject, the sample providing a source of protein, and determining a quantitative measure of the amount of at least one constituent of any constituent (*e.g.*, prostate cancer survivability protein) of Table 20, and arriving at a measure of each constituent.

In one embodiment, the method comprises detecting the presence or an absence of at least one protein constituent of Table 20 using immunoassays based on antibodies to proteins encoded by the genes described herein as predictive of prostate cancer survability (*e.g.*, one or more constituents of Tables 20). For example, the method comprises contacting a sample from said subject (e.g., whole blood or blood fraction (e.g., serum or plasma) with an antibody which specifically binds to at least one protein constituent of Table 20 to form an antibody/protein complex, and detecting the presence or absence of said complex in said sample, wherein a detectable complex is indicative of the presence said constituent in said sample, and wherein the presence of said constituent is indicative of increased survival time of said subject. In one embodiment, at least 6 protein constituents detected using immunoassays based on antibodies to proteins, wherein the proteins are are ABL2, SEMA4D, ITGAL, C1QA, TIMP1 and CDKN1A.

Also provided are methods of assessing the effect of a particular variable, including but not limited to age, PSA level, therapeutic agent, body mass index, ethnicity, and CTC count, on the precited survivability and/or survival time of a subject based on a sample from the subject, the sample providing a source of RNAs and/or protein, and determining a quantitative measure of the amount of at least one constituent of any constituent (*e.g.*, prostate cancer survivability gene or protein) of Table 1 and/or 20 as a distinct RNA and/or protein constituent in a sample obtained at a first period of time to produce a first subject data set and determining a quantitative measure of the amount of at least one constituent of any constituent of Table 1 and/or 20 as a distinct RNA and/or constituent in a sample obtained at a second period of time (*e.g.*, after administration of a therapeutic agent to said subject) to produce a second subject data set.

In a further aspect the invention provides methods of monitoring the progression of prostate cancer in a subject, based on a sample from the subject, the sample providing a source of RNAs and/or proteins, by determining a quantitative measure of the amount of at least one constituent of any constituent of Table 1 and/or 20 as a distinct RNA and/or protein constituent in a sample obtained at a first period of time to produce a first subject data set and determining a quantitative measure of the amount of at least one constituent of any constituent of Table 1 and/or 20 as a distinct RNA and/or protein constituent in a sample obtained at a second period of time to produce a second subject data set. Optionally, the constituents measured in the first sample are the same constituents measured in the second sample. The first subject data set and the second subject data set are compared allowing effect of the agent on the predicted survivability and/or survival time to be determined. The second subject sample is taken *e.g.*, one day, one week, one month, two months, three months, 1 year, 2 years, or more after the first subject sample. Optionally the first subject sample is taken prior to the subject receiving treatment, *e.g.* chemotherapy, radiation therapy, or surgery and the second subject sample is taken after treatment.

In various aspects the invention provides a method for determining a profile data set, *i.e.,* a prostate cancer survivability profile, for characterizing the predicted survivability and/or survival time of a subject with prostate cancer based on a sample from the subject, the sample providing a source of RNAs and/or, by using amplification for measuring the amount of RNA and/or protein in a panel of constituents including at least 1 constituent from Table 1 and/or 20, and arriving at a measure of each constituent. The profile data set contains the measure of each constituent of the panel.

In various aspects, the invention also provides a method for providing an index that is indicative of the predicted survivability or survival time of a prostate-cancer diagnosed subject, based on a sample from the subject, the method comprising: using amplification for measuring the amount of at least one constituent of Table 1 and/or 20 as a distinct RNA and/or protein constituent in the subject sample, wherein such measure is obtained under measurement conditions that are substantially repeatable to form a first profile data set, and applying values from said first profile data set to an index function, thereby providing a single-valued measure of the predicted probability of survivability or survival time so as to produce an index pertinent to the predicted survivability or survival time of the subject.

The methods of the invention further include comparing the quantitative measure of the constituent in the subject derived sample to a reference value. The reference value is for example an index value. Comparison of the subject measurements to a reference value allows for the the prediction of the primary endpoints of prostate cancer progression (*e.g.*, metastasis and/or survivability) to be determined.

In various aspects of the invention the methods are carried out wherein the measurement conditions are substantially repeatable, particularly within a degree of repeatability of better than ten percent, five percent or more particularly within a degree of repeatability of better than three percent, and/or wherein efficiencies of amplification for all constituents are substantially similar, more particularly wherein the efficiency of amplification is within ten percent, more particularly wherein the efficiency of amplification for all constituents is within five percent, and still more particularly wherein the efficiency of amplification for all constituents is within three percent or less.

In addition, the one or more different subjects may have in common with the subject at least one of age group, gender, ethnicity, geographic location, nutritional history, medical condition, clinical indicator, medication, physical activity, body mass, and environmental exposure. A clinical indicator may be used to assess the predicted survivability and/or survival time of the one or more different subjects, and may also include interpreting the calibrated profile data set in the context of at least one other clinical indicator, wherein the at least one other clinical indicator includes blood chemistry, X-ray or other radiological or metabolic imaging technique, molecular markers in the blood, other chemical assays, and physical findings.

At least 1, 2, 3, 4, 5, 6, 7, 8, 9, 10, 15, 20, 30 40, 50 or more constituents are measured.

Preferably, at least one constituent is measured. For example the constituent is selected from Table 1 and is selected from:

ABL2, BCAM, BCL2, C1QA, C1QB, CAV2, CDKN1A, CREBBP, E2F1, ELA2, FGF2, ITGAL, MYC, NCOA4, NFATC2, NUDT4, RP51077B9.4, SEMA4D, SPARC, TIMP1 or XK. In one embodiment, the constituent is ABL2.

In one aspect, two constituents from Table 1 are measured. The first constituent is i) ABL2, BCAM, BCL2, C1QA, C1QB, CAV2, CDKN1A, CREBBP, E2F1, ELA2, FGF2, ITGAL, MYC, NCOA4, NFATC2, NUDT4, RP51077B9.4, SEMA4D, SPARC, TIMP1 or XK; and the second constituent is ACPP AKT1, C1QA, C1QB, CA4, CASP9, CAV2, CCND2, CD44, CD48, CD59, CDC25A, CDH1, CDK2, CDK5, CDKN1A, CDKN1A, CDKN2A, CDKN2D, CEACAM1, COL6A2, COVA1, CREBBP, CTNNA1, CTSD, DAD1, DLC1, E2F1, E2F5, ELA2, EP300, EPAS1, ERBB2, ETS2, FAS, FGF2, FOS, G1P3, G6PD, GNB1, GSK3B, GSTT1, HMGA1, HRAS, HSPA1A, ICAM1, IFI16, IFITM1, IGF1R, IGF2BP2, IGFBP3, IL1B, IQGAP1, IRF1, ITGA1, ITGAL, ITGB1, JUN, KAI1, LGALS8, MAP2K1, MAPK1, MAPK14, MEIS1, MMP9, MNDA, MTA1, MTF1, MYC, MYD88, NAB1, NCOA1, NCOA4, NEDD4L, NFATC2, NFKB1, NME1, NOTCH2, NR4A2, NRAS, NRP1, NUDT4, PDGFA, PLAU, PLXDC2, PTCH1, PTEN, PTGS2, PTPRC, PYCARD, RAF1, RB1, RBM5, RHOA, RHOC, RP51077B9.4, S100A11, S100A6, SEMA4D, SERPINA1, SERPINE1, SERPING1, SIAH2, SKIL, SMAD3, SMAD4, SMARCD3, SOCS1, SOX4, SP1, SPARC, SRC, SRF, ST14, STAT3, SVIL, TEGT, TGFB1, THBS1, TIMP1, TLR2, TNF, TNFRSF1A, TOPBP1, TP53, TXNRD1, UBE2C, USP7, VEGF, VHL, VIM, XK, XRCC1, ZNF185, or ZNF350. For example, the first constituent is ABL2 and the second constituent is C1QA. In another embodiment, the first constituent is SEMA4D and the second constituent is TIMP1. In still another embodiment, the first constituent is ITGAL and the second constituent is CDKN1A. In yet another embodiment, the first constituent is CDKN1A and the second constituent is ITGAL.

In yet another aspect, at least six constituents from Table 1 are measured. For example, ABL2, SEMA4D, ITGAL, C1QA, TIMP1 and CDKN1A are measured.

The constituents are selected so as to predict the survivability and/or survival time of a prostate cancer-diagnosed subject with statistically significant accuracy. The prostate cancer-diagnosed subject is diagnosed with different stages of cancer. In one embodiment, the prostate cancer-diagnosed subject is hormone or taxane refractory (with or without bone metastasis).

Preferably, the constituents are selected so as to predict the survivability and/or survival time or a prostate cancer-diagnosed subject with at least 75%, 80%, 85%, 90%, 95%, 97%, 98%, 99% or greater accuracy. By "accuracy" is meant that the method has the ability to correctly predict the survivability status and/or survival time of a prostate-cancer diagnosed subject. Accuracy is determined for example by comparing the results of the Gene Precision Profiling™ to the survivability status of the subject (*i.e.,* alive or dead).

For example the combination of constituents are selected according to any of the models enumerated in Tables 5, 7A-7D or 8. In some embodiments, any of the models enumerated in any of Tables 5, 7A-7D or 8 are combined (*e.g..*, averaged) to form additional multi-gene models capable of predict the survivability and/or survival time or a prostate cancer-diagnosed subject with at least 75%, 80%, 85%, 90%, 95%, 97%, 98%, 99% or greater accuracy.

By prostate cancer or conditions related to prostate cancer is meant the malignant growth of abnormal cells in the prostate gland, capable of invading and destroying other prostate cells, and spreading (metastasizing) to other parts of the body, including bones and lymph nodes.

The sample is any sample derived from a subject which contains RNA and/or protein. For example, the sample is blood, a blood fraction, body fluid, a population of cells or tissue from the subject, a prostate cell, or a rare circulating tumor cell or circulating endothelial cell found in the blood.

Optionally one or more other samples can be taken over an interval of time that is at least one month between the first sample and the one or more other samples, or taken over an interval of time that is at least twelve months between the first sample and the one or more samples, or they may be taken pre-therapy intervention or post-therapy intervention. In such embodiments, the first sample may be derived from blood and the baseline profile data set may be derived from tissue or body fluid of the subject other than blood. Alternatively, the first sample is derived from tissue or bodily fluid of the subject and the baseline profile data set is derived from blood.

Also included in the invention are kits for predicting the survivability and/or survival time of prostate cancer-diagnosed subject, containing at least one reagent for the detection or quantification of any constituent measured according to the methods of the invention and instructions for using the kit.

Unless otherwise defined, all technical and scientific terms used herein have the same meaning as commonly understood by one of ordinary skill in the art to which this invention belongs. Although methods and materials similar or equivalent to those described herein can be used in the practice or testing of the present invention, suitable methods and materials are described below. All publications, patent applications, patents, and other references mentioned herein are incorporated by reference in their entirety. In case of conflict, the present specification, including definitions, will control. In addition, the materials, methods, and examples are illustrative only and not intended to be limiting.

Other features and advantages of the invention will be apparent from the following detailed description and claims.

## BRIEF DESCRIPTION OF THE DRAWINGS

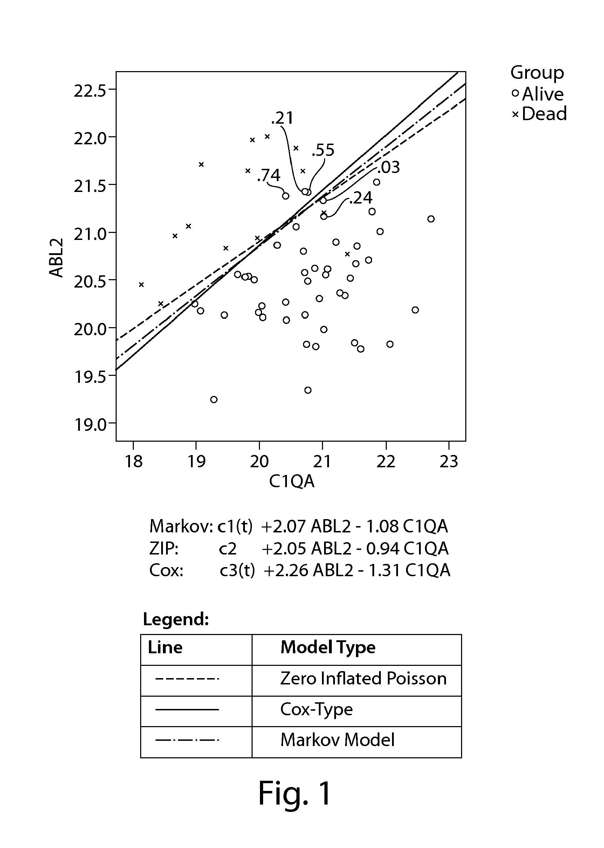

Figure 1 is a graphical representation of a 2-gene model, ABL2 and C1QA, based on the Precision Profile™ for Prostate Cancer Survivability (Table 1), identified using Cox-Type, Zero-Inflation Poisson, and Markov survival models, capable of predicting the survivability status of hormone or taxane refractory prostate cancer (with or without bone metastasis) (cohort 4) with statistically significant accuracy. The discrimination lines for each type of survival model superimposed onto the graph is an example of the Index Function evaluated at a particular value. Values below and to the right of the line represent subjects predicted to be alive. Values to the above and to the left of the line represent subjects predicted to be dead. ABL2 values are plotted along the Y-axis, C1QA values are plotted along the X-axis.

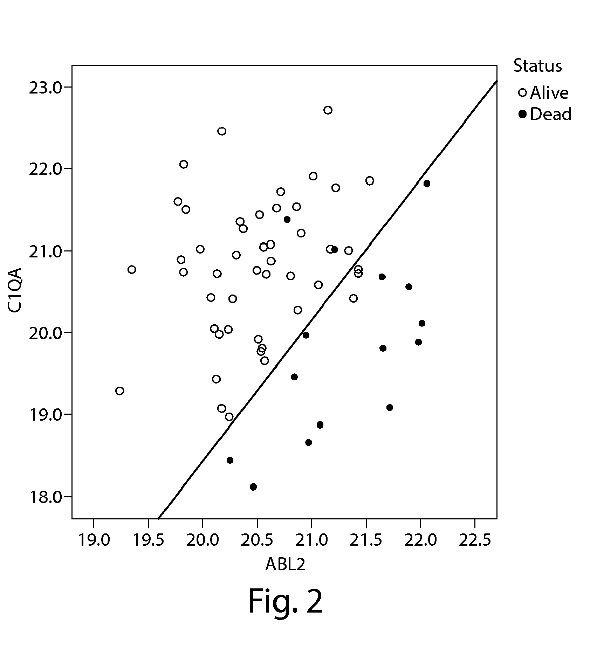

Figure 2 is a graphical representation of a 2-gene model, ABL2 and C1QA, based on the Precision Profile for Prostate Cancer Survivability (Table 1), identified using a Cox-Type survival model, capable of predicting the survivability status of hormone or taxane refractory prostate cancer (with or without bone metastasis) (cohort 4) with statistically significant accuracy. The discrimination lines for each type of survival model superimposed onto the graph is an example of the Index Function evaluated at a particular value. Values above and to the left of the line represent subjects predicted to be alive. Values to the below and to the right of the line represent subjects predicted to be dead. ABL2 values are plotted along the X-axis, C1QA values are plotted along the Y-axis.

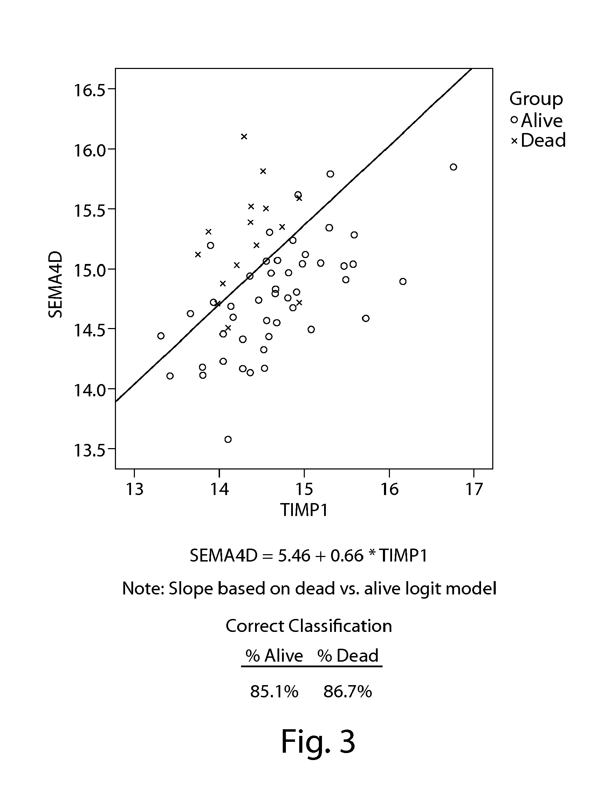

Figure 3 is a graphical representation of a 2-gene model, SEMA4D and TIMP1, based on the Precision Profile for Prostate Cancer Survivability (Table 1), identified using Cox-Type and Zero-Inflation Poisson survival models, capable of predicting the survivability status of hormone or taxane refractory prostate cancer (with or without bone metastasis) (cohort 4) with statistically significant accuracy. The discrimination line is based on a dead vs. alive logit model. Values below and to the right of the line represent subjects predicted to be alive. Values to the above and to the left of the line represent subjects predicted to be dead. SEMA4D values are plotted along the Y-axis, TIMP1 values are plotted along the X-axis.

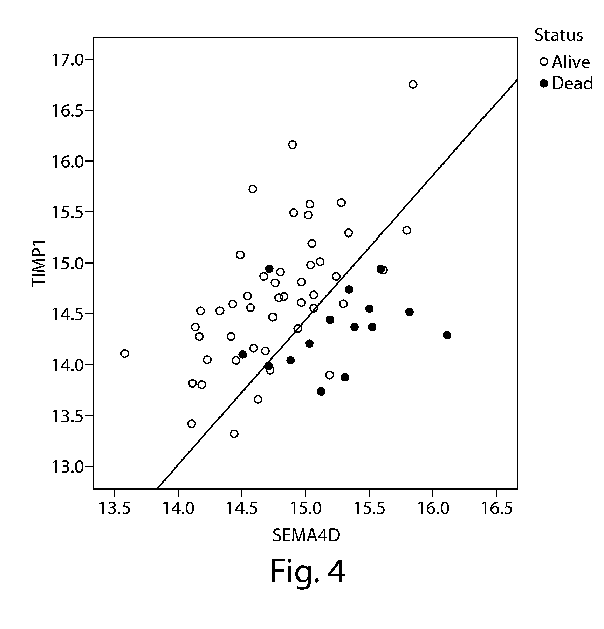

Figure 4 is a graphical representation of a 2-gene model, SEMA4D and TIMP1, based on the Precision Profile™ for Prostate Cancer Survivability (Table 1), identified using Cox-Type and Zero-Inflation Poisson survival models, capable of predicting the survivability status of hormone or taxane refractory prostate cancer (with or without bone metastasis) (cohort 4) with statistically significant accuracy. The discrimination line is based on a dead vs. alive logit model. Values above and to the left of the line represent subjects predicted to be alive. Values to the below and to the right of the line represent subjects predicted to be dead. SEMA4D values are plotted along the X-axis, TIMP1 values are plotted along the Y-axis.

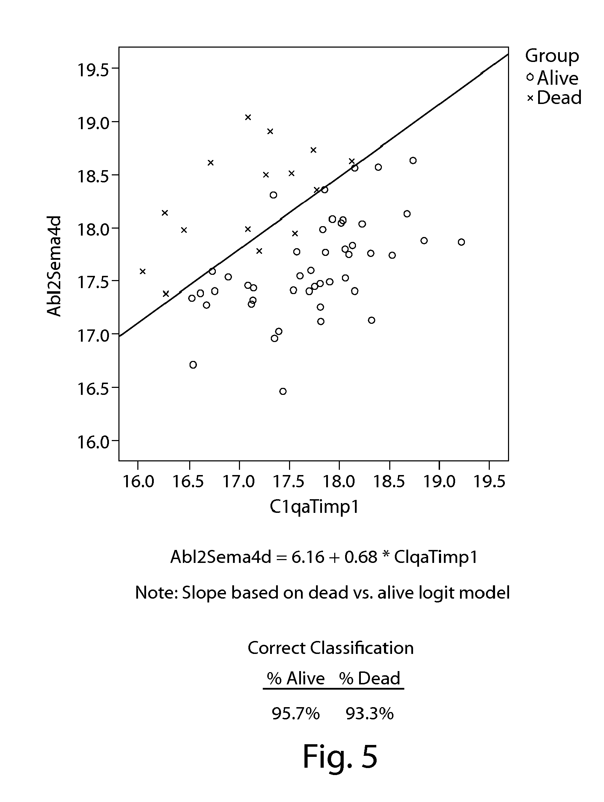

Figure 5 is a graphical representation of a 4-gene model, ABL2, SEMA4D, C1QA and TIMP1, based on the Precision Profile™ for Prostate Cancer Survivability (Table 1), identified using Cox-Type and Zero-Inflation Poisson survival models, capable of predicting the survivability status of hormone or taxane refractory prostate cancer (with or without bone metastasis) (cohort 4) with statistically significant accuracy. The discrimination line is based on a dead vs. alive logit model. Values below and to the right of the line represent subjects predicted to be alive. Values to the above and to the left of the line represent subjects predicted to be dead. The combined average of ABL2 and SEMA4D values are plotted along the Y-axis. The combined average of C1QA and TIMP1 values are plotted along the X-axis.

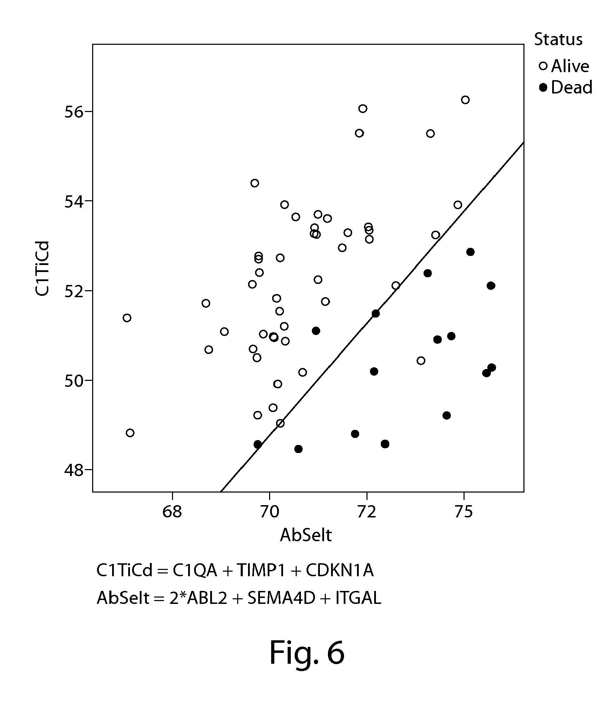

Figure 6 is a graphical representation of a 6-gene model, ABL2, SEMA4D, ITGAL and C1QA, TIMP1, CDKN1A, based on the Precision Profile™ for Prostate Cancer Survivability (Table 1), identified using a Cox-Type survival model, capable of predicting the survivability status of hormone or taxane refractory prostate cancer (with or without bone metastasis) (cohort 4) with statistically significant accuracy. The discrimination line is based on a dead vs. alive logit model. Values above and to the left of the line represent subjects predicted to be alive. Values to the below and to the right of the line represent subjects predicted to be dead. The combined average of ABL2, SEMA4D and ITGAL values (denoted as AbSeIt) are plotted along the X-axis. The combined average of C1QA, TIMP1 and CDKN1A values (denoted as C1TiCd) are plotted along the Y-axis.

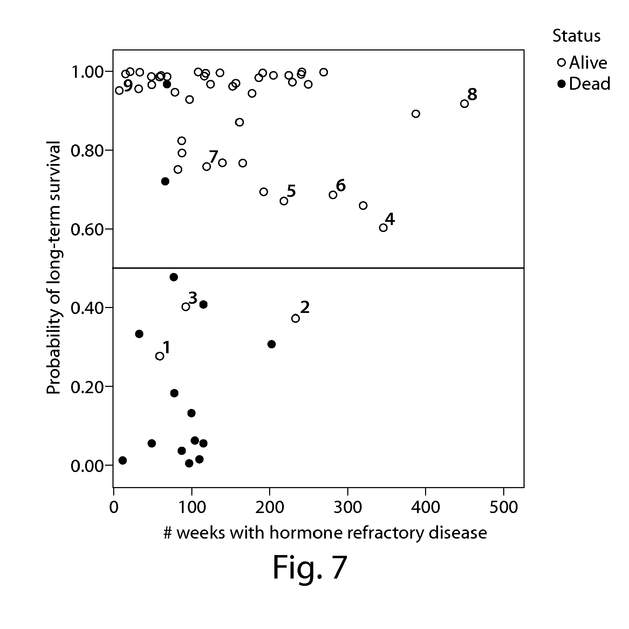

Figure 7 is an example of index, based on a 2-gene model, ABL2 and C1QA, capable of predicting the probability of long term survival in hormone or taxane refractory prostate cancer subjects with statistically significant accuracy. Prostate cancer subjects who were alive (denoted as open circles) as of the designated survival date of the study (June 20, 2008) were correctly classified by the index having increased probability of long-term survival, subjects who were dead (denoted as filled circles) as of the designated survival date of the study were correctly classified by the index as having a decreased probability of long-term survivability.

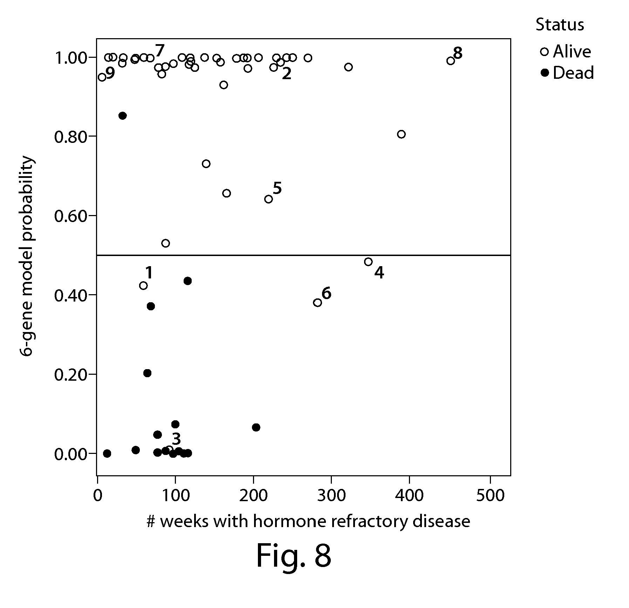

Figure 8 is an example of index, based on a 6-gene model, ABL2, SEMA4D, ITGAL and C1QA, TIMP1, CDKN1A, capable of predicting the probability of long term survival in hormone or taxane refractory prostate cancer subjects with statistically significant accuracy. Prostate cancer subjects who were alive (denoted as open circles) as of the designated survival date of the study (June 20, 2008) were correctly classified by the index having increased probability of long-term survival, subjects who were dead (denoted as filled circles) as of the designated survival date of the study were correctly classified by the index as having a decreased probability of long-term survivability.

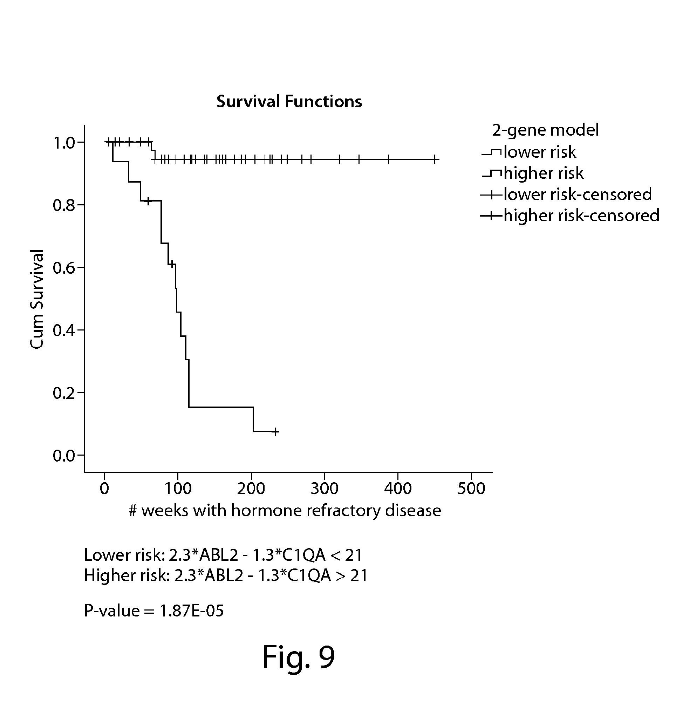

Figure 9 is a cumulative survival curve (Meier Kaplan) based on a 2-gene model, ABL2 and C1QA, obtained with survival time definition #1 (date classified as cohort 4 status).

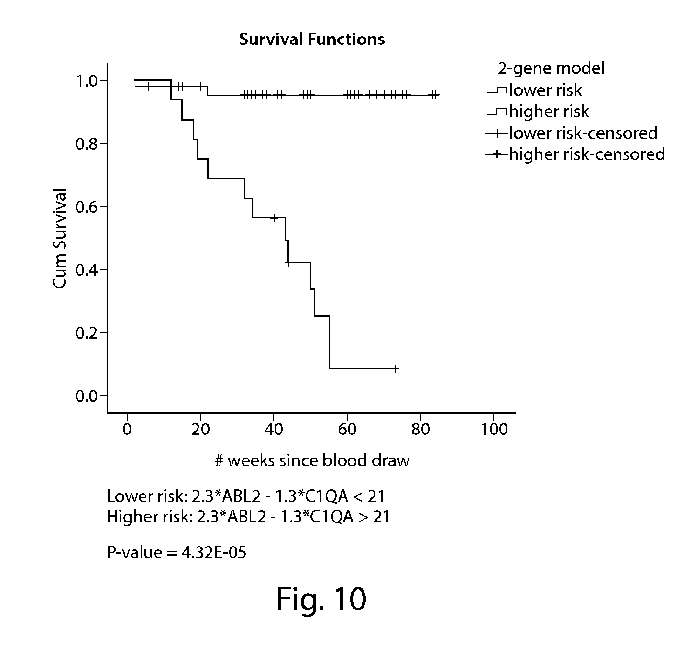

Figure 10 is a cumulative survival curve (Meier Kaplan) based on a 2-gene model, ABL2 and C1QA, obtained with survival time definition #2 (date of blood draw).

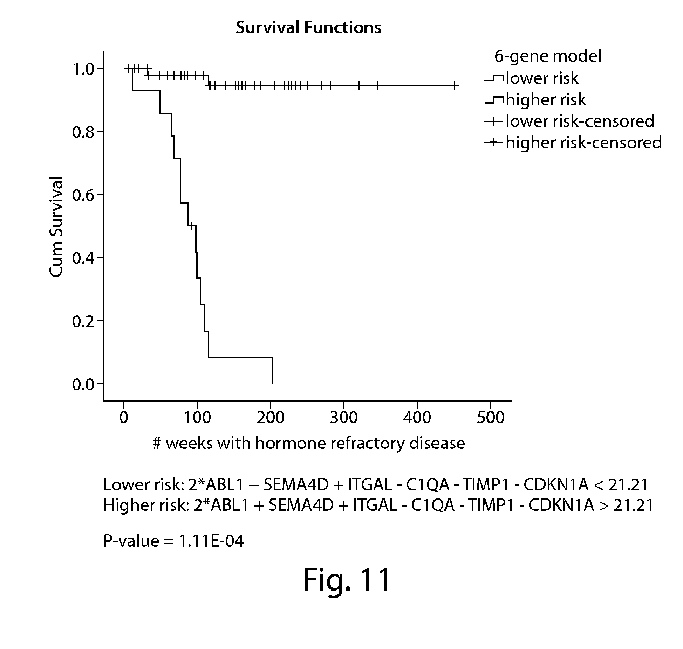

Figure 11 is a cumulative survival curve (Meier Kaplan) based on a 6-gene model, ABL2, SEMA4D, ITGAL and C1QA, TIMP1 CDKN1A, obtained with survival time definition #1 (date classified as cohort 4 status).

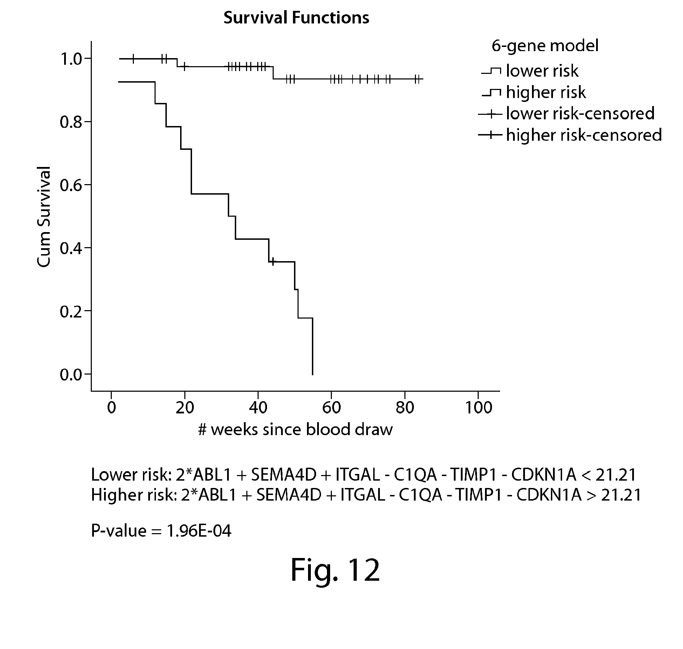

Figure 12 is a cumulative survival curve (Meier Kaplan) based on a a 6-gene model, ABL2, SEMA4D, ITGAL and C1QA, TIMP1 CDKN1A, obtained with survival time definition #2 (date of blood draw).

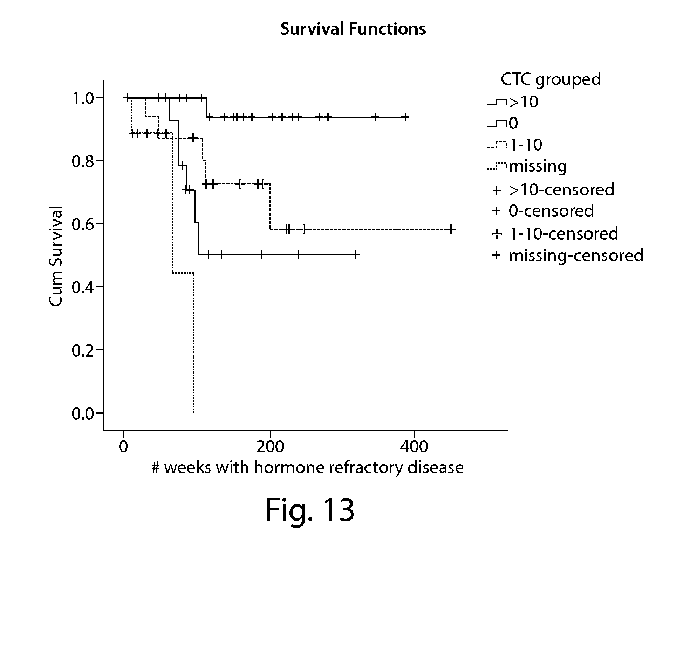

Figure 13 is a cumulative survival curve (Meier Kaplan) based CTC enumeration for various hormone refractory prostate cancer patients.

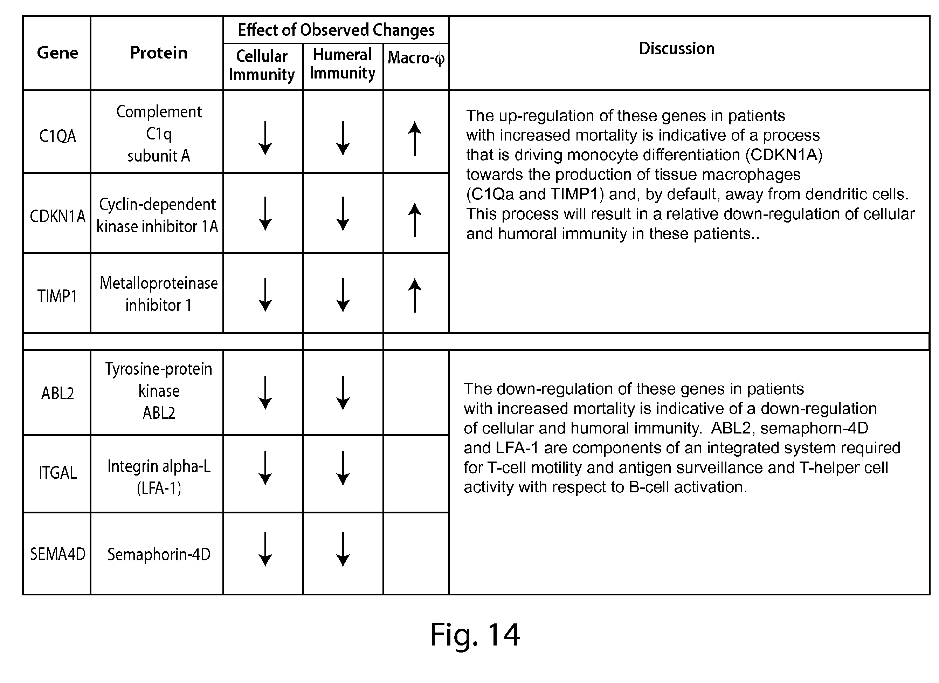

Figure 14 is a chart summarizing the observed effects of six-genes from the Precision Profile for Prostate Cancer Survivability (Table 1) on cellular and humoral immunity and macrophages.

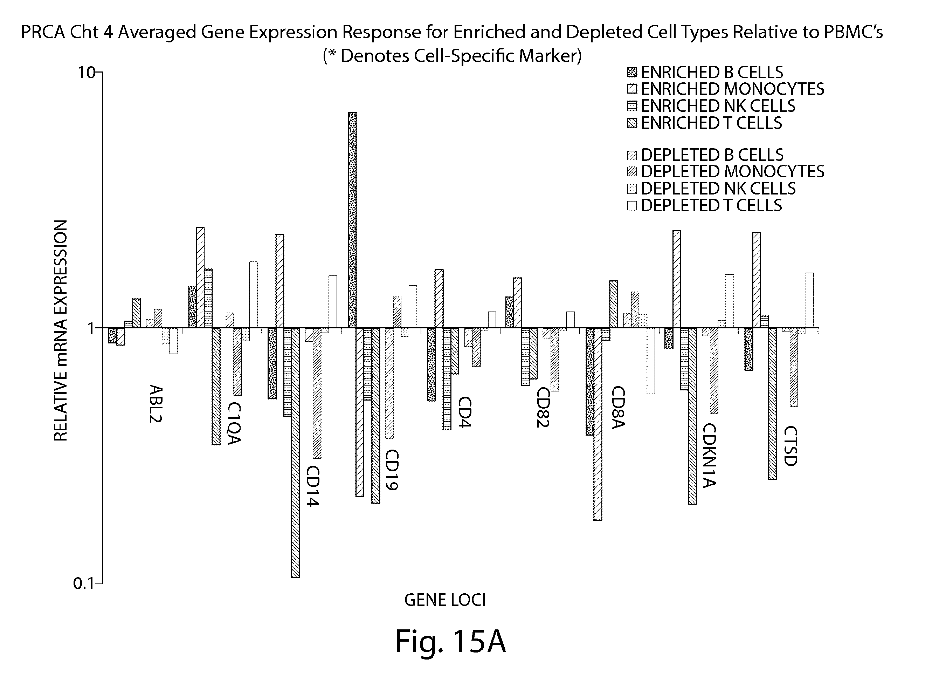

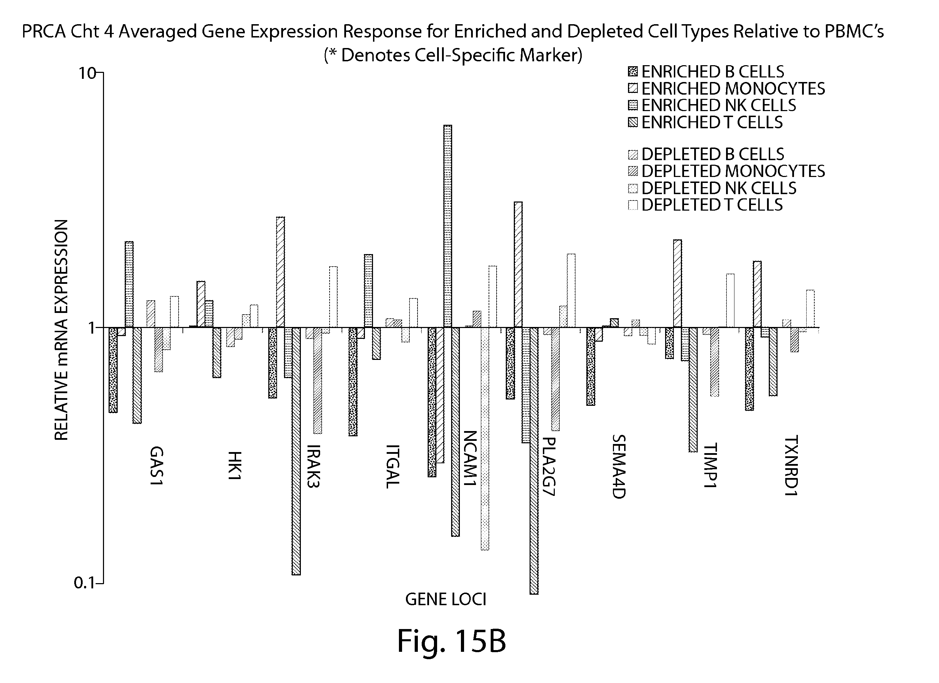

Figures 15A and 15B are bar graphs showing a quantitative comparison of gene expression levels between fractionated cell samples (B-cells, monocytes, T-cells, NK cells) from eleven hormone refractory prostate cancer cohort 4 subjects on a gene-by-gene basis for a panel of 18-genes.

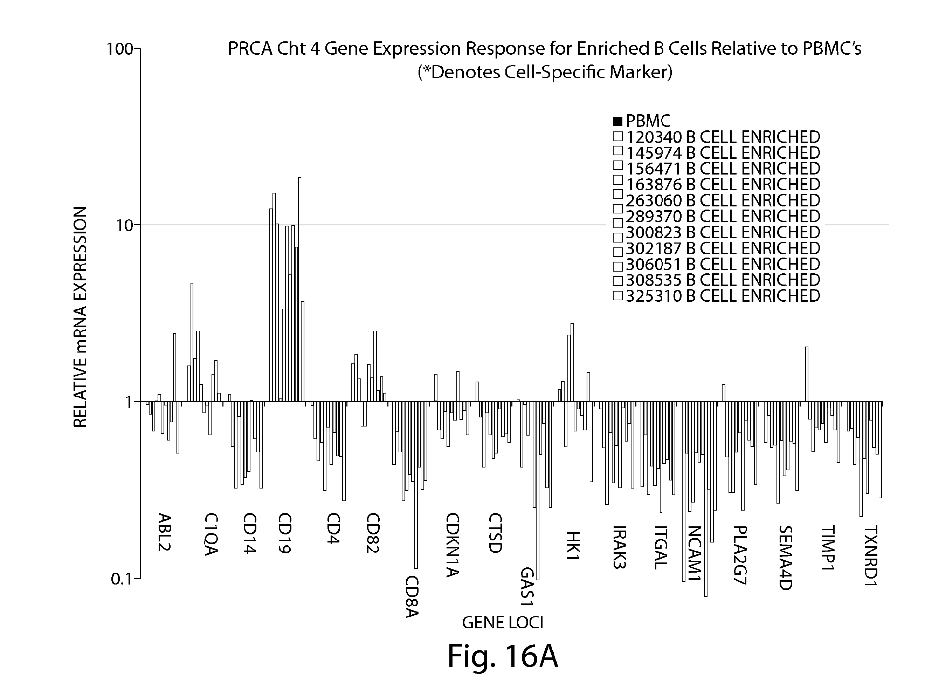

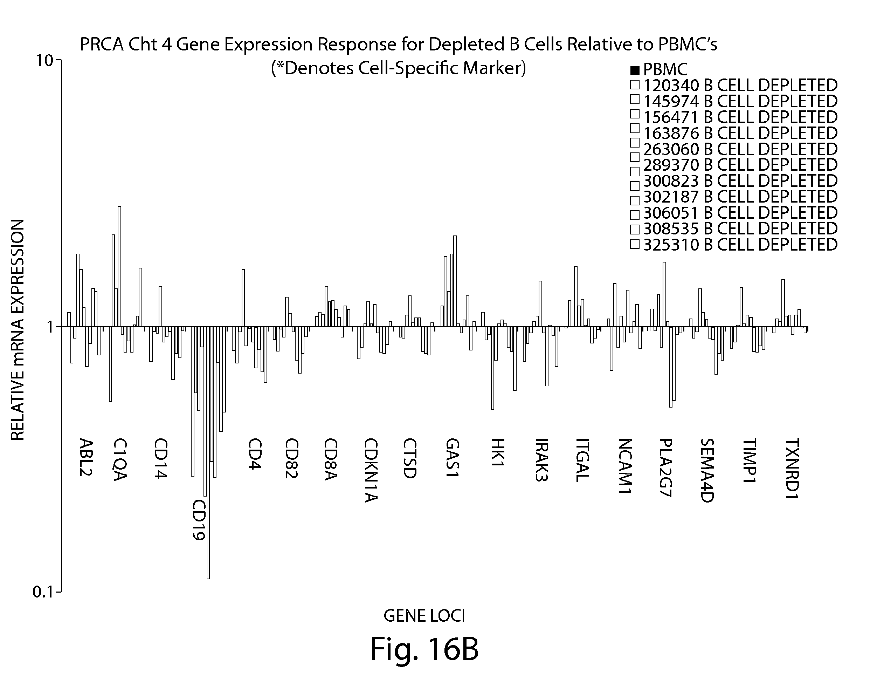

Figures 16A is a bar graph showing gene expression response for a panel of 18 genes in enriched B-cells relative to PBMC's obtained from eleven hormone refractory prostate cancer cohort 4 subj ects; Figure 16B is a bar graph showing gene expression response for a panel of 18 8 genes in depleted B-cells relative to PBMC's obtained from eleven hormone refractory prostate cancer cohort 4 subjects.

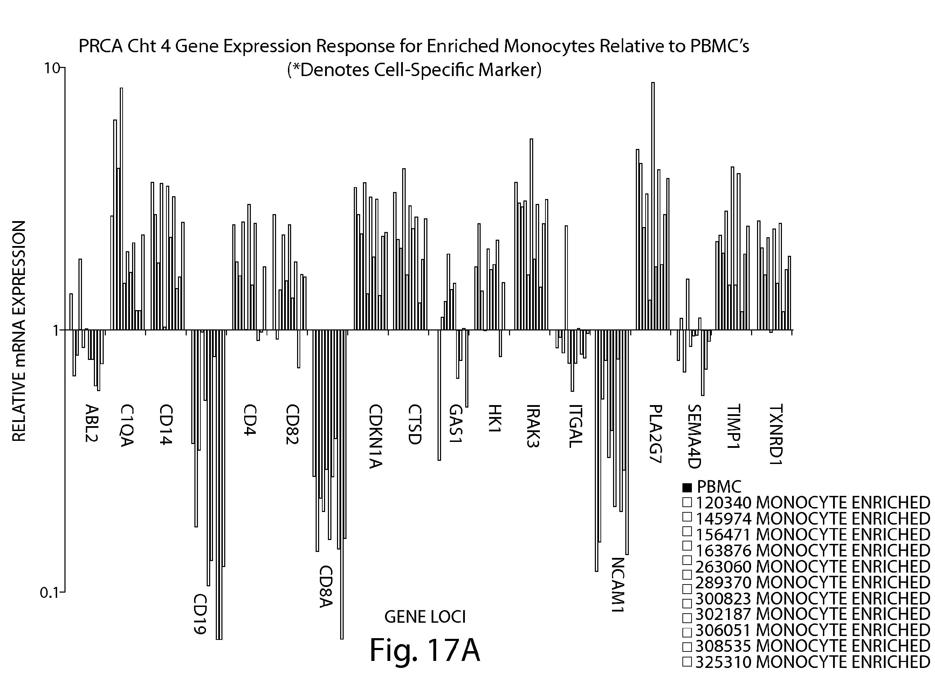

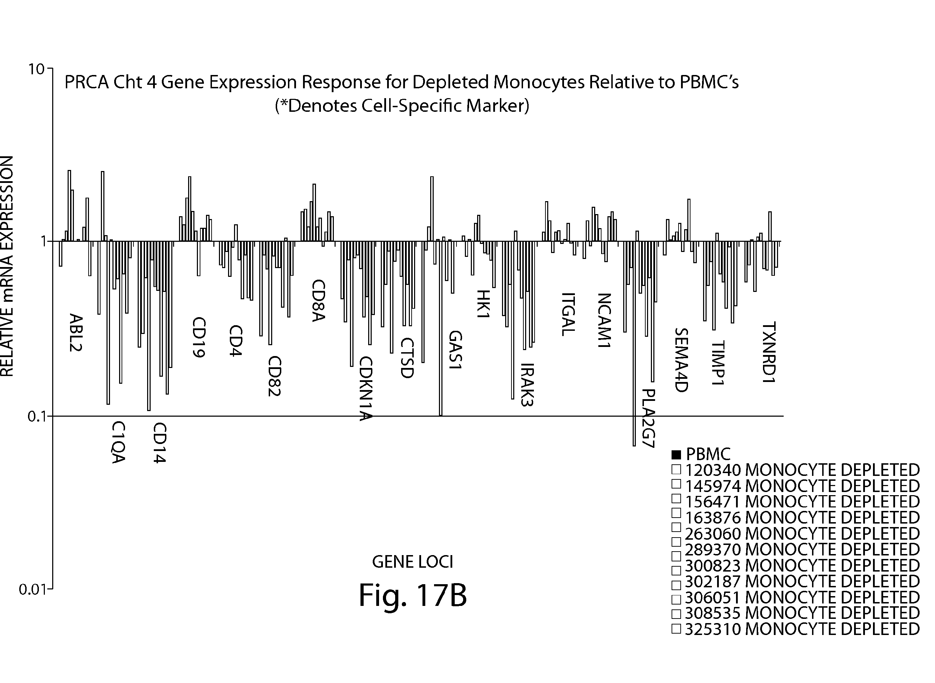

Figures 17A is a bar graph showing gene expression response for a panel of 18 genes in enriched monocytes relative to PBMC's obtained from eleven hormone refractory prostate cancer cohort 4 subjects; Figure 17B is a bar graph showing gene expression response for a panel of 18 genes in depleted monocytes relative to PBMC's obtained from eleven hormone refractory prostate cancer cohort 4 subjects

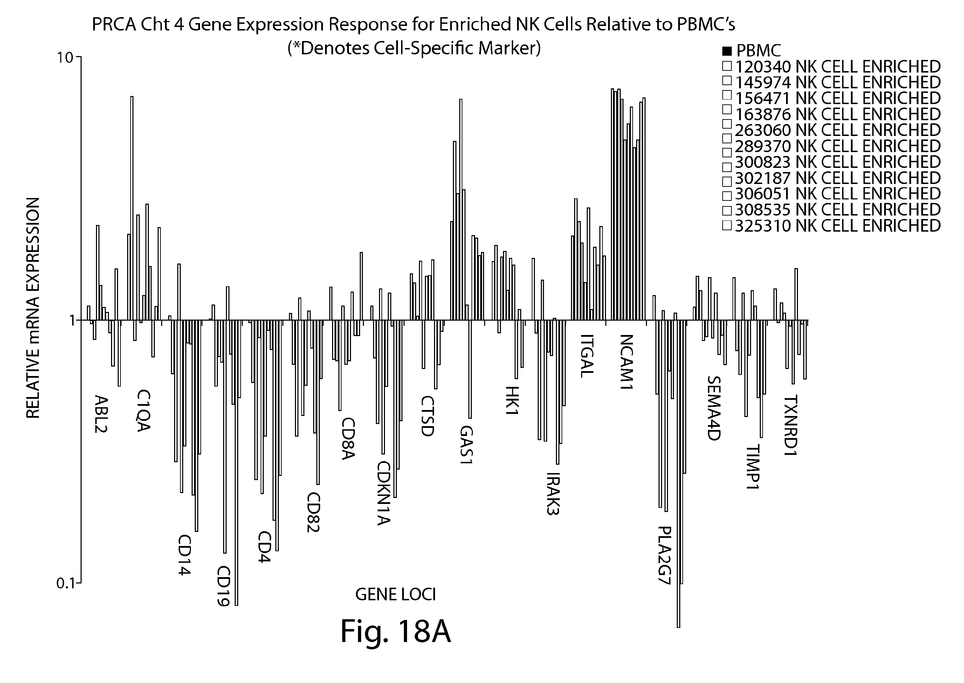

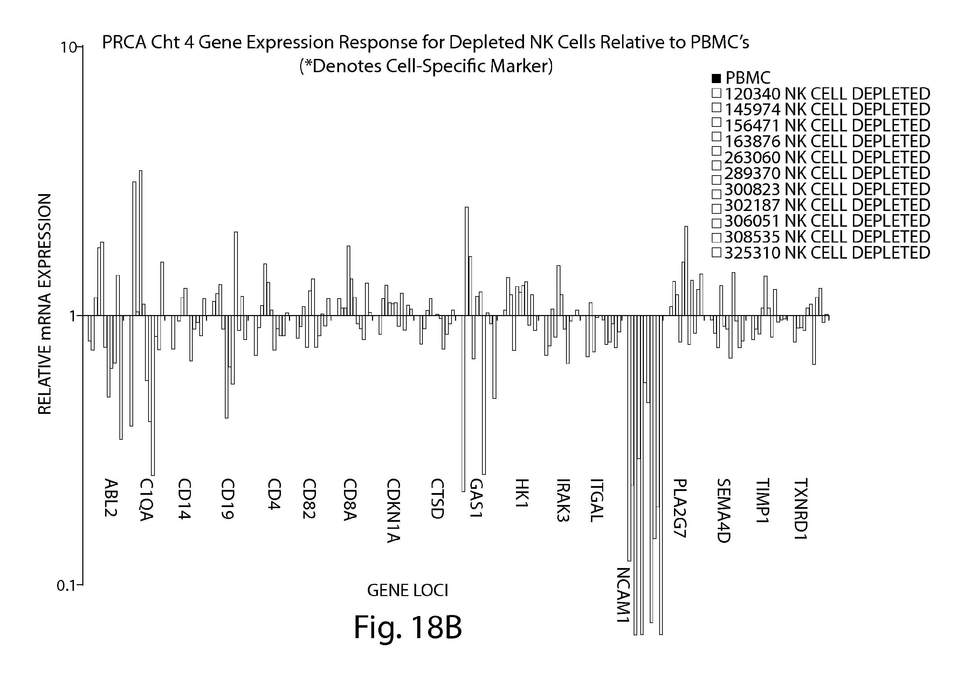

Figures 18A is a bar graph showing gene expression response for a panel of 18 genes in enriched NK-cells relative to PBMC's obtained from eleven hormone refractory prostate cancer cohort 4 subjects; Figure 18B is a bar graph showing gene expression response for a panel of 18 genes in depleted NK-cells relative to PBMC's obtained from eleven hormone refractory prostate cancer cohort 4 subjects.

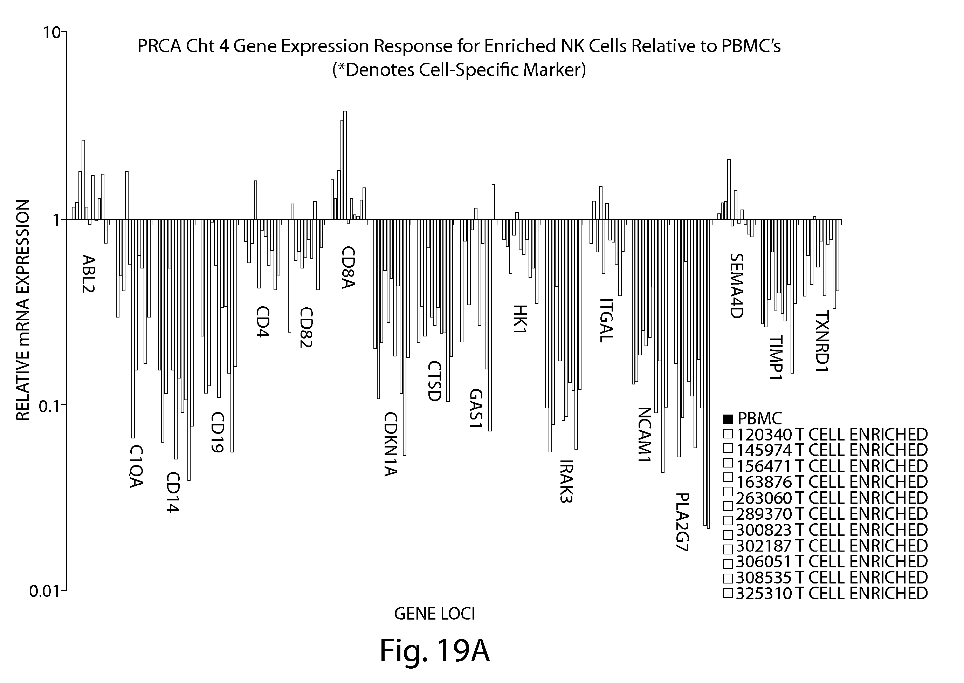

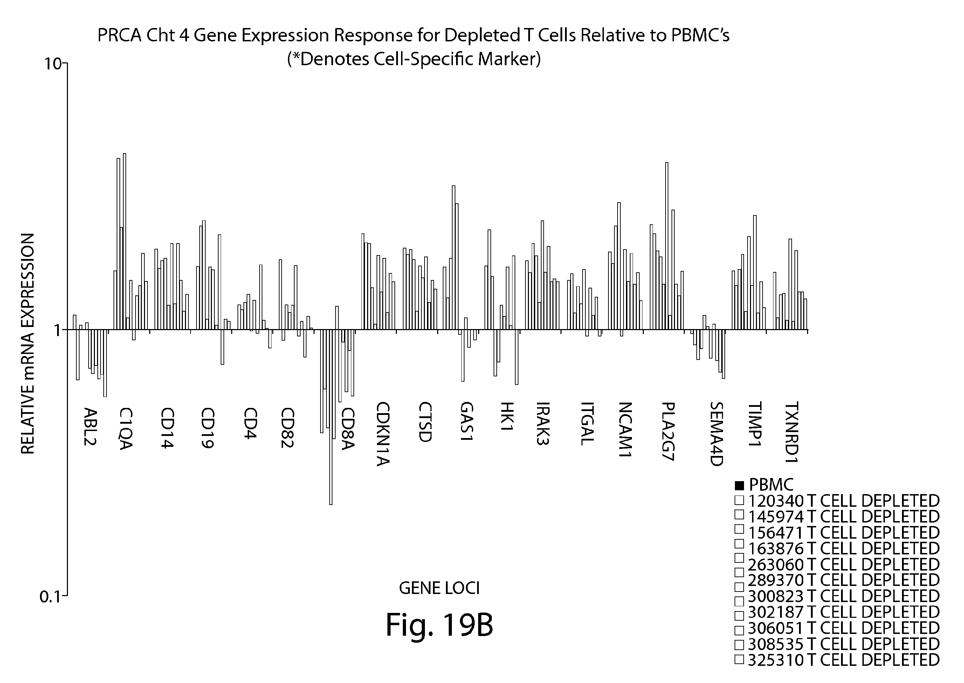

Figure 19A is a bar graph showing gene expression response for a panel of 18 genes in enriched T-cells relative to PBMC's obtained from eleven hormone refractory prostate cancer cohort 4 subjects; Figure 19B is a bar graph showing gene expression response for a panel of 18 genes in depleted T-cells relative to PBMC's obtained from eleven hormone refractory prostate cancer cohort 4 subjects

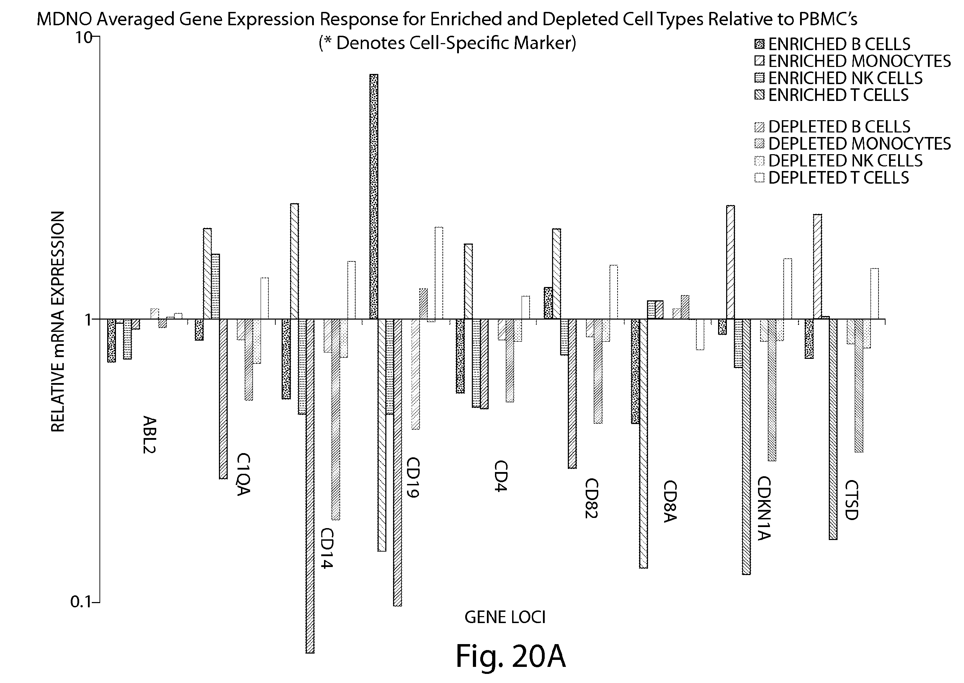

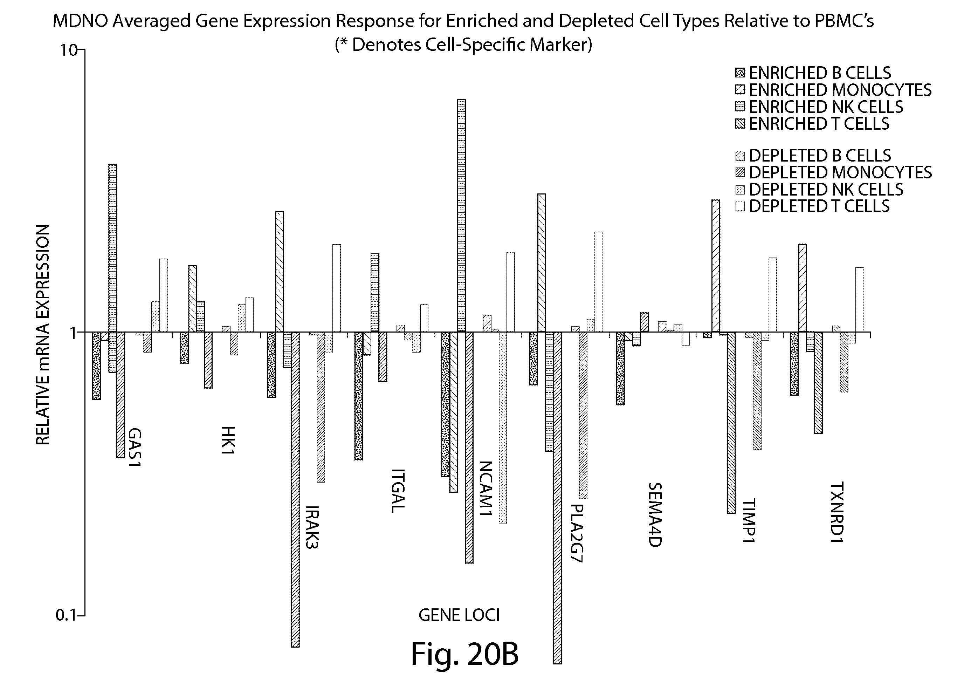

Figures 20A and 20B are bar graphs showing a quantitative comparison of gene expression levels between fractionated cell samples (B-cells, monocytes, T-cells, NK cells) from seven medically defined normal subjects (MDNO) on a gene-by-gene basis for a panel of 18 genes.

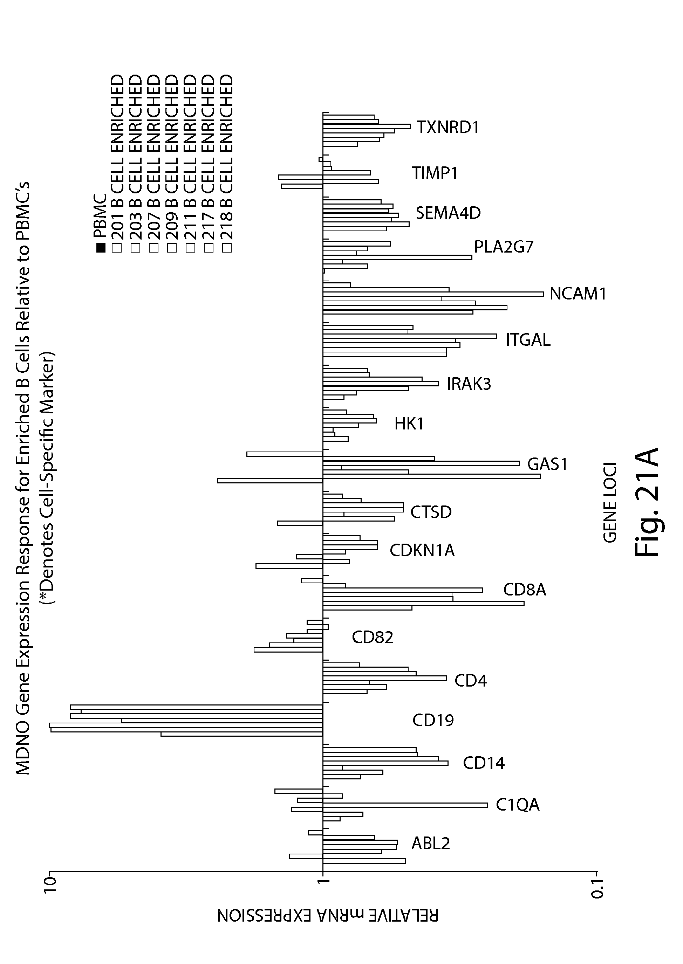

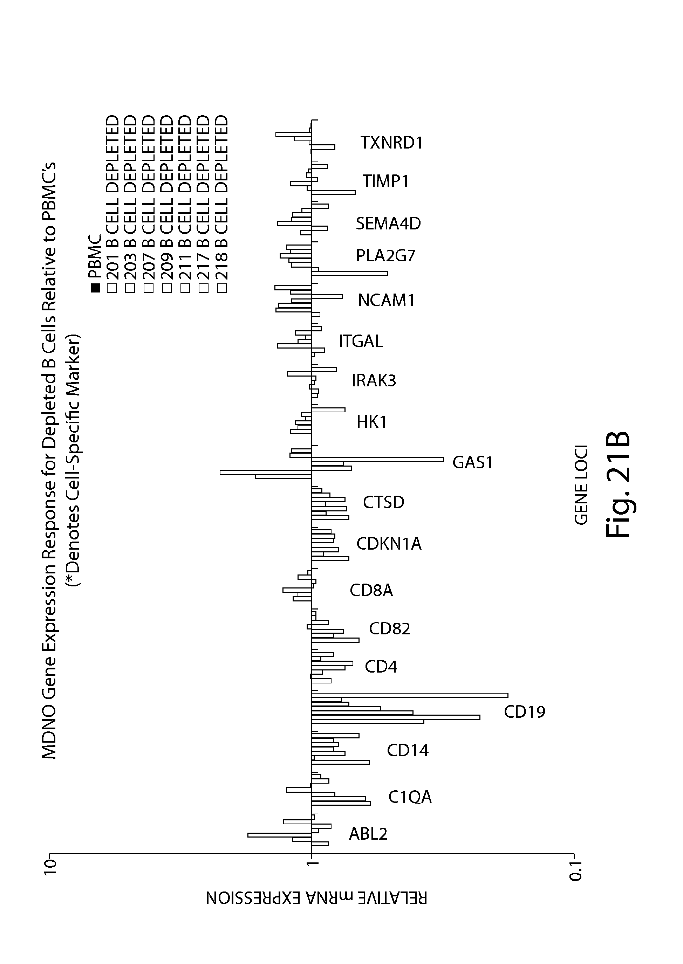

Figure 21A is a bar graph showing gene expression response for a panel of 18 genes in enriched B-cells relative to PBMC's obtained from seven medically defined normal subjects (MDNO); Figure 21B is a bar graph showing gene expression response for a panel of 18 genes in depleted B-cells relative to PBMC's obtained from seven medically defined normal subjects.

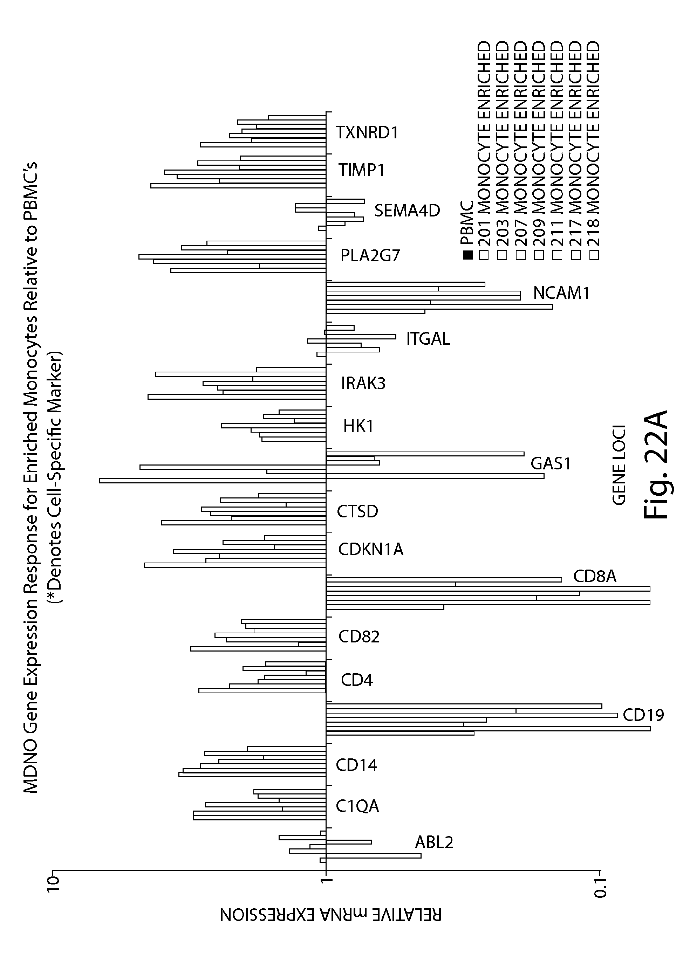

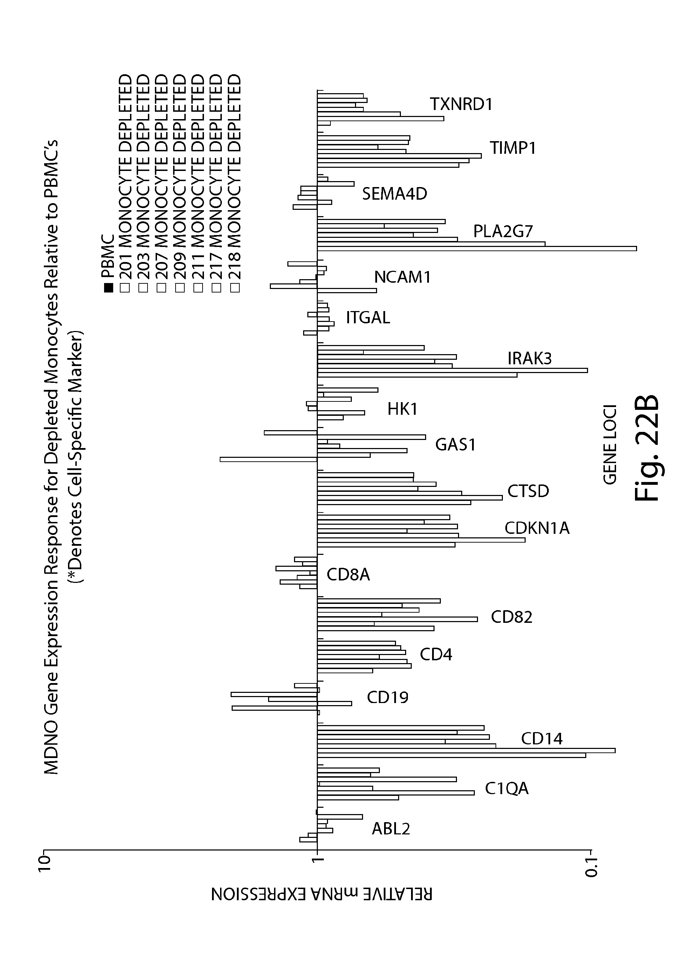

Figure 22A is a bar graph showing gene expression response for a panel of 18 genes in enriched monocytes cells relative to PBMC's obtained from seven medically defined normal subjects (MDNO); Figure 22B is a bar graph showing gene expression response for a panel of 18 genes in depleted monocytes cells relative to PBMC's obtained from seven medically defined normal subjects.

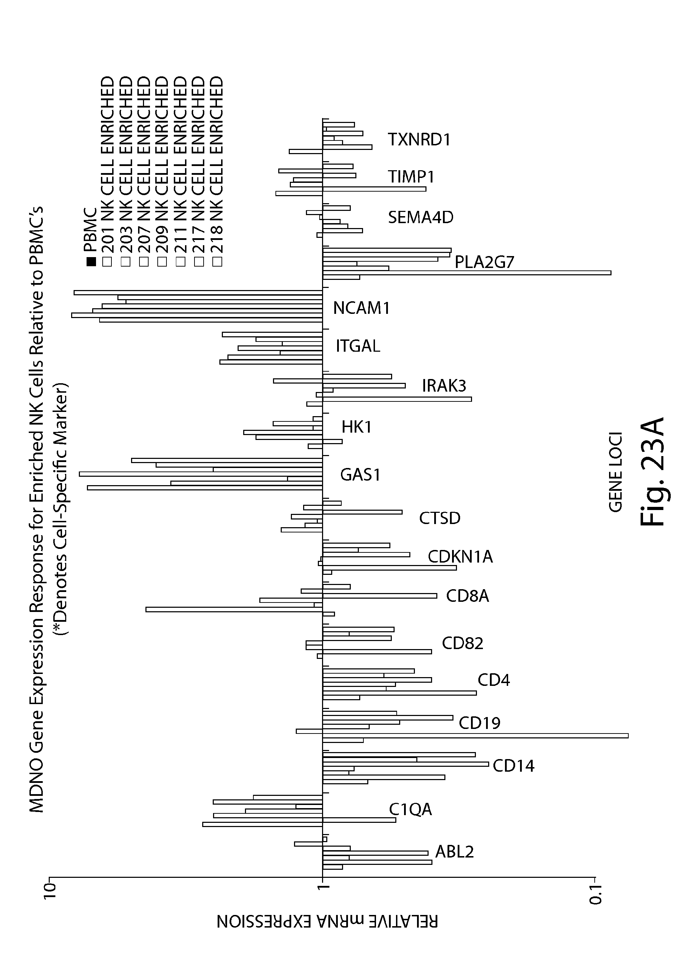

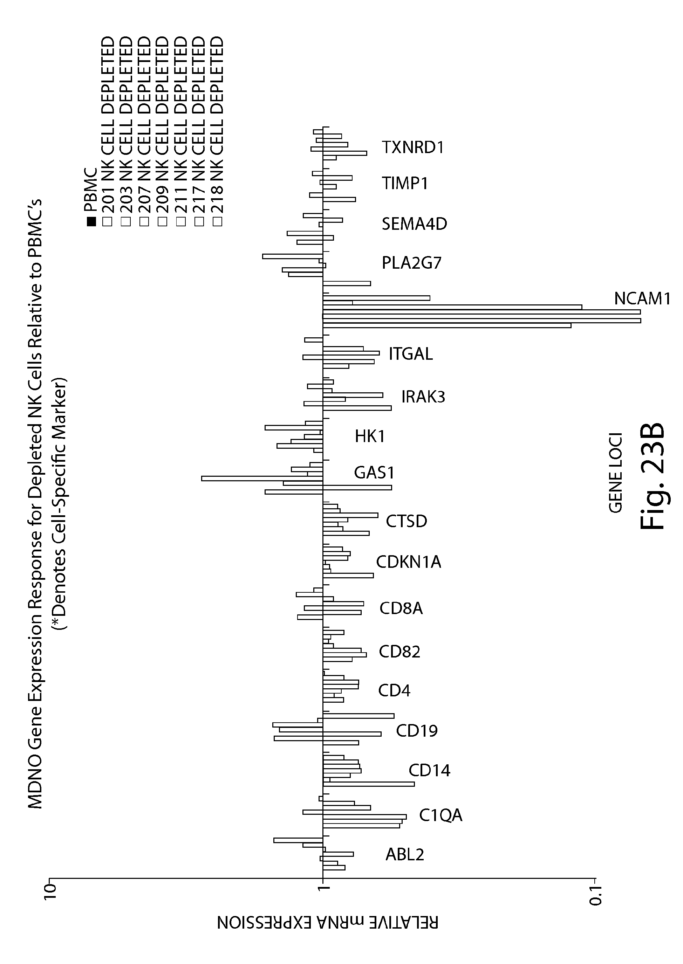

Figure 23A is a bar graph showing gene expression response for a panel of 18 genes in enriched NK-cells relative to PBMC's obtained from seven medically defined normal subjects (MDNO); Figure 23B is a bar graph showing gene expression response for a panel of 18 genes in depleted NK-cells relative to PBMC's obtained from seven medically defined normal subjects.

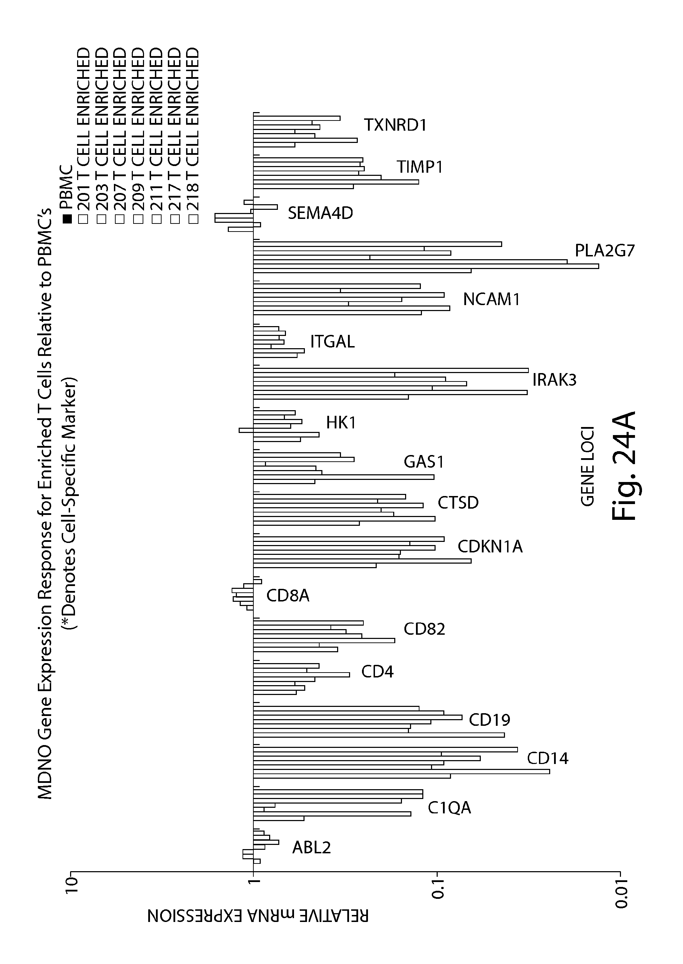

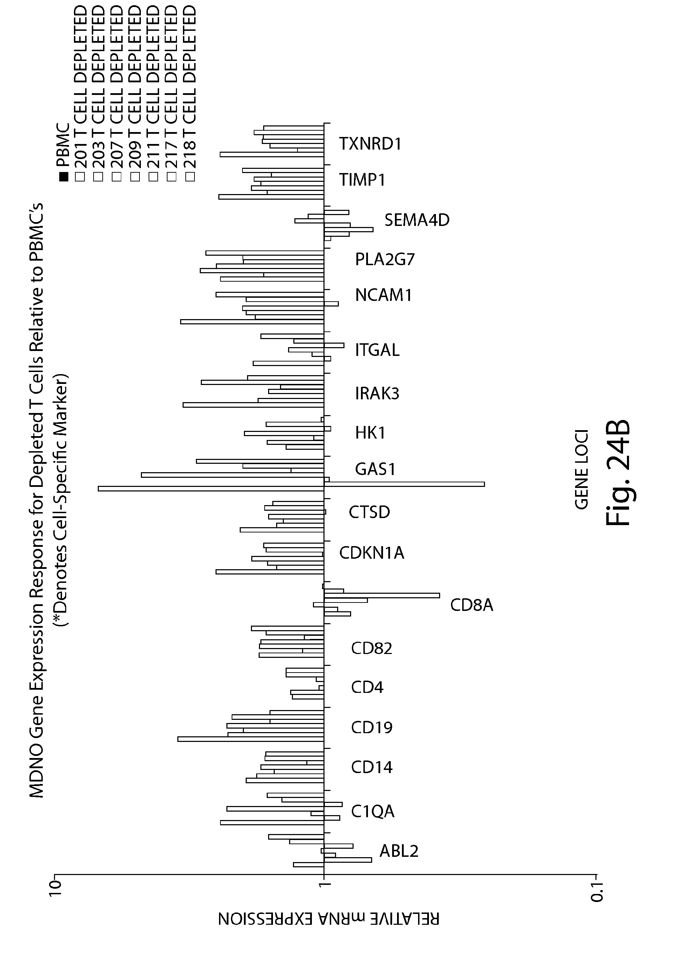

Figure 24A is a bar graph showing gene expression response for a panel of 18 genes in enriched T-cells relative to PBMC's obtained from seven medically defined normal subjects (MDNO); Figure 24B is a bar graph showing gene expression response for a panel of 18 genes in depleted T-cells relative to PBMC's obtained from seven medically defined normal subjects.

## DETAILED DESCRIPTION

*Definitions*

The following terms shall have the meanings indicated unless the context otherwise requires:

"*Accuracy*" refers to the degree of conformity of a measured or calculated quantity (a test reported value) to its actual (or true) value. Clinical accuracy relates to the proportion of true outcomes (true positives (TP) or true negatives (TN)) versus misclassified outcomes (false positives (FP) or false negatives (FN)), and may be stated as a sensitivity, specificity, positive predictive values (PPV) or negative predictive values (NPV), or as a likelihood, odds ratio, among other measures.

"*Algorithm*" is a set of rules for describing a biological condition or for describing the predicted survivability or survival time of a subject having a biological condition. The rule set may be defined exclusively algebraically but may also include alternative or multiple decision points requiring domain-specific knowledge, expert interpretation or other clinical indicators.

An "*agent*" is a "*composition*" or a "*stimulus*", as those terms are defined herein, or a combination of a *composition* and a *stimulus.*

"*Amplification*" in the context of a quantitative RT-PCR assay is a function of the number of DNA replications that are required to provide a quantitative determination of its concentration. "*Amplification*" here refers to a degree of sensitivity and specificity of a quantitative assay technique. Accordingly, amplification provides a measurement of concentrations of constituents that is evaluated under conditions wherein the efficiency of amplification and therefore the degree of sensitivity and reproducibility for measuring all constituents is substantially similar.

A "*baseline profile data set*" is a set of values associated with constituents of a *Gene Expression Panel* (Precision Profile™) resulting from evaluation of a biological sample (or population or set of samples) under a desired *biological condition* that is used for mathematically normative purposes. The desired biological condition may be, for example, the condition of a subject (or population or set of subjects) before exposure to an agent or in the presence of an untreated disease or in the absence of a disease. Alternatively, or in addition, the desired biological condition may be health of a subject or a population or set of subjects. Alternatively, or in addition, the desired biological condition may be that associated with a population or set of subjects selected on the basis of at least one of age group, gender, ethnicity, geographic location, nutritional history, medical condition, clinical indicator, medication, physical activity, body mass, and environmental exposure.

A "*biological condition*" of a subject is the condition of the subject in a pertinent realm that is under observation, and such realm may include any aspect of the subject capable of being monitored for change in condition, such as health; disease including cancer; trauma; aging; infection; tissue degeneration; developmental steps; physical fitness; obesity; and mood. As can be seen, a condition in this context may be chronic or acute or simply transient. Moreover, a targeted biological condition may be manifest throughout the organism or population of cells or may be restricted to a specific organ (such as skin, heart, eye or blood), but in either case, the condition may be monitored directly by a sample of the affected population of cells or indirectly by a sample derived elsewhere from the subject. The term "*biological condition*" includes a "*physiological condition*".

"*Bodyfluid*" of a subject includes blood, urine, spinal fluid, lymph, mucosal secretions, prostatic fluid, semen, haemolymph or any other body fluid known in the art for a subject.

"*Calibrated profile data set*" is a function of a member of a first *profile data set* and a corresponding member of a *baseline profile data set* for a given constituent in a panel.

A "*circulating endothelial cell*" ("CEC") is an endothelial cell from the inner wall of blood vessels which sheds into the bloodstream under certain circumstances, including inflammation, and contributes to the formation of new vasculature associated with cancer pathogenesis. CECs may be useful as a marker of tumor progression and/or response to antiangiogenic therapy.

A "*circulating tumor cell*" ("CTC") is a tumor cell of epithelial origin which is shed from the primary tumor upon metastasis, and enters the circulation. The number of circulating tumor cells in peripheral blood is associated with prognosis in patients with metastatic cancer. These cells can be separated and quantified using immunologic methods that detect epithelial cells.

A "*clinical indicator*" is any physiological datum used alone or in conjunction with other data in evaluating the *physiological condition* of a collection of cells or of an organism. This term includes pre-clinical indicators.

"*Clinical parameters*" encompasses all non-sample or non-Precision Profiles™ of a subject's health status or other characteristics, such as, without limitation, age (AGE), ethnicity (RACE), gender (SEX), and family history of cancer.

A "*composition*" includes a chemical compound, a nutraceutical, a pharmaceutical, a homeopathic formulation, an allopathic formulation, a naturopathic formulation, a combination of compounds, a toxin, a food, a food supplement, a mineral, and a complex mixture of substances, in any physical state or in a combination of physical states.

To "*derive*" a profile data set from a *sample* includes determining a set of values associated with constituents of a *Gene Expression Panel* (Precision Profile™) either (i) by direct measurement of such constituents in a biological *sample.*

"*Distinct RNA or protein constituent*" in a panel of constituents is a distinct expressed product of a gene, whether RNA or protein. An "*expression*" product of a gene includes the gene product whether RNA or protein resulting from translation of the messenger RNA.

"*FN*" is false negative, which for a disease state test means classifying a disease subject incorrectly as non-disease or normal.

"*FP*" is false positive, which for a disease state test means classifying a normal subject incorrectly as having disease.

A "*formula*," "*algorithm*," or "*model*" is any mathematical equation, algorithmic, analytical or programmed process, statistical technique, or comparison, that takes one or more continuous or categorical inputs (herein called "*parameters*") and calculates an output value, sometimes referred to as an "*index*" or "*index value.*" Non-limiting examples of "*formulas*" include comparisons to reference values or profiles, sums, ratios, and regression operators, such as coefficients or exponents, value transformations and normalizations (including, without limitation, those normalization schemes based on clinical parameters, such as gender, age, or ethnicity), rules and guidelines, statistical classification models, and neural networks trained on historical populations. Of particular use in combining constituents of a Gene Expression Panel (Precision Profile™) are linear and non-linear equations and statistical significance and classification analyses to determine the relationship between levels of constituents of a Gene Expression Panel (Precision Profile™) detected in a subject sample and the survivability of the subject. Techniques which may be used in survival and time to event hazard analysis, include but are not limited to Cox, Zero-Inflation Poisson, Markov, Weibull, Kaplan-Meier and Greenwood models, well known to those of skill in the art. In panel and combination construction, of particular interest are structural and synactic statistical classification algorithms, and methods of risk index construction, utilizing pattern recognition features, including, without limitation, such established techniques such as cross-correlation, Principal Components Analysis (PCA), factor rotation, Logistic Regression Analysis (LogReg), Kolmogorov Smirnoff tests (KS), Linear Discriminant Analysis (LDA), Eigengene Linear Discriminant Analysis (ELDA), Support Vector Machines (SVM), Random Forest (RF), Recursive Partitioning Tree (RPART), as well as other related decision tree classification techniques (CART, LART, LARTree, FlexTree, amongst others), Shrunken Centroids (SC), StepAIC, K-means, Kth-Nearest Neighbor, Boosting, Decision Trees, Neural Networks, Bayesian Networks, Support Vector Machines, and Hidden Markov Models, among others. Many of these techniques are useful either combined with a consituentes of a Gene Expression Panel (Precision Profile™) selection technique, such as forward selection, backwards selection, or stepwise selection, complete enumeration of all potential panels of a given size, genetic algorithms, voting and committee methods, or they may themselves include biomarker selection methodologies in their own technique. These may be coupled with information criteria, such as Akaike's Information Criterion (AIC) or Bayes Information Criterion (BIC), in order to quantify the tradeoff between additional biomarkers and model improvement, and to aid in minimizing overfit. The resulting predictive models may be validated in other clinical studies, or cross-validated within the study they were originally trained in, using such techniques as Bootstrap, Leave-One-Out (LOO) and 10-Fold cross-validation (10-Fold CV). At various steps, false discovery rates (FDR) may be estimated by value permutation according to techniques known in the art.

A "*Gene Expression Panel*" (Precision Profile™) is an experimentally verified set of constituents, each constituent being a distinct expressed product of a gene, whether RNA or protein, wherein constituents of the set are selected so that their measurement provides a measurement of the predicted survivability of a subject.

A "*Gene Expression Profile*" is a set of values associated with constituents of a *Gene Expression Panel* (Precision Profile™) resulting from evaluation of a biological sample (or population or set of samples).

A *Gene Expression Profile Survivability Index*" is the value of an index function that provides a mapping from an instance of a *Gene Expression Profile* into a single-valued measure of the survivability of a subject.

The "*health*" of a subject includes mental, emotional, physical, spiritual, allopathic, naturopathic and homeopathic condition of the subject.

"*Index*" is an arithmetically or mathematically derived numerical characteristic developed for aid in simplifying or disclosing or informing the analysis of more complex quantitative information. A survivability and/or survival time index may be determined by the application of a specific algorithm to a plurality of subjects or samples with a common biological condition.

"*Inflammation*" is used herein in the general medical sense of the word and may be an acute or chronic; simple or suppurative; localized or disseminated; cellular and tissue response initiated or sustained by any number of chemical, physical or biological agents or combination of agents.

"*Inflammatory state*" is used to indicate the relative biological condition of a subject resulting from inflammation, or characterizing the degree of inflammation.

A "*large number*" of data sets based on a common panel of genes is a number of data sets sufficiently large to permit a statistically significant conclusion to be drawn with respect to an instance of a data set based on the same panel.

"*Negative predictive value*" or "*NPV*" is calculated by TN/(TN + FN) or the true negative fraction of all negative test results. It also is inherently impacted by the prevalence of the disease and pre-test probability of the population intended to be tested.

See, *e.g.*, O'Marcaigh AS, Jacobson RM, "Estimating the Predictive Value of a Diagnostic Test, How to Prevent Misleading or Confusing Results," Clin. Ped. 1993, 32(8): 485-491, which discusses specificity, sensitivity, and positive and negative predictive values of a test, *e.g.*, a clinical diagnostic test. Often, for binary disease state classification approaches using a continuous diagnostic test measurement, the sensitivity and specificity is summarized by Receiver Operating Characteristics (ROC) curves according to Pepe et al., "Limitations of the Odds Ratio in Gauging the Performance of a Diagnostic, Prognostic, or Screening Marker," Am. J. Epidemiol 2004, 159 (9): 882-890, and summarized by the Area Under the Curve (AUC) or c-statistic, an indicator that allows representation of the sensitivity and specificity of a test, assay, or method over the entire range of test (or assay) cut points with just a single value. See also, *e.g.*, Shultz, "Clinical Interpretation of Laboratory Procedures," chapter 14 in Teitz, Fundamentals of Clinical Chemistry, Burtis and Ashwood (eds.), 4th edition 1996, W.B. Saunders Company, pages 192-199; and Zweig et al., "ROC Curve Analysis: An Example Showing the Relationships Among Serum Lipid and Apolipoprotein Concentrations in Identifying Subjects with Coronory Artery Disease," Clin. Chem., 1992, 38(8): 1425-1428. An alternative approach using likelihood functions, BIC, odds ratios, information theory, predictive values, calibration (including goodness-of-fit), and reclassification measurements is summarized according to Cook, "Use and Misuse of the Receiver Operating Characteristic Curve in Risk Prediction," Circulation 2007, 115: 928-935.

A "*normal*" subject is a subject who is generally in good health, has not been diagnosed with prostate cancer, is asymptomatic for prostate cancer, and lacks the traditional laboratory risk factors for prostate cancer.

A "*normative*" condition of a subject to whom a composition is to be administered means the condition of a subject before administration, even if the subject happens to be suffering from a disease.

A "*panel*" of genes is a set of genes including at least two constituents.

A "*population of cells*" refers to any group of cells wherein there is an underlying commonality or relationship between the members in the population of cells, including a group of cells taken from an organism or from a culture of cells or from a biopsy, for example.

"*Positive predictive value*" or "*PPV*" is calculated by TP/(TP+FP) or the true positive fraction of all positive test results. It is inherently impacted by the prevalence of the disease and pre-test probability of the population intended to be tested.

"*Prostate cancer*" is the malignant growth of abnormal cells in the prostate gland, capable of invading and destroying other prostate cells, and spreading (metastasizing) to other parts of the body, including bones and lymph nodes. As defined herein, the term "*prostate cancer*" includes Stage 1, Stage 2, Stage 3, and Stage 4 prostate cancer as determined by the Tumor/Nodes/Metastases ("TNM") system which takes into account the size of the tumor, the number of involved lymph nodes, and the presence of any other metastases; or Stage A, Stage B, Stage C, and Stage D, as determined by the Jewitt-Whitmore system.

"*Risk*" in the context of the present invention, relates to the probability that an event will occur over a specific time period, and can mean a subject's "absolute" risk or "relative" risk. Absolute risk can be measured with reference to either actual observation post-measurement for the relevant time cohort, or with reference to index values developed from statistically valid historical cohorts that have been followed for the relevant time period. Relative risk refers to the ratio of absolute risks of a subject compared either to the absolute risks of lower risk cohorts, across population divisions (such as tertiles, quartiles, quintiles, or deciles, etc.) or an average population risk, which can vary by how clinical risk factors are assessed. Odds ratios, the proportion of positive events to negative events for a given test result, are also commonly used (odds are according to the formula p/(1-p) where p is the probability of event and (1- p) is the probability of no event) to no-conversion.

"*Risk evaluation*," or "*evaluation of risk*" in the context of the present invention encompasses making a prediction of the probability, odds, or likelihood that an event (*e.g.*, death) or disease state may occur, and/or the rate of occurrence of the event (*e.g.*, death) or conversion from one disease state to another, *i.e.*, from a normal condition to cancer or from cancer remission to cancer, or from primary cancer occurrence to occurrence of a cancer metastasis. Risk evaluation can also comprise prediction of future clinical parameters, traditional laboratory risk factor values, or other indices of cancer results, either in absolute or relative terms in reference to a previously measured population. Such differing use may require different consituentes of a Gene Expression Panel (Precision Profile™) combinations and individualized panels, mathematical algorithms, and/or cut-off points, but be subject to the same aforementioned measurements of accuracy and performance for the respective intended use.

A "*sample*" from a subject may include a single cell or multiple cells or fragments of cells or an aliquot of body fluid, taken from the subject, by means including venipuncture, excretion, ejaculation, massage, biopsy, needle aspirate, lavage sample, scraping, surgical incision or intervention or other means known in the art. The sample is blood, urine, spinal fluid, lymph, mucosal secretions, prostatic fluid, semen, haemolymph or any other body fluid known in the art for a subject. The sample is also a tissue sample. The sample is or contains a circulating endothelial cell or a circulating tumor cell.

"*Sensitivity*" is calculated by TP/(TP+FN) or the true positive fraction of disease subjects.

"*Specificity*" is calculated by TN/(TN+FP) or the true negative fraction of non-disease or normal subjects.

By "*statistically significant*", it is meant that the alteration is greater than what might be expected to happen by chance alone (which could be a "false positive"). Statistical significance can be determined by any method known in the art. Commonly used measures of significance include the *p*-value, which presents the probability of obtaining a result at least as extreme as a given data point, assuming the data point was the result of chance alone. A result is often considered highly significant at a *p*-value of 0.05 or less and statistically significant at a *p*-value of 0.10 or less. Such *p*-values depend significantly on the power of the study performed.

A "*set*" or "*population*" of samples or subjects refers to a defined or selected group of samples or subjects wherein there is an underlying commonality or relationship between the members included in the set or population of samples or subjects.

A "*Signature Profile*" is an experimentally verified subset of a *Gene Expression Profile* selected to discriminate a biological condition, agent or physiological mechanism of action, or predict the survivability and/or survival time of a subject having a biological condition.

A "*Signature Panel*" is a subset of a *Gene Expression Panel (Precision Profile™ )*, the constituents of which are selected to permit discrimination of a *biological condition*, *agent* or physiological mechanism of action or to precit the survivability and/or survival time of a subject having a biological condition.

A "*subject*" is a cell, tissue, or organism, human or non-human, whether *in vivo,* ex vivo or *in vitro,* under observation. As used herein, reference to predicting the survivability and/or survival time of a subject based on a sample from the subject, includes using blood or other tissue sample from a human subject to evaluate the human subject's predicted survivability and/or survival time; it also includes, for example, using a blood sample itself as the subject to evaluate, for example, the effect of therapy or an agent upon the sample.

A "*stimulus*" includes (i) a monitored physical interaction with a subject, for example ultraviolet A or B, or light therapy for seasonal affective disorder, or treatment of psoriasis with psoralen or treatment of cancer with embedded radioactive seeds, other radiation exposure, and (ii) any monitored physical, mental, emotional, or spiritual activity or inactivity of a subject.

"*Survivability*" refers to the ability to remain alive or continue to exist (*i.e.*, alive or dead).

"*Survival time*" refers to the length or period of time a subject is able to remain alive or continue to exist as measured from an initial date (*e.g.*, date of birth, date of diagnosis of a particular disease or stage of disease, date of initiating a therapeutic regimen, date of blood draw for clinical analysis, etc.) to a later date in time (*e.g.*, date of death, date of termination of a particular therapeutic regimen, or an arbitrary date). As used herein, survival time can be a period of up to 6 months, 12 months, 18 months, 20 months, 24 months, 30 months, 36 months, 42 months, 48 months, 54 months, 60 months, 66 months, 72 months, 78 months, 84 months, 90 months, 96 months, 102 months, 108 months, 114 months, 120 months, or greater.

"*Therapy*" or "therapeutic regimen" includes all interventions whether biological, chemical, physical, metaphysical, or combination of the foregoing, intended to sustain or alter the monitored biological condition of a subject.

"*TN*" is *true negative*, which for a disease state test means classifying a non-disease or normal subject correctly.

"*TP*" is *true positive*, which for a disease state test means correctly classifying a disease subject.

The PCT patent application publication number WO 01/25473, published April 12, 2001, entitled "Systems and Methods for Characterizing a Biological Condition or Agent Using Calibrated Gene Expression Profiles," which is herein incorporated by reference, discloses the use of Gene Expression Panels (Precision Profiles™) for the evaluation of (i) biological condition (including with respect to health and disease) and (ii) the effect of one or more agents on biological condition (including with respect to health, toxicity, therapeutic treatment and drug interaction). The PCT patent application PCT/ US2007/023425, filed November 6, 2007, entitled "Gene Expression Profiling for Identification, Monitoring and Treatment of Prostate Cancer", filed for an invention by the inventors herein, and which is herein incorporated by reference in its entirety, discloses the use of Gene Expression Panels (Precision Profiles™) for evaluating the presence or likelihood of prostate cancer in a subject, and for monitoring response to therapy in a prostate cancer-diagnosed subject, and for monitoring the progression of prostate cancer in a prostate-cancer-diagnosed subject (*i.e.*, cancer versus a normal, healthy, disease free state).

The present invention provides a Gene Expression Panel (Precision Profile™) for predicting the survivability and/or survival time of a prostate cancer-diagnosed subject and for evaluating the effect of one or more variables on the predicted survivability and/or survival time of a prostate cancer-diagnosed subject. The Gene Expression Panel (Precision Profile ™) described herein may be used for identifying and assessing predictive relationships between RNA-transcript-based gene expression and predicted survivability and/or survival time of a prostate cancer diagnosed subject (either direct relationship or indirect relationship, *e.g.*, affecting the latent classes). For example, the Gene Expression Panel (Precision Profile ™) described herein may be used, without limitation, for measurement of the following with respect to a prostate cancer-diagnosed subject: predicting the survivability, predicting the expected survival time, predicting the probability of long-term survivability, predicting the effect of one or more variables (including without limitiation, age, PSA level, therapeutic regimen, body mass index, ethnicity, family history of cancer) on survivability and/or survival time, and for predicting the survivability and/or survival time of latent classes (*e.g.*, distinguishing the predicted survivability and/or survival times of a set or population of prostate cancer-diagnosed subjects having the same or different clinical presentation (*e.g.*, tumor volume, tumor location, stage of disease, etc.)). The Gene Expression Panel (Precision Profile™) may be employed with respect to samples derived from subjects in order to evaluate their predicted survivability and/or survival time.

The Gene Expression Panel (Precision Profile™) is referred to herein as the Precision Profile™ for Prostate Cancer Survivability (Table 1), which includes one or more genes, *e.g.*, constituents, whose expression is associated with prostate cancer survivability.. Each gene of the Precision Profile™ for Prostate Cancer Survivability is referred to herein as a prostate cancer survivability gene or a prostate cancer survivability constituent.

In addition to the the Precision Profile™ for Prostate Cancer Survivability, (Table 1), the invention provides a Protein Expression Panel for predicting the survivability and/or survival time of a prostate cancer-diagnosed subject and for evaluating the effect of one or more variables on the predicted survivability and/or survival time of a prostate cancer-diagnosed subject. The Protein Expression Panel described herein may be used for identifying and assessing predictive relationships between protein expression and predicted survivability and/or survival time of a prostate cancer diagnosed subject (either direct relationship or indirect relationship, *e.g.*, affecting the latent classes). For example, the Protein Expression Panel described herein may be used, without limitation, for measurement of the following with respect to a prostate cancer-diagnosed subject: predicting the survivability, predicting the expected survival time, predicting the probability of long-term survivability, predicting the effect of one or more variables (including without limitiation, age, PSA level, therapeutic regimen, body mass index, ethnicity, family history of cancer) on survivability and/or survival time, and for predicting the survivability and/or survival time of latent classes (*e.g.*, distinguishing the predicted survivability and/or survival times of a set or population of prostate cancer-diagnosed subjects having the same or different clinical presentation (*e.g.*, tumor volume, tumor location, stage of disease, etc.)). The Protein Expression Panel may be employed with respect to samples derived from subjects in order to evaluate their predicted survivability and/or survival time.

The Protein Expression Panel is referred to herein as the Precision Protein Panel for Prostate Cancer Survivability (Table 20), which includes proteins whose expression is associated with prostate cancer survival rates and may be useful in predicting the survivability and/or survival time of prostate cancer subjects.

It has been discovered that valuable and unexpected results may be achieved when the quantitative measurement of constituents is performed under repeatable conditions (within a degree of repeatability of measurement of better than twenty percent, preferably ten percent or better, more preferably five percent or better, and more preferably three percent or better). For the purposes of this description and the following claims, a degree of repeatability of measurement of better than twenty percent may be used as providing measurement conditions that are "substantially repeatable". In particular, it is desirable that each time a measurement is obtained corresponding to the level of expression of a constituent in a particular sample, substantially the same measurement should result for substantially the same level of expression. In this manner, expression levels for a constituent in a Gene Expression Panel (Precision Profile™) may be meaningfully compared from sample to sample. Even if the expression level measurements for a particular constituent are inaccurate (for example, say, 30% too low), the criterion of repeatability means that all measurements for this constituent, if skewed, will nevertheless be skewed systematically, and therefore measurements of expression level of the constituent may be compared meaningfully. In this fashion valuable information may be obtained and compared concerning expression of the constituent under varied circumstances.

In addition to the criterion of repeatability, it is desirable that a second criterion also be satisfied, namely that quantitative measurement of constituents is performed under conditions wherein efficiencies of amplification for all constituents are substantially similar as defined herein. When both of these criteria are satisfied, then measurement of the expression level of one constituent may be meaningfully compared with measurement of the expression level of another constituent in a given sample and from sample to sample.

The prediction of the survivability of a prostate cancer-diagnosed subject is defined to be a prediction of the survivability and/or survival time of the subject and/or the assessment of the effect of a particular variable (*e.g.*, age, PSA level, therapeutic agent, body mass index, ethnicity, CTC count) on the predicted survivability and/or survival time.

The agent to be evaluated for its effect on the survivability of a prostate cancer-diagnosed subject may be a compound known to treat prostate cancer or compounds that have been not shown to treat prostate cancer. For example, the agent may be an alkylating agent (*e.g.*, Cisplatin, Carboplatin, Oxaliplatin, BBR3464, Chlorambucil, Chlormethine, Cyclophosphamides, Ifosmade, Melphalan, Carmustine, Fotemustine, Lomustine, Streptozocin, Busulfan, Dacarbazine, Mechlorethamine, Procarbazine, Temozolomide, ThioTPA, and Uramustine); an anti-metabolite (*e.g.*, purine (azathioprine, mercaptopurine), pyrimidine (Capecitabine, Cytarabine, Fluorouracil, Gemcitabine), and folic acid (Methotrexate, Pemetrexed, Raltitrexed)); a vinca alkaloid (*e.g.*, Vincristine, Vinblastine, Vinorelbine, Vindesine); a taxane (*e.g.*, paclitaxel, docetaxel, BMS-247550); an anthracycline (*e.g.*, Daunorubicin, Doxorubicin, Epirubicin, Idarubicin, Mitoxantrone, Valrubicin, Bleomycin, Hydroxyurea, and Mitomycin); a topoisomerase inhibitor (*e.g.*, Topotecan, Irinotecan Etoposide, and Teniposide); a monoclonal antibody (*e.g.*, Alemtuzumab, Bevacizumab, Cetuximab, Gemtuzumab, Panitumumab, Rituximab, and Trastuzumab); a photosensitizer (*e.g.*, Aminolevulinic acid, Methyl aminolevulinate, Porfimer sodium, and Verteporfin); a tyrosine kinase inhibitor (*e.g.*, Gleevec™); an epidermal growth factor receptor inhibitor (*e.g.*, Iressa™, erlotinib (Tarceva™), gefitinib); an FPTase inhibitor (*e.g.*, FTIs (R115777, SCH66336, L-778,123)); a KDR inhibitor (*e.g.*, SU6668, PTK787); aproteosome inhibitor (*e.g.*, PS341); a TS/DNA synthesis inhibitor (*e.g.*, ZD9331, Raltirexed (ZD1694, Tomudex), ZD9331, 5-FU)); an S-adenosyl-methionine decarboxylase inhibitor (*e.g.*, SAM468A); a DNA methylating agent *(e.g.,* TMZ); a DNA binding agent *(e.g.,* PZA); an agent which binds and inactivates $O^6$-alkylguanine AGT (*e.g.*, BG); a c-*raf*- 1 antisense oligo-deoxynucleotide *(e.g.,* ISIS-5132 (CGP-69846A)); tumor immunotherapy; a steroidal and/or non-steroidal anti-inflammatory agent (*e.g.*, corticosteroids, COX-2 inhibitors); or other agents such as Alitretinoin, Altretamine, Amsacrine, Anagrelide, Arsenic trioxide, Asparaginase, Bexarotene, Bortezomib, Celecoxib, Dasatinib, Denileukin Diftitox, Estramustine, Hydroxycarbamide, Imatinib, Pentostatin, Masoprocol, Mitotane, Pegaspargase, and Tretinoin.

The predicted survivability and/or survival time of a prostate cancer-diagnosed subject is evaluated by determining the level of expression (*e.g.*, a quantitative measure) of an effective number (*e.g.*, one or more) of constituents of the Precision Profile™ for Prostate Cancer Survivability (Table 1) and/or the Precision Protein Panel™ for Prostate Cancer Survivability (Table 20) and assessing the effects of constituent expression on the hazard rate for statistical survival models (e.g., Cox-Type Proporational Hazards, Zero-Inflated Poisson model, and Markov models). By an effective number is meant the number of constituents that need to be measured in order to predict the survivability and/or survival time of a prostate cancer-diagnosed subject, and/or to predict the survivability and/or survival time of latent classes (*e.g.*, prostate cancer subject having the same or different clinical presentation). Preferably, the selected constituents are incrementally significant at the .05 level (i.e., incremental p-value < 0.05). In one embodiment, the constituents are selected as to predict the survivability and/or survival time of a prostate cancer-diagnosed subject with least 75% accuracy, more preferably 80%, 85%, 90%, 95%, 97%, 98%, 99% or greater accuracy.

The level of expression is determined by any means known in the art. For example, the level of expression of one or more constituents of the Precision Profile™ for Prostate Cancer Survivability (Table 1) is measure by quantitative PCR, and the level of expression of one or more constituents of the Precision Protein Patent for Prostate Cancer Survivability (Table 20) is measured electrophoretically or immunochemically. Immunochemical detection includes for example, radio-immunoassay, immunofluorescence assay, or enzyme-linked immunosorbant assay. The measurement is obtained under conditions that are substantially repeatable. Optionally, the qualitative measure of the constituent is compared to a reference or baseline level or value (*e.g.* a baseline profile set). In one embodiment, the reference or baseline level is the predicted survivability and/or survival time as a function of variable subject factors such as age, PSA level, metastatic status and/or treatment, without the use of constituent measurements. In another embodiment, the reference or baseline level is derived from the same subject from which the first measure is derived. For example, the baseline is taken from a subject at different time periods, (*e.g.*, prior to receiving treatment or surgery for prostate cancer, or at different time periods during a course of treatment). Such methods allow for the evaluation of the effect of a particular variable (*e.g.*, treatment for a selected individual) on the survivability of a prostate-cancer diagnosed subject. Such methods also allow for the evaluation of the effect of a particular variable (*e.g.*, treatment) on the expression levels of one or more constituents which are capable of predicting the survivability of a prostate cancer diagnosed subject. Comparison can be performed on test (*e.g.,* patient) and reference samples (*e.g.*, baseline) measured concurrently or at temporally distinct times. An example of the latter is the use of compiled expression information, *e.g.*, a gene expression database, which assembles information about expression levels of cancer survivability associated genes.

A reference or baseline level or value as used herein can be used interchangeably and is meant to be relative to a number or value derived from population studies, including without limitation, such subjects having similar age range, disease status (*e.g.*, stage), subjects in the same or similar ethnic group, or relative to the starting sample of a subject undergoing treatment for prostate cancer. Such reference values can be derived from statistical analyses and/or risk prediction data of populations obtained from mathematical algorithms and computed indices of prostate cancer. Reference indices can also be constructed and used using algorithms and other methods of statistical and structural classification.

In one embodiment of the present invention, the reference or baseline value is the amount of expression of a cancer survivability associated gene in a control sample derived from one or more prostate cancer-diagnosed subjects who have not received any treatment for prostate cancer.

In another embodiment of the present invention, the reference or baseline value is the level of cancer survivability associated genes in a control sample derived from one or more prostate-cancer diagnosed subjects who have received a therapeutic regimen to treat prostate cancer.

In a further embodiment, such subjects are monitored and/or periodically retested for a diagnostically relevant period of time ("longitudinal studies") following such test to verify continued survivability, or lack thereof. Such period of time may be one year, two years, two to five years, five years, five to ten years, ten years, or ten or more years from the initial testing date for determination of the reference or baseline value. Furthermore, retrospective measurement of cancer survivability associated genes in properly banked historical subject samples may be used in establishing these reference or baseline values, thus shortening the study time required, presuming the subjects have been appropriately followed during the intervening period through the intended horizon of the product claim.

A reference or baseline value can also comprise the amounts of cancer survivability associated genes derived from subjects who show an improvement in cancer status as a result of treatments and/or therapies for the cancer being treated and/or evaluated.

For example, where the reference or baseline level is comprised of the amounts of cancer survivability associated genes derived from one or more prostate-cancer diagnosed subjects who have not received any treatment for prostate cancer, a change (*e.g.*, increase or decrease) in the expression level of a cancer survivability associated gene in the patient-derived sample as compared to the expression level of such gene in the reference or baseline level indicates that the particular therapeutic may have an effect on the predicted survivability and/or survival time of the subject.

Expression of a prostate cancer survivability gene also allows for the course of treatment of prostate cancer to be monitored and evaluated for an effect on the predicted survivability and/or survival time of a prostate-cancer-diagnosed subject In this method, a biological sample is provided from a subject undergoing treatment, *e.g.*, if desired, biological samples are obtained from the subject at various time points before, during, or after treatment. Expression of a prostate cancer survivability gene is then determined and compared to a reference or baseline profile. The baseline profile may be taken or derived from one or more individuals who have been exposed to the treatment. Alternatively, the baseline level may be taken or derived from one or more individuals who have not been exposed to the treatment. For example, samples may be collected from subjects who have received initial treatment for prostate cancer and subsequent treatment for prostate cancer to monitor whether the course of treatment has an affect on the predicted survivability and/or survival time of the subject..

A Gene Expression Panel (Precision Profile™) is selected in a manner so that quantitative measurement of RNA or protein constituents in the Panel constitutes a measurement of of the predicted survivability and/or survival time of a subject. In one kind of arrangement, a calibrated profile data set is employed. Each member of the calibrated profile data set is a function of (i) a measure of a distinct constituent of a Gene Expression Panel (Precision Profile™) and (ii) a baseline quantity.

Additional embodiments relate to the use of an index or algorithm resulting from quantitative measurement of constituents, and optionally in addition, derived from either statistical analysis (e.g. predicted probability) or computational biology, useful as a prognostic tool for predicting the survivability and/or survival times of a prostate cancer-diagnosed subject (*e.g.*, as a direct effect or affecting latent classes).

Gene expression profiling and the use of index characterization for a particular condition or agent or both may be used to reduce the cost of Phase 3 clinical trials and may be used beyond Phase 3 trials; labeling for approved drugs; selection of suitable medication in a class of medications for a particular patient that is directed to their unique physiology; diagnosing or determining a prognosis of a medical condition or an infection which may precede onset of symptoms or alternatively diagnosing adverse side effects associated with administration of a therapeutic agent; managing the health care of a patient; and quality control for different batches of an agent or a mixture of agents.

The subject

The methods disclosed herein may be applied to cells of humans, mammals or other organisms without the need for undue experimentation by one of ordinary skill in the art because all cells transcribe RNA and it is known in the art how to extract RNA from all types of cells.

A subject can include those who have already been diagnosed as having prostate cancer or a condition related to prostate cancer. Subjects diagnosed with prostate cancer include those who have localized prostate cancer or prostate cancer metastasis (*e.g.*, bones and lymph nodes metastasis). Alterrnatively, a subject can include those who have been diagnosed with different stages of prostate cancer (*e.g.*, Stage 1, Stage 2, Stage 3, and Stage 4 prostate cancer as determined by the Tumor/Nodes/Metastases ("TNM") system; or Stage A, Stage B, Stage C, and Stage D, as determined by the Jewitt-Whitmore system). Diagnosis of prostate cancer is made, for example, from any one or combination of the following procedures: a medical history, physical examination, *e.g.*, digital rectal examination, blood tests, *e.g.*, a PSA test, and screening tests and tissue sampling procedures *e.g.*, cytoscopy and transrectal ultrasonography, and biopsy, in conjunction with Gleason Score. Alternatively, a subject can include those with hormone-refractory prostate cancer.

Optionally, the subject has been previously treated with a surgical procedure for removing prostate cancer or a condition related to prostate cancer, including but not limited to any one or combination of the following treatments: prostatectomy (including radical retropubic and radical perineal prostatectomy), transurethral resection, orchiectomy, and cryosurgery. Optionally, the subject has previously been treated with radiation therapy including but not limited to external beam radiation therapy and brachytherapy). Optionally, the subject has been treated with hormonal therapy, including but not limited to orchiectomy, anti-androgen therapy (*e.g.*, flutamide, bicalutamide, nilutamide, cyproterone acetate, ketoconazole and aminoglutethimide), and GnRH agonists (*e.g.*, leuprolide, goserelin, triptorelin, and buserelin). Optionally, the subject has previously been treated with chemotherapy for palliative care (*e.g.*, docetaxel with a corticosteroid such as prednisone). Optionally, the subject has previously been treated with any one or combination of such radiation therapy, hormonal therapy, and chemotherapy, as previously described, alone, in combination, or in succession with a surgical procedure for removing prostate cancer as previously described. Optionally, the subject may be treated with any of the agents previously described; alone, or in combination with a surgical procedure for removing prostate cancer and/or radiation therapy as previously described.

A subject can also include those who are suffering from, or at risk of developing prostate cancer or a condition related to prostate cancer, such as those who exhibit known risk factors for prostate cancer or conditions related to prostate cancer. Known risk factors for prostate cancer include, but are not limited to: age (increased risk above age 50), race (higher prevalence among African American men), nationality (higher prevalence in North America and northwestern Europe), family history, and diet (increased risk with a high animal fat diet).

Selecting Constituents of a Gene Expression Panel (Precision Profile™)

The general approach to selecting constituents of a Gene Expression Panel (Precision Profile™) has been described in PCT application publication number WO 01/25473, incorporated herein in its entirety. A wide range of Gene Expression Panels (Precision Profiles™) have been designed and experimentally validated, each panel providing a quantitative measure of biological condition that is derived from a sample of blood or other tissue. For each panel, experiments have verified that a Gene Expression Profile using the panel's constituents is informative of a biological condition (it has also been demonstrated that in being informative of biological condition, the Gene Expression Profile is used, among other things, to measure the effectiveness of therapy, as well as to provide a target for therapeutic intervention).

Gene Expression Profiles Based on Gene Expression Panels (Precision Profiles™) of the Present Invention

Tables 5-12 and 19-20 were derived from a study of the gene expression patterns based on the Precision Profile for Prostate Cancer survivability (Table 1) in hormone or taxane refractory prostate cancer patients, described in the Examples below.

Table 5 describes all statistically significant 1 and 2-gene models based on genes from the Precision Profile™ for Prostate Cancer Survivability (Table 1) which were identified by using a Cox-type Model as capable of predicting the survivability of a prostate cancer-diagnosed subject. For example, the first row of Table 5, describes a 2-gene model, ABL2 and C1QA, capable of predicting the survivability status of hormone or taxane refractory prostate cnacer subjects (cohort 4). The 2-gene model ABL2 and C1QA was also identified using a Zero-Inflation Poission model and a Markov model as a gene model capable of predicting the survivability of hormone or taxane refractory prostate cancer-diagnosed subjects with statistically significant accuracy, as described in Example below. Table 6 summarizes the mean expression and likelihood ratio p-values of the genes obtained from the Cox-type survival model.

Tables 7A-7D describe examples of statistically significant 1 and 2-gene models based on genes from the Precision Profile™ for Prostate Cancer Survivability (Table 1) which were identified by using a Zero Inflated Poisson survival model as capable of predicting the probability of being long-term survivor among prostate-cancer diagnosed subjects. Table 8 describes a comparison of various gene models identified using the Zero Inflated Poisson survival model.

Table 9 describes an example of a statistically significant 2 gene model identified by using a Markov survival model capable of predicting the probability of transitioning from their current state of health to the state of being dead.

Table 10 describes the differential expression of RNA transcripts in prostate cancer patients with a high vs. low risk of death, as predicted by the survivability models described herein.

Table 11 summarizes the waldp-values for two 2-gene models, ABL2 and C1QA, and SEMA4D and TIMP1, and for one 1-gene model, ABL2, obtained using the Cox-Type, Zero Inflated Poisson, and Markov survival models.

Table 12 summarizes a comparison of the 2-gene model, ABL2 and C1QA, obtained using the Cox-Type, Zero Inflated Poisson, and Markov survival models, sorted by exposure. Table 13 summarizes a comparison of the 2-gene model, ABL2 and C1QA, obtained using the Cox-Type, Zero Inflated Poisson, and Markov survival models, sorted by risk score.

Table 19 describes the mean differences in target gene expression in alive vs. dead prostate cancer subjects for the top 25 genes ranked by the Cox-Type model by p-value.

Table 20 describes a list of proteins which correspond to the RNA transcripts which exhibit differential expression in long-term prostate cancer survivors as opposed to short term survivors.

As indicated in the Tables listed above, several gene expression profiles have been derived from the Gene Expression Panel (Precision Profile™) described herein and experimentally validated as described herein, as being caable of providing a quantitative measure of the predicted survival rate and/or survival time of hormone or taxane refractory prostate cancer subjects. As described herein, several of the genes (*i.e.*, constituents) of the Precision Profile™ for Prostate Cancer Survivability are differentially expressed in long-term versus short term prostate cancer survivors. Without intending to be bound by theory, such differentially expressed genes may reflect an increased "bias" of the immune system towards phagocytosis and inflammation as reflected by increased production and activation of tissue macrophages and a decrease in both cell-mediated and humoral immunity. Examples of such differentially expressed genes include ABL2, C1QA, CDKN1A, ITGAL, SEMA4D, and TIMP1. Surprisingly, several of the most statistically significant gene expression profiles (*i.e.*, gene models) described herein comprise one or more of these six genes.

### ABL2

ABL2, also known as Tyrosine-protein kinase ABL2, is a membrane associated, nonreceptor tyrosine kinase which regulates cytoskeleton remodeling during cell differentiation, cell division and cell adhesion. It also localizes to dynamic actin structures, and phosphorylates CRK and CRKL, DOK1, and other proteins controlling cytoskeleton dynamics. ABL2 expression is closely correlated with semaphorin expression (T-cells > B-cells >> moncytes). It is activated in response to "outside - in" signalling mediated by LFA-1 / integrin interactions, and is involed in directed migration and integrated into receptor mediated GTP-ase activity. Without intending to be bound by theory, the differential expression of ABL2 seen between long term vs. short term prostate cancer survivors may be due to a decreased T-cell "surveillance" of antigen presenting cells, decreased cellular immunity and T-helper cell activity.

### CIQA

C1QA, also known as Complement C1q subcomponent subunit A, associates with the proenzymes C1r and C1s to yield C1, the first component of the serum complement system. The collagen-like regions of C1q interact with the Ca(2+)-dependent C1r(2)C1s(2) proenzyme complex, and efficient activation of C1 takes place on interaction of the globular heads of C1q with the Fc regions of IgG or IgM antibody present in immune complexes. C1q is required for phagocytotic clearance of apoptotic cells. C1QA is secreted extracellularly by monocytes and tissue macrophages. Expression levels increase as monocytes are transformed into tissue macrophages. Protein expression by macrophages is enhanced by IFNG. Without intending to be bound by theory, the differential expression of C1QA seen between long term vs. short term prostate cancer survivors may be due to the fact that one of the final steps in maturation of peripheral blood monocyte to tissue macrophage is the upregulation of C1q expression. C1q is not expressed in dendritic cells, therefore, there is a scewing of the immune system away from antigen presentation (dendritic cells) to phagocytosis / inflammation (macrophages).

### CDKN1A

CDKN1A expression and activation is required for maturation of the peripehral blood monocyte into tissue macrophages and dendritic cells. CDKN1A, also known as Cyclin-dependent kinase Inhibitor 1A, may promote cell cycle arrest by enhancing the inhibition of CDK2 activity by CDKN1A. It also may be required for repair of DNA damage by homologous recombination in conjunction with BRCA2. CDKN1A is expressed at high levels in testis and skeletal muscle and at lower levels in brain, heart, kidney, liver, lung, ovary, pancreas, placenta, and spleen. It is also seen in proliferating lymphocytes; associated with EGR gene expression in response to radiation challenge. Without intending to be bound by theory, the differential expression of CDKN1A seen between long term vs. short term prostate cancer survivors may be a reflection of augmented tissue macrophage production.

## ITGAL

ITGAL, also known as Integrin alpha-L (Leukocyte adhesion glycoprotein LFA-1), is a receptor for ICAM1, ICAM2, ICAM3 and ICAM4. It is involved in a variety of immune phenomena including leukocyte-endothelial cell interaction, cytotoxic T-cell mediated killing, and antibody dependent killing by granulocytes and monocytes. ITGAL is expressed in leukocytes. While found on all leukocyte subtypes, it has been reported to be highly expressed in T-cells and monocytes/macrophages. Without intending to be bound by theory, the differential expression of LFA-1 seen between long term vs. short term prostate cancer survivors may be a combination of effects in both the T-cells (decreased motility and antigen surveillance) and monocyte/ macrophage (increased tissue macrophage production and migration) populations. The overall decrease in LFA-1 expression is most likely due to a relatively greater decrease in T-cell mobility and antigen surveillance as refected in decreases in ABL2 and SEMA4D expression).

## SEMA4D

SEMA4D, also known as Semaphorin-4D, is involved in B-cell activation in the context of B-B and B-T cell interations and T-cell immunity. In the context of the immune response it binds to CD72 expressed on B-cells. In non-immune cells SEMA4D will bind to Plexin-B1 and is inovled in directed migration. SEMA4D is strongly expressed in skeletal muscle, peripheral blood lymphocytes, spleen, and thymus and also expressed at lower levels in testes, brain, kidney, small intestine, prostate, heart, placenta, lung and pancreas, but not in colon and liver. It is constitutively expressed on T-cells, upregulated in B-cells when activated. It is not found on monocyte-derived cells (dendritic cells or macrophages). Without intending to be bound by theory, the differential expression of SEMA4D seen between long term vs. short term prostate cancer survivors may be due to decreased helper T-cell activity and reflective of an overall decrease in cell-mediated and humeral immunity.

## TIMP1

TIMP1, also known as Metalloproteinase inhibitor 1, complexes with metalloproteinases (such as collagenases) and irreversibly inactivates them. The N-terminal domain is known to inhibit all MMPs except for the MT-MMPs and MMP-19. The C-terminal domain mediates numerous "non-MMP dependent" activities including signficant "anti-apoptosis" in a variety of cell types, inlcuding tumor cells (breast, prostate, and others). Binding of the zymogen form of MMP-9 (pro-MMP9) by the C-terminal domain may allow for the display of active enzyme on the cell surface of macrophages (directed migration) and tumor cells (metastasis). TIMP1 is secreted. It is expressed by monocytes, macrophages, fibroblasts and tumor stromal cells. Generally, in cancer, TIMP-1 protein expression in the tumor and in blood inversely correlated with clinical outcome. Without intending to be bound by theory, the differential expression of SEMA4D seen between long term vs. short term prostate cancer survivors may be due to TIMP1 and MMP9 upregulation in a coordinated fashion as peripheral blood monocytes mature into tissue macrophages.

### Design of assays

Typically, a sample is run through a panel in replicates of three for each target gene (assay); that is, a sample is divided into aliquots and for each aliquot the concentrations of each constituent in a Gene Expression Panel (Precision Profile™) is measured. From over thousands of constituent assays, with each assay conducted in triplicate, an average coefficient of variation was found (standard deviation/average)* 100, of less than 2 percent among the normalized ΔCt measurements for each assay (where normalized quantitation of the target mRNA is determined by the difference in threshold cycles between the internal control (*e.g.*, an endogenous marker such as 18S rRNA, or an exogenous marker) and the gene of interest. This is a measure called "intra-assay variability". Assays have also been conducted on different occasions using the same sample material. This is a measure of "inter-assay variability". Preferably, the average coefficient of variation of intra- assay variability or inter-assay variability is less than 20%, more preferably less than 10%, more preferably less than 5%, more preferably less than 4%, more preferably less than 3%, more preferably less than 2%, and even more preferably less than 1%.

It has been determined that it is valuable to use the quadruplicate or triplicate test results to identify and eliminate data points that are statistical "outliers"; such data points are those that differ by a percentage greater, for example, than 3% of the average of all three or four values. Moreover, if more than one data point in a set of three or four is excluded by this procedure, then all data for the relevant constituent is discarded.

Measurement of Gene Expression for a Constituent in the Panel

For measuring the amount of a particular RNA in a sample, methods known to one of ordinary skill in the art were used to extract and quantify transcribed RNA from a sample with respect to a constituent of a Gene Expression Panel (Precision Profile™). (See detailed protocols below. Also see PCT application publication number WO 98/24935 herein incorporated by reference for RNA analysis protocols). Briefly, RNA is extracted from a sample such as any tissue, body fluid, cell (*e.g.*, circulating tumor cell) or culture medium in which a population of cells of a subject might be growing. For example, cells may be lysed and RNA eluted in a suitable solution in which to conduct a DNAse reaction. Subsequent to RNA extraction, first strand synthesis may be performed using a reverse transcriptase. Gene amplification, more specifically quantitative PCR assays, can then be conducted and the gene of interest calibrated against an internal marker such as 18S rRNA (Hirayama et al., Blood 92, 1998: 46-52). Any other endogenous marker can be used, such as 28S-25S rRNA and 5S rRNA. Samples are measured in multiple replicates, for example, 3 replicates. In an embodiment of the invention, quantitative PCR is performed using amplification, reporting agents and instruments such as those supplied commercially by Applied Biosystems (Foster City, CA). Given a defined efficiency of amplification of target transcripts, the point (*e.g.*, cycle number) that signal from amplified target template is detectable may be directly related to the amount of specific message transcript in the measured sample. Similarly, other quantifiable signals such as fluorescence, enzyme activity, disintegrations per minute, absorbance, etc., when correlated to a known concentration of target templates (*e.g.*, a reference standard curve) or normalized to a standard with limited variability can be used to quantify the number of target templates in an unknown sample.

Although not limited to amplification methods, quantitative gene expression techniques may utilize amplification of the target transcript. Alternatively or in combination with amplification of the target transcript, quantitation of the reporter signal for an internal marker generated by the exponential increase of amplified product may also be used. Amplification of the target template may be accomplished by isothermic gene amplification strategies or by gene amplification by thermal cycling such as PCR.

It is desirable to obtain a definable and reproducible correlation between the amplified target or reporter signal, i.e., internal marker, and the concentration of starting templates. It has been discovered that this objective can be achieved by careful attention to, for example, consistent primer-template ratios and a strict adherence to a narrow permissible level of experimental amplification efficiencies (for example 80.0 to 100% +/- 5% relative efficiency, typically 90.0 to 100% +/- 5% relative efficiency, more typically 95.0 to 100% +/- 2 %, and most typically 98 to 100% +/- 1 % relative efficiency). In determining gene expression levels with regard to a single Gene Expression Profile, it is necessary that all constituents of the panels, including endogenous controls, maintain similar amplification efficiencies, as defined herein, to permit accurate and precise relative measurements for each constituent. Amplification efficiencies are regarded as being "substantially similar", for the purposes of this description and the following claims, if they differ by no more than approximately 10%, preferably by less than approximately 5%, more preferably by less than approximately 3%, and more preferably by less than approximately 1%. Measurement conditions are regarded as being "substantially repeatable, for the purposes of this description and the following claims, if they differ by no more than approximately +/- 10% coefficient of variation (CV), preferably by less than approximately +/- 5% CV, more preferably +/- 2% CV. These constraints should be observed over the entire range of concentration levels to be measured associated with the relevant biological condition. While it is thus necessary for various embodiments herein to satisfy criteria that measurements are achieved under measurement conditions that are substantially repeatable and wherein specificity and efficiencies of amplification for all constituents are substantially similar, nevertheless, it is within the scope of the present invention as claimed herein to achieve such measurement conditions by adjusting assay results that do not satisfy these criteria directly, in such a manner as to compensate for errors, so that the criteria are satisfied after suitable adjustment of assay results.

In practice, tests are run to assure that these conditions are satisfied. For example, the design of all primer-probe sets are done in house, experimentation is performed to determine which set gives the best performance. Even though primer-probe design can be enhanced using computer techniques known in the art, and notwithstanding common practice, it has been found that experimental validation is still useful. Moreover, in the course of experimental validation, the selected primer-probe combination is associated with a set of features:

The reverse primer should be complementary to the coding DNA strand. In one embodiment, the primer should be located across an intron-exon junction, with not more than four bases of the three-prime end of the reverse primer complementary to the proximal exon. (If more than four bases are complementary, then it would tend to competitively amplify genomic DNA.)

In an embodiment of the invention, the primer probe set should amplify cDNA of less than 110 bases in length and should not amplify, or generate fluorescent signal from, genomic DNA or transcripts or cDNA from related but biologically irrelevant loci.

A suitable target of the selected primer probe is first strand cDNA, which in one embodiment may be prepared from whole blood as follows:

(a) <u>Use of whole blood for *ex vivo* assessment of predicted survivability and/or survival time</u>

Human blood is obtained by venipuncture and prepared for assay. The aliquots of heparinized, whole blood are mixed with additional test therapeutic compounds and held at 37°C in an atmosphere of 5% $CO_2$ for 30 minutes. Cells are lysed and nucleic acids, *e.g.*, RNA, are extracted by various standard means.

Nucleic acids, RNA and or DNA, are purified from cells, tissues or fluids of the test population of cells. RNA is preferentially obtained from the nucleic acid mix using a variety of standard procedures (or RNA Isolation Strategies, pp. 55-104, in RNA Methodologies, A laboratory guide for isolation and characterization, 2nd edition, 1998, Robert E. Farrell, Jr., Ed., Academic Press), in the present using a filter-based RNA isolation system from Ambion (RNAqueous ™, Phenol-free Total RNA Isolation Kit, Catalog #1912, version 9908; Austin, Texas).

(b) <u>Amplification strategies.</u>

Specific RNAs are amplified using message specific primers or random primers. The specific primers are synthesized from data obtained from public databases (*e.g.*, Unigene, National Center for Biotechnology Information, National Library of Medicine, Bethesda, MD), including information from genomic and cDNA libraries obtained from humans and other animals. Primers are chosen to preferentially amplify from specific RNAs obtained from the test or indicator samples (see, for example, RT PCR, Chapter 15 in RNA Methodologies, A Laboratory Guide for Isolation and Characterization, 2nd edition, 1998, Robert E. Farrell, Jr., Ed., Academic Press; or Chapter 22 pp.143-151, RNA Isolation and Characterization Protocols, Methods in Molecular Biology, Volume 86, 1998, R. Rapley and D. L. Manning Eds., Human Press, or Chapter 14 Statistical refinement of primer design parameters; or Chapter 5, pp.55-72, PCR Applications: protocols for functional genomics, M.A.Innis, D.H. Gelfand and J.J. Sninsky, Eds., 1999, Academic Press). Amplifications are carried out in either isothermic conditions or using a thermal cycler (for example, a ABI 9600 or 9700 or 7900 obtained from Applied Biosystems, Foster City, CA; see Nucleic acid detection methods, pp. 1-24, in Molecular Methods for Virus Detection, D.L.Wiedbrauk and D.H., Farkas, Eds., 1995, Academic Press). Amplified nucleic acids are detected using fluorescent-tagged detection oligonucleotide probes (see, for example, TaqmanTM PCR Reagent Kit, Protocol, part number 402823, Revision A, 1996, Applied Biosystems, Foster City CA) that are identified and synthesized from publicly known databases as described for the amplification primers.

For example, without limitation, amplified cDNA is detected and quantified using detection systems such as the ABI Prism® 7900 Sequence Detection System (Applied Biosystems (Foster City, CA)), the Cepheid SmartCycler® and Cepheid GeneXpert® Systems, the Fluidigm BioMark™ System, and the Roche LightCycler® 480 Real-Time PCR System. Amounts of specific RNAs contained in the test sample can be related to the relative quantity of fluorescence observed (see for example, Advances in Quantitative PCR Technology: 5' Nuclease Assays, Y.S. Lie and C.J. Petropolus, Current Opinion in Biotechnology, 1998, 9:43-48, or Rapid Thermal Cycling and PCR Kinetics, pp. 211-229, chapter 14 in PCR applications: protocols for functional genomics, M.A. Innis, D.H. Gelfand and J.J. Sninsky, Eds., 1999, Academic Press). Examples of the procedure used with several of the above-mentioned detection systems are described below. In some embodiments, these procedures can be used for both whole blood RNA and RNA extracted from cultured cells (*e.g.*, without limitation, CTCs, and CECs). In some embodiments, any tissue, body fluid, or cell(s) (*e.g.*, circulating tumor cells (CTCs) or circulating endothelial cells (CECs)) may be used for *ex vivo* assessment of predicted survivability and/or survival time affected by an agent. Methods herein may also be applied using proteins where sensitive quantitative techniques, such as an Enzyme Linked ImmunoSorbent Assay (ELISA) or mass spectroscopy, are available and well-known in the art for measuring the amount of a protein constituent (see WO 98/24935 herein incorporated by reference).

An example of a procedure for the synthesis of first strand cDNA for use in PCR amplification is as follows:

<u>Materials</u>

1. Applied Biosystems TAQMAN Reverse Transcription Reagents Kit (P/N 808-0234). Kit Components: 10X TaqMan RT Buffer, 25 mM Magnesium chloride, deoxyNTPs mixture, Random Hexamers, RNase Inhibitor, MultiScribe Reverse Transcriptase (50 U/mL) (2) RNase / DNase free water (DEPC Treated Water from Ambion (P/N 9915G), or equivalent).

<u>Methods</u>

1. Place RNase Inhibitor and MultiScribe Reverse Transcriptase on ice immediately. All other reagents can be thawed at room temperature and then placed on ice.

2. Remove RNA samples from -80oC freezer and thaw at room temperature and then place immediately on ice.

3. Prepare the following cocktail of Reverse Transcriptase Reagents for each 100 mL RT reaction (for multiple samples, prepare extra cocktail to allow for pipetting error):

|  | 1 reaction (mL) | 11X, e.g. 10 samples ($\mu$L) |
|---|---|---|
| 10X RT Buffer | 10.0 | 110.0 |
| 25 mM MgCl$_2$ | 22.0 | 242.0 |
| dNTPs | 20.0 | 220.0 |
| Random Hexamers | 5.0 | 55.0 |
| RNAse Inhibitor | 2.0 | 22.0 |
| Reverse Transcriptase | 2.5 | 27.5 |
| Water | 18.5 | 203.5 |
| Total: | 80.0 | 880.0 (80 $\mu$L per sample) |

4. Bring each RNA sample to a total volume of 20 $\mu$L in a 1.5 mL microcentrifuge tube (for example, remove 10 $\mu$L RNA and dilute to 20 $\mu$L with RNase / DNase free water, for whole blood RNA use 20 $\mu$L total RNA) and add 80 $\mu$L RT reaction mix from step 5,2,3. Mix by pipetting up and down.

5. Incubate sample at room temperature for 10 minutes.

6. Incubate sample at 37°C for 1 hour.

7. Incubate sample at 90°C for 10 minutes.

8. Quick spin samples in microcentrifuge.

9. Place sample on ice if doing PCR immediately, otherwise store sample at -20°C for future use.

10. PCR QC should be run on all RT samples using 18S and β-action.

Following the synthesis of first strand cDNA, one particular embodiment of the approach for amplification of first strand cDNA by PCR, followed by detection and quantification of constituents of a Gene Expression Panel (Precision Profil™) is performed using the ABI Prism® 7900 Sequence Detection System as follows:

Materials

1. 20X Primer/Probe Mix for each gene of interest.

2. 20X Primer/Probe Mix for 18S endogenous control.

3. 2X Taqman Universal PCR Master Mix.

4. cDNA transcribed from RNA extracted from cells.

5. Applied Biosystems 96-Well Optical Reaction Plates.

6. Applied Biosystems Optical Caps, or optical-clear film.

7. Applied Biosystem Prism® 7700 or 7900 Sequence Detector.

Methods

1. Make stocks of each Primer/Probe mix containing the Primer/Probe for the gene of interest, Primer/Probe for 18S endogenous control, and 2X PCR Master Mix as follows. Make sufficient excess to allow for pipetting error e.g., approximately 10% excess. The following example illustrates a typical set up for one gene with quadruplicate samples testing two conditions (2 plates).

|  | 1X (1 well) ($\mu$L) |
|---|---|
| 2X Master Mix | 7.5 |
| 20X 18S Primer/Probe Mix | 0.75 |
| 20X Gene of interest Primer/Probe Mix | 0.75 |
| Total | 9.0 |

2. Make stocks of cDNA targets by diluting 95$\mu$L of cDNA into 2000$\mu$L of water. The amount of cDNA is adjusted to give Ct values between 10 and 18, typically between 12 and 16.

3. Pipette 9 μL of Primer/Probe mix into the appropriate wells of an Applied Biosystems 384-Well Optical Reaction Plate.

4. Pipette 10μL of cDNA stock solution into each well of the Applied Biosystems 384-Well Optical Reaction Plate.

5. Seal the plate with Applied Biosystems Optical Caps, or optical-clear film.

6. Analyze the plate on the ABI Prism® 7900 Sequence Detector.

In another embodiment of the invention, the use of the primer probe with the first strand cDNA as described above to permit measurement of constituents of a Gene Expression Panel (Precision Profile™) is performed using a QPCR assay on Cepheid SmartCycler® and GeneXpert® Instruments as follows:

I. To run a QPCR assay in duplicate on the Cepheid SmartCycler® instrument containing three target genes and one reference gene, the following procedure should be followed.

A. With 20X Primer/Probe Stocks.

<u>Materials</u>

1. SmartMix™-HM lyophilized Master Mix.

2. Molecular grade water.

3. 20X Primer/Probe Mix for the 18S endogenous control gene. The endogenous control gene will be dual labeled with VIC-MGB or equivalent.

4. 20X Primer/Probe Mix for each for target gene one, dual labeled with FAM-BHQ1 or equivalent.

5. 20X Primer/Probe Mix for each for target gene two, dual labeled with Texas Red-BHQ2 or equivalent.

6. 20X Primer/Probe Mix for each for target gene three, dual labeled with Alexa 647-BHQ3 or equivalent.

7. Tris buffer, pH 9.0

8. cDNA transcribed from RNA extracted from sample.

9. SmartCycler 25 μL tube.

10. Cepheid SmartCycler® instrument.

<u>Methods</u>

1. For each cDNA sample to be investigated, add the following to a sterile 650 μL tube.

| | |
|---|---|
| SmartMix™-HM lyophilized Master Mix | 1 bead |
| 20X 18S Primer/Probe Mix | 2.5 μL |
| 20X Target Gene 1 Primer/Probe Mix | 2.5 μL |
| 20X Target Gene 2 Primer/Probe Mix | 2.5 μL |
| 20X Target Gene 3 Primer/Probe Mix | 2.5 μL |
| Tris Buffer, pH 9.0 | 2.5 μL |
| Sterile Water | 34.5 μL |
| Total | 47 μL |

Vortex the mixture for 1 second three times to completely mix the reagents. Briefly centrifuge the tube after vortexing.

2. Dilute the cDNA sample so that a 3 μL addition to the reagent mixture above will give an 18S reference gene CT value between 12 and 16.

3. Add 3 μL of the prepared cDNA sample to the reagent mixture bringing the total volume to 50 μL. Vortex the mixture for 1 second three times to completely mix the reagents. Briefly centrifuge the tube after vortexing.

4. Add 25 μL of the mixture to each of two SmartCycler® tubes, cap the tube and spin for 5 seconds in a microcentrifuge having an adapter for SmartCycler® tubes.

5. Remove the two SmartCycler tubes from the microcentrifuge and inspect for air bubbles. If bubbles are present, re-spin, otherwise, load the tubes into the SmartCycler® instrument.

6. Run the appropriate QPCR protocol on the SmartCycler®, export the data and analyze the results.

B. With Lyophilized SmartBeads™.

<u>Materials</u>

    1. SmartMix™-HM lyophilized Master Mix.
    2. Molecular grade water.
    3. SmartBeads™ containing the 18S endogenous control gene dual labeled with VIC-MGB or equivalent, and the three target genes, one dual labeled with FAM-BHQ1 or equivalent, one dual labeled with Texas Red-BHQ2 or equivalent and one dual labeled with Alexa 647-BHQ3 or equivalent.
    4. Tris buffer, pH 9.0
    5. cDNA transcribed from RNA extracted from sample.
    6. SmartCycler® 25 $\mu$L tube.
    7. Cepheid SmartCycler® instrument.

<u>Methods</u>

1. For each cDNA sample to be investigated, add the following to a sterile 650 $\mu$L tube.

| | |
|---|---|
| SmartMix™-HM lyophilized Master Mix | 1 bead |
| SmartBead™ containing four primer/probe sets | 1 bead |
| Tris Buffer, pH 9.0 | 2.5 $\mu$L |
| Sterile Water | 44.5 $\mu$L |
| Total | 47 $\mu$L |

Vortex the mixture for 1 second three times to completely mix the reagents. Briefly centrifuge the tube after vortexing.
2. Dilute the cDNA sample so that a 3 $\mu$L addition to the reagent mixture above will give an 18S reference gene CT value between 12 and 16.
3. Add 3 $\mu$L of the prepared cDNA sample to the reagent mixture bringing the total volume to 50 $\mu$L. Vortex the mixture for 1 second three times to completely mix the reagents. Briefly centrifuge the tube after vortexing.
4. Add 25 $\mu$L of the mixture to each of two SmartCycler tubes, cap the tube and spin for 5 seconds in a microcentrifuge having an adapter for SmartCycler® tubes.
5. Remove the two SmartCycler® tubes from the microcentrifuge and inspect for air bubbles. If bubbles are present, re-spin, otherwise, load the tubes into the SmartCycler® instrument.
6. Run the appropriate QPCR protocol on the SmartCycler®, export the data and analyze the results.

II. To run a QPCR assay on the Cepheid GeneXpert® instrument containing three target genes and one reference gene, the following procedure should be followed. Note that to do duplicates, two self contained cartridges need to be loaded and run on the GeneXpert® instrument.

<u>Materials</u>

    1. Cepheid GeneXpert® self contained cartridge preloaded with a lyophilized SmartMix™-HM master mix bead and a lyophilized SmartBead™ containing four primer/probe sets.
    2. Molecular grade water, containing Tris buffer, pH 9.0.
    3. Extraction and purification reagents.
    4. Clinical sample (whole blood, RNA, etc.)
    5. Cepheid GeneXpert® instrument.

<u>Methods</u>

    1. Remove appropriate GeneXpert® self contained cartridge from packaging.

2. Fill appropriate chamber of self contained cartridge with molecular grade water with Tris buffer, pH 9.0.

3. Fill appropriate chambers of self contained cartridge with extraction and purification reagents.

4. Load aliquot of clinical sample into appropriate chamber of self contained cartridge.

5. Seal cartridge and load into GeneXpert® instrument.

6. Run the appropriate extraction and amplification protocol on the GeneXpert® and analyze the resultant data.

In yet another embodiment of the invention, the use of the primer probe with the first strand cDNA as described above to permit measurement of constituents of a Gene Expression Panel (Precision Profile™) is performed using a QPCR assay on the Roche LightCycler 480 Real-Time PCR System as follows:

Materials

1. 20X Primer/Probe stock for the 18S endogenous control gene. The endogenous control gene may be dual labeled with either VIC-MGB or VIC-TAMRA.

2. 20X Primer/Probe stock for each target gene, dual labeled with either FAM-TAMRA or FAM-BHQ1.

3. 2X LightCycler® 490 Probes Master (master mix).

4. 1X cDNA sample stocks transcribed from RNA extracted from samples.

5. 1X TE buffer, pH 8.0.

6. LightCycler® 480 384-well plates.

7. Source MDx 24 gene Precision Profile™ 96-well intermediate plates.

8. RNase/DNase free 96-well plate.

9. 1.5 mL microcentrifuge tubes.

10. Beckman/Coulter Biomek® 3000 Laboratory Automation Workstation.

11. Velocity 11 Bravo™ Liquid Handling Platform.

12. LightCycler 480 Real-Time PCR System.

Methods

1. Remove a Source MDx 24 gene Precision Profile™ 96-well intermediate plate from the freezer, thaw and spin in a plate centrifuge.

2. Dilute four (4) 1X cDNA sample stocks in separate 1.5 mL microcentrifuge tubes with the total final volume for each of 540 μL.

3. Transfer the 4 diluted cDNA samples to an empty RNase/DNase free 96-well plate using the Biomek® 3000 Laboratory Automation Workstation.

4. Transfer the cDNA samples from the cDNA plate created in step 3 to the thawed and centrifuged Source MDx 24 gene Precision Profile™ 96-well intermediate plate using Biomek® 3000 Laboratory Automation Workstation. Seal the plate with a foil seal and spin in a plate centrifuge.

5. Transfer the contents of the cDNA-loaded Source MDx 24 gene Precision Profile™ 96-well intermediate plate to a new LightCycler® 480 384-well plate using the Bravo™ Liquid Handling Platform. Seal the 384-well plate with a LightCycler® 480 optical sealing foil and spin in a plate centrifuge for 1 minute at 2000 rpm.

6. Place the sealed in a dark 4°C refrigerator for a minimum of 4 minutes.

7. Load the plate into the LightCycler® 480 Real-Time PCR System and start the LightCycler® 480 software. Chose the appropriate run parameters and start the run.

8. At the conclusion of the run, analyze the data and export the resulting CP values to the database.

In some instances, target gene FAM measurements may be beyond the detection limit of the particular platform instrument used to detect and quantify constituents of a Gene Expression Panel (Precision Profile™). To address the issue of "undetermined" gene expression measures as lack of expression for a particular gene, the detection limit may be reset and the "undetermined" constituents may be "flagged". For example without limitation, the ABI Prism® 7900HT Sequence Detection System reports target gene FAM measurements that are beyond the detection limit of the instrument (>40 cycles) as "undetermined". Detection Limit Reset is performed when at least 1 of 3 target gene FAM $C_T$ replicates are not detected after 40 cycles and are designated as "undetermined". "Undetermined" target gene FAM $C_T$ replicates are re-set to 40 and flagged. $C_T$ normalization ($\Delta C_T$) and relative expression calculations that have used re-set FAM $C_T$ values are also flagged.

For measuring the amount of a particular protein in a sample, methods known to one of ordinary skill in the art can be used to extract and quantify protein from a sample with respect to a constituent of a Protein Expression Panel (*e.g.*, the Precistion Protein Panel for Prostate Cancer Survivability in Table 20). The sample may be any tissue, body fluid (*e.g.*, whole blood, blood fraction (*e.g.*, serum, plasma, leukocytes), urine, semen), cell (*e.g.*, circulating tumor cell) or culture medium in which a population of cells of a subject might be growing. Expression determined at the protein level, *i.e.*, by measuring the levels of polypeptides encoded by the gene products described herein, or activities thereof. Such methods are well known in the art and include, *e.g.*, immunoassays based on antibodies to proteins encoded by the genes described herein as predictive of prostate cancer survability (*e.g.*, one or more constituents of Tables 20). Such antibodies may be obtained by methods known to one of ordinary skill in the art. Alternatively, such antibodies may be commercially available. Examples of such commercially available antibodies include, without limitation, the ABL2 antibody IHB11 (ab54209, Abcam, Cambridge, MA), the CD100 [A8] (SEMA4D) antibody (ab33260, Abcam, Cambridge MA), the CD11a [EP1285Y] (ITGAL) antibody (ab52895, Abcam, Cambridge, MA), the C1QA antibody (ab14004, Abcam, Cambridge, MA), the TIMP1 antibody (ab38978 or ab1827, Abcam, Cambridge, MA), and the CDKN1A [2186C2a] antibody (ab51332, Abcam, Cambridge, MA). Alternatively, a suitable method can be selected to determine the activity of proteins encoded by the marker genes according to the activity of each protein analyzed.

Immunoassays carried out in accordance with the present invention may be homogeneous assays or heterogeneous assays. In a homogeneous assay the immunological reaction usually involves the specific antibody, a labeled analyte, and the sample of interest. The signal arising from the label is modified, directly or indirectly, upon the binding of the antibody to the labeled analyte. Both the immunological reaction and detection of the extent thereof are carried out in a homogeneous solution. Immunochemical labels which may be employed include free radicals, radioisotopes, fluorescent dyes, enzymes, bacteriophages, or coenzymes.

In a heterogeneous assay approach, the reagents are usually the sample, the antibody, and means for producing a detectable signal. Samples as described above may be used. The antibody is generally immobilized on a support, such as a bead, plate, slide, or column, and contacted with the specimen suspected of containing the antigen in a liquid phase. The support is then separated from the liquid phase and either the support phase or the liquid phase is examined for a detectable signal employing means for producing such signal. The signal is related to the presence of the analyte in the sample. Means for producing a detectable signal include the use of radioactive labels, fluorescent labels, or enzyme labels. For example, if the antigen to be detected contains a second binding site, an antibody which binds to that site can be conjugated to a detectable group and added to the liquid phase reaction solution before the separation step. The presence of the detectable group on the solid support indicates the presence of the antigen in the test sample. Examples of suitable immunoassays are radioimmunoassays, immunofluorescence methods, or enzyme-linked immunoassays.

Those skilled in the art will be familiar with numerous specific immunoassay formats and variations thereof which may be useful for carrying out the method disclosed herein. See generally E. Maggio, Enzyme-Immunoassay, (1980) (CRC Press, Inc., Boca Raton, Fla.); see also U.S. Pat. No. 4,727,022 to Skold et al. titled "Methods for Modulating Ligand-Receptor Interactions and their Application," U.S. Pat. No. 4,659,678 to Forrest et al. titled "Immunoassay of Antigens," U.S. Pat. No. 4,376,110 to David et al., titled "Immunometric Assays Using Monoclonal Antibodies," U.S. Pat. No. 4,275,149 to Litman et al., titled "Macromolecular Environment Control in Specific Receptor Assays," U.S. Pat. No. 4,233,402 to Maggio et al., titled "Reagents and Method Employing Channeling," and U.S. Pat. No. 4,230,767 to Boguslaski et al., titled "Heterogenous Specific Binding Assay Employing a Coenzyme as Label."

Baseline profile data sets

The analyses of samples from single individuals and from large groups of individuals provide a library of profile data sets relating to a particular panel or series of panels. These profile data sets may be stored as records in a library for use as baseline profile data sets. As the term "baseline" suggests, the stored baseline profile data sets serve as comparators for providing a calibrated profile data set that is informative about the predicted survivability and/or survival time, or the effect of a variable on (*e.g.*, the effect of an therapeutic agent) on the predicted survivability and/or survival time of a subject. Baseline profile data sets may be stored in libraries and classified in a number of cross-referential ways. One form of classification may rely on the characteristics of the panels from which the data sets are derived. The libraries may also be accessed for records associated with a single subject or particular clinical trial. The classification of baseline profile data sets may further be annotated with medical information about a particular subject, a medical condition, and/or a particular agent.

The choice of a baseline profile data set for creating a calibrated profile data set is related to the survivability and/or survival time to be evaluated, monitored, or predicted, as well as, the intended use of the calibrated panel (*e.g.*, as to monitor the affect of a therapeutic agent on predicted survivability and/or survival time of a subject over time). It may be desirable to access baseline profile data sets from the same subject for whom a first profile data set is obtained or from different subject at varying times, exposures to stimuli, drugs or complex compounds; or may be derived from like or dissimilar populations or sets of subjects.

The profile data set may arise from the same subject for which the first data set is obtained, where the sample is taken at a separate or similar time, a different or similar site or in a different or similar biological condition. For example, a sample may be taken before stimulation or after stimulation with an exogenous compound or substance, such as before or after therapeutic treatment. Alternatively the sample is taken before or include before or after a surgical procedure for prostate cancer. The profile data set obtained from the unstimulated sample may serve as a baseline profile data set for the sample taken after stimulation. The baseline data set may also be derived from a library containing profile data sets of a population or set of subjects having some defining characteristic or biological condition. The baseline profile data set may also correspond to some *ex vivo* or *in vitro* properties associated with an *in vitro* cell culture. The resultant calibrated profile data sets may then be stored as a record in a database or library along with or separate from the baseline profile data base and optionally the first profile data *set al.*though the first profile data set would normally become incorporated into a baseline profile data set under suitable classification criteria. The remarkable consistency of Gene Expression Profiles associated with predicted survivability and/or survival times makes it valuable to store profile data, which can be used, among other things for normative reference purposes. The normative reference can serve to indicate the degree to which a subject conforms to a given prediction (e.g., survivability and/or survival time).

Calibrated data

Given the repeatability achieved in measurement of gene expression, described above in connection with "Gene Expression Panels" (Precision Profiles™) and "gene amplification", it was concluded that where differences occur in measurement under such conditions, the differences are attributable to differences in biological condition. Thus, it has been found that calibrated profile data sets are highly reproducible in samples taken from the same individual under the same conditions. Similarly, it has been found that calibrated profile data sets are reproducible in samples that are repeatedly tested. Also found have been repeated instances wherein calibrated profile data sets obtained when samples from a subject are exposed *ex vivo* to a compound are comparable to calibrated profile data from a sample that has been exposed to a sample *in vivo.*

Calculation of calibrated profile data sets and computational aids

The calibrated profile data set may be expressed in a spreadsheet or represented graphically for example, in a bar chart or tabular form but may also be expressed in a three dimensional representation. The function relating the baseline and profile data may be a ratio expressed as a logarithm. The constituent may be itemized on the x-axis and the logarithmic scale may be on the y-axis. Members of a calibrated data set may be expressed as a positive value representing a relative enhancement of gene expression or as a negative value representing a relative reduction in gene expression with respect to the baseline.

Each member of the calibrated profile data set should be reproducible within a range with respect to similar samples taken from the subject under similar conditions. For example, the calibrated profile data sets may be reproducible within 20%, and typically within 10%. In accordance with embodiments of the invention, a pattern of increasing, decreasing and no change in relative gene expression from each of a plurality of gene loci examined in the Gene Expression Panel (Precision Profile™) may be used to prepare a calibrated profile set that is informative with regards to predicted survivability and/or survival time of a subject or populations or sets of subjects or samples. Patterns of this nature may be used to identify likely candidates for a drug trial, used alone or in combination with other clinical indicators to be prognostic with respect to predicted survivability and/or survival time or may be used to guide the development of a pharmaceutical or nutraceutical through manufacture, testing and marketing.

The numerical data obtained from quantitative gene expression and numerical data from calibrated gene expression relative to a baseline profile data set may be stored in databases or digital storage mediums and may be retrieved for purposes including managing patient health care or for conducting clinical trials or for characterizing a drug. The data may be transferred in physical or wireless networks via the World Wide Web, email, or internet access site for example or by hard copy so as to be collected and pooled from distant geographic sites.

The method also includes producing a calibrated profile data set for the panel, wherein each member of the calibrated profile data set is a function of a corresponding member of the first profile data set and a corresponding member of a baseline profile data set for the panel, and wherein the baseline profile data set is related to the predicted survivability and/or survival time of a prostate cancer diagnosed subject, with the calibrated profile data set being a comparison between the first profile data set and the baseline profile data set, thereby providing evaluation of the predicted survivability and/or survival time of a prostate cancer-diagnosed subject.

In yet other embodiments, the function is a mathematical function and is other than a simple difference, including a second function of the ratio of the corresponding member of first profile data set to the corresponding member of the baseline profile data set, or a logarithmic function. In such embodiments, the first sample is obtained and the first profile data set quantified at a first location, and the calibrated profile data set is produced using a network to access a database stored on a digital storage medium in a second location, wherein the database may be updated to reflect the first profile data set quantified from the sample. Additionally, using a network may include accessing a global computer network.

In an embodiment of the present invention, a descriptive record is stored in a single database or multiple databases where the stored data includes the raw gene expression data (first profile data set) prior to transformation by use of a baseline profile data set, as well as a record of the baseline profile data set used to generate the calibrated profile data set including for example, annotations regarding whether the baseline profile data set is derived from a particular Signature Panel and any other annotation that facilitates interpretation and use of the data.

Because the data is in a universal format, data handling may readily be done with a computer. The data is organized so as to provide an output optionally corresponding to a graphical representation of a calibrated data set.

The above described data storage on a computer may provide the information in a form that can be accessed by a user. Accordingly, the user may load the information onto a second access site including downloading the information. However, access may be restricted to users having a password or other security device so as to protect the medical records contained within. A feature of this embodiment of the invention is the ability of a user to add new or annotated records to the data set so the records become part of the biological information.

The various embodiments of the invention may be also implemented as a computer program product for use with a computer system. The product may include program code for deriving a first profile data set and for producing calibrated profiles. Such implementation may include a series of computer instructions fixed either on a tangible medium, such as a computer readable medium (for example, a diskette, CD-ROM, ROM, or fixed disk), or transmittable to a computer system via a modem or other interface device, such as a communications adapter coupled to a network. The network coupling may be for example, over optical or wired communications lines or via wireless techniques (for example, microwave, infrared or other transmission techniques) or some combination of these. The series of computer instructions preferably embodies all or part of the functionality previously described herein with respect to the system. Those skilled in the art should appreciate that such computer instructions can be written in a number of programming languages for use with many computer architectures or operating systems. Furthermore, such instructions may be stored in any memory device, such as semiconductor, magnetic, optical or other memory devices, and may be transmitted using any communications technology, such as optical, infrared, microwave, or other transmission technologies. It is expected that such a computer program product may be distributed as a removable medium with accompanying printed or electronic documentation (for example, shrink wrapped software), preloaded with a computer system (for example, on system ROM or fixed disk), or distributed from a server or electronic bulletin board over a network (for example, the Internet or World Wide Web). In addition, a computer system is further provided including derivative modules for deriving a first data set and a calibration profile data set.

The calibration profile data sets in graphical or tabular form, the associated databases, and the calculated index or derived algorithm, together with information extracted from the panels, the databases, the data sets or the indices or algorithms are commodities that can be sold together or separately for a variety of purposes as described in WO O1/25473.

In other embodiments, a clinical indicator may be used to assess the survivability of a prostate cancer diagnosed subject by interpreting the calibrated profile data set in the context of at least one other clinical indicator, wherein the at least one other clinical indicator is selected from the group consisting of blood chemistry, (*e.g.*, PSA levels) X-ray or other radiological or metabolic imaging technique, molecular markers in the blood, other chemical assays, and physical findings.

Index construction

In combination, (i) the remarkable consistency of Gene Expression Profiles with respect to the predicted survivability and/or survival time across a population or set of subject or samples, or across a population of cells and (ii) the use of procedures that provide substantially reproducible measurement of constituents in a Gene Expression Panel (Precision Profile™) giving rise to a Gene Expression Profile, under measurement conditions wherein specificity and efficiencies of amplification for all constituents of the panel are substantially similar, make possible the use of an index that characterizes a Gene Expression Profile, and which therefore provides a predicted measurement of survivability and/or survival time.

An index may be constructed using an index function that maps values in a Gene Expression Profile into a single value that is pertinent to the predicted survivability and/or survival time of a subject. The values in a Gene Expression Profile are the amounts of each constituent of the Gene Expression Panel (Precision Profile™). These constituent amounts form a profile data set, and the index function generates a single value—the index— from the members of the profile data set.

The index function may conveniently be constructed as a linear sum of terms, each term being what is referred to herein as a "contribution function" of a member of the profile data set. For example, the contribution function may be a constant times a power of a member of the profile data set. So the index function would have the form

$$I = \sum C_i M_i^{P(i)},$$

where I is the index, Mi is the value of the member *i* of the profile data set, *Ci* is a constant, and P(i) is a power to which Mi is raised, the sum being formed for all integral values of *i* up to the number of members in the data set. We thus have a linear polynomial expression. The role of the coefficient Ci for a particular gene expression specifies whether a higher ∆Ct value for this gene either increases (a positive Ci) or decreases (a lower value) the likelihood of prostate cancer, the ∆Ct values of all other genes in the expression being held constant.

The values *Ci* and P(i) may be determined in a number of ways, so that the index *I* is informative of the predicted survivability and/or survival time of a subject. One way is to apply statistical techniques, such as latent class modeling, to the profile data sets to correlate clinical data or experimentally derived data, or other data pertinent to the predicted survivability and/or survival time. In this connection, for example, may be employed the software from Statistical Innovations, Belmont, Massachusetts, called Latent Gold®. Alternatively, other simpler modeling techniques may be employed in a manner known in the art. The index function for predicting the survivability and/or survival time of a prostate cancer-diagnosed subject may be constructed, for example, in a manner that a greater degree of survivability and/or survival time (as determined by the profile data set for the Precision Profile™ described herein (Table 1)) correlates with a large value of the index function.

Just as a baseline profile data set, discussed above, can be used to provide an appropriate normative reference, and can even be used to create a Calibrated profile data set, as discussed above, based on the normative reference, an index that characterizes a Gene Expression Profile can also be provided with a normative value of the index function used to create the index. This normative value can be determined with respect to a relevant population or set of subjects or samples or to a relevant population of cells, so that the index may be interpreted in relation to the normative value. The relevant population or set of subjects or samples, or relevant population of cells may have in common a property that is at least one of age range, gender, ethnicity, geographic location, nutritional history, medical condition (*e.g.*, prostate cancer), clinical indicator (*e.g.*, PSA level), medication (*e.g.*, chemotherapy or radiotherapy), physical activity, body mass, and environmental exposure.

As an example, for illustrative purposes only, the index can be constructed, in relation to a normative Gene Expression Profile for a population or set of prostate cancer subjects, in such a way that a reading of approximately 1 characterizes normative Gene Expression Profiles of healthy subjects. Let us further assume that the predicted survivability that is the subject of the index is "less than three years survival time"; a reading of 1 in this example thus corresponds to a Gene Expression Profile that matches the norm for prostate cancer subjects who will survive less than three years. A substantially higher reading then may identify a subject experiencing prostate cancer who is predicted to survive greater than three years. The use of 1 as identifying a normative value, however, is only one possible choice; another logical choice is to use 0 as identifying the normative value. With this choice, deviations in the index from zero can be indicated in standard deviation units (so that values lying between -1 and +1 encompass 90% of a normally distributed reference population or set of subjects. Since it was determined that Gene Expression Profile values (and accordingly constructed indices based on them) tend to be normally distributed, the 0-centered index constructed in this manner is highly informative. It therefore facilitates use of the index in diagnosis or prognosis of disease and setting objectives for treatment.

Still another embodiment is a method of providing an index pertinent to predicting the survivability and/or surivival time of prostate cancer-diagnosed subjects based on a first sample from the subject, the first sample providing a source ofRNAs, the method comprising deriving from the first sample a profile data set, the profile data set including a plurality of members, each member being a quantitative measure of the amount of a distinct RNA constituent in a panel of constituents selected so that measurement of the constituents is indicative of the predicted survivability and/or survival time of the subject, the panel including at least one constituent of any of the genes listed in the Precision Profile™ for Predicting Prostate Cancer Survivability (Table 1). In deriving the profile data set, such measure for each constituent is achieved under measurement conditions that are substantially repeatable, at least one measure from the profile data set is applied to an index function that provides a mapping from at least one measure of the profile data set into one measure of the predicted survivability and/or survival time of a prostate cancer-diagnosed subject, so as to produce an index pertinent to the survivability and/or survival time of the subject.

Performance and Accuracy Measures of the Invention

The performance and thus absolute and relative clinical usefulness of the invention may be assessed in multiple ways as noted above. Amongst the various assessments of performance, the invention is intended to provide accuracy in clinical diagnosis and prognosis. The accuracy of a diagnostic or prognostic test, assay, or method concerns the ability of the test, assay, or method to distinguish between the survivability and/or survival times of subjects having prostate cancer is based on whether the subjects have an "effective amount" or a "significant alteration" in the levels of a cancer survivability associated gene. By "effective amount" or "significant alteration", it is meant that the measurement of an appropriate number of cancer survivability associated gene (which may be one or more) is different than the predetermined cut-off point (or threshold value) for that cancer survivability associated gene and therefore indicates that the subjects survivability and/or survival time for which the cancer survivability associated gene(s) is a determinant.

The difference in the level of cancer survivability associated gene(s) between normal and abnormal is preferably statistically significant. As noted below, and without any limitation of the invention, achieving statistical significance, and thus the preferred analytical and clinical accuracy, generally but not always requires that combinations of several cancer survivability associated gene(s) be used together in panels and combined with mathematical algorithms in order to achieve a statistically significant predicted survivability and/or survival time associated gene index.

In the categorical diagnosis of a disease state, changing the cut point or threshold value of a test (or assay) usually changes the sensitivity and specificity, but in a qualitatively inverse relationship. Therefore, in assessing the accuracy and usefulness of a proposed medical test, assay, or method for assessing a subject's condition, one should always take both sensitivity and specificity into account and be mindful of what the cut point is at which the sensitivity and specificity are being reported because sensitivity and specificity may vary significantly over the range of cut points. Use of statistics such as AUC, encompassing all potential cut point values, is preferred for most categorical risk measures using the invention, while for continuous risk measures, statistics of goodness-of-fit and calibration to observed results or other gold standards, are preferred.

Using such statistics, an "acceptable degree of diagnostic or prognostic accuracy", is herein defined as a test or assay (such as the test of the invention for determining an effective amount or a significant alteration of cancer survivability associated gene(s), which thereby indicates the predicted survivability and/or survival time of a prostate cancer-diagnosed subject) in which the AUC (area under the ROC curve for the test or assay) is at least 0.60, desirably at least 0.65, more desirably at least 0.70, preferably at least 0.75, more preferably at least 0.80, and most preferably at least 0.85.

By a "very high degree of diagnostic or prognostic accuracy", it is meant a test or assay in which the AUC (area under the ROC curve for the test or assay) is at least 0.75, desirably at least 0.775, more desirably at least 0.800, preferably at least 0.825, more preferably at least 0.850, and most preferably at least 0.875.

The predictive value of any test depends on the sensitivity and specificity of the test, and on the prevalence of the condition in the population being tested. This notion, based on Bayes' theorem, provides that the greater the likelihood that the condition being screened for is present in an individual or in the population (pre-test probability), the greater the validity of a positive test and the greater the likelihood that the result is a true positive. Thus, the problem with using a test in any population where there is a low likelihood of the condition being present is that a positive result has limited value (*i.e.,* more likely to be a false positive). Similarly, in populations at very high risk, a negative test result is more likely to be a false negative.

As a result, ROC and AUC can be misleading as to the clinical utility of a test in low disease prevalence tested populations (defined as those with less than 1% rate of occurrences (incidence) per annum, or less than 10% cumulative prevalence over a specified time horizon). Alternatively, absolute risk and relative risk ratios as defined elsewhere in this disclosure can be employed to determine the degree of clinical utility. Populations of subjects to be tested can also be categorized into quartiles by the test's measurement values, where the top quartile (25% of the population) comprises the group of subjects with the highest relative risk for developing prostate cancer, and the bottom quartile comprising the group of subjects having the lowest relative risk for developing prostate cancer. Generally, values derived from tests or assays having over 2.5 times the relative risk from top to bottom quartile in a low prevalence population are considered to have a "high degree of diagnostic accuracy," and those with five to seven times the relative risk for each quartile are considered to have a "very high degree of diagnostic accuracy." Nonetheless, values derived from tests or assays having only 1.2 to 2.5 times the relative risk for each quartile remain clinically useful are widely used as risk factors for a disease. Often such lower diagnostic accuracy tests must be combined with additional parameters in order to derive meaningful clinical thresholds for therapeutic intervention, as is done with the aforementioned global risk assessment indices.

A health economic utility function is yet another means of measuring the performance and clinical value of a given test, consisting of weighting the potential categorical test outcomes based on actual measures of clinical and economic value for each. Health economic performance is closely related to accuracy, as a health economic utility function specifically assigns an economic value for the benefits of correct classification and the costs of misclassification of tested subjects. As a performance measure, it is not unusual to require a test to achieve a level of performance which results in an increase in health economic value per test (prior to testing costs) in excess of the target price of the test.

In general, alternative methods of determining diagnostic or prognostic accuracy are commonly used for continuous measures, when a disease category or risk category (such as those at risk for dying within a short period of time from hormone refractory prostate cancer, or those who may survive a long period of time with hormone refractory prostate cancer) has not yet been clearly defined by the relevant medical societies and practice of medicine, where thresholds for therapeutic use are not yet established, or where there is no existing gold standard for diagnosis or prognosis of the condition For continuous measures of risk, measures of diagnostic or prognostic accuracy for a calculated index are typically based on curve fit and calibration between the predicted continuous value and the actual observed values (or a historical index calculated value) and utilize measures such as R squared, Hosmer-Lemeshow P-value statistics and confidence intervals. It is not unusual for predicted values using such algorithms to be reported including a confidence interval (usually 90% or 95% CI) based on a historical observed cohort's predictions, as in the test for risk of future breast cancer recurrence commercialized by Genomic Health, Inc. (Redwood City, California).

In general, by defining the degree of diagnostic or prognostic accuracy, *i.e.*, cut points on a ROC curve, defining an acceptable AUC value, and determining the acceptable ranges in relative concentration of what constitutes an effective amount of the cancer survivability associated gene(s) of the invention allows for one of skill in the art to use the cancer survivability associated gene(s) to identify, diagnose, or prognose subjects with a pre-determined level of predictability and performance.

Results from the cancer survivability associated gene(s) indices thus derived can then be validated through their calibration with actual results, that is, by comparing the predicted versus observed rate of survivability and/or survival time in a given population, and the best predictive cancer survivability associated gene(s) selected for and optimized through mathematical models of increased complexity. Many such formula may be used; beyond the simple non-linear transformations, such as logistic regression, of particular interest in this use of the present invention are structural and synactic classification algorithms, and methods of risk index construction, utilizing pattern recognition features, including established techniques such as the Kth-Nearest Neighbor, Boosting, Decision Trees, Neural Networks, Bayesian Networks, Support Vector Machines, and Hidden Markov Models, as well as other formula described herein.

Furthermore, the application of such techniques to panels of multiple cancer survivability associated gene(s) is provided, as is the use of such combination to create single numerical "risk indices" or "risk scores" encompassing information from multiple cancer survivability associated gene(s) inputs. Individual B cancer survivability associated gene(s) may also be included or excluded in the panel of cancer survivability associated gene(s) used in the calculation of the cancer survivability associated gene(s) indices so derived above, based on various measures of relative performance and calibration in validation, and employing through repetitive training methods such as forward, reverse, and stepwise selection, as well as with genetic algorithm approaches, with or without the use of constraints on the complexity of the resulting cancer survivability associated gene(s) indices.

The above measurements of diagnostic or prognostic accuracy for cancer survivability associated gene(s) are only a few of the possible measurements of the clinical performance of the invention. It should be noted that the appropriateness of one measurement of clinical accuracy or another will vary based upon the clinical application, the population tested, and the clinical consequences of any potential misclassification of subjects. Other important aspects of the clinical and overall performance of the invention include the selection of cancer survivability associated gene(s) so as to reduce overall cancer survivability associated gene(s) variability (whether due to method (analytical) or biological (pre-analytical variability, for example, as in diurnal variation), or to the integration and analysis of results (post-analytical variability) into indices and cut-off ranges), to assess analyte stability or sample integrity, or to allow the use of differing sample matrices amongst blood, cells, serum, plasma, urine, etc.

Kits

The invention also includes a prostate cancer survivability detection reagent. In some embodiments, the detection reagent is one or more nucleic acids that specifically identify one or more prostate cancer survivability nucleic acids (*e.g.*, any gene listed in Table 1, sometimes referred to herein as prostate cancer survivability associated genes or prostate cancer survivability associated constituents) by having homologous nucleic acid sequences, such as oligonucleotide sequences, complementary to a portion of the prostate cancer survivability genes nucleic acids or antibodies to proteins encoded by the prostate cancer survivability gene nucleic acids packaged together in the form of a kit. The oligonucleotides can be fragments of the prostate cancer survivability genes. For example the oligonucleotides can be 200, 150, 100, 50, 25, 10 or less nucleotides in length. In another embodiment, the detection reagen is one or more antibodies that specifically identify one or more prostate cancer survivability proteins (e.g., any protein listed in Table 20).

The kit may contain in separate containers a nucleic acid or antibody (either already bound to a solid matrix or packaged separately with reagents for binding them to the matrix), control formulations (positive and/or negative), and/or a detectable label. The reagents may also include ancillary agents such as buffering agents and stabilizing agents, *e.g.*, polysaccharides and the like. Instructions (*i.e.*, written, tape, VCR, CD-ROM, etc.) for carrying out the assay may be included in the kit. The assay may for example be in the form of PCR, a Northern hybridization or a sandwich ELISA, as known in the art.

For example, the kit may comprise one or more antibodies or antibody fragments which specifically bind to a protein constituent of the Protein Expression Panels described herein (*e.g.*, the Precision Protein Panel for Prostate Cancer Survivability in Table 20). The antibodies may be conjugated conjugated to a solid support suitable for a diagnostic assay (*e.g.*, beads, plates, slides or wells formed from materials such as latex or polystyrene) in accordance with known techniques, such as precipitation. Antibodies as described herein may likewise be conjugated to detectable groups such as radiolabels (*e.g.,* 35 S, 125 I, 131 I), enzyme labels (*e.g.*, horseradish peroxidase, alkaline phosphatase), and fluorescent labels (*e.g.*, fluorescein) in accordance with known techniques. Alternatively the kit comprises (a) an antibody conjugated to a solid support and (b) a second antibody of the invention conjugated to a detectable group, or (a) an antibody, and (b) a specific binding partner for the antibody conjugated to a detectable group.

In another embodiment, prostate cancer survivability detection reagents can be immobilized on a solid matrix such as a porous strip to form at least one prostate cancer survivability gene detection site. The measurement or detection region of the porous strip may include a plurality of sites containing a nucleic acid. A test strip may also contain sites for negative and/or positive controls. Alternatively, control sites can be located on a separate strip from the test strip. Optionally, the different detection sites may contain different amounts of immobilized nucleic acids, *i.e.*, a higher amount in the first detection site and lesser amounts in subsequent sites. Upon the addition of test sample, the number of sites displaying a detectable signal provides a quantitative indication of the amount of prostate cancer survivability genes present in the sample. The detection sites may be configured in any suitably detectable shape and are typically in the shape of a bar or dot spanning the width of a test strip.

Alternatively, prostate cancer survivability detection reagents can be labeled (*e.g.*, with one or more fluorescent dyes) and immobilized on lyophilized beads to form at least one prostate cancer survivability gene detection site. The beads may also contain sites for negative and/or positive controls. Upon addition of the test sample, the number of sites displaying a detectable signal provides a quantitative indication of the amount of prostate cancer survivability genes present in the sample.

Alternatively, the kit contains a nucleic acid substrate array comprising one or more nucleic acid sequences. The nucleic acids on the array specifically identify one or more nucleic acid sequences represented by prostate cancer survivability genes (see Table 1). In various embodiments, the expression of 1, 2, 3, 4, 5, 6, 7, 8, 9, 10, 15, 20, 25, 40 or 50 or more of the sequences represented by prostate cancer survivability genes (see Table 1) can be identified by virtue of binding to the array. The substrate array can be on, *i.e.,* a solid substrate, *i.e.,* a "chip" as described in U.S. Patent No. 5,744,305. Alternatively, the substrate array can be a solution array, *i.e.,* Luminex, Cyvera, Vitra and Quantum Dots' Mosaic.

The skilled artisan can routinely make antibodies, nucleic acid probes, *i.e.,* oligonucleotides, aptamers, siRNAs, antisense oligonucleotides, against any of the prostate cancer survivability genes and/or proteins listed in Tables 1 and 20.

## OTHER EMBODIMENTS

While the invention has been described in conjunction with the detailed description thereof, the foregoing description is intended to illustrate and not limit the scope of the invention, which is defined by the scope of the appended claims. Other aspects, advantages, and modifications are within the scope of the following claims.

## EXAMPLES

**Example 1:** Gene Expression Profiles for Predicting the Survivability of Hormone or Taxane Refractory Prostate Cancer Subjects

The following study was conducted to investigate whether any of the genes *(i.e.,* RNA- based transcripts) shown in the Precision Profile™ for Prostate Cancer Survivablity (Table 1), individually or when paired with another gene, are predictive of primary endpoints of prostate cancer progression (*i.e.*, survival time). The survivability (*i.e.*, whether each subject was alive or dead) of 62 hormone or taxane refractory prostate cancer subjects (with or without bone metastases) was measured as of June 20, 2008. A summary of any therapy each of the 62 subjects were receiving during the study period is shown in Table 2 (*e.g.*, hormone therapy, radiotherapy, chemotherapy, other therapy, and/or a combination thereof). A summary of the date each patient became hormone or taxane refractory (*i.e.* classified as cohort 4), their survivability status *(i.e.,* alive or dead) and survival date as of June 20, 2008 is shown in Table 3. As shown in Table 3, a total of 47 of the 62 cohort 4 prostate cancer subjects were alive and 15 of the 62 cohort 4 prostate cancer subjects were dead as of June 20, 2008. 14 of the 15 dead subjects died within 2.2 years (115 weeks) since entering hormone refractory status. The median survival time of those who died was 20 months from the date each patient became hormone refractory (Table 4). Overall, 30 of the 47 alive subjects lived beyond 2.2 years and up to 8.6 years (450 weeks).

RNA was isolated using the PAXgene System from whole blood samples obtained (at a single time-point) from a total of 66 subjects with hormone or taxane refractory prostate cancer (with or without bone metastatis) (sometimes referred to herein as "Cohort 4"). Circulating tumor cells were also enumerated using CellSave tubes, and isolated using EDTA tubes, from the whole blood samples. It was assumed that the 66 subjects from which the blood samples were obtained have gene expression data representative of the clinical study population.

Custom primers and probes were prepared for the targeted 174 genes shown in the Precision Profile™ for Prostate Cancer Survivability(shown in Table 1), selected to be informative relative to the survivability and/or survival times of prostate cancer patients. Gene expression profiles for the 174 prostate cancer specific genes were analyzed using the RNA samples obtained from the cohort 4 prostate cancer subjects.

1 and 2-gene models yielding the best prediction of the survivability of hormone or taxane refractory prostate cancer subjects (cohort 4) were generated using survival analysis as described below.

## Survival Models:

When time from an initial (baseline) state to some event (*e.g.*, death) is known, it is possible to examine the predictive relationship between the gene expressions and the time to the event (*i.e.*, survival time). Survival analysis can be used to quantify and assess the effects of the genes in statistical models, typically which predict the hazard ratio for each subject based on predictors such as the subjects' gene expressions and other risk factors. The hazard rate is the probability of the event occurring during the next time period t+1 given that it has not occurred as of time period t.

Three survival models were employed to examine the predictive relationship between gene expression (*i.e.*, them genes shown in Table 1) and the time to the event (*i.e.*, survival time).

1) Cox-type Proportional Hazards model. The genes enter directly as predictors in a log-linear model consisting of an intercept (the baseline hazard rate which may vary over time period t) plus other terms such as the gene expressions and other time constant or time varying predictors. For example, if multiple blood draws are available at different times leading to multiple expressions for a given gene, the gene can be included in the model as a time varying predictor. In such models, a significant gene effect means that subjects with a higher expression on that gene have a significantly higher (lower) probability of experiencing the event (*e.g.*, death) in the next period t, than those with a lower expression but otherwise the same on the other risk factors in the model.

2) Zero-Inflated Poisson (ZIP) model. For this type of model, the gene expressions effect survival time indirectly through a latent variable which posits 2 or more hypothetical patient types, one of which has a hazard ratio of 0, reflecting a zero risk of experiencing the event (*e.g.*, death) during the time span of the study. This subject type may be referred to as 'long-term survivors'. Each of the other types have different but non-zero hazard functions. In ZIP models, a significant gene effect means that subjects with a higher expression on that gene have a significantly higher (lower) probability of being a long-term survivor, than those with a lower expression but otherwise the same on the other risk factors in the model. Unlike the Cox model, this predicted probability does not depend on time.

3) Markov model. This type of model is similar to the ZIP model in that the genes do not have a direct effect on survival time. However, rather than assuming that a subject's membership in one of the types is fixed but unknown (latent), the Markov model re-is expressed in terms of states. Specifically, the genes affect a subject's probability of transition from the state Alive to the state Dead. (The transition parameters associated with the transition from Dead to Alive are fixed at zero). For this type of model, a significant gene effect means that subjects with a higher expression on that gene have a significantly higher (lower) probability of transition from their current state of Alive to the state of being Dead, than those with a lower expression on that gene but who otherwise are the same with respect to the other risk factors in the model. (For a more detailed description of these survival models, see *e.g.*, Jewell, 2004; Jewell, et. al, 1998; Vermunt, 2008).

In these models, the parameter estimates can also be used to obtain predictions for the expected survival time. Model development consisted of a two-step process.

### Step 1: Development of baseline survival models without gene expression data

Two initial baseline models were developed to predict survival time as a function of age, treatment, and metastatic status. Thus, these baseline models were developed without the use of any gene expression data. For the 1st such model, survival time was measured from the date the patient was determined to be hormone or taxane refractory (Definition #1). For the 2nd such model, survival time was measured from the date of the blood draw (Definition #2). If any significant predictive relationships were found, it was expected to be stronger in the first baseline model, because the time of the blood draw should not be relevant in these baseline models.

The predictive relationships in these models were estimated using the 3 different survival models described above. For each type of analysis, each period was set to correspond to approximately 4 months (16 weeks).

### Step 2: Target genes as additional predictor variables in baseline survival models

For each of the most significant baseline models developed in Step 1, target genes were included as additional predictor variables, and allowed to affect survival times directly (based on the baseline models developed using Cox-type models) or affecting the latent classes (based on baseline models developed using ZIP and/or Markov models). The genes were entered into these models in the following way:

1. Separate models were developed for each of the 174 genes, with one of the genes included in each of these models.
2. Separate models were developed for each gene pair.

Final gene models summarized and interpreted were those for which all genes in the model were incrementally significant at the .05 level. As mentioned above, various comparisons were made between different models and examined for consistency. For example, if expected survival times were found to be significantly longer for patients with higher expression on gene Y, it was determined whether this relationship holds true for different subsets of patients defined by a) treatment, b) age range, or c) recent PSA score range. In addition, the p-values for the significant gene effects were compared and examined for consistent patterns when the survival time was measured from the time the patient was determined to be refractory, as opposed to the time of the blood draw.

Results based on the Cox-Type Model (estimated using 4 month periods)

A listing of all 1 and 2-gene models capable of predicting the survivability of hormone or taxane refractory prostate cancer subjects (cohort 4) is shown in Table 5 (read from left to right). As shown in Table 5, the 1 and 2-gene models are identified in the first two columns on the left side of Table 5, ranked from best to worst by their entropy $R^2$ value (shown in column 3, ranked from high to low). The incremental p-value for each first and second gene in the 1 or 2-gene model is shown in columns 3-4. As previously indicated, a total number of 47 RNA samples from subjects who were alive as of June 20, 2008 and a total of 15 RNA samples from subjects who were dead as of June 20, 2008 were analyzed. No samples or values were exluded.

The number of subjects correctly classified or misclassified by the top two "best" gene models (as defined by having the highest entropy $R^2$ values, *i.e.,* 2-gene model ABL2 and C1QA and 2-gene model SEMA4D and TIMP1) were calculated, respectively. The "best" 2-gene model ABL2 and C1QA, as defined by the entropy $R^2$ value, was capable of accurately predicting the survivability status of 44 of the 47 alive subjects (93.6% classification accuracy) 13 of the 15 dead subjects (86.7% classification accuracy). The next best 2-gene model SEMA4D and TIMP1 (as ranked by the entropy $R^2$ value), was capable of accurately predicting the survivability status of 40 of the 47 alive subjects (85.1% correct classification) and 13 of the 15 dead subjects (86.7% correct classification).

Two or more of the gene models enumerated in Table 5 can also be averaged together to create additional multi-gene models capable of accurately predicting the survivability of prostate cancer subjects. For example, averaging the top two "best" gene models together creates a 4-gene model *(i.e.,* ABL2, SEMA4D, C1QA and TIMP1) capable of correctly predicting 45 of the 47 subjects still alive *(i.e.,* 95.7% correct classification), and only 13 of the 15 subjects who died *(i.e.,* 86.7% classification). As another example, averaging three of the top 2-gene models, such as 2-gene model ABL2 and C1QA, 2-gene model SEMA4D and TIMP1, and 2-gene model CDKN1A and ITGAL, yields a 6-gene model ABL2, SEMA4D, ITGAL, C1QA, TIMP1 and CDKNA, capable of correctly predicting 45 of the 47 alive subjects *(i.e.,* 95.7% correct classification), and 14 of the 15 dead prostate cancer subjects *(i.e.,* 93.3% correct classification).

A ranking of the 174 prostate cancer survivability genes for which gene expression profiles were obtained, from most to least significant (as ranked by their entropy $R^2$ value), is shown in Table 6. Table 6 summarizes the likelihood ratio p-values for the difference in the mean expression levels for alive and dead cohort 4 prostate cancer subjects, obtained from the Cox-type survival model. As shown in Table 6, there are 20 genes that are significant at the 0.05 level (highlighted in gray).

The predicted probability based on each of the gene models enumerated in Table 5, alone or in combination, can be used to create a prostate cancer survivability index that can be used as a tool by a practitioner (*e.g.*, primary care physician, oncologist, etc.) for predicting the prognosis and survival times of prostate cancer-diagnosed subjects and to ascertain the necessity of future screening or treatment options (see *e.g.*, Figures 7 and 8).

Results Based on the Zero Inflated Poisson (ZIP) Model (estimated using 4 month periods)

An example of the four ZIP gene models capable of predicting the probability of being a long term survivor for each subject still alive as of June 20, 2008, is shown in Tables 7A-7D. The first model contains only gene ABL2 (see Table 7A), the second ABL2 and C1QA (see Table 7B), and the 3rd SEMA4D and TIMP1 (see Table 7C). The 4th model is based on the averaged gene expressions of two 2-gene models *(i.e.,* a 4-gene model) -- the average gene expressions of the first gene in each of models 2 and 3 *(i.e.,* ABL2 and SEMA4D), and the average gene expressions of the second gene in each of models 2 and 3 *(i.e.,* C1QA and TIMP1) (see Table 7D). For each model, subjects are sorted from high to low. Note that all 4 ZIP models rank subject #272956 as having a low probability of being a long term survivor -- 0.38, 0.45, 0.12 and 0.10 respectively. A comparison of two different 2-gene ZIP models (2-gene model C1QA and ABL2, and 2-gene model SEMA4D and TIMP1) is shown in Table 8.

Results Based on the Markov Model (estimated using 4 month periods)

An example of the 2-gene model, C1QA and ABL2, capable of predicting transition probabilities obtained from using the Markov Model analysis is shown in Table 9. For example, for subject #44, the probability of dying during the initial period is predicted to be 0.19. Given that this subject does not die in period 1 the probability of transitioning from the Alive to the Dead state in period 2 is 0.166, and given that this subject remains alive at the end of period 2, the probability of transitioning to the state Dead in period 3 remains at 0.1872. This transition probability then increases to 0.2979 in period 4, 0.307 in period 5 and 0.3801 for each period after period 5. Blank probability cells indicate that the subject no further data is available on this subject. This is because the subject qualified for Cohort 4 status quite recently, or the person died at an earlier time. For example, subject # 9 qualified for Cohort 4 status on July 13 2006, and died on June 27 2007. Thus, this subject was not alive during period 4. Subject #322324 entered Cohort 4 status on November 5 2007, and since period 4 does not begin until after June 20 2008, period 4 as well as future periods were left blank. The 3 misclassified dead subjects and the 4 misclassified alive subjects are highlighted in in gray in Table 9.

Summary of Results

Each three types of survival models when applied to the gene expression data give similar results. Each yielded the 1-gene model ABL2 as a highly significant 1-gene model, and the 2-gene models ABL2 and C1QA, and SEMA4D and TIMP1 as the top two highly significant 2-gene models (as ranked by their entropy $R^2$ values), capable of predicting the survivability of hormone or taxane refractory prostate cancer subjects. These "best" models all showed similar structure, *i.e.*, patients with the highest risk of death had low expression of 1 gene relative to the other model gene (see Table 10). Additionally, risk scores obtained from each of many 2-gene models were highly predictive of those who died, and the number and significance of such models indicates that the results are well beyond chance.

A summary of the entropy $R^2$ and wald p-values for these 1 and 2-gene models obtained by the three different survival models is shown in Table 11 As shown in Table 11, the wald p-values obtained by each of the three models are very similar.

The linear component for each of these models is:

Markov: c1(t)+2.07 ABL2 - 1.08 C1QA
ZIP: c2 + 2.05 ABL2 - 0.94 C1QA
Cox: c3(t) + 2.26 ABL2 -1.31 C1QA

which can be used as risk scores; the higher the score, the greater the risk of dying in the next period t+1, given that the subject is alive during period t.

A discrimination plot of the 2-gene model, ABL2 and C1QA, is shown in Figure 1. As shown in Figure 1, the cohort 4 prostate cancer subjects who were alive as of June 20, 2008 are represented by circles, whereas the cohort 4 prostate cancer subjects who were dead as of June 20, 2008 are represented by X's. The lines superimposed on the discrimination graph in Figure 1 illustrates how well the 2-gene model discriminates between the 2 groups as estimated by the Cox-Type model, the Zero-Inflated Poisson Model, and the Markov Model. The discrimination lines were superimposed by setting each of the risk scores listed above to 0, solving for ABL2 as a function of C1QA and setting c1(t), c2 and c3(t) equal to constants that maximize the correct classification rates of those subjects who died and those who were still alive as of June 20 2008. As shown in Figure 1, each of the three methodologies yielded very similar results. Values below and to the right of the lines represent subjects predicted by the 2-gene model to be in the alive population. Values above and to the left of the lines represent subjects predicted by the 2-gene model to be in the dead population. Each of the three survival models misclassifies only 2 of the 15 subjects who have died and only 3 of the 47 subjects still alive.

A comparison of each of the Cox-Type, Markov, and ZIP models for 2-gene model ABL2 and C1QA sorted by exposure and by Cox-score within status (*i.e.*, alive vs. dead) is shown in Tables 12 and 13, respectively.

A discrimination plot of the 2-gene model, ABL2 and C1QA, is also shown re-plotted in Figure 2. As shown in Figure 2, the cohort 4 prostate cancer subjects who were alive as of June 20, 2008 are represented by open circles, whereas the cohort 4 prostate cancer subjects who were dead as of June 20, 2008 are represented by filled circles. The equation for the cut-off line shown in Figure 2 is 2.3*ABL2-1.3*C1QA=21. Values above and to the left of the line represent subjects predicted by this 2-gene model to be in the alive population. Values below and to the right of the line represent subjects predicted to be in the dead population. As shown in Figure 2, this 2-gene model misclassifies only 3 of the 47 alive subjects (*i.e.,* 93.6% correct classification, and only 2 of the 15 subjects who have died (*i.e.,* (86.7% classification accuracy).

A discrimination plot of the second "best" 2-gene model, SEMA4D and TIMP1, as identified by the Cox-Type and ZIP survival models, is shown in Figure 3. As shown in Figure 3, the cohort 4 prostate cancer subjects who were alive as of June 20, 2008 are represented by circles, whereas the subjects who were dead as of June 20, 2008 are represented by X's. The line superimposed on the discrimination graph in Figure 3 illusrates how well this 2-gene model discriminates between the 2-groups as estimated by a dead vs. alive logit model. The equation for the cut-off line shown in Figure 3 is SEMA4D=5.46+0.66*TIMP1 (slope is based on dead vs. alive logit model). Values below and to the right of the line represent subjects predicted by the 2-gene model to be in the alive population. Subjects above and to the left of the line represent subjects to be in the dead population. As shown in Figure 3, this 2-gene model misclassifies 7 of the 47 subjects still alive *(i.e.,* 85.1% correct classification), and only 2 of the 15 subjects who have died *(i.e.,* 86.7% correct classification). A discrimination plot of the 2-gene model, SEMA4D and TIMP1, is also shown re-plotted in Figure 4.

A discrimination plot of the averaged gene expressions of the two "best" models from Table 5 *(i.e.,* 4-gene model ABL2, SEMA4D, C1QA and TIMP1) is shown in Figure 5. As shown in Figure 5, the cohort 4 prostate cancer subjects who were alive as of June 20, 2008 are represented by circles, whereas the subjects who were dead as of June 20, 2008 are represented by X's. The line superimposed on the discrimination graph in Figure 5 illusrates how well this 4-gene model discriminates between the 2-groups as estimated by a dead vs. alive logit model. The equation for the cut-off line shown in Figure 5 is Abl2Sema4D=6.16+0.68*ClqaTimp1 (slope is based on dead vs. alive logit model). Values below and to the right of the line represent subjects predicted by the 4-gene model to be in the alive population. Subjects above and to the left of the line represent subjects to be in the dead population. As shown in Figure 5, this 4-gene model misclassifies only 2 of the 47 subjects still alive (*i.e.,* 95.7% correct classification), and only 2 of the 15 subjects who have died (*i.e.,* 86.7% classification).

A discrimination plot of the averaged gene expressions of three of the top models used to create the 6-gene model ABL2, SEMA4D, ITGAL, C1QA, TIMP1 and CDKN1A, is shown in Figure 6. As shown in Figure 6, the cohort 4 prostate cancer subjects who were alive as of June 20, 2008 are represented by open circles, whereas the subjects who were dead as of June 20, 2008 are represented by filled circles. The line superimposed on the discrimination graph in Figure 6 illusrates how well this 6-gene model discriminates between the 2-groups as estimated by a dead vs. alive logit model (C1TiCd=C1QA+TIMP1 +CDKN1A; AbSeIt=2*ABL2+SEMA4D+ITGAL). Values below and to the right of the line represent subjects predicted by the 6-gene model to be in the alive population. Subjects above and to the left of the line represent subjects to be in the dead population. As shown in Figure 6, this 6-gene model misclassifies only 2 of the 47 subjects still alive (*i.e.,* 95.7% correct classification), and only 2 of the 15 subjects who have died (*i.e.,* 86.7% classification).

Figure 7 is an example of an index based on the 2-gene model ABL2 and C1QA, which can be used by practiotioners to predict the probability of long term survival of prostate cancer subjects. Figure 8 is an example of an index based on the 6-gene model ABL2, SEMA4D, ITGAL and C1QA, TIMP1, CDKN1A, which can be used by practiotioners (*e.g.*, primary care physician, oncologist, etc.) to predicting the prognosis and survival times of prostate cancer-diagnosed subjects and to ascertain the necessity of future screening or treatment options.

Risk Scores Obtained from Additional Blood Draw

A second blood draw was used to obtain gene measurements on 3 of cohort 4 prostate cancer subjects and gene expression profiles for the 174 prostate cancer specific genes (Table 1) were analyzed using RNA samples obtained from the these blood samples. Survival estimates were generated as previously described based on the Cox-Type, ZIP and Markov models.

As shown in Table 14, each of the three types of survival models yielded similar risk scores with the measurements obtained from the second blood draw.

**Example 2:** Re-estimation of Cox-Type Model Using Weekly Periods

The Cox-Type model described in Example 1 was re-estimated using weekly periods (rather than quarterly periods, as used in Example 1). Re-estimation based on weekly periods resulted in lower (more significant) p-values as well as some other minor changes.

The Cox-Type model when estimated based on weekly periods yields 28 genes that are significant at the .05 level, as compared to only 20 genes that were significant at the 0.05 level when survival estimates were based on quarterly periods, as shown in Table 6. Again, ABL2 was the most significant when used to define a 1-gene model, but now it is more significant (p = 8.1E-5 rather than p = .0001). Also, as before, CAV2 is the 2nd most significant (p = 7.9E-4). The order of the other genes was similar but somewhat different than that obtained with the quarterly period definition.

These calculations were repeated using time since the blood draw ("survival time Definition #2") rather than time since entering cohort 4 status ("survival time Definition #1"). As expected, the results were very similar. A comparison of the p-values for the top 28 most significant genes as estimated using survival time Definition #1 (as estimated using weekly periods) and as estimated using survival time Definition #2 is shown in Table 15. As before, ABL2 was the most significant gene estimated using time since blood draw (p = 3.1E-4), but not as significant as when using estimated using weekly periods (p = 8.1E-5). The p-values for other genes also differed from those obtained under survival time Definition #1 (as estimated using weekly periods) -- some were higher and some lower (see Table 15). Surprisingly, more genes (42) were significant under this Definition #2. Regardless of the survival time definition, the best 2-gene model contained ABL2 & C1QA as previously identified, and the risk score functions were similar:

RISK1 (ABL2, C1QA) = 2.09*ABL2 -1.08*C1Qa
RISK2 (ABL2, C1QA) = 1.91*ABL2 - 1.15*C1Qa

Under both definitions, the unique p-value associated with ABL2 in this 2-gene model was slightly more significant (p = 1.9E-5, and p = 2.3E-5) than in the 1-gene model, and the same was true for C1Qa (p = .00029 and p = .00042) (See Table 16). Table 17 shows that the results are also very similar for the 2nd best 2-gene model, SEMA4D and TIMP1.

Note that the Cox model assumes that the hazard function is proportional to the values of the predictors (covariates). For the 2-gene models that were re-estimated, the proportional hazards assumption was found to be consistent with the data. Without intending to be bound by any theory, there was no evidence that the effects of the genes (labeled b in Tables 16 and 17) varied over time.

Figures 9 and 10 show a Kaplan Meier survival assessment of the 2-gene model ABL2 and C1QA, based on survival time definition #1 and #2, respectively. The cumulative survival curve was smoother when based on survival time definition #1 (Figure 9), as most deaths occur between weeks 64 and 115 (steep decline in curve) following the beginning of cohort 4 status. In Figures 9 and 10, the cumulative survival function was plotted for a hypothetical patient with gene measurements at the mean of the genes (lower risk: 2.3*ABL2-1.3*C1QA < 21; higher risk: 2.3*ABL2-1.3*C1QA > 21; p-values = 1.87E-05 and 4.32E-05 respectively). Without intending to be bound by any theory for patients with different values on the genes, these curves may shift somewhat up or down but the general shape should remain.

Kaplan Meier Survival Assessment also confirmed prediction of the 6-gene model ABL2, SEMA4D, ITGAL and C1QA, TIMP1, CDKN1A, based on time of hormone-refractory diagnosis (*i.e.*, survival time definition #1, Figure 11) and time of blood draw (*i.e.*, survival time definition #2, Figure 12) (lower risk: 2*ABL1+SEMA4D+ITGAL-C1QA-TIMP1-CDKN1A < 21.21; higher risk: 2*ABL1+SEMA4D+ITGAL-C1QA-TIMP1-CDKN1A > 21.21; p-values = 1.11E-04 and 1.96E-04, respectively).

Regardless of the survival time definition, CTC was found not to be a significant predictor of survival time. CTC enumeration from whole blood was performed using CellSave tubes and the Immunicon platform. CTC counts from 12 of 15 Dead CaP subjects ranged from 0 to 152 CTCs with an average of 44 CTCs. Blood samples for CTC enumeration were not available from 3 Dead CaP subjects. CTC counts from 42 of 47 Alive CaP subjects ranged from 0 to 931 CTCs with an average of 39 CTCs. Blood samples for CTC enumeration were not available from 5 Alive CaP subjects. Interestingly, the highest CTC counts (931 and 263) were evident in patients from the Alive CaP subject group. These same subjects were also classified in the low risk group from both 2 and 6 gene models. In Cox models using CTCs as the sole predictor, p-values were 0.42 and 0.96 under the survival time definitions from hormone refractory diagnosis (*i.e.*, definition #1) and from blood draw (*i.e.*, definition #2), respectively. Also, when entered as an additional predictor in the 2-gene model along with ABL2 and C1Qa, CTC had no effect. As shown in Figure 13, Kaplan Meier survival curves differ across patients with varying CTCs and are weaker than those provided by 2-Gene model, confirming that CTCs were not a significant predictor of survival time.

Likewise, treatment type was found not to be a significant predictior of survival, regardless of the survival time definition used. Study results indicate that the survival assessment described herein is independent of treatment type, and is an independent prognostic tool for hormone refractory prostate cancer.

One hundred permuted data sets were generated by randomly assigning 15 "dead" and 47 "alive" subjects from the entire pool of subjects (dead and alive) and re-estimating 2-gene models. Conclusion of permutation tests is that only a very small chance exists that the results of the Source MDx 2-gene models were by chance.

Follow-up validation studies of 125 subjects will be designed to confirm the results of the above analyses, as shown in Table 18. Survival prediction will enable patient stratification in clinical trials.

**Example 3:** Protein Expression Profiles for Predicting the Survivability of Hormone or Taxane Refractory Prostate Cancer Subjects

As indicated in Example 1, many of the top gene-models enumerated in Table 5 showed similar structure, *i.e.*, patients with the highest risk of death had low expression of 1 gene relative to the other model gene (see Table 10). An analysis of the target gene mean differences ($\Delta\Delta C_T$ difference) for the top 25 genes ranked by the Cox-Type model by p-value revealed survival rates that are associated with higher and lower gene expression, as shown in Table 19. Without intending to be bound by theory, such differentially expressed genes appear to reflect an increased "bias" of the immune system towards phagocytosis and inflammation as reflected by increased production and activation of tissue macrophages and a decrease in both cell-mediated and humoral immunity. Examples of such differentially expressed genes include ABL2, C1QA, CDKN1A, ITGAL, SEMA4D, and TIMP1. Surprisingly, several of the most statistically significant gene expression profiles (*i.e.*, gene models) described herein comprised one or more of these six genes. A summary of these six genes and the observed effect each gene had on cellular and humoral immunity and macrophages is shown in Figure 14.

A list of proteins which correspond to these RNA transcripts which exhibit differential expression in long-term prostate cancer survivors as opposed to short term survivors is shown in Table 20 (*i.e.,* the Precision Protein Panel for Prostate Cancer Survivability).

The proteins shown in Table 20 are analyzed in both retrospective blood samples from prostate cancer patients (banked serum and plasma) and prospective studies from cancer patients- in serum, plasma and leukocytes. In one embodiment, the presence of one or more constituents of the Precision Protein Panel for Prostate Cancer Survivability (Table 20) in a sample (*e.g.* serum and/or plasma) obtained from a prostate-cancer subject is analyzed using standard immunoassay techniques well known to one of ordinary skill in the art. A microtiter plate is prepared by conjugating one or more antibodies which specifically bind to one or more of constituents of the Precision Protein Panel for Prostate Cancer Survivability (Table 20) to one or more wells of the microtiter plate using techniques known to one of ordinary skill in the art. For example, the ABL2 antibody IHB11 (ab54209), the CD100 [A8] (SEMA4D) antibody (ab33260), the CD11a [EP1285Y] (ITGAL) antibody (ab52895), the C1QA antibody (ab14004), the TIMP1 antibody (ab38978 or ab1827), and/or the CDKN1A [2186C2a] antibody (ab51332) (Abeam, Cambridge, MA) may be immobilized to one or more wells of the microtiter plate. The wells are then incubated with a solution of bovine serum albumin (BSA) or casein to block non-specific adsorption of other proteins to the plate. The serum and/or plasma is introduced to the antibody-conjugated microplate to allow protein binding to the antibody conjugated well. Non- bound proteins are removed by washing the wells using known a mild detergent solution. One or more appropriate protein-specific antibodies are added to each respective well (*i.e.,* the antibody which recognizes the protein of interest) and incubated to allow binding to the protein of interest (if present). An enzyme-linked secondary antibody which is specific to the primary antibodies is applied to each respective well. The plate is washed to remove unbound antibody-enzyme conjugates. A substrate is added to convert the enzyme into a color, fluorescent, or electrochemical signal. The absorbance or fluorescence or electrochemical signal (*e.g.*, current) of the plate wells is measured to determine the presence and quantity of the protein(s) of interest.

In another embodiment, the presence of one or more constituents of the Precision Protein Panel for Prostate Cancer Survivability (Table 20) in a whole blood sample obtained from a prostate cancer subject is analyzed according to the methods disclosed in U.S. Patent No. 7,326,579 as follows. Whole blood is obtained from a relevant subject and subjected to forcible hemolysis in a manner not to affect agglutination reaction (*e.g.*, by mixing whole blood with a low osmotic solution, mixing blood with a solution of saponins for hemolysis, freezing and thawing whole blood, and/or ultrasonicating whole blood). The hemolysis is then subjected to an agglutination reaction with an insoluble particle suspension reagent (*e.g.*, a latex reagent) onto which one or more antibodies specifically reacting with the protein(s) of interest have been immobilized (*e.g.*, the ABL2 antibody IHB11 (ab54209), the CD100 [A8] (SEMA4D) antibody (ab33260), the CD11a [EP1285Y] (ITGAL) antibody (ab52895), the C1QA antibody (ab14004), the TIMP1 antibody (ab38978 or ab1827), and/or the CDKN1A [2186C2a] antibody (ab51332) (Abeam, Cambridge, MA)). The resulting agglutination mixture is analyzed for a change in its absorbance or in its scattered light by irradiation with light at a wavelength which is substantially free from absorption by both hemoglobin and the hemolysis reagent to determine the quantity of the amount of protein of interest in the sample. The method may optionally be combined with known techniques for quantitating the amount of protein in a sample, *e.g.*, immunoturbidimetry.

These data support that Gene Expression Profiles with sufficient precision and calibration as described herein (1) can predict the survivability/and or survival time of prostate cancer-diagnosed subjects; (2) predict the probability of long term survivability and identify subsets of individuals among prostate-cancer diagnosed subjects with a higher probability of long-term survivability based on their gene expression patterns; (3) may be used to monitor the affect of a therapeutic regimen on the survivability and/or survival time of prostate-cancer diagnosed subjects; and (4) may be used to guide the medical management of a patient by adjusting therapy to bring one or more relevant Gene Expression Profiles closer to a target set of values, which may be normative values or other desired or achievable values.

**Example 4:** Cell Fractionation Study-Hormone Refractory Prostate Cancer Subjects (cohort 4)

A cell fractionation study was designed to investigate the cellular origin of the gene expression signal observed in whole blood for a custom Precision Profile™ containing 18 select genes identified as having statistically significant differences in mean levels of expression in hormone refractory prostate cancer subjects who may be at high risk of dying. Whole blood samples from eleven individuals with hormone refractory prostate cancer were collected in CPT tubes for purification of peripheral blood mononuclear cells (PBMC's). Four different cell types were subsequently enriched from the purified PBMC fraction and levels of gene transcripts from both enriched and depleted B cells, monocytes, NK cells, T cells and the original PBMC fraction, were quantitatively analyzed using Source MDx's optimized QPCR assays (Precision Profiles™). In addition, whole blood samples from seven medically defined Normal subjects (i.e., normal, healthy subjects) were collected. The same four cell types were again enriched from purified PBMC's and cell specific gene expression determined using Source MDx Precision Profiles™.

Normalized target gene expression values from PBMC samples were compared to those from enriched (and depleted) cell fractions to determine whether an increase in expression was observed in a specific cellular fraction(s). Expression levels of cell specific markers were also analyzed in parallel for each cellular fraction generated in the enrichment process, to determine the fold-enrichment of specific cell types.

A comparison of the averaged relative expression values for individual genes in enriched cell fractions from both normal and disease cohorts was made to determine whether the level of expression or even the cell type in which the gene was expressed were different.

*Cell Enrichment and RNA Extraction:*

Becton Dickinson IMag™ Cell Separation Reagents were used to magnetically enrich the four different cell types (B cells, monocytes, NK cells, T cells) isolated from the PBMC fraction of whole blood following the manufacturers recommended protocol.

*RNA Quality Assessment:*

Integrity of purified RNA samples was visualized with electropherograms and gel-like images produced using the Bioanalyzer 2100 (Agilent Technologies) in combination with the RNA 6000 Nano or Pico LabChip.

*cDNA First Strand Synthesis and QC:*

First strand cDNA was synthesized from random hexamer-primed RNA templates using TaqMan® Reverse Transcription reagents. Quantitative PCR (QPCR) analysis of the 18S rRNA content of newly synthesized cDNA, using the ABI Prism® 7900 Sequence Detection System, served as a quality check of the first strand synthesis reaction.

*Quantitative PCR:*

Target gene amplification was performed in a QPCR reaction using Applied Biosystem's TaqMan® 2X Universal Master Mix and custom designed primer-probe sets. Individual target gene amplification was multiplexed with the 18S rRNA endogenous control and run in a 384- well format on the ABI Prism® 7900HT Sequence Detection System.

*QPCR Data Analysis:*

QPCR Sequence Detection System data files generated consisted of triplicate target gene cycle threshold, or CT values (FAM) and triplicate 18S rRNA endogenous control CT values (VIC). Normalized, delta CT ($\Delta C_T$) gene expression values for each amplified gene were calculated by taking the difference between CT values of the target gene and its endogenous control. All replicate CT values (target gene and endogenous control) were quality control checked to ensure that predefined criteria are met. An average delta $C_T$ value was then calculated for individual gene FAM and VIC replicate sets. The difference in normalized gene expression values ($\Delta C_T$) between samples was calculated to obtain a delta delta CT ($\Delta\Delta C_T$) value: $\Delta C_T$ (enriched sample)- $\Delta C_T$ (PBMC control sample). The $\Delta\Delta C_T$ value was then used for the calculation of a relative expression value with the following equation: $2^{-(\Delta\Delta CT)}$. Therefore, a difference of one $C_T$, as determined by the $\Delta\Delta C_T$ calculation, is equivalent to a two-fold difference in expression. Relative expression values were calculated for the enriched and depleted samples compared to the PBMC starting material to determine cell specific expression for the genes analyzed.

*Gene expression Analysis of Fractionated Cell samples from whole blood samples obtained from hormone refractory PRCA subjects (cohort 4)*

A quantitative comparison of gene expression levels between fractionated cell samples from eleven prostate cancer, cohort 4 subjects on a gene-by-gene basis for a panel of 18 genes was made using data obtained from the optimized Source MDx quantitative PCR assay (Precision Profile™).

Averaged relative expression values, calculated for each of the 18 genes analyzed from all eleven prostate cancer cohort 4 (PRCA Cht 4) patient samples, are presented in Table 22 (expression values shaded and bolded denotes ≥ 2-fold increased expression; expression values shaded, italicized and underlined denotes ≥ 2-fold decreased expression; * denotes cell marker). A graphical representation of the data is shown in Figures 15A & 15B.

Without intending to be bound by any theory, a differential pattern of expression across the four enriched cell types was observed in a heat map of the averaged relative expression values for each of the 18 genes analyzed (Table 21), indicating that some genes are more highly expressed in specific cell types upon enrichment from PBMC's. Not unexpectedly, cell specific marker genes exhibited a greatly increased expression in their enriched, cell specific fraction and a concomitant decrease in expression was observed in enriched, non-specific cell fractions. For example, the B cell marker CD19 was induced almost 7-fold in enriched B cells and had a decreased expression in enriched monocytes, NK cells and T cells (0.15-fold, 0.46-fold and 0.10- fold, respectively).

Many genes other than cell-specific markers also exhibited an increased expression in only one enriched cell fraction, potentially indicating that these genes may be preferentially expressed in one specific cell type. The genes IRAK3 and PLA2G7 showed a 2.72 and 3.11-fold increase in expression in enriched monocytes, respectively and a decrease in expression in the three other enriched cell types, possibly indicating that monocytes may be responsible for the majority of expression observed for these genes in whole blood.

A few genes also exhibited increased expression in multiple enriched fractions, indicating that the origin of the expression in whole blood originates from multiple cell types. C1QA and HK1 are examples of such genes as both are induced in enriched B cells, monocytes and NK cells also.

The majority of genes analyzed exhibited an increased expression in enriched monocytes (C1QA, CD4, CD82, CDKN1A, CTSD, HK1, IRAK3, PLA2G7, TIMP1 and TXNRD1), while fewer genes exhibited increased expression in enriched B cells (C1QA, CD82 and HK1), NK cells (ABL2, C1QA, GAS1 and ITGAL) and T cells (ABL2 and SEMA4D).

A graphical representation of the gene expression response for individual PRCA cohort 4 subjects in both enriched and depleted cells is presented in Figures 16A& 16B through Figures 19A & 19B (all "A" figures show the response in enriched fractions and "B" figures the depleted fractions).

As shown throughout these Figures, the gene expression profile was very similar between the eleven prostate cancer patient samples for the majority of genes in all cell fractions, indicating a consistency in cell-specific expression for genes across individuals, although the magnitude of response was slightly variable between patient samples. Additionally, genes showing an induction in enriched cell fractions, exhibited a corresponding decrease in expression in the depleted cell fraction for the same cell type.

*Gene expression Analysis of Fractionated Cell samples from Whole Blood samples obtained from medically defined Normal subjects*

A quantitative comparison of gene expression levels between fractionated cell samples was also conducted from seven medically defined normal (MDNO) subjects on a gene-by-gene basis for the 18 gene panel (Table 21) using data obtained from the optimized Source MDx quantitative PCR assay (Precision Profile™).

Averaged relative expression values, calculated for each of the 18 genes analyzed from all seven medically defined normal (MDNO) patient samples, are presented in Table 23 (expression values shaded and bolded denotes ≥ 2-fold increased expression; expression values shaded, italicized and underlined denotes ≥ 2-fold decreased expression; * denotes cell marker). A graphical representation of the data is shown in Figures 20A & 20B. Many of the same findings as in the PRCA Cohort 4 patient sample analysis were observed.

For example, a differential pattern of expression across the four enriched cell types was observed in a heat map of the averaged relative expression values for each of the 18 genes analyzed (Table 21). Many genes other than cell-specific markers also exhibited an increased expression in only one enriched cell fraction. A few genes also exhibited an increased expression in multiple enriched fractions. Overall, the majority of genes analyzed exhibited an increased expression in enriched monocytes.

A graphical representation of the gene expression response for individual MDNO subjects in both enriched and depleted cells is presented in Figures 21A & 21B through Figures 24A & 24B (all "A" figures show the response in enriched fractions and "B" figures the depleted fractions). Again, many of the same findings as in the PRCA Cohort 4 patient sample analysis were observed. For example, the gene expression profile was very similar between the seven MDNO patient samples for the majority of genes in all cell fractions. Additionally, the magnitude of response was slightly variable between patient samples. Genes showing an induction in enriched cell fractions exhibited a corresponding decrease in expression in the depleted cell fraction for the same cell type.

*Summary*

A comparison of gene expression profiles between disease and normal subjects reveal a strong similarity in expression patterns in all enriched cell types. Though there does not appear to be a difference in cell-specific gene expression, a number a genes may have slightly differing magnitudes of expression in certain enriched fractions -between prostate cancer and normal subjects, though it has not been determined whether these differences are in fact statistically significant. Genes having potentially different magnitudes of expression in enriched fractions include ABL2, C1QA, GAS1, CD82 and TIMP1. ABL2 had an average 1.31-fold increased expression in enriched T cells from prostate cancer patients compared to a 0.93-fold decrease in expression in enriched T cells from normal subjects. C1QA had an average 1.45-fold increased expression in enriched B cells from prostate cancer patients compared to a 0.85-fold decrease in expression in enriched B cells from normal subjects. GAS1 had an average 2.18-fold increased expression in enriched NK cells from prostate cancer patients compared to a 3.94-fold increased expression in enriched NK cells from normal subjects. CD82 has an average 1.58-fold increased expression in enriched monocytes from prostate cancer patients compared to a 2.10-fold increase in expression in enriched monocytes from normal subjects. TIMP1 had an average 2.21-fold increased expression in enriched monocytes from prostate cancer patients compared to a 2.93- fold increase in expression in enriched monocytes from normal subjects.

The references listed below are hereby incorporated herein by reference.

## References

Magidson, J. GOLDMineR User's Guide (1998). Belmont, MA: Statistical Innovations Inc.

Vermunt and Magidson (2005). Latent GOLD 4.0 Technical Guide, Belmont MA: Statistical Innovations.

Vermunt and Magidson (2007). LG-Syntax™ User's Guide: Manual for Latent GOLD® 4.5 Syntax Module, Belmont MA: Statistical Innovations.

Vermunt J.K. and J. Magidson. Latent Class Cluster Analysis in (2002) J. A. Hagenaars and A. L. McCutcheon (eds.), Applied Latent Class Analysis, 89-106. Cambridge: Cambridge University Press.

Magidson, J. "Maximum Likelihood Assessment of Clinical Trials Based on an Ordered Categorical Response." (1996) Drug Information Journal, Maple Glen, PA: Drug Information Association, Vol. 30, No. 1, pp 143-170.

The contents of EP 09790154.0, from which this application is divided, are incorporated herein by reference in their entirety.

**TABLE 1:** Precision Profile™ for Prostate Cancer Survivability

| Gene Symbol | Gene Name | Gene Accession Number |
|---|---|---|
| ABCC1 | ATP-binding cassette, sub-family C (CFTR/MRP), member 1 | NM_004996 |
| ABL1 | v-abl Abelson murine leukemia viral oncogene homolog 1 | NM_005157 |
| ABL2 | v-abl Abelson murine leukemia viral oncogene homolog 2 (arg, Abelson-related gene) | NM_005158 |
| ACPP | acid phosphatase, prostate | NM_001099 |
| ADAM17 | a disintegrin and metalloproteinase domain 17 (tumor necrosis factor, alpha, converting enzyme) | NM_003183 |
| ADAMTS1 | A disintegrin-like and metalloprotease (reprolysin type) with thrombospondin type 1 motif, 1 | NM_006988 |
| AKT1 | v-akt murine thymoma viral oncogene homolog 1 | NM_005163 |
| ALOX5 | arachidonate 5-lipoxygenase | NM_000698 |
| ANGPT1 | angiopoietin 1 | NM_001146 |
| ANLN | anillin, actin binding protein (scraps homolog, Drosophila) | NM_018685 |
| AOC3 | amine oxidase, copper containing 3 (vascular adhesion protein 1) | NM_003734 |
| APAF1 | apoptotic Protease Activating Factor 1 | NM_013229 |

(continued)

| Gene Symbol | Gene Name | Gene Accession Number |
|---|---|---|
| APC | adenomatosis polyposis coli | NM_000038 |
| BCAM | basal cell adhesion molecule (Lutheran blood group) | NM_005581 |
| BCL2 | B-cell CLL/lymphoma 2 | NM_000633 |
| BRAF | v-raf murine sarcoma viral oncogene homolog B1 | NM_004333 |
| BRCA1 | breast cancer 1, early onset | NM_007294 |
| C1 QA | complement component 1, q subcomponent, A chain | NM-015991 |
| C1QB | complement component 1, q subcomponent, B chain | NM_000491 |
| CA4 | carbonic anhydrase IV | NM_000717 |
| CASP1 | caspase 1, apoptosis-related cysteine peptidase (interleukin 1, beta, convertase) | NM_033292 |
| CASP9 | caspase 9, apoptosis-related cysteine peptidase | NM_001229 |
| CAV2 | caveolin 2 | NM_001233 |
| CCL3 | chemokine (C-C motif) ligand 3 | NM_002983 |
| CCL5 | chemokine (C-C motif) ligand 5 | NM_002985 |
| CCND2 | cyclin D2 | NM_001759 |
| CCNE1 | Cyclin E1 | NM_001238 |
| CD19 | CD19 Antigen | NM_001770 |
| CD44 | CD44 antigen (homing function and Indian blood group system) | NM_000610 |
| CD48 | CD48 antigen (B-cell membrane protein) | NM_001778 |
| CD59 | CD59 antigen p18-20 | NM_000611 |
| CD82 (KAI1) | CD82 antigen | NM_002231 |
| CD97 | CD97 molecule | NM_078481 |
| CDC25A | cell division cycle 25A | NM_001789 |
| CDH1 | cadherin 1, type 1, E-cadherin (epithelial) | NM_004360 |
| CDK2 | cyclin-dependent kinase 2 | NM_001798 |
| CDK5 | cyclin-dependent kinase 5 | NM_004935 |
| CDKN1A | cyclin-dependent kinase inhibitor 1A (p21, Cip1) | NM_000389 |
| CDKN2A | cyclin-dependent kinase inhibitor 2A (melanoma, p16, inhibits CDK4) | NM_000077 |
| CDKN2D | cyclin-dependent kinase inhibitor 2D (p19, inhibits CDK4) | NM_001800 |
| CEACAM1 | carcinoembryonic antigen-related cell adhesion molecule 1 (biliary glycoprotein) | NM_001712 |
| CEBPB | CCAAT/enhancer binding protein (C/EBP), beta | NM_005194 |
| CFLAR | CASP8 and FADD-like apoptosis regulator | NM_003879 |
| COL6A2 | collagen, type VI, alpha 2 | NM_001849 |
| COVA1 | cytosolic ovarian carcinoma antigen 1 | NM_006375 |
| CREBBP | CREB binding protein | NM_004380 |
| CTNNA1 | catenin (cadherin-associated protein), alpha 1, 102kDa | NM_001903 |
| CTSD | cathepsin D (lysosomal aspartyl peptidase) | NM_001909 |

(continued)

| Gene Symbol | Gene Name | Gene Accession Number |
|---|---|---|
| DAD1 | defender against cell death 1 | NM_001344 |
| DLC1 | deleted in liver cancer 1 | NM_182643 |
| E2F1 | E2F transcription factor 1 | NM_005225 |
| E2F5 | E2F transcription factor 5, p130-binding | NM_001951 |
| EGR1 | Early growth response-1 | NM_001964 |
| EGR3 | early growth response 3 | NM_004430 |
| ELA2 | Elastase 2, neutrophil | NM_001972 |
| EP300 | E1A binding protein p300 | NM_001429 |
| EPAS1 | endothelial PAS domain protein 1 | NM_001430 |
| ERBB2 | V-erb-b2 erythroblastic leukemia viral oncogene homolog 2, neuro/glioblastoma derived oncogene homolog (avian) | NM_004448 |
| ETS2 | v-ets erythroblastosis virus E26 oncogene homolog 2 (avian) | NM_005239 |
| FAS | Fas (TNF receptor superfamily, member 6) | NM_000043 |
| FGF2 | Fibroblast growth factor 2 (basic) | NM_002006 |
| FOS | v-fos FBJ murine osteosarcoma viral oncogene homolog | NM_005252 |
| G6PD | glucose-6-phosphate dehydrogenase | NM_000402 |
| GADD45A | growth arrest and DNA-damage-inducible, alpha | NM_001924 |
| GNB1 | guanine nucleotide binding protein (G protein), beta polypeptide 1 | NM_002074 |
| GSK3B | glycogen synthase kinase 3 beta | NM_002093 |
| GSTT1 | glutathione S-transferase theta 1 | NM_000853 |
| HMGA1 | high mobility group AT-hook 1 | NM_145899 |
| HRAS | v-Ha-ras Harvey rat sarcoma viral oncogene homolog | NM_005343 |
| HSPA1A | Heat shock protein 70 | NM_005345 |
| ICAM1 | Intercellular adhesion molecule 1 | NM_000201 |
| IFI16 | Interferon inducible protein 16, gamma | NM_005531 |
| IFI6 (G1P3) | interferon, alpha-inducible protein 6 | NM_002038 |
| IFITM1 | interferon induced transmembrane protein 1 (9-27) | NM_003641 |
| IFNG | interferon gamma | NM_000619 |
| IGF1R | insulin-like growth factor 1 receptor | NM_000875 |
| IGF2BP2 | insulin-like growth factor 2 mRNA binding protein 2 | NM_006548 |
| IGFBP3 | insulin-like growth factor binding protein 3 | NM_001013398 |
| IL10 | interleukin 10 | NM_000572 |
| IL18 | Interleukin 18 | NM_001562 |
| IL1B | Interleukin 1, beta | NM_000576 |
| IL1RN | interleukin 1 receptor antagonist | NM_173843 |
| IL8 | interleukin 8 | NM_000584 |
| IQGAP1 | IQ motif containing GTPase activating protein 1 | NM_003870 |

(continued)

| Gene Symbol | Gene Name | Gene Accession Number |
|---|---|---|
| IRF1 | interferon regulatory factor 1 | NM_002198 |
| ITGA1 | integrin, alpha 1 | NM_181501 |
| ITGAL | integrin, alpha L (antigen CD11A (p180), lymphocyte function-associated antigen 1; alpha polypeptide) | NM_002209 |
| ITGB1 | integrin, beta 1 (fibronectin receptor, beta polypeptide, antigen CD29 includes MDF2, MSK12) | NM_002211 |
| JUN | v-jun sarcoma virus 17 oncogene homolog (avian) | NM_002228 |
| KLK3 | kallikrein 3, (prostate specific antigen) | NM_001648 |
| KRT5 | keratin 5 (epidermolysis bullosa simplex, Dowling-Meara/Kobner/Weber-Cockayne types) | NM_000424 |
| LGALS8 | lectin, galactoside-binding, soluble, 8 (galectin 8) | NM_006499 |
| MAP2K1 | mitogen-activated protein kinase kinase 1 | NM_002755 |
| MAPK1 | mitogen-activated protein kinase 1 | NM_138957 |
| MAPK14 | mitogen-activated protein kinase 14 | NM_001315 |
| MEIS1 | Meis1, myeloid ecotropic viral integration site 1 homolog (mouse) | NM_002398 |
| MME | membrane metallo-endopeptidase (neutral endopeptidase, enkephalinase, CALLA, CD10) | NM_000902 |
| MMP9 | matrix metallopeptidase 9 (gelatinase B, 92kDa gelatinase, 92kDa type IV collagenase) | NM_004994 |
| MNDA | myeloid cell nuclear differentiation antigen | NM_002432 |
| MTA1 | metastasis associated 1 | NM_004689 |
| MTF1 | metal-regulatory transcription factor 1 | NM_005955 |
| MYC | v-myc myelocytomatosis viral oncogene homolog (avian) | NM_002467 |
| MYD88 | myeloid differentiation primary response gene (88) | NM_002468 |
| NAB1 | NGFI-A binding protein 1 (EGR1 binding protein 1) | NM_005966 |
| NAB2 | NGFI-A binding protein 2 (EGR1 binding protein 2) | NM_005967 |
| NCOA1 | nuclear receptor coactivator 1 | NM_003743 |
| NCOA4 | nuclear receptor coactivator 4 | NM_005437 |
| NEDD4L | neural precursor cell expressed, developmentally down-regulated 4-like | NM_015277 |
| NFATC2 | nuclear factor of activated T-cells, cytoplasmic, calcineurin-dependent 2 | NM_012340 |
| NFKB1 | nuclear factor of kappa light polypeptide gene enhancer in B-cells 1 (p105) | NM_003998 |
| NME1 | non-metastatic cells 1, protein (NM23A) expressed in | NM_198175 |
| NME4 | non-metastatic cells 4, protein expressed in | NM_005009 |
| NOTCH2 | Notch homolog 2 | NM_024408 |
| NR4A2 | nuclear receptor subfamily 4, group A, member 2 | NM_006186 |
| NRAS | neuroblastoma RAS viral (v-ras) oncogene homolog | NM_002524 |
| NRP1 | neuropilin 1 | NM_003873 |

(continued)

| Gene Symbol | Gene Name | Gene Accession Number |
|---|---|---|
| NUDT4 | nudix (nucleoside diphosphate linked moiety X)-type motif 4 | NM_019094 |
| PDGFA | platelet-derived growth factor alpha polypeptide | NM_002607 |
| PLAU | plasminogen activator, urokinase | NM_002658 |
| PLEK2 | pleckstrin 2 | NM_016445 |
| PLXDC2 | plexin domain containing 2 | NM_032812 |
| POV1 | solute carrier family 43, member 1 | NM_003627 |
| PTCH1 | patched homolog 1 (Drosophila) | NM_000264 |
| PTEN | phosphatase and tensin homolog (mutated in multiple advanced cancers 1) | NM_000314 |
| PTGS2 | prostaglandin-endoperoxide synthase 2 (prostaglandin G/H synthase and cyclooxygenase) | NM_000963 |
| PTPRC | protein tyrosine phosphatase, receptor type, C | NM_002838 |
| PYCARD | PYD and CARD domain containing | NM_013258 |
| RAF1 | v-raf-1 murine leukemia viral oncogene homolog 1 | NM_002880 |
| RB1 | retinoblastoma 1 (including osteosarcoma) | NM_000321 |
| RBM5 | RNA binding motif protein 5 | NM_005778 |
| RHOA | ras homolog gene family, member A | NM_001664 |
| RHOC | ras homolog gene family, member C | NM_175744 |
| RP5-1077B9.4 | invasion inhibitory protein 45 | NM_001025374 |
| S100A11 | S100 calcium binding protein A11 | NM_005620 |
| S100A6 | S100 calcium binding protein A6 | NM_014624 |
| SEMA4D | sema domain, immunoglobulin domain (Ig), transmembrane domain (TM) and short cytoplasmic domain, (semaphorin) 4D | NM_006376 |
| SERPINA1 | serpin peptidase inhibitor, clade A (alpha-1 antiproteinase, antitrypsin), member 1 | NM_001002235 |
| SERPINE1 | serpin peptidase inhibitor, clade E (nexin, plasminogen activator inhibitor type 1), member 1 | NM_000602 |
| SERPING1 | serpin peptidase inhibitor, clade G (C1 inhibitor), member 1, (angioedema, hereditary) | NM_000062 |
| SIAH2 | seven in absentia homolog 2 (Drosophila) | NM_005067 |
| SKIL | SKI-like oncogene | NM_005414 |
| SMAD3 | SMAD, mothers against DPP homolog 3 (Drosophila) | NM_005902 |
| SMAD4 | SMAD family member 4 | NM_005359 |
| SMARCD3 | SWI/SNF related, matrix associated, actin dependent regulator of chromatin, subfamily d, member 3 | NM_001003801 |
| SOCS1 | suppressor of cytokine signaling 1 | NM_003745 |
| SORBS1 | sorbin and SH3 domain containing 1 | NM_001034954 |
| SOX4 | SRY (sex determining region Y)-box 4 | NM_003107 |
| SP1 | Sp1 transcription factor | NM_138473 |
| SPARC | secreted protein, acidic, cysteine-rich (osteonectin) | NM_004598 |

(continued)

| Gene Symbol | Gene Name | Gene Accession Number |
|---|---|---|
| SRC | v-src sarcoma (Schmidt-Ruppin A-2) viral oncogene homolog (avian) | NM_198291 |
| SRF | serum response factor (c-fos serum response element-binding transcription factor) | NM_003131 |
| ST14 | suppression of tumorigenicity 14 (colon carcinoma) | NM_021978 |
| STAT3 | signal transducer and activator of transcription 3 (acute-phase response factor) | NM_003150 |
| SVIL | supervillin | NM_003174 |
| TEGT | testis enhanced gene transcript (BAX inhibitor 1) | NM_003217 |
| TGFB1 | transforming growth factor, beta 1 (Camurati-Engelmann disease) | NM_000660 |
| THBS1 | thrombospondin 1 | NM_003246 |
| TIMP1 | tissue inhibitor of metalloproteinase 1 | NM_003254 |
| TLR2 | toll-like receptor 2 | NM_003264 |
| TNF | tumor necrosis factor (TNF superfamily, member 2) | NM_000594 |
| TNFRSF1A | tumor necrosis factor receptor superfamily, member 1A | NM_001065 |
| TNFRSF13B | tumor necrosis factor receptor superfamily, member 13B | NM_012452 |
| TOPBP1 | topoisomerase (DNA) II binding protein 1 | NM_007027 |
| TP53 | tumor protein p53 (Li-Fraumeni syndrome) | NM_000546 |
| TXNRD1 | thioredoxin reductase | NM_003330 |
| UBE2C | ubiquitin-conjugating enzyme E2C | NM_007019 |
| USP7 | ubiquitin specific peptidase 7 (herpes virus-associated) | NM_003470 |
| VEGF | vascular endothelial growth factor | NM_003376 |
| VHL | von Hippel-Lindau tumor suppressor | NM_000551 |
| VIM | vimentin | NM_003380 |
| XK | X-linked Kx blood group (McLeod syndrome) | NM_021083 |
| XRCC1 | X-ray repair complementing defective repair in Chinese hamster cells 1 | NM_006297 |
| ZNF185 | zinc finger protein 185 (LIM domain) | NM_007150 |
| ZNF350 | zinc finger protein 350 | NM_021632 |

**Table 2:** Types of Therapy for 62 Cohort 4 Prostate Cancer Subjects

| Patients | 1st Line Treatment | 2nd Line | 3rd Line | 4 th Line |
|---|---|---|---|---|
| 16 | Hormone Rx | none | none | none |
| 12 | Chemotherapy | none | none | none |
| 6 | Hormone Rx | Hormone Rx | none | none |
| 4 | Hormone Rx | Hormone Rx | Hormone Rx | none |
| 3 | Hormone Rx | Chemotherapy | none | none |
| 3 | Other Rx | none | none | none |

(continued)

| Patients | 1st Line Treatment | 2nd Line | 3rd Line | 4 th Line |
|---|---|---|---|---|
| 2 | Hormone Rx | Hormone Rx | Chemotherapy | none |
| 2 | Radiotherapy | none | none | none |
| 1 | Chemotherapy | Hormone Rx | none | none |
| 1 | Hormone Rx | Hormone Rx | Hormone Rx | Hormone Rx |
| 1 | Hormone Rx | Hormone Rx | Other Rx | none |
| 1 | Hormone Rx | Hormone Rx | Radiotherapy | none |
| 1 | Hormone Rx | Other Rx | Chemotherapy | Chemotherapy |
| 1 | Hormone Rx | Other Rx | Chemotherapy | no |
| 1 | Hormone Rx | Other Rx | Hormone Rx | Chemotherapy |
| 1 | Hormone Rx | Other Rx | Hormone Rx | Hormone Rx |
| 1 | Hormone Rx | Other Rx | Hormone Rx | none |
| 1 | Hormone Rx | Radiotherapy | Hormone Rx | none |
| 1 | Other Rx | Hormone Rx | none | none |
| 1 | Other Rx | Hormone Rx | Radiotherapy | none |
| 1 | Radiotherapy | Hormone Rx | Hormone Rx | Hormone Rx |
| 1 | Radiotherapy | Hormone Rx | none | none |
| **62** | **Total Patients** | | | |

**Table 3:** Summary of Patient Survivability Status and Survival Date

| cohort IV | Pt ID | Initial date classified as Cohort 4 | status | survival date |
|---|---|---|---|---|
| 154:001 | 163876 | 10/11/2004 | ALIVE | 6/20/2008 |
| 154:006 | 278209 | 1/5/2006 | DEAD | 2/17/2008 |
| 154:009 | 204342 | 7/13/2006 | DEAD | 6/27/2007 |
| 154:016 | 290733 | 10/16/2006 | ALIVE | 6/20/2008 |
| 154:026 | 289486 | 10/17/2006 | ALIVE | 6/20/2008 |
| 154:031 | N/A | 7/10/2006 | DEAD | 3/12/2008 |
| 154:032 | 255270 | 6/6/2005 | DEAD | 6/8/2007 |
| 154:044 | 64177 | 7/8/2005 | DEAD | 5/22/2007 |
| 154:046 | 258871 | 12/22/2005 | DEAD | 11/15/2007 |
| 154:048 | 131732 | 4/21/2005 | ALIVE | 6/20/2008 |
| 154:056 | 232308 | 12/29/2003 | ALIVE | 6/20/2008 |
| 154:057 | N/A | 1/5/2006 | DEAD | 7/3/2007 |
| 154:059 | 112938 | 5/19/2005 | ALIVE | 6/20/2008 |
| 154:063 | 236369 | 3/15/2004 | DEAD | 2/1/2008 |
| 154:072 / 154:111457 | 111457 | 1/27/2003 | ALIVE | 6/20/2008 |
| 154:078 | 295798 | 2/2/2006 | ALIVE | 6/20/2008 |
| 154:088 | 265599 | 7/14/2005 | DEAD | 9/27/2007 |
| 154:099 | 199885 | 7/12/2004 | ALIVE | 6/20/2008 |
| 154:113 | 102903 | 11/2/1999 | ALIVE | 6/20/2008 |
| 154:124 / 154:250157 | 250157 | 7/13/2006 | DEAD | 1/8/2008 |
| 154:155 | 250196 | 6/23/2005 | ALIVE | 6/20/2008 |
| 154:156 / 154:279014 | 279014 | 3/1/2007 | ALIVE | 6/20/2008 |
| 154:103398 | 103398 | 4/16/2004 | ALIVE | 6/20/2008 |
| 154:109722 | 109722 | 10/29/2001 | ALIVE | 6/20/2008 |
| 154:137633 | 137633 | 7/21/2005 | ALIVE | 6/20/2008 |

| cohort IV | Pt ID | Initial date classified as Cohort 4 | status | survival date |
|---|---|---|---|---|
| 154:152331 | 152331 | 1/24/2005 | ALIVE | 6/20/2008 |
| 154:164406 | 164406 | 11/10/2005 | ALIVE | 6/20/2008 |
| 154:178930 | 178930 | 1/18/2001 | ALIVE | 6/20/2008 |
| 154:185401 | 185401 | 10/20/2005 | ALIVE | 6/20/2008 |
| 154:187129 | 187129 | 11/22/2004 | ALIVE | 6/20/2008 |
| 154:187770 | 187770 | 4/25/2003 | ALIVE | 6/20/2008 |
| 154:196141 | 196141 | 4/29/2002 | ALIVE | 6/20/2008 |
| 154:196262 | 196262 | 2/26/2004 | ALIVE | 6/20/2008 |
| 154:200871 | 200871 | 6/20/2005 | ALIVE | 6/20/2008 |
| 154:208893 | 208893 | 7/16/2007 | ALIVE | 6/20/2008 |
| 154:219180 | 219180 | 2/16/2006 | DEAD | 5/7/2008 |
| 154:221617 | 221617 | 8/7/2006 | ALIVE | 6/20/2008 |
| 154:223748 | 223748 | 9/8/2003 | ALIVE | 6/20/2008 |
| 154:224210 | 224210 | 5/26/2006 | ALIVE | 6/20/2008 |
| 154:229247 | 229247 | 11/10/2003 | ALIVE | 6/20/2008 |
| 154:229664 | 229664 | 11/3/2003 | ALIVE | 6/20/2008 |
| 154:233923 | 233923 | 2/2/2004 | ALIVE | 6/20/2008 |
| 154:244769 | 244769 | 3/13/2006 | ALIVE | 6/20/2008 |
| 154:249044 | 249044 | 10/21/2004 | ALIVE | 6/20/2008 |
| 154:252906 | 252906 | 3/30/2006 | ALIVE | 6/20/2008 |

| cohort IV | Pt ID | Initial date classified as Cohort 4 | status | survival date |
|---|---|---|---|---|
|  |  |  |  |  |
| I54:261891 | 261891 | 7/12/2007 | ALIVE | 6/20/2008 |
| I54:272956 | 272956 | 9/11/2006 | ALIVE | 6/20/2008 |
| I54:275979 | 275979 | 1/4/2007 | DEAD | 4/28/2008 |
| I54:279316 | 279316 | 3/9/2006 | ALIVE | 6/20/2008 |
| I54:290701 | 290701 | 1/11/2007 | DEAD | 9/5/2007 |
| I54:294238 | 294238 | 11/20/2006 | DEAD | 2/14/2008 |
| I54:295740 | 295740 | 11/20/2006 | ALIVE | 6/20/2008 |
| I54:303333 | 303333 | 5/3/2007 | ALIVE | 6/20/2008 |
| I54:322324 | 322324 | 11/5/2007 | ALIVE | 6/20/2008 |
| I54:323394 | 323394 | 12/6/2007 | DEAD | 3/5/2008 |
| I54:330355 | 330355 | 1/31/2008 | ALIVE | 6/20/2008 |
| I54:50223520 | 50223520 | 12/21/2006 | ALIVE | 6/20/2008 |
| I54:50254384 | 50254384 | 4/30/2007 | ALIVE | 6/20/2008 |
| I54:50796156 | 50796156 | 4/23/2007 | ALIVE | 6/20/2008 |
| I54:334666 | 334666 | 3/13/2008 | ALIVE | 6/20/2008 |
| 322703 | 322703 | 11/2/2007 | ALIVE | 6/20/2008 |
| 336476 | 336476 | 5/5/2008 | ALIVE | 6/20/2008 |
| 329976 | 329976 | 1/31/2008 | ALIVE | 6/20/2008 |

**Table 4:**

DF Cohort 4 Prostate Cancer Subjects: $N_1$ = 15 dead and $N_2$ = 47 alive as of June 20, 2008

## Median survival time of those who died was 20 months

| elapsed time | | subjects remaining | | | deaths | censored | cumulative | | % of | |
|---|---|---|---|---|---|---|---|---|---|---|
| years | months | period | Total | Percent | | | deaths | censored | Deaths | Alive |
| | | 1 | 62 | 100% | --------- | --------- | --------- | --------- | | |
| 0 | 3 | 2 | 60 | 97% | 1 | 1 | 1 | 1 | 7% | 2% |
| | 6 | 3 | 58 | 94% | 0 | 2 | 1 | 3 | 7% | 7% |
| | 9 | 4 | 55 | 89% | 1 | 2 | 2 | 5 | 13% | 11% |
| 1 | 12 | 5 | 52 | 84% | 1 | 2 | 3 | 7 | 20% | 15% |
| | 15 | 6 | 48 | 77% | 1 | 3 | 4 | 10 | 27% | 23% |
| | 18 | 7 | 43 | 69% | 3 | 2 | 7 | 12 | 47% | 26% |
| | 21 | 8 | 39 | 63% | 1 | 3 | 8 | 15 | 53% | 33% |
| 2 | 24 | 9 | 34 | 55% | 3 | 2 | 11 | 17 | 73% | 37% |
| | 27 | 10 | 29 | 47% | 3 | 2 | 14 | 19 | 93% | 41% |
| | 30 | 11 | 26 | 42% | 0 | 3 | 14 | 22 | 93% | 48% |
| | 33 | 12 | 24 | 39% | 0 | 2 | 14 | 24 | 93% | 52% |
| 3 | 36 | 13 | 21 | 34% | 0 | 3 | 14 | 27 | 93% | 59% |
| | 39 | 14 | 19 | 31% | 0 | 2 | 14 | 29 | 93% | 63% |
| | 42 | 15 | 18 | 29% | 0 | 1 | 14 | 30 | 93% | 65% |
| | 45 | 16 | 15 | 24% | 0 | 3 | 14 | 33 | 93% | 72% |
| 4 | 48 | 17 | 13 | 21% | 1 | 1 | 15 | 34 | 100% | 74% |
| | 51 | 18 | 12 | 19% | 0 | 1 | 15 | 35 | 100% | 76% |
| | 54 | 19 | 9 | 15% | 0 | 3 | 15 | 38 | 100% | 83% |
| | 57 | 20 | 7 | 11% | 0 | 2 | 15 | 40 | 100% | 87% |
| 5 | 60 | 21 | 6 | 10% | 0 | 1 | 15 | 41 | 100% | 89% |
| | 63 | 22 | 5 | 8% | 0 | 1 | 15 | 42 | 100% | 91% |
| | 66 | 23 | 4 | 6% | 0 | 1 | 15 | 43 | 100% | 93% |
| | 69 | 24 | 4 | 6% | 0 | 0 | 15 | 43 | 100% | 93% |
| 6 | 72 | 25 | 4 | 6% | 0 | 0 | 15 | 43 | 100% | 93% |
| | 75 | 26 | 3 | 5% | 0 | 1 | 15 | 44 | 100% | 96% |
| | 78 | 27 | 3 | 5% | 0 | 0 | 15 | 44 | 100% | 96% |
| | 81 | 28 | 2 | 3% | 0 | 1 | 15 | 45 | 100% | 98% |
| 7 | 84 | 29 | 2 | 3% | 0 | 0 | 15 | 45 | 100% | 98% |
| | 87 | 30 | 2 | 3% | 0 | 0 | 15 | 45 | 100% | 98% |
| | 90 | 31 | 1 | 2% | 0 | 1 | 15 | 46 | 100% | 100% |
| | 93 | 32 | 1 | 2% | 0 | 0 | 15 | 46 | 100% | 100% |
| 8 | 96 | 33 | 1 | 2% | 0 | 0 | 15 | 46 | 100% | 100% |
| | 99 | 34 | 1 | 2% | 0 | 0 | 15 | 46 | 100% | 100% |
| | 102 | 35 | 1 | 2% | 0 | 0 | 15 | 46 | 100% | 100% |

**Table 5:** 1 and 2-gene Cox-Type Survival Models

| 2-gene models and | | Entropy | | |
| --- | --- | --- | --- | --- |
| 1-gene models | | R-sq | p-val 1 | p-val 2 |
| ABL2 | C1QA | 0.21 | 3.0E-07 | 2.2E-06 |
| SEMA4D | TIMP1 | 0.19 | 1.2E-07 | 1.3E-06 |
| MYD88 | SEMA4D | 0.18 | 1.7E-06 | 1.6E-08 |
| SEMA4D | SVIL | 0.17 | 5.3E-08 | 4.1E-06 |
| CDKN1A | ITGAL | 0.17 | 2.9E-07 | 1.7E-05 |
| ABL2 | C1QB | 0.17 | 4.4E-05 | 0.0001 |
| ABL2 | PYCARD | 0.17 | 5.0E-08 | 0.0001 |
| ABL2 | MNDA | 0.17 | 5.7E-08 | 0.0001 |
| CDKN1A | SMAD3 | 0.16 | 3.7E-07 | 4.0E-05 |
| ABL2 | CDKN1A | 0.16 | 4.2E-05 | 0.0002 |
| S100A11 | SEMA4D | 0.16 | 1.3E-05 | 1.1E-07 |
| CCL5 | CDKN1A | 0.16 | 4.8E-05 | 1.1E-07 |
| ABL2 | ST14 | 0.16 | 1.6E-07 | 0.0003 |
| C1QB | SEMA4D | 0.16 | 1.7E-05 | 0.0001 |
| ABL2 | TIMP1 | 0.16 | 1.8E-06 | 0.0004 |
| NFATC2 | RHOC | 0.16 | 2.1E-07 | 2.0E-06 |
| CDKN1A | TGFB1 | 0.15 | 2.5E-07 | 9.7E-05 |
| CDKN1A | NFATC2 | 0.15 | 2.7E-06 | 0.0001 |
| MNDA | SEMA4D | 0.15 | 3.2E-05 | 2.7E-07 |
| ABL1 | CDKN1A | 0.15 | 0.0001 | 9.4E-07 |
| SEMA4D | TEGT | 0.15 | 3.0E-07 | 3.5E-05 |
| BRCA1 | C1QB | 0.15 | 0.0003 | 4.9E-07 |
| SEMA4D | SERPINA1 | 0.15 | 3.4E-07 | 4.1E-05 |
| C1QB | SERPING1 | 0.15 | 5.9E-07 | 0.0004 |
| RBM5 | TIMP1 | 0.15 | 4.8E-06 | 3.0E-06 |
| PYCARD | SEMA4D | 0.15 | 6.3E-05 | 5.3E-07 |
| C1QB | SOCS1 | 0.15 | 3.4E-06 | 0.0005 |
| C1QB | PLXDC2 | 0.14 | 1.0E-06 | 0.0006 |
| ABL2 | VIM | 0.14 | 5.8E-07 | 0.0014 |
| ABL2 | TXNRD1 | 0.14 | 5.9E-07 | 0.0014 |
| C1QA | SEMA4D | 0.14 | 7.6E-05 | 0.0002 |
| CDKN1A | SEMA4D | 0.14 | 7.9E-05 | 0.0003 |
| ABL2 | FAS | 0.14 | 6.8E-07 | 0.0016 |
| ABL2 | CDK5 | 0.14 | 6.8E-07 | 0.0016 |
| IFITM1 | SEMA4D | 0.14 | 8.7E-05 | 7.5E-07 |
| ABL2 | MYD88 | 0.14 | 8.8E-07 | 0.0019 |
| CDKN1A | TP53 | 0.14 | 1.3E-06 | 0.0003 |
| CDKN1A | SMAD4 | 0.14 | 2.9E-06 | 0.0004 |

| 2-gene models and | | Entropy | | |
|---|---|---|---|---|
| 1-gene models | | R-sq | p-val 1 | p-val 2 |
| C1QA | ITGAL | 0.14 | 6.5E-06 | 0.0003 |
| SMAD4 | TIMP1 | 0.14 | 1.1E-05 | 3.3E-06 |
| ABL1 | C1QA | 0.14 | 0.0003 | 3.3E-06 |
| ABL2 | CAV2 | 0.14 | 0.0012 | 0.0025 |
| CDKN1A | HMGA1 | 0.14 | 2.9E-06 | 0.0005 |
| CDKN1A | MAP2K1 | 0.14 | 2.0E-06 | 0.0005 |
| CDKN1A | XRCC1 | 0.14 | 2.3E-06 | 0.0005 |
| CAV2 | SEMA4D | 0.14 | 0.0002 | 0.0015 |
| C1QB | CREBBP | 0.14 | 1.3E-05 | 0.0013 |
| CDKN1A | VHL | 0.14 | 2.5E-06 | 0.0006 |
| C1QB | EP300 | 0.14 | 2.9E-06 | 0.0013 |
| C1QA | SMAD3 | 0.14 | 5.2E-06 | 0.0004 |
| ABL2 | S100A6 | 0.14 | 1.5E-06 | 0.0033 |
| BCL2 | CDKN1A | 0.14 | 0.0006 | 1.7E-05 |
| CDK2 | CDKN1A | 0.14 | 0.0006 | 2.4E-06 |
| ABL2 | ICAM1 | 0.13 | 5.9E-06 | 0.0038 |
| ABL2 | ITGA1 | 0.13 | 2.7E-06 | 0.0027 |
| GNB1 | TIMP1 | 0.13 | 1.8E-05 | 3.7E-06 |
| CDKN1A | NFKB1 | 0.13 | 4.1E-06 | 0.0007 |
| ABL2 | TOPBP1 | 0.13 | 2.8E-06 | 0.0044 |
| C1QA | MYC | 0.13 | 1.5E-05 | 0.0006 |
| C1QB | MTF1 | 0.13 | 5.3E-06 | 0.0019 |
| CDKN1A | SRC | 0.13 | 2.8E-06 | 0.0009 |
| RHOA | SEMA4D | 0.13 | 0.0002 | 2.1E-06 |
| ABL2 | RHOA | 0.13 | 2.1E-06 | 0.0051 |
| C1QA | SMAD4 | 0.13 | 6.9E-06 | 0.0006 |
| ABL2 | CD48 | 0.13 | 2.1E-06 | 0.0053 |
| C1QA | ICAM1 | 0.13 | 8.2E-06 | 0.0007 |
| CDKN1A | ICAM1 | 0.13 | 8.1E-06 | 0.0009 |
| CDKN1A | IRF1 | 0.13 | 3.7E-06 | 0.0010 |
| C1QB | RBM5 | 0.13 | 1.5E-05 | 0.0022 |
| CDKN1A | PTCH1 | 0.13 | 1.4E-05 | 0.0010 |
| TIMP1 | XRCC1 | 0.13 | 4.4E-06 | 2.7E-05 |
| ABL2 | NAB1 | 0.13 | 5.0E-06 | 0.0064 |
| CDKN1A | MYC | 0.13 | 2.1E-05 | 0.0012 |
| ABL2 | LGALS8 | 0.13 | 3.2E-06 | 0.0066 |
| ABL2 | SP1 | 0.13 | 1.5E-05 | 0.0151 |
| ABCC1 | CDKN1A | 0.13 | 0.0012 | 6.8E-06 |
| ABL2 | ITGB1 | 0.13 | 3.6E-06 | 0.0072 |
| C1QB | ITGAL | 0.13 | 2.0E-05 | 0.0030 |
| CD97 | SEMA4D | 0.13 | 0.0004 | 2.9E-06 |

| 2-gene models and | | Entropy | | |
|---|---|---|---|---|
| 1-gene models | | R-sq | p-val 1 | p-val 2 |
| CAV2 | ITGAL | 0.13 | 2.1E-05 | 0.0035 |
| CDKN1A | MTA1 | 0.13 | 5.7E-06 | 0.0014 |
| CDKN1A | RBM5 | 0.13 | 2.1E-05 | 0.0014 |
| CREBBP | TIMP1 | 0.13 | 3.5E-05 | 3.2E-05 |
| CDKN1A | CDKN2A | 0.13 | 3.3E-06 | 0.0014 |
| SEMA4D | VIM | 0.13 | 3.3E-06 | 0.0004 |
| ABL2 | TEGT | 0.13 | 3.5E-06 | 0.0093 |
| SEMA4D | STAT3 | 0.13 | 8.9E-06 | 0.0004 |
| SP1 | TIMP1 | 0.13 | 6.3E-05 | 2.0E-05 |
| C1QA | RBM5 | 0.13 | 2.6E-05 | 0.0012 |
| C1QA | JUN | 0.13 | 1.5E-05 | 0.0012 |
| ABL2 | IL1B | 0.12 | 4.9E-06 | 0.0105 |
| ABL2 | ZNF185 | 0.12 | 5.7E-06 | 0.0107 |
| CDKN1A | TNF | 0.12 | 4.7E-06 | 0.0019 |
| C1QB | VEGF | 0.12 | 6.2E-06 | 0.0043 |
| PTPRC | SEMA4D | 0.12 | 0.0005 | 5.2E-06 |
| SRF | TIMP1 | 0.12 | 4.8E-05 | 1.6E-05 |
| C1QA | CASP9 | 0.12 | 6.5E-06 | 0.0013 |
| ABL2 | SKIL | 0.12 | 5.3E-06 | 0.0114 |
| CDKN1A | SRF | 0.12 | 1.6E-05 | 0.0020 |
| ABL2 | PTPRC | 0.12 | 5.4E-06 | 0.0117 |
| C1QB | CASP9 | 0.12 | 6.8E-06 | 0.0048 |
| ABL2 | CD44 | 0.12 | 5.5E-06 | 0.0121 |
| AKT1 | CDKN1A | 0.12 | 0.0022 | 1.6E-05 |
| C1QB | MYC | 0.12 | 3.9E-05 | 0.0051 |
| APC | C1QA | 0.12 | 0.0016 | 1.6E-05 |
| CDKN1A | GNB1 | 0.12 | 1.1E-05 | 0.0023 |
| ABL2 | MTF1 | 0.12 | 1.4E-05 | 0.0134 |
| ABL2 | CTSD | 0.12 | 7.3E-06 | 0.0138 |
| CDKN1A | COVA1 | 0.12 | 7.1E-06 | 0.0024 |
| RBM5 | TEGT | 0.12 | 5.4E-06 | 3.8E-05 |
| C1QB | CTSD | 0.12 | 7.8E-06 | 0.0059 |
| CDKN1A | E2F5 | 0.12 | 1.5E-05 | 0.0026 |
| C1QA | MTA1 | 0.12 | 1.1E-05 | 0.0018 |
| C1QA | NFATC2 | 0.12 | 6.5E-05 | 0.0018 |
| BRAF | C1QB | 0.12 | 0.0062 | 1.2E-05 |
| CDKN1A | MTF1 | 0.12 | 1.6E-05 | 0.0027 |
| CAV2 | CCND2 | 0.12 | 1.9E-05 | 0.0073 |
| BRAF | CAV2 | 0.12 | 0.0076 | 1.3E-05 |
| C1QA | ZNF350 | 0.12 | 1.3E-05 | 0.0019 |
| C1QA | COVA1 | 0.12 | 8.3E-06 | 0.0019 |

| 2-gene models and | | Entropy | | |
|---|---|---|---|---|
| 1-gene models | | R-sq | p-val 1 | p-val 2 |
| C1QA | SOX4 | 0.12 | 9.0E-06 | 0.0020 |
| C1QA | SRF | 0.12 | 2.4E-05 | 0.0021 |
| PTEN | SEMA4D | 0.12 | 0.0008 | 6.7E-06 |
| ABL2 | SMARCD3 | 0.12 | 6.7E-06 | 0.0189 |
| ABL2 | NOTCH2 | 0.12 | 2.0E-05 | 0.0193 |
| CDKN1A | NRP1 | 0.12 | 1.4E-05 | 0.0033 |
| NOTCH2 | TIMP1 | 0.12 | 8.2E-05 | 2.1E-05 |
| CAV2 | SRF | 0.12 | 2.7E-05 | 0.0093 |
| ABL2 | AKT1 | 0.12 | 2.4E-05 | 0.0203 |
| ABL2 | CASP9 | 0.12 | 1.1E-05 | 0.0208 |
| NCOA1 | TIMP1 | 0.12 | 8.4E-05 | 2.4E-05 |
| ABL2 | ACPP | 0.12 | 7.5E-06 | 0.0210 |
| CEBPB | SEMA4D | 0.12 | 0.0010 | 7.8E-06 |
| C1QA | MTF1 | 0.12 | 2.1E-05 | 0.0025 |
| ABL2 | RAF1 | 0.12 | 1.7E-05 | 0.0216 |
| CDKN1A | LGALS8 | 0.12 | 9.7E-06 | 0.0037 |
| EP300 | TIMP1 | 0.12 | 8.9E-05 | 1.8E-05 |
| C1QB | SP1 | 0.12 | 4.2E-05 | 0.0172 |
| C1QA | IRF1 | 0.12 | 1.4E-05 | 0.0026 |
| C1QA | SP1 | 0.12 | 4.4E-05 | 0.0143 |
| ABL2 | CTNNA1 | 0.12 | 8.4E-06 | 0.0240 |
| CDKN1A | JUN | 0.12 | 3.4E-05 | 0.0041 |
| NFKB1 | TIMP1 | 0.12 | 0.0001 | 2.2E-05 |
| ITGAL | TIMP1 | 0.12 | 0.0001 | 6.4E-05 |
| C1QA | VHL | 0.12 | 1.8E-05 | 0.0030 |
| BCL2 | RHOC | 0.12 | 1.1E-05 | 0.0001 |
| CDKN1A | NOTCH2 | 0.12 | 2.7E-05 | 0.0044 |
| CDKN1A | SP1 | 0.12 | 4.9E-05 | 0.0081 |
| ABL2 | DLC1 | 0.12 | 5.4E-05 | 0.0273 |
| CDKN1A | RB1 | 0.12 | 1.4E-05 | 0.0047 |
| C1QB | GNB1 | 0.12 | 2.3E-05 | 0.0109 |
| ABL2 | PTGS2 | 0.12 | 1.5E-05 | 0.0286 |
| C1QB | SRF | 0.12 | 3.8E-05 | 0.0113 |
| ABL2 | SOX4 | 0.12 | 1.5E-05 | 0.0296 |
| MAPK1 | SEMA4D | 0.11 | 0.0014 | 1.1E-05 |
| CCND2 | CDKN1A | 0.11 | 0.0051 | 3.5E-05 |
| CAV2 | GNB1 | 0.11 | 2.5E-05 | 0.0146 |
| ABCC1 | C1QA | 0.11 | 0.0037 | 2.9E-05 |
| C1QB | GSK3B | 0.11 | 3.6E-05 | 0.0125 |
| C1QA | PTCH1 | 0.11 | 7.1E-05 | 0.0038 |
| CAV2 | VHL | 0.11 | 2.2E-05 | 0.0153 |

| 2-gene models and | | Entropy | | |
| 1-gene models | | R-sq | p-val 1 | p-val 2 |
|---|---|---|---|---|
| IQGAP1 | TIMP1 | 0.11 | 0.0001 | 3.4E-05 |
| CDKN1A | CREBBP | 0.11 | 0.0001 | 0.0057 |
| C1QB | G6PD | 0.11 | 1.2E-05 | 0.0135 |
| MTF1 | TIMP1 | 0.11 | 0.0001 | 3.4E-05 |
| C1QA | CREBBP | 0.11 | 0.0001 | 0.0041 |
| C1QB | STAT3 | 0.11 | 3.1E-05 | 0.0139 |
| SEMA4D | SP1 | 0.11 | 6.4E-05 | 0.0022 |
| C1QA | TP53 | 0.11 | 2.1E-05 | 0.0041 |
| ABL2 | SRF | 0.11 | 4.7E-05 | 0.0366 |
| ABL2 | G1P3 | 0.11 | 1.9E-05 | 0.0374 |
| CDKN1A | NRAS | 0.11 | 1.5E-05 | 0.0047 |
| C1QB | SMAD4 | 0.11 | 4.5E-05 | 0.0151 |
| ABL2 | RHOC | 0.11 | 1.5E-05 | 0.0394 |
| ABL2 | RP51077B9.4 | 0.11 | 0.0001 | 0.0394 |
| CAV2 | RBM5 | 0.11 | 9.5E-05 | 0.0181 |
| CDKN1A | SOX4 | 0.11 | 1.9E-05 | 0.0065 |
| C1QB | USP7 | 0.11 | 1.6E-05 | 0.0162 |
| ABL2 | TNF | 0.11 | 1.6E-05 | 0.0426 |
| BCAM | CAV2 | 0.11 | 0.0194 | 0.0002 |
| C1QA | TOPBP1 | 0.11 | 2.2E-05 | 0.0048 |
| C1QB | IFI16 | 0.11 | 3.4E-05 | 0.0165 |
| C1QA | SRC | 0.11 | 2.1E-05 | 0.0048 |
| C1QA | HMGA1 | 0.11 | 3.7E-05 | 0.0049 |
| BCAM | C1QA | 0.11 | 0.0050 | 0.0002 |
| APC | C1QB | 0.11 | 0.0171 | 5.0E-05 |
| TIMP1 | VHL | 0.11 | 3.0E-05 | 0.0002 |
| ABL2 | FGF2 | 0.11 | 0.0005 | 0.0377 |
| BCL2 | C1QA | 0.11 | 0.0053 | 0.0002 |
| C1QB | EPAS1 | 0.11 | 2.8E-05 | 0.0184 |
| CAV2 | CDK2 | 0.11 | 2.5E-05 | 0.0214 |
| ABL2 | GNB1 | 0.11 | 3.6E-05 | 0.0479 |
| C1QA | HRAS | 0.11 | 3.8E-05 | 0.0053 |
| C1QB | FOS | 0.11 | 2.1E-05 | 0.0185 |
| AKT1 | C1QA | 0.11 | 0.0054 | 5.3E-05 |
| C1QA | XRCC1 | 0.11 | 3.0E-05 | 0.0055 |
| C1QA | CAV2 | 0.11 | 0.0222 | 0.0055 |
| C1QB | ICAM1 | 0.11 | 6.4E-05 | 0.0193 |
| CAV2 | IQGAP1 | 0.11 | 4.8E-05 | 0.0226 |
| C1QA | NAB1 | 0.11 | 3.4E-05 | 0.0057 |
| CDKN1A | HRAS | 0.11 | 4.2E-05 | 0.0086 |
| C1QB | MAPK1 | 0.11 | 1.7E-05 | 0.0204 |

| 2-gene models and | | Entropy | | |
|---|---|---|---|---|
| 1-gene models | | R-sq | p-val 1 | p-val 2 |
| MME | SEMA4D | 0.11 | 0.0023 | 1.7E-05 |
| BRAF | CDKN1A | 0.11 | 0.0088 | 3.8E-05 |
| CAV2 | CREBBP | 0.11 | 0.0002 | 0.0248 |
| CDKN1A | TOPBP1 | 0.11 | 2.9E-05 | 0.0091 |
| CAV2 | MAP2K1 | 0.11 | 3.3E-05 | 0.0262 |
| CAV2 | MYC | 0.11 | 0.0002 | 0.0265 |
| CDKN1A | NCOA1 | 0.11 | 6.1E-05 | 0.0096 |
| CAV2 | EPAS1 | 0.11 | 3.4E-05 | 0.0271 |
| CAV2 | SP1 | 0.11 | 9.8E-05 | 0.0331 |
| SEMA4D | TXNRD1 | 0.11 | 2.0E-05 | 0.0026 |
| CAV2 | SMAD4 | 0.11 | 6.7E-05 | 0.0276 |
| BCAM | CDKN1A | 0.11 | 0.0099 | 0.0003 |
| CDKN1A | IGFBP3 | 0.11 | 3.7E-05 | 0.0101 |
| CDKN1A | NAB1 | 0.11 | 4.1E-05 | 0.0101 |
| CAV2 | NFATC2 | 0.11 | 0.0002 | 0.0283 |
| C1QB | CEACAM1 | 0.11 | 2.0E-05 | 0.0245 |
| RBM5 | VIM | 0.11 | 2.1E-05 | 0.0001 |
| CAV2 | JUN | 0.11 | 8.4E-05 | 0.0296 |
| CAV2 | USP7 | 0.11 | 2.4E-05 | 0.0298 |
| CAV2 | RB1 | 0.11 | 3.0E-05 | 0.0298 |
| C1QA | NOTCH2 | 0.11 | 6.2E-05 | 0.0073 |
| G6PD | SEMA4D | 0.11 | 0.0029 | 2.2E-05 |
| CD44 | CDKN1A | 0.11 | 0.0110 | 2.7E-05 |
| CFLAR | SEMA4D | 0.11 | 0.0029 | 3.1E-05 |
| C1QB | IQGAP1 | 0.11 | 6.4E-05 | 0.0263 |
| C1QB | VHL | 0.11 | 4.2E-05 | 0.0264 |
| ALOX5 | C1QB | 0.11 | 0.0267 | 3.2E-05 |
| CAV2 | MTF1 | 0.11 | 6.2E-05 | 0.0313 |
| CDK5 | CDKN1A | 0.11 | 0.0112 | 2.2E-05 |
| C1QA | MAP2K1 | 0.11 | 3.9E-05 | 0.0077 |
| CAV2 | EP300 | 0.11 | 5.1E-05 | 0.0324 |
| ABCC1 | CAV2 | 0.11 | 0.0327 | 5.9E-05 |
| C1QA | RB1 | 0.11 | 3.2E-05 | 0.0080 |
| CAV2 | LGALS8 | 0.11 | 2.9E-05 | 0.0327 |
| C1QB | XRCC1 | 0.11 | 4.3E-05 | 0.0280 |
| CASP9 | CDKN1A | 0.11 | 0.0118 | 3.6E-05 |
| SEMA4D | ZNF185 | 0.11 | 3.3E-05 | 0.0031 |
| C1QB | TOPBP1 | 0.11 | 3.7E-05 | 0.0282 |
| C1QB | IGF1R | 0.11 | 4.7E-05 | 0.0282 |
| C1QB | IRF1 | 0.11 | 4.2E-05 | 0.0299 |
| CAV2 | VEGF | 0.11 | 3.8E-05 | 0.0357 |

| 2-gene models and | | Entropy | | |
|---|---|---|---|---|
| 1-gene models | | R-sq | p-val 1 | p-val 2 |
| C1QB | CDKN1A | 0.11 | 0.0127 | 0.0306 |
| C1QB | NCOA1 | 0.11 | 8.2E-05 | 0.0314 |
| C1QA | RAF1 | 0.11 | 5.8E-05 | 0.0090 |
| MAP2K1 | TIMP1 | 0.11 | 0.0003 | 4.6E-05 |
| C1QA | KAI1 | 0.11 | 0.0001 | 0.0090 |
| C1QB | HSPA1A | 0.11 | 8.5E-05 | 0.0320 |
| TGFB1 | TIMP1 | 0.11 | 0.0003 | 2.8E-05 |
| C1QB | CAV2 | 0.11 | 0.0384 | 0.0326 |
| ABL1 | CAV2 | 0.11 | 0.0387 | 9.1E-05 |
| C1QA | GNB1 | 0.10 | 6.3E-05 | 0.0094 |
| C1QB | RB1 | 0.10 | 3.8E-05 | 0.0333 |
| C1QA | NFKB1 | 0.10 | 6.9E-05 | 0.0095 |
| C1QB | NOTCH2 | 0.10 | 8.0E-05 | 0.0338 |
| CAV2 | XRCC1 | 0.10 | 5.1E-05 | 0.0395 |
| CAV2 | E2F5 | 0.10 | 7.8E-05 | 0.0410 |
| CAV2 | ICAM1 | 0.10 | 0.0001 | 0.0435 |
| CREBBP | S100A11 | 0.10 | 3.0E-05 | 0.0003 |
| CAV2 | SERPING1 | 0.10 | 4.2E-05 | 0.0444 |
| FAS | SEMA4D | 0.10 | 0.0041 | 3.1E-05 |
| ICAM1 | TIMP1 | 0.10 | 0.0004 | 0.0001 |
| CDKN1A | EP300 | 0.10 | 6.8E-05 | 0.0157 |
| CAV2 | IRF1 | 0.10 | 5.3E-05 | 0.0449 |
| CAV2 | SMAD3 | 0.10 | 0.0001 | 0.0453 |
| C1QB | RAF1 | 0.10 | 7.0E-05 | 0.0389 |
| C1QA | GSK3B | 0.10 | 0.0001 | 0.0111 |
| AKT1 | CAV2 | 0.10 | 0.0470 | 0.0001 |
| CAV2 | IFI16 | 0.10 | 7.5E-05 | 0.0466 |
| CAV2 | NCOA1 | 0.10 | 0.0001 | 0.0469 |
| PYCARD | RBM5 | 0.10 | 0.0002 | 3.2E-05 |
| C1QB | CCND2 | 0.10 | 0.0001 | 0.0404 |
| CDKN1A | ZNF350 | 0.10 | 7.3E-05 | 0.0173 |
| PDGFA | SEMA4D | 0.10 | 0.0046 | 8.6E-05 |
| SEMA4D | SPARC | 0.10 | 0.0003 | 0.0049 |
| CDKN1A | IFI16 | 0.10 | 8.6E-05 | 0.0190 |
| CDKN1A | IQGAP1 | 0.10 | 0.0001 | 0.0192 |
| C1QB | SVIL | 0.10 | 5.5E-05 | 0.0466 |
| RAF1 | SEMA4D | 0.10 | 0.0050 | 8.4E-05 |
| IQGAP1 | SEMA4D | 0.10 | 0.0051 | 0.0001 |
| CDKN1A | COL6A2 | 0.10 | 9.1E-05 | 0.0197 |
| CDKN1A | VEGF | 0.10 | 5.8E-05 | 0.0198 |
| C1QA | XK | 0.10 | 0.0013 | 0.0141 |

| 2-gene models and | | Entropy | | |
| 1-gene models | | R-sq | p-val 1 | p-val 2 |
|---|---|---|---|---|
| CDKN1A | USP7 | 0.10 | 4.4E-05 | 0.0208 |
| CDKN1A | PTGS2 | 0.10 | 6.0E-05 | 0.0208 |
| NCOA1 | SEMA4D | 0.10 | 0.0055 | 0.0001 |
| RHOA | TIMP1 | 0.10 | 0.0005 | 4.3E-05 |
| C1QB | FGF2 | 0.10 | 0.0013 | 0.0436 |
| MYC | TIMP1 | 0.10 | 0.0005 | 0.0004 |
| MEIS1 | SEMA4D | 0.10 | 0.0061 | 0.0002 |
| CDKN2D | SEMA4D | 0.10 | 0.0063 | 0.0001 |
| CDKN1A | DAD1 | 0.10 | 4.7E-05 | 0.0245 |
| C1QA | PTGS2 | 0.10 | 7.0E-05 | 0.0168 |
| ITGAL | VIM | 0.10 | 4.6E-05 | 0.0003 |
| C1QA | VEGF | 0.10 | 7.3E-05 | 0.0172 |
| C1QA | SERPING1 | 0.10 | 6.6E-05 | 0.0174 |
| C1QA | NRP1 | 0.10 | 9.7E-05 | 0.0175 |
| CDKN1A | GSK3B | 0.10 | 0.0002 | 0.0273 |
| APAF1 | C1QA | 0.10 | 0.0191 | 8.3E-05 |
| CDKN1A | RAF1 | 0.10 | 0.0001 | 0.0280 |
| C1QA | CDK2 | 0.10 | 8.6E-05 | 0.0195 |
| SEMA4D | THBS1 | 0.10 | 0.0001 | 0.0076 |
| PTGS2 | SEMA4D | 0.10 | 0.0077 | 8.3E-05 |
| C1QA | HSPA1A | 0.10 | 0.0002 | 0.0203 |
| BRCA1 | C1QA | 0.10 | 0.0215 | 9.6E-05 |
| AKT1 | TIMP1 | 0.10 | 0.0007 | 0.0002 |
| CDKN1A | SERPING1 | 0.10 | 8.2E-05 | 0.0322 |
| TEGT | TIMP1 | 0.10 | 0.0007 | 6.1E-05 |
| CA4 | SEMA4D | 0.10 | 0.0085 | 8.4E-05 |
| C1QA | CTSD | 0.10 | 8.7E-05 | 0.0225 |
| CDKN1A | VIM | 0.10 | 6.1E-05 | 0.0341 |
| CDKN1A | NR4A2 | 0.10 | 0.0001 | 0.0343 |
| RP51077B9.4 | SEMA4D | 0.10 | 0.0091 | 0.0005 |
| CDKN1A | ZNF185 | 0.10 | 9.2E-05 | 0.0355 |
| CDKN1A | KAI1 | 0.10 | 0.0004 | 0.0356 |
| C1QA | SOCS1 | 0.10 | 0.0004 | 0.0245 |
| CDKN1A | HSPA1A | 0.10 | 0.0002 | 0.0370 |
| C1QA | STAT3 | 0.10 | 0.0002 | 0.0256 |
| GSK3B | TIMP1 | 0.10 | 0.0008 | 0.0002 |
| CASP9 | TIMP1 | 0.10 | 0.0008 | 0.0001 |
| CDKN1A | STAT3 | 0.10 | 0.0002 | 0.0393 |
| C1QA | LGALS8 | 0.09 | 9.0E-05 | 0.0274 |
| CDKN1A | RHOA | 0.09 | 7.6E-05 | 0.0411 |
| CDKN1A | MYD88 | 0.09 | 8.4E-05 | 0.0424 |

| 2-gene models and | | Entropy | | |
|---|---|---|---|---|
| 1-gene models | | R-sq | p-val 1 | p-val 2 |
| CDKN1A | PTPRC | 0.09 | 9.4E-05 | 0.0426 |
| CDKN1A | ITGA1 | 0.09 | 0.0001 | 0.0287 |
| CDKN1A | CTSD | 0.09 | 0.0001 | 0.0432 |
| CDKN1A | ERBB2 | 0.09 | 0.0002 | 0.0439 |
| C1QA | COL6A2 | 0.09 | 0.0002 | 0.0301 |
| SEMA4D | SERPINE1 | 0.09 | 0.0003 | 0.0114 |
| C1QA | NRAS | 0.09 | 9.5E-05 | 0.0223 |
| HSPA1A | SEMA4D | 0.09 | 0.0118 | 0.0003 |
| IL1B | SEMA4D | 0.09 | 0.0123 | 0.0001 |
| CDKN1A | XK | 0.09 | 0.0028 | 0.0488 |
| CDKN1A | TEGT | 0.09 | 8.6E-05 | 0.0488 |
| CDKN1A | IL1B | 0.09 | 0.0001 | 0.0492 |
| CDK5 | ITGAL | 0.09 | 0.0007 | 8.9E-05 |
| C1QA | GSTT1 | 0.09 | 0.0002 | 0.0345 |
| RAF1 | TIMP1 | 0.09 | 0.0011 | 0.0002 |
| C1QA | S100A6 | 0.09 | 9.8E-05 | 0.0346 |
| CDK5 | MYC | 0.09 | 0.0008 | 9.1E-05 |
| C1QA | SIAH2 | 0.09 | 0.0006 | 0.0360 |
| DLC1 | SEMA4D | 0.09 | 0.0136 | 0.0005 |
| C1QA | SKIL | 0.09 | 0.0001 | 0.0369 |
| C1QA | TGFB1 | 0.09 | 0.0001 | 0.0373 |
| C1QA | NUDT4 | 0.09 | 0.0013 | 0.0373 |
| C1QA | CDH1 | 0.09 | 0.0002 | 0.0392 |
| RP51077B9.4 | SOCS1 | 0.09 | 0.0007 | 0.0008 |
| C1QA | EP300 | 0.09 | 0.0002 | 0.0410 |
| CREBBP | MYD88 | 0.09 | 0.0001 | 0.0012 |
| C1QA | TNF | 0.09 | 0.0001 | 0.0419 |
| HSPA1A | TIMP1 | 0.09 | 0.0013 | 0.0004 |
| C1QA | NCOA1 | 0.09 | 0.0004 | 0.0442 |
| AOC3 | SEMA4D | 0.09 | 0.0167 | 0.0001 |
| PTPRC | TIMP1 | 0.09 | 0.0014 | 0.0001 |
| C1QA | IFI16 | 0.09 | 0.0003 | 0.0451 |
| ABL1 | RHOC | 0.09 | 0.0001 | 0.0004 |
| C1QA | CTNNA1 | 0.09 | 0.0001 | 0.0471 |
| C1QA | IQGAP1 | 0.09 | 0.0004 | 0.0473 |
| ITGB1 | NFATC2 | 0.09 | 0.0015 | 0.0002 |
| PTGS2 | TIMP1 | 0.09 | 0.0014 | 0.0002 |
| ABL1 | CDK5 | 0.09 | 0.0001 | 0.0004 |
| CREBBP | SERPINA1 | 0.09 | 0.0001 | 0.0013 |
| ABL2 | | 0.09 | 0.0003 | |
| BRAF | XK | 0.09 | 0.0045 | 0.0003 |

| 2-gene models and | | Entropy | | |
| --- | --- | --- | --- | --- |
| 1-gene models | | R-sq | p-val 1 | p-val 2 |
| C1QA | FGF2 | 0.09 | 0.0042 | 0.0436 |
| SORBS1 | XK | 0.09 | 0.0045 | 0.0002 |
| FOS | SEMA4D | 0.09 | 0.0198 | 0.0002 |
| BRAF | TIMP1 | 0.09 | 0.0016 | 0.0003 |
| IGF1R | SEMA4D | 0.09 | 0.0205 | 0.0003 |
| FAS | ITGAL | 0.09 | 0.0011 | 0.0001 |
| NFATC2 | TNF | 0.09 | 0.0002 | 0.0018 |
| CTSD | TIMP1 | 0.09 | 0.0018 | 0.0002 |
| HMGA1 | TIMP1 | 0.09 | 0.0018 | 0.0004 |
| ITGAL | PYCARD | 0.09 | 0.0002 | 0.0012 |
| ITGAL | ST14 | 0.09 | 0.0002 | 0.0012 |
| APAF1 | SEMA4D | 0.09 | 0.0240 | 0.0003 |
| FAS | RBM5 | 0.09 | 0.0012 | 0.0002 |
| JUN | TIMP1 | 0.09 | 0.0020 | 0.0007 |
| RBM5 | RHOA | 0.09 | 0.0002 | 0.0013 |
| CDK5 | SMAD3 | 0.09 | 0.0007 | 0.0002 |
| ABL1 | TIMP1 | 0.09 | 0.0022 | 0.0006 |
| TIMP1 | TOPBP1 | 0.09 | 0.0003 | 0.0022 |
| BCL2 | NME1 | 0.09 | 0.0002 | 0.0025 |
| E2F1 | SEMA4D | 0.08 | 0.0297 | 0.0018 |
| ACPP | SEMA4D | 0.08 | 0.0305 | 0.0002 |
| RB1 | TIMP1 | 0.08 | 0.0024 | 0.0003 |
| NFATC2 | NME1 | 0.08 | 0.0002 | 0.0025 |
| FGF2 | SEMA4D | 0.08 | 0.0272 | 0.0067 |
| NOTCH2 | SEMA4D | 0.08 | 0.0322 | 0.0006 |
| GSK3B | SEMA4D | 0.08 | 0.0328 | 0.0007 |
| ADAM17 | SEMA4D | 0.08 | 0.0334 | 0.0003 |
| IFI16 | SEMA4D | 0.08 | 0.0337 | 0.0005 |
| SMAD3 | TIMP1 | 0.08 | 0.0026 | 0.0009 |
| CREBBP | SEMA4D | 0.08 | 0.0346 | 0.0025 |
| RBM5 | TXNRD1 | 0.08 | 0.0002 | 0.0017 |
| PLXDC2 | SEMA4D | 0.08 | 0.0360 | 0.0004 |
| IRF1 | TIMP1 | 0.08 | 0.0029 | 0.0004 |
| ABCC1 | TIMP1 | 0.08 | 0.0029 | 0.0006 |
| MTF1 | SEMA4D | 0.08 | 0.0376 | 0.0007 |
| PLAU | SEMA4D | 0.08 | 0.0400 | 0.0004 |
| CDK2 | TIMP1 | 0.08 | 0.0030 | 0.0004 |
| EP300 | SEMA4D | 0.08 | 0.0389 | 0.0006 |
| CAV2 | | 0.08 | 0.0008 | |
| CDK5 | NFATC2 | 0.08 | 0.0032 | 0.0003 |
| ANLN | XK | 0.08 | 0.0090 | 0.0003 |

| 2-gene models and | | Entropy | | |
|---|---|---|---|---|
| 1-gene models | | R-sq | p-val 1 | p-val 2 |
| NFATC2 | TIMP1 | 0.08 | 0.0033 | 0.0034 |
| BRCA1 | TIMP1 | 0.08 | 0.0033 | 0.0004 |
| CREBBP | RHOA | 0.08 | 0.0003 | 0.0031 |
| STAT3 | TIMP1 | 0.08 | 0.0034 | 0.0007 |
| MAPK14 | SEMA4D | 0.08 | 0.0451 | 0.0003 |
| GNB1 | SEMA4D | 0.08 | 0.0457 | 0.0007 |
| PTCH1 | RHOC | 0.08 | 0.0003 | 0.0019 |
| CREBBP | TEGT | 0.08 | 0.0003 | 0.0032 |
| C1QB | | 0.08 | 0.0006 | |
| ITGAL | ITGB1 | 0.08 | 0.0004 | 0.0023 |
| BCL2 | TNF | 0.08 | 0.0004 | 0.0041 |
| CASP9 | SEMA4D | 0.08 | 0.0494 | 0.0005 |
| SEMA4D | XK | 0.08 | 0.0109 | 0.0497 |
| BCAM | FGF2 | 0.08 | 0.0107 | 0.0082 |
| FGF2 | NFATC2 | 0.08 | 0.0036 | 0.0108 |
| ITGA1 | SEMA4D | 0.08 | 0.0395 | 0.0004 |
| ABL1 | ITGB1 | 0.08 | 0.0004 | 0.0012 |
| CDKN2D | CREBBP | 0.08 | 0.0038 | 0.0008 |
| SMAD4 | VIM | 0.08 | 0.0003 | 0.0012 |
| RHOC | SMAD3 | 0.08 | 0.0014 | 0.0004 |
| MYD88 | TIMP1 | 0.08 | 0.0047 | 0.0004 |
| CREBBP | RP51077B9.4 | 0.08 | 0.0031 | 0.0044 |
| LGALS8 | TIMP1 | 0.08 | 0.0048 | 0.0005 |
| CCND2 | TIMP1 | 0.08 | 0.0049 | 0.0014 |
| CREBBP | MAPK1 | 0.08 | 0.0004 | 0.0045 |
| CDKN2A | NFATC2 | 0.08 | 0.0051 | 0.0004 |
| MYC | NRAS | 0.08 | 0.0005 | 0.0069 |
| RBM5 | RP51077B9.4 | 0.08 | 0.0035 | 0.0032 |
| CREBBP | IFITM1 | 0.08 | 0.0004 | 0.0049 |
| CDK5 | VHL | 0.08 | 0.0009 | 0.0004 |
| CREBBP | PTEN | 0.08 | 0.0005 | 0.0051 |
| BCAM | TIMP1 | 0.08 | 0.0055 | 0.0074 |
| APC | TIMP1 | 0.08 | 0.0057 | 0.0016 |
| SOCS1 | XK | 0.08 | 0.0167 | 0.0030 |
| FGF2 | ITGAL | 0.08 | 0.0032 | 0.0158 |
| FGF2 | MYC | 0.08 | 0.0035 | 0.0159 |
| CD59 | SOCS1 | 0.08 | 0.0030 | 0.0020 |
| CREBBP | PYCARD | 0.08 | 0.0005 | 0.0057 |
| MYC | RP51077B9.4 | 0.08 | 0.0041 | 0.0045 |
| ITGAL | RP51077B9.4 | 0.07 | 0.0042 | 0.0040 |
| ITGAL | RHOC | 0.07 | 0.0006 | 0.0042 |

| 2-gene models and | | Entropy | | |
| --- | --- | --- | --- | --- |
| 1-gene models | | R-sq | p-val 1 | p-val 2 |
| IGF2BP2 | XK | 0.07 | 0.0199 | 0.0013 |
| IFI16 | TIMP1 | 0.07 | 0.0068 | 0.0013 |
| PLXDC2 | TIMP1 | 0.07 | 0.0070 | 0.0010 |
| TIMP1 | TXNRD1 | 0.07 | 0.0006 | 0.0070 |
| BCL2 | TIMP1 | 0.07 | 0.0070 | 0.0078 |
| CD44 | TIMP1 | 0.07 | 0.0073 | 0.0007 |
| EPAS1 | TIMP1 | 0.07 | 0.0074 | 0.0011 |
| BRAF | RP51077B9.4 | 0.07 | 0.0052 | 0.0014 |
| BCL2 | FGF2 | 0.07 | 0.0219 | 0.0073 |
| BCL2 | CDK5 | 0.07 | 0.0006 | 0.0089 |
| CREBBP | SPARC | 0.07 | 0.0056 | 0.0075 |
| ELA2 | FGF2 | 0.07 | 0.0228 | 0.0105 |
| CDKN1A | | 0.07 | 0.0063 | |
| BRAF | CD59 | 0.07 | 0.0030 | 0.0015 |
| KAI1 | TIMP1 | 0.07 | 0.0086 | 0.0040 |
| SRF | VIM | 0.07 | 0.0007 | 0.0028 |
| TIMP1 | VIM | 0.07 | 0.0007 | 0.0088 |
| BCL2 | ITGB1 | 0.07 | 0.0009 | 0.0099 |
| SVIL | TIMP1 | 0.07 | 0.0092 | 0.0011 |
| BRAF | SPARC | 0.07 | 0.0062 | 0.0016 |
| FGF2 | JUN | 0.07 | 0.0030 | 0.0258 |
| FGF2 | PTCH1 | 0.07 | 0.0051 | 0.0276 |
| NR4A2 | TIMP1 | 0.07 | 0.0102 | 0.0014 |
| BRAF | NUDT4 | 0.07 | 0.0121 | 0.0018 |
| BRAF | DLC1 | 0.07 | 0.0053 | 0.0019 |
| MYC | ST14 | 0.07 | 0.0010 | 0.0080 |
| BRAF | NCOA4 | 0.07 | 0.0078 | 0.0020 |
| CREBBP | THBS1 | 0.07 | 0.0017 | 0.0104 |
| HRAS | NME1 | 0.07 | 0.0009 | 0.0022 |
| E2F5 | FGF2 | 0.07 | 0.0332 | 0.0025 |
| RHOC | TP53 | 0.07 | 0.0016 | 0.0011 |
| ELA2 | SOCS1 | 0.07 | 0.0062 | 0.0081 |
| SMAD3 | TNF | 0.07 | 0.0011 | 0.0039 |
| CREBBP | DLC1 | 0.07 | 0.0058 | 0.0111 |
| C1QA | | 0.07 | 0.0015 | |
| ACPP | ITGAL | 0.07 | 0.0081 | 0.0010 |
| ITGB1 | PTCH1 | 0.07 | 0.0068 | 0.0013 |
| CDH1 | XK | 0.07 | 0.0398 | 0.0026 |
| TIMP1 | ZNF350 | 0.07 | 0.0023 | 0.0132 |
| SOX4 | TIMP1 | 0.07 | 0.0134 | 0.0016 |
| FGF2 | SMAD3 | 0.07 | 0.0037 | 0.0377 |

| 2-gene models and 1-gene models | | Entropy R-sq | p-val 1 | p-val 2 |
|---|---|---|---|---|
| MYD88 | SP1 | 0.07 | 0.0045 | 0.0014 |
| G6PD | TIMP1 | 0.07 | 0.0140 | 0.0011 |
| ABCC1 | FGF2 | 0.07 | 0.0405 | 0.0028 |
| BRAF | E2F1 | 0.07 | 0.0108 | 0.0025 |
| MTA1 | TIMP1 | 0.07 | 0.0148 | 0.0023 |
| CREBBP | MEIS1 | 0.07 | 0.0062 | 0.0138 |
| DLC1 | MYC | 0.07 | 0.0107 | 0.0072 |
| FGF2 | XK | 0.07 | 0.0484 | 0.0425 |
| MTF1 | RP51077B9.4 | 0.07 | 0.0100 | 0.0035 |
| CREBBP | VIM | 0.07 | 0.0012 | 0.0142 |
| BCAM | ITGB1 | 0.07 | 0.0016 | 0.0208 |
| UBE2C | XK | 0.07 | 0.0472 | 0.0012 |
| TIMP1 | VEGF | 0.07 | 0.0019 | 0.0157 |
| DLC1 | SOCS1 | 0.07 | 0.0081 | 0.0075 |
| CREBBP | ZNF185 | 0.07 | 0.0018 | 0.0146 |
| NFATC2 | TP53 | 0.07 | 0.0022 | 0.0166 |
| CREBBP | PDGFA | 0.07 | 0.0033 | 0.0150 |
| BRAF | ELA2 | 0.07 | 0.0111 | 0.0028 |
| ITGB1 | JUN | 0.07 | 0.0054 | 0.0017 |
| DLC1 | RBM5 | 0.07 | 0.0100 | 0.0079 |
| TIMP1 | USP7 | 0.07 | 0.0015 | 0.0169 |
| EP300 | RP51077B9.4 | 0.07 | 0.0112 | 0.0031 |
| DAD1 | NFATC2 | 0.07 | 0.0182 | 0.0014 |
| BRAF | MEIS1 | 0.07 | 0.0073 | 0.0030 |
| AOC3 | TIMP1 | 0.06 | 0.0181 | 0.0016 |
| NAB1 | TIMP1 | 0.06 | 0.0180 | 0.0029 |
| VIM | XRCC1 | 0.06 | 0.0027 | 0.0014 |
| TIMP1 | TP53 | 0.06 | 0.0025 | 0.0185 |
| ITGAL | SPARC | 0.06 | 0.0130 | 0.0119 |
| NUDT4 | SOCS1 | 0.06 | 0.0098 | 0.0227 |
| CREBBP | MNDA | 0.06 | 0.0015 | 0.0179 |
| SOCS1 | TIMP1 | 0.06 | 0.0195 | 0.0100 |
| ITGB1 | SMAD4 | 0.06 | 0.0054 | 0.0020 |
| PTCH1 | TIMP1 | 0.06 | 0.0198 | 0.0103 |
| RP51077B9.4 | SRF | 0.06 | 0.0062 | 0.0130 |
| NFATC2 | PYCARD | 0.06 | 0.0015 | 0.0207 |
| BCL2 | DAD1 | 0.06 | 0.0016 | 0.0224 |
| MTF1 | VIM | 0.06 | 0.0016 | 0.0046 |
| CD97 | CREBBP | 0.06 | 0.0191 | 0.0016 |
| BRAF | SIAH2 | 0.06 | 0.0111 | 0.0035 |
| MAPK1 | TIMP1 | 0.06 | 0.0209 | 0.0016 |

| 2-gene models and | | Entropy | | |
|---|---|---|---|---|
| 1-gene models | | R-sq | p-val 1 | p-val 2 |
| ALOX5 | TIMP1 | 0.06 | 0.0212 | 0.0024 |
| APAF1 | TIMP1 | 0.06 | 0.0211 | 0.0026 |
| SRF | TEGT | 0.06 | 0.0016 | 0.0066 |
| RBM5 | SPARC | 0.06 | 0.0145 | 0.0129 |
| MYC | RHOC | 0.06 | 0.0019 | 0.0153 |
| DAD1 | MYC | 0.06 | 0.0156 | 0.0017 |
| IGF1R | TIMP1 | 0.06 | 0.0221 | 0.0035 |
| BCAM | SPARC | 0.06 | 0.0148 | 0.0299 |
| NFATC2 | ST14 | 0.06 | 0.0020 | 0.0229 |
| CDC25A | SOCS1 | 0.06 | 0.0115 | 0.0031 |
| NOTCH2 | PYCARD | 0.06 | 0.0017 | 0.0052 |
| CREBBP | G6PD | 0.06 | 0.0018 | 0.0216 |
| BCAM | PDGFA | 0.06 | 0.0047 | 0.0321 |
| COVA1 | TIMP1 | 0.06 | 0.0237 | 0.0025 |
| FAS | NFATC2 | 0.06 | 0.0253 | 0.0019 |
| FAS | SRF | 0.06 | 0.0076 | 0.0019 |
| RP51077B9.4 | XRCC1 | 0.06 | 0.0037 | 0.0162 |
| ITGAL | TEGT | 0.06 | 0.0019 | 0.0155 |
| NCOA1 | SPARC | 0.06 | 0.0172 | 0.0065 |
| MEIS1 | RBM5 | 0.06 | 0.0154 | 0.0106 |
| ITGB1 | SMAD3 | 0.06 | 0.0083 | 0.0026 |
| CREBBP | SVIL | 0.06 | 0.0030 | 0.0241 |
| CREBBP | PTPRC | 0.06 | 0.0025 | 0.0244 |
| JUN | ST14 | 0.06 | 0.0024 | 0.0087 |
| CREBBP | SERPINE1 | 0.06 | 0.0073 | 0.0251 |
| SIAH2 | SOCS1 | 0.06 | 0.0141 | 0.0147 |
| RP51077B9.4 | SMAD4 | 0.06 | 0.0076 | 0.0181 |
| SERPINA1 | TIMP1 | 0.06 | 0.0285 | 0.0021 |
| CREBBP | PLAU | 0.06 | 0.0036 | 0.0271 |
| NFATC2 | SRC | 0.06 | 0.0032 | 0.0299 |
| DAD1 | ITGAL | 0.06 | 0.0182 | 0.0023 |
| NCOA1 | S100A11 | 0.06 | 0.0023 | 0.0077 |
| E2F1 | SERPING1 | 0.06 | 0.0032 | 0.0225 |
| BCL2 | CDKN2A | 0.06 | 0.0024 | 0.0338 |
| MNDA | RBM5 | 0.06 | 0.0185 | 0.0023 |
| CDK5 | TP53 | 0.06 | 0.0041 | 0.0023 |
| ABL1 | NME1 | 0.06 | 0.0024 | 0.0084 |
| E2F1 | SOCS1 | 0.06 | 0.0158 | 0.0231 |
| BCL2 | RP51077B9.4 | 0.06 | 0.0200 | 0.0347 |
| ITGAL | MEIS1 | 0.06 | 0.0128 | 0.0193 |
| MYC | NME1 | 0.06 | 0.0024 | 0.0222 |

| 2-gene models and | | Entropy | | |
|---|---|---|---|---|
| 1-gene models | | R-sq | p-val 1 | p-val 2 |
| GNB1 | RP51077B9.4 | 0.06 | 0.0207 | 0.0058 |
| SRC | TIMP1 | 0.06 | 0.0324 | 0.0036 |
| E2F5 | TIMP1 | 0.06 | 0.0325 | 0.0070 |
| ETS2 | TIMP1 | 0.06 | 0.0329 | 0.0041 |
| IQGAP1 | S100A11 | 0.06 | 0.0024 | 0.0075 |
| SP1 | TEGT | 0.06 | 0.0028 | 0.0100 |
| DLC1 | EP300 | 0.06 | 0.0056 | 0.0153 |
| FOS | TIMP1 | 0.06 | 0.0331 | 0.0034 |
| EP300 | SPARC | 0.06 | 0.0222 | 0.0057 |
| BCAM | ZNF185 | 0.06 | 0.0036 | 0.0451 |
| SEMA4D | | 0.06 | 0.0034 | |
| PDGFA | RBM5 | 0.06 | 0.0200 | 0.0066 |
| DLC1 | ITGAL | 0.06 | 0.0204 | 0.0156 |
| ITGB1 | MYC | 0.06 | 0.0239 | 0.0033 |
| ABCC1 | CDK5 | 0.06 | 0.0025 | 0.0068 |
| AKT1 | PYCARD | 0.06 | 0.0025 | 0.0090 |
| NFATC2 | RP51077B9.4 | 0.06 | 0.0220 | 0.0354 |
| SP1 | SPARC | 0.06 | 0.0207 | 0.0106 |
| DLC1 | SRF | 0.06 | 0.0105 | 0.0161 |
| BCAM | THBS1 | 0.06 | 0.0049 | 0.0475 |
| NCOA4 | SOCS1 | 0.06 | 0.0177 | 0.0238 |
| S100A11 | TIMP1 | 0.06 | 0.0352 | 0.0026 |
| EPAS1 | SPARC | 0.06 | 0.0238 | 0.0048 |
| MTF1 | PYCARD | 0.06 | 0.0026 | 0.0078 |
| ANLN | ELA2 | 0.06 | 0.0243 | 0.0027 |
| CDK5 | MAP2K1 | 0.06 | 0.0049 | 0.0027 |
| ELA2 | RBM5 | 0.06 | 0.0219 | 0.0247 |
| NRAS | TIMP1 | 0.06 | 0.0299 | 0.0032 |
| IQGAP1 | SPARC | 0.06 | 0.0250 | 0.0085 |
| RBM5 | ZNF185 | 0.06 | 0.0040 | 0.0220 |
| CREBBP | E2F1 | 0.06 | 0.0276 | 0.0343 |
| GNB1 | SPARC | 0.06 | 0.0250 | 0.0067 |
| MEIS1 | SMAD4 | 0.06 | 0.0102 | 0.0153 |
| CA4 | CREBBP | 0.06 | 0.0349 | 0.0039 |
| SMAD4 | TEGT | 0.06 | 0.0028 | 0.0102 |
| SPARC | SRF | 0.06 | 0.0116 | 0.0255 |
| CD59 | UBE2C | 0.06 | 0.0028 | 0.0128 |
| RP51077B9.4 | SP1 | 0.06 | 0.0116 | 0.0228 |
| CD59 | CREBBP | 0.06 | 0.0357 | 0.0130 |
| RP51077B9.4 | VHL | 0.06 | 0.0058 | 0.0253 |
| ITGAL | TNF | 0.06 | 0.0034 | 0.0245 |

| 2-gene models and | | Entropy | | |
|---|---|---|---|---|
| 1-gene models | | R-sq | p-val 1 | p-val 2 |
| E2F1 | ITGAL | 0.06 | 0.0244 | 0.0297 |
| ITGB1 | RBM5 | 0.06 | 0.0244 | 0.0040 |
| CD97 | TIMP1 | 0.06 | 0.0414 | 0.0031 |
| NRP1 | TIMP1 | 0.06 | 0.0417 | 0.0064 |
| GNB1 | RHOA | 0.06 | 0.0032 | 0.0074 |
| ITGAL | TXNRD1 | 0.06 | 0.0031 | 0.0254 |
| BCL2 | G1P3 | 0.06 | 0.0046 | 0.0469 |
| CCL5 | NFATC2 | 0.06 | 0.0440 | 0.0031 |
| CFLAR | TIMP1 | 0.06 | 0.0426 | 0.0046 |
| BCL2 | TLR2 | 0.06 | 0.0035 | 0.0480 |
| RP51077B9.4 | USP7 | 0.06 | 0.0036 | 0.0277 |
| E2F1 | RBM5 | 0.06 | 0.0256 | 0.0320 |
| CDK5 | MTA1 | 0.06 | 0.0064 | 0.0032 |
| CD48 | NFATC2 | 0.06 | 0.0458 | 0.0033 |
| CD48 | MYC | 0.06 | 0.0312 | 0.0033 |
| NFKB1 | VIM | 0.06 | 0.0032 | 0.0085 |
| CREBBP | TXNRD1 | 0.06 | 0.0033 | 0.0410 |
| IFITM1 | STAT3 | 0.06 | 0.0087 | 0.0034 |
| PLAU | RBM5 | 0.06 | 0.0266 | 0.0055 |
| ITGAL | NME1 | 0.06 | 0.0034 | 0.0271 |
| TIMP1 | TNFRSF1A | 0.06 | 0.0062 | 0.0449 |
| NME1 | SMAD3 | 0.06 | 0.0141 | 0.0034 |
| IFI16 | IFITM1 | 0.06 | 0.0035 | 0.0082 |
| ACPP | CREBBP | 0.06 | 0.0421 | 0.0034 |
| ITGB1 | ZNF350 | 0.06 | 0.0076 | 0.0044 |
| SMAD4 | SPARC | 0.06 | 0.0309 | 0.0124 |
| RBM5 | ST14 | 0.06 | 0.0039 | 0.0273 |
| CREBBP | ELA2 | 0.06 | 0.0311 | 0.0428 |
| NFATC2 | SPARC | 0.06 | 0.0309 | 0.0480 |
| CTNNA1 | TIMP1 | 0.06 | 0.0468 | 0.0034 |
| HRAS | TIMP1 | 0.06 | 0.0467 | 0.0086 |
| CDKN2D | IQGAP1 | 0.06 | 0.0106 | 0.0088 |
| MEIS1 | NCOA1 | 0.06 | 0.0116 | 0.0188 |
| DLC1 | HMGA1 | 0.06 | 0.0093 | 0.0217 |
| ITGAL | PDGFA | 0.06 | 0.0093 | 0.0292 |
| NOTCH2 | SPARC | 0.06 | 0.0321 | 0.0109 |
| CCND2 | SPARC | 0.06 | 0.0323 | 0.0127 |
| MEIS1 | MYC | 0.06 | 0.0339 | 0.0193 |
| MYC | SPARC | 0.06 | 0.0322 | 0.0340 |
| ANLN | SIAH2 | 0.06 | 0.0262 | 0.0037 |
| EGR1 | TIMP1 | 0.06 | 0.0499 | 0.0037 |

| 2-gene models and | | Entropy | | |
| 1-gene models | | R-sq | p-val 1 | p-val 2 |
|---|---|---|---|---|
| HRAS | RHOC | 0.06 | 0.0043 | 0.0093 |
| MTF1 | SPARC | 0.06 | 0.0338 | 0.0110 |
| MEIS1 | SP1 | 0.06 | 0.0151 | 0.0192 |
| FAS | SMAD4 | 0.06 | 0.0137 | 0.0038 |
| HMGA1 | RP51077B9.4 | 0.06 | 0.0329 | 0.0102 |
| ELA2 | MYC | 0.06 | 0.0365 | 0.0347 |
| ICAM1 | MEIS1 | 0.06 | 0.0208 | 0.0161 |
| ABCC1 | ITGB1 | 0.05 | 0.0051 | 0.0104 |
| CCND2 | E2F1 | 0.05 | 0.0391 | 0.0138 |
| ITGAL | RHOA | 0.05 | 0.0040 | 0.0324 |
| PYCARD | SP1 | 0.05 | 0.0161 | 0.0045 |
| CCND2 | RP51077B9.4 | 0.05 | 0.0353 | 0.0144 |
| MYC | VIM | 0.05 | 0.0039 | 0.0388 |
| BRCA1 | ELA2 | 0.05 | 0.0378 | 0.0069 |
| SP1 | ZNF185 | 0.05 | 0.0068 | 0.0165 |
| RBM5 | S100A11 | 0.05 | 0.0041 | 0.0334 |
| RHOA | SP1 | 0.05 | 0.0167 | 0.0047 |
| CD44 | ITGAL | 0.05 | 0.0351 | 0.0051 |
| MAP2K1 | VIM | 0.05 | 0.0041 | 0.0075 |
| NCOA1 | RP51077B9.4 | 0.05 | 0.0368 | 0.0142 |
| MEIS1 | SRF | 0.05 | 0.0172 | 0.0231 |
| S100A11 | SP1 | 0.05 | 0.0170 | 0.0046 |
| DLC1 | MTF1 | 0.05 | 0.0125 | 0.0268 |
| ACPP | RBM5 | 0.05 | 0.0347 | 0.0042 |
| MAP2K1 | RP51077B9.4 | 0.05 | 0.0376 | 0.0077 |
| BRAF | NEDD4L | 0.05 | 0.0223 | 0.0097 |
| CDKN2D | EPAS1 | 0.05 | 0.0078 | 0.0112 |
| RP51077B9.4 | SMAD3 | 0.05 | 0.0185 | 0.0382 |
| GADD45A | SOCS1 | 0.05 | 0.0306 | 0.0122 |
| MEIS1 | SOCS1 | 0.05 | 0.0306 | 0.0244 |
| ITGB1 | VHL | 0.05 | 0.0089 | 0.0058 |
| NCOA1 | SERPINE1 | 0.05 | 0.0159 | 0.0151 |
| SMAD3 | ST14 | 0.05 | 0.0051 | 0.0191 |
| ELA2 | UBE2C | 0.05 | 0.0044 | 0.0410 |
| DLC1 | SMAD4 | 0.05 | 0.0164 | 0.0287 |
| SOCS1 | SPARC | 0.05 | 0.0415 | 0.0312 |
| ACPP | SRF | 0.05 | 0.0189 | 0.0046 |
| AKT1 | ZNF185 | 0.05 | 0.0066 | 0.0164 |
| ABCC1 | RP51077B9.4 | 0.05 | 0.0409 | 0.0125 |
| IFI16 | SPARC | 0.05 | 0.0430 | 0.0115 |
| NRAS | RP51077B9.4 | 0.05 | 0.0458 | 0.0055 |

| 2-gene models and | | Entropy | | |
| --- | --- | --- | --- | --- |
| 1-gene models | | R-sq | p-val 1 | p-val 2 |
| APC | FAS | 0.05 | 0.0047 | 0.0173 |
| ITGAL | PLAU | 0.05 | 0.0079 | 0.0367 |
| RBM5 | THBS1 | 0.05 | 0.0089 | 0.0385 |
| ELA2 | ITGAL | 0.05 | 0.0399 | 0.0440 |
| MYC | TNF | 0.05 | 0.0054 | 0.0462 |
| ALOX5 | RP51077B9.4 | 0.05 | 0.0423 | 0.0070 |
| NCOA1 | THBS1 | 0.05 | 0.0090 | 0.0163 |
| DLC1 | SP1 | 0.05 | 0.0191 | 0.0274 |
| ITGAL | ZNF185 | 0.05 | 0.0069 | 0.0401 |
| CDK5 | COVA1 | 0.05 | 0.0069 | 0.0049 |
| ITGAL | MAP2K1 | 0.05 | 0.0088 | 0.0412 |
| G1P3 | MYC | 0.05 | 0.0481 | 0.0073 |
| CDK2 | RP51077B9.4 | 0.05 | 0.0436 | 0.0081 |
| NFKB1 | RP51077B9.4 | 0.05 | 0.0440 | 0.0130 |
| HMGA1 | MEIS1 | 0.05 | 0.0274 | 0.0135 |
| ICAM1 | SPARC | 0.05 | 0.0456 | 0.0209 |
| IFI16 | RP51077B9.4 | 0.05 | 0.0449 | 0.0125 |
| ITGAL | THBS1 | 0.05 | 0.0095 | 0.0423 |
| NCOA1 | PDGFA | 0.05 | 0.0136 | 0.0174 |
| CEACAM1 | ELA2 | 0.05 | 0.0480 | 0.0051 |
| EP300 | MEIS1 | 0.05 | 0.0283 | 0.0119 |
| NOTCH2 | ZNF185 | 0.05 | 0.0074 | 0.0159 |
| MEIS1 | PTCH1 | 0.05 | 0.0365 | 0.0285 |
| DLC1 | NCOA1 | 0.05 | 0.0174 | 0.0329 |
| CDKN2D | SOCS1 | 0.05 | 0.0365 | 0.0135 |
| RP51077B9.4 | TOPBP1 | 0.05 | 0.0084 | 0.0469 |
| HSPA1A | RP51077B9.4 | 0.05 | 0.0475 | 0.0185 |
| RP51077B9.4 | VEGF | 0.05 | 0.0082 | 0.0473 |
| RHOA | SRF | 0.05 | 0.0222 | 0.0056 |
| MYD88 | RBM5 | 0.05 | 0.0447 | 0.0060 |
| GSK3B | RP51077B9.4 | 0.05 | 0.0485 | 0.0189 |
| CDKN2D | EP300 | 0.05 | 0.0127 | 0.0141 |
| NEDD4L | SOCS1 | 0.05 | 0.0382 | 0.0282 |
| GNB1 | TEGT | 0.05 | 0.0055 | 0.0133 |
| SP1 | VIM | 0.05 | 0.0060 | 0.0221 |
| ICAM1 | PYCARD | 0.05 | 0.0055 | 0.0238 |
| CD59 | RBM5 | 0.05 | 0.0462 | 0.0257 |
| PTCH1 | SOCS1 | 0.05 | 0.0392 | 0.0398 |
| HSPA1A | S100A11 | 0.05 | 0.0055 | 0.0196 |
| DLC1 | NFKB1 | 0.05 | 0.0150 | 0.0366 |
| GNB1 | VIM | 0.05 | 0.0056 | 0.0139 |

| 2-gene models and | | Entropy | | |
| 1-gene models | | R-sq | p-val 1 | p-val 2 |
|---|---|---|---|---|
| DLC1 | SMAD3 | 0.05 | 0.0247 | 0.0371 |
| PTGS2 | RBM5 | 0.05 | 0.0488 | 0.0090 |
| MEIS1 | MTF1 | 0.05 | 0.0175 | 0.0328 |
| MEIS1 | NFKB1 | 0.05 | 0.0156 | 0.0329 |
| CDK5 | JUN | 0.05 | 0.0259 | 0.0059 |
| MNDA | SP1 | 0.05 | 0.0235 | 0.0065 |
| IFI16 | MEIS1 | 0.05 | 0.0332 | 0.0147 |
| ICAM1 | ITGB1 | 0.05 | 0.0077 | 0.0253 |
| CDKN2D | STAT3 | 0.05 | 0.0160 | 0.0155 |
| HSPA1A | SERPINA1 | 0.05 | 0.0059 | 0.0209 |
| IQGAP1 | THBS1 | 0.05 | 0.0115 | 0.0190 |
| MEIS1 | SMAD3 | 0.05 | 0.0266 | 0.0345 |
| ADAMTS1 | SOCS1 | 0.05 | 0.0433 | 0.0396 |
| FAS | JUN | 0.05 | 0.0273 | 0.0062 |
| ALOX5 | CD59 | 0.05 | 0.0288 | 0.0092 |
| CA4 | IGF1R | 0.05 | 0.0133 | 0.0089 |
| PDGFA | SP1 | 0.05 | 0.0250 | 0.0186 |
| SRF | ST14 | 0.05 | 0.0072 | 0.0262 |
| MEIS1 | NOTCH2 | 0.05 | 0.0197 | 0.0355 |
| DLC1 | GSK3B | 0.05 | 0.0220 | 0.0409 |
| NME1 | PTCH1 | 0.05 | 0.0460 | 0.0065 |
| DLC1 | ICAM1 | 0.05 | 0.0273 | 0.0413 |
| MTA1 | RHOC | 0.05 | 0.0075 | 0.0126 |
| CDKN2D | IFI16 | 0.05 | 0.0162 | 0.0169 |
| DLC1 | GNB1 | 0.05 | 0.0161 | 0.0430 |
| CDK5 | SMAD4 | 0.05 | 0.0248 | 0.0067 |
| PYCARD | SMAD4 | 0.05 | 0.0250 | 0.0067 |
| MAP2K1 | ST14 | 0.05 | 0.0078 | 0.0122 |
| DLC1 | XRCC1 | 0.05 | 0.0131 | 0.0441 |
| SMAD4 | TXNRD1 | 0.05 | 0.0068 | 0.0252 |
| GNB1 | MEIS1 | 0.05 | 0.0392 | 0.0170 |
| NOTCH2 | VIM | 0.05 | 0.0069 | 0.0219 |
| ABL1 | ST14 | 0.05 | 0.0081 | 0.0260 |
| BRCA1 | CD59 | 0.05 | 0.0327 | 0.0119 |
| MTF1 | RHOA | 0.05 | 0.0074 | 0.0214 |
| IQGAP1 | MEIS1 | 0.05 | 0.0404 | 0.0224 |
| DLC1 | JUN | 0.05 | 0.0314 | 0.0466 |
| NAB2 | RHOC | 0.05 | 0.0084 | 0.0172 |
| RHOC | SRC | 0.05 | 0.0106 | 0.0085 |
| CD59 | EP300 | 0.05 | 0.0171 | 0.0339 |
| ITGB1 | NAB1 | 0.05 | 0.0154 | 0.0097 |

| 2-gene models and | | Entropy | | |
|---|---|---|---|---|
| 1-gene models | | R-sq | p-val 1 | p-val 2 |
| COL6A2 | RHOC | 0.05 | 0.0086 | 0.0187 |
| NCOA1 | ZNF185 | 0.05 | 0.0107 | 0.0254 |
| JUN | MEIS1 | 0.05 | 0.0417 | 0.0326 |
| FAS | ICAM1 | 0.05 | 0.0320 | 0.0074 |
| HRAS | ITGB1 | 0.05 | 0.0098 | 0.0189 |
| CDKN2D | ETS2 | 0.05 | 0.0124 | 0.0192 |
| MTF1 | TEGT | 0.05 | 0.0074 | 0.0224 |
| NOTCH2 | PDGFA | 0.05 | 0.0199 | 0.0232 |
| APC | CDKN2D | 0.05 | 0.0196 | 0.0280 |
| PYCARD | SRF | 0.05 | 0.0322 | 0.0076 |
| PDGFA | SMAD4 | 0.05 | 0.0287 | 0.0209 |
| ICAM1 | ZNF185 | 0.05 | 0.0113 | 0.0338 |
| CD48 | SMAD3 | 0.05 | 0.0342 | 0.0078 |
| CDKN2D | GSK3B | 0.05 | 0.0276 | 0.0203 |
| CDK5 | SRF | 0.05 | 0.0332 | 0.0079 |
| CDKN2D | SP1 | 0.05 | 0.0310 | 0.0209 |
| SRF | ZNF185 | 0.05 | 0.0115 | 0.0333 |
| ICAM1 | PDGFA | 0.05 | 0.0213 | 0.0344 |
| CDKN2A | SMAD3 | 0.05 | 0.0348 | 0.0084 |
| BRAF | PLAU | 0.05 | 0.0134 | 0.0178 |
| HSPA1A | MEIS1 | 0.05 | 0.0454 | 0.0282 |
| ICAM1 | VIM | 0.05 | 0.0078 | 0.0346 |
| AKT1 | ITGB1 | 0.05 | 0.0106 | 0.0295 |
| EP300 | PLAU | 0.05 | 0.0138 | 0.0185 |
| BRAF | SERPINE1 | 0.05 | 0.0300 | 0.0186 |
| AKT1 | MEIS1 | 0.05 | 0.0467 | 0.0300 |
| CD59 | PLXDC2 | 0.05 | 0.0150 | 0.0387 |
| SERPINA1 | SP1 | 0.05 | 0.0329 | 0.0089 |
| ST14 | VHL | 0.05 | 0.0168 | 0.0096 |
| CCND2 | MEIS1 | 0.05 | 0.0477 | 0.0307 |
| IQGAP1 | SERPINE1 | 0.05 | 0.0312 | 0.0268 |
| ICAM1 | ST14 | 0.05 | 0.0099 | 0.0372 |
| ABCC1 | ST14 | 0.05 | 0.0099 | 0.0235 |
| IQGAP1 | PDGFA | 0.05 | 0.0233 | 0.0271 |
| GSK3B | TXNRD1 | 0.05 | 0.0085 | 0.0303 |
| ITGB1 | SRF | 0.05 | 0.0368 | 0.0114 |
| HSPA1A | PDGFA | 0.05 | 0.0242 | 0.0316 |
| ITGB1 | MAP2K1 | 0.05 | 0.0165 | 0.0119 |
| NCOA1 | SERPINA1 | 0.05 | 0.0089 | 0.0314 |
| MTF1 | PDGFA | 0.05 | 0.0248 | 0.0277 |
| HSPA1A | SERPINE1 | 0.05 | 0.0334 | 0.0322 |

| 2-gene models and | | Entropy | | |
|---|---|---|---|---|
| 1-gene models | | R-sq | p-val 1 | p-val 2 |
| IQGAP1 | ZNF185 | 0.05 | 0.0135 | 0.0294 |
| SP1 | THBS1 | 0.05 | 0.0172 | 0.0367 |
| FAS | GSK3B | 0.05 | 0.0334 | 0.0094 |
| NFKB1 | PYCARD | 0.05 | 0.0095 | 0.0259 |
| SERPINE1 | SP1 | 0.05 | 0.0379 | 0.0366 |
| SERPINE1 | STAT3 | 0.05 | 0.0262 | 0.0357 |
| CD59 | ETS2 | 0.05 | 0.0166 | 0.0467 |
| CDKN2D | SRF | 0.05 | 0.0425 | 0.0261 |
| SMAD4 | ZNF185 | 0.05 | 0.0147 | 0.0381 |
| ICAM1 | TXNRD1 | 0.05 | 0.0101 | 0.0448 |
| NOTCH2 | THBS1 | 0.05 | 0.0197 | 0.0326 |
| FAS | SP1 | 0.05 | 0.0413 | 0.0118 |
| MTF1 | PLAU | 0.05 | 0.0179 | 0.0329 |
| XK | | 0.05 | 0.0140 | |
| GNB1 | PYCARD | 0.04 | 0.0106 | 0.0266 |
| AKT1 | ST14 | 0.04 | 0.0124 | 0.0397 |
| CDKN2D | GNB1 | 0.04 | 0.0268 | 0.0285 |
| SRF | TXNRD1 | 0.04 | 0.0109 | 0.0468 |
| SP1 | TXNRD1 | 0.04 | 0.0115 | 0.0434 |
| IFI16 | SERPINE1 | 0.04 | 0.0413 | 0.0281 |
| CDKN2D | IGF1R | 0.04 | 0.0240 | 0.0297 |
| CDK5 | NFKB1 | 0.04 | 0.0309 | 0.0114 |
| FGF2 | | 0.04 | 0.0250 | |
| ITGB1 | NFKB1 | 0.04 | 0.0314 | 0.0153 |
| GSK3B | PLAU | 0.04 | 0.0195 | 0.0451 |
| ABCC1 | RHOC | 0.04 | 0.0137 | 0.0325 |
| NOTCH2 | RHOA | 0.04 | 0.0122 | 0.0374 |
| MTF1 | ST14 | 0.04 | 0.0138 | 0.0365 |
| APC | ITGB1 | 0.04 | 0.0157 | 0.0452 |
| GSK3B | SERPINE1 | 0.04 | 0.0452 | 0.0436 |
| AKT1 | CDK5 | 0.04 | 0.0120 | 0.0450 |
| G1P3 | SERPING1 | 0.04 | 0.0173 | 0.0185 |
| IQGAP1 | PTEN | 0.04 | 0.0123 | 0.0392 |
| ABL1 | TNF | 0.04 | 0.0142 | 0.0467 |
| HSPA1A | PYCARD | 0.04 | 0.0122 | 0.0444 |
| CDK5 | XRCC1 | 0.04 | 0.0244 | 0.0124 |
| ST14 | TOPBP1 | 0.04 | 0.0203 | 0.0145 |
| MTF1 | ZNF185 | 0.04 | 0.0182 | 0.0384 |
| CASP9 | PYCARD | 0.04 | 0.0126 | 0.0201 |
| SERPINE1 | SMAD4 | 0.04 | 0.0483 | 0.0474 |
| NOTCH2 | SERPINE1 | 0.04 | 0.0483 | 0.0413 |

| 2-gene models and | | Entropy | | |
|---|---|---|---|---|
| 1-gene models | | R-sq | p-val 1 | p-val 2 |
| CA4 | STAT3 | 0.04 | 0.0360 | 0.0186 |
| IFI16 | PDGFA | 0.04 | 0.0366 | 0.0338 |
| CDK5 | HRAS | 0.04 | 0.0348 | 0.0134 |
| ALOX5 | MMP9 | 0.04 | 0.0207 | 0.0200 |
| GNB1 | ZNF185 | 0.04 | 0.0195 | 0.0332 |
| GSK3B | PYCARD | 0.04 | 0.0133 | 0.0485 |
| VHL | VIM | 0.04 | 0.0133 | 0.0276 |
| CDKN2D | MAPK14 | 0.04 | 0.0165 | 0.0357 |
| NRP1 | RHOC | 0.04 | 0.0161 | 0.0293 |
| HRAS | ST14 | 0.04 | 0.0159 | 0.0356 |
| NME1 | TP53 | 0.04 | 0.0247 | 0.0142 |
| CDKN2D | PLXDC2 | 0.04 | 0.0254 | 0.0367 |
| ALOX5 | CDKN2D | 0.04 | 0.0371 | 0.0207 |
| ITGB1 | TP53 | 0.04 | 0.0255 | 0.0189 |
| CA4 | ETS2 | 0.04 | 0.0244 | 0.0204 |
| FAS | NFKB1 | 0.04 | 0.0398 | 0.0147 |
| CA4 | MAPK14 | 0.04 | 0.0181 | 0.0209 |
| GNB1 | PDGFA | 0.04 | 0.0407 | 0.0369 |
| NFKB1 | ST14 | 0.04 | 0.0172 | 0.0404 |
| PYCARD | RAF1 | 0.04 | 0.0361 | 0.0148 |
| NFKB1 | TEGT | 0.04 | 0.0148 | 0.0405 |
| MTF1 | THBS1 | 0.04 | 0.0291 | 0.0467 |
| S100A11 | STAT3 | 0.04 | 0.0428 | 0.0154 |
| RHOC | VHL | 0.04 | 0.0324 | 0.0186 |
| BRCA1 | CDKN2D | 0.04 | 0.0422 | 0.0271 |
| BRAF | PDGFA | 0.04 | 0.0446 | 0.0371 |
| FAS | MAP2K1 | 0.04 | 0.0303 | 0.0165 |
| PLAU | PLXDC2 | 0.04 | 0.0297 | 0.0277 |
| BRCA1 | PLAU | 0.04 | 0.0280 | 0.0290 |
| MTA1 | NME1 | 0.04 | 0.0174 | 0.0342 |
| NME1 | VHL | 0.04 | 0.0350 | 0.0175 |
| EP300 | PDGFA | 0.04 | 0.0473 | 0.0410 |
| GNB1 | THBS1 | 0.04 | 0.0330 | 0.0429 |
| CDKN2D | TLR2 | 0.04 | 0.0191 | 0.0458 |
| NFKB1 | PDGFA | 0.04 | 0.0474 | 0.0468 |
| ACPP | GNB1 | 0.04 | 0.0441 | 0.0174 |
| ADAM17 | CDKN2D | 0.04 | 0.0474 | 0.0220 |
| PDGFA | STAT3 | 0.04 | 0.0490 | 0.0493 |
| FAS | NAB1 | 0.04 | 0.0398 | 0.0187 |
| CA4 | FOS | 0.04 | 0.0254 | 0.0265 |
| RAF1 | ZNF185 | 0.04 | 0.0276 | 0.0459 |

| 2-gene models and | | Entropy | | |
|---|---|---|---|---|
| 1-gene models | | R-sq | p-val 1 | p-val 2 |
| ALOX5 | CA4 | 0.04 | 0.0268 | 0.0281 |
| MTA1 | ST14 | 0.04 | 0.0219 | 0.0381 |
| BRAF | IGF2BP2 | 0.04 | 0.0476 | 0.0441 |
| COVA1 | NME1 | 0.04 | 0.0197 | 0.0274 |
| MAP2K1 | PYCARD | 0.04 | 0.0191 | 0.0356 |
| IFI16 | ZNF185 | 0.04 | 0.0281 | 0.0494 |
| EP300 | THBS1 | 0.04 | 0.0376 | 0.0468 |
| BRAF | CDC25A | 0.04 | 0.0375 | 0.0460 |
| COVA1 | ITGB1 | 0.04 | 0.0268 | 0.0288 |
| IRF1 | PYCARD | 0.04 | 0.0215 | 0.0394 |
| ITGB1 | SOX4 | 0.04 | 0.0334 | 0.0295 |
| PLAU | VEGF | 0.04 | 0.0338 | 0.0380 |
| CTSD | PLAU | 0.04 | 0.0402 | 0.0350 |
| BCAM | | 0.04 | 0.0240 | |
| CASP9 | VIM | 0.04 | 0.0236 | 0.0383 |
| ITGB1 | MTA1 | 0.04 | 0.0497 | 0.0324 |
| RHOC | XRCC1 | 0.04 | 0.0493 | 0.0290 |
| MMP9 | PLXDC2 | 0.04 | 0.0469 | 0.0390 |
| PLXDC2 | S100A11 | 0.04 | 0.0251 | 0.0473 |
| ITGB1 | TOPBP1 | 0.04 | 0.0441 | 0.0355 |
| MAP2K1 | RHOC | 0.04 | 0.0313 | 0.0496 |
| NUDT4 | | 0.04 | 0.0260 | |
| ITGB1 | SRC | 0.04 | 0.0407 | 0.0362 |
| CDK2 | CDK5 | 0.04 | 0.0278 | 0.0472 |
| BCL2 | | 0.04 | 0.0360 | |
| COVA1 | ST14 | 0.04 | 0.0334 | 0.0411 |
| RHOC | TNF | 0.04 | 0.0348 | 0.0353 |
| COVA1 | RHOC | 0.03 | 0.0364 | 0.0444 |
| NFATC2 | | 0.03 | 0.0340 | |
| TIMP1 | | 0.03 | 0.0470 | |
| CA4 | TLR2 | 0.03 | 0.0367 | 0.0471 |
| CREBBP | | 0.03 | 0.0380 | |
| E2F1 | | 0.03 | 0.0260 | |
| MYC | | 0.03 | 0.0520 | |
| NCOA4 | | 0.03 | 0.0460 | |
| ELA2 | | 0.03 | 0.0510 | |
| SPARC | | 0.03 | 0.0530 | |
| RP51077B9.4 | | 0.03 | 0.0640 | |

**Table 6:** Means and Likelihood Ratio P-Values Ranked by Entropy $R^2$ for the Cox-Type Survival Model

| | | | Likelihood | Entropy |
|---|---|---|---|---|
| Gene | Dead Mean | Alive Mean | Ratio p-val | R-sq |
| ABL2 | 21.3 | 20.5 | 0.0001 | 0.09 |

(continued)

| Gene | Dead Mean | Alive Mean | Likelihood Ratio p-val | Entropy R-sq |
|---|---|---|---|---|
| CAV2 | 22.8 | 24.1 | 0.0003 | 0.08 |
| C1QB | 20.1 | 21.2 | 0.0003 | 0.08 |
| CDKN1A | 16.2 | 16.8 | 0.0007 | 0.07 |
| C1QA | 19.9 | 20.8 | 0.0010 | 0.07 |
| SEMA4D | 15.2 | 14.8 | 0.0025 | 0.06 |
| XK | 16.5 | 17.7 | 0.0105 | 0.05 |
| FGF2 | 23.4 | 24.4 | 0.0119 | 0.04 |
| BCAM | 18.8 | 20.3 | 0.0229 | 0.04 |
| NUDT4 | 15.0 | 15.9 | 0.0263 | 0.04 |
| BCL2 | 18.6 | 17.7 | 0.0280 | 0.04 |
| NFATC2 | 17.4 | 16.6 | 0.0303 | 0.03 |
| TIMP1 | 14.3 | 14.7 | 0.0312 | 0.03 |
| CREBBP | 15.5 | 15.1 | 0.0339 | 0.03 |
| E2F1 | 20.1 | 20.7 | 0.0423 | 0.03 |
| MYC | 18.9 | 18.3 | 0.0444 | 0.03 |
| NCOA4 | 11.2 | 11.9 | 0.0460 | 0.03 |
| ELA2 | 18.3 | 19.6 | 0.0467 | 0.03 |
| SPARC | 14.6 | 15.2 | 0.0469 | 0.03 |
| RP51077B9.4 | 16.6 | 16.8 | 0.0489 | 0.03 |
| ITGAL | 15.6 | 15.1 | 0.0516 | 0.03 |
| RBM5 | 16.3 | 15.9 | 0.0531 | 0.03 |
| SIAH2 | 12.9 | 13.8 | 0.0599 | 0.03 |
| PTCH1 | 21.3 | 20.5 | 0.0618 | 0.03 |
| SOCS1 | 17.3 | 17.0 | 0.0628 | 0.03 |
| DLC1 | 23.0 | 23.4 | 0.0685 | 0.03 |
| ADAMTS1 | 22.9 | 22.3 | 0.0690 | 0.03 |
| KAI1 | 15.9 | 15.5 | 0.0722 | 0.03 |
| MEIS1 | 21.5 | 21.8 | 0.0798 | 0.03 |
| NEDD4L | 17.8 | 18.4 | 0.0862 | 0.03 |
| CD59 | 17.6 | 17.9 | 0.0982 | 0.02 |
| JUN | 21.9 | 21.4 | 0.1036 | 0.02 |
| SMAD3 | 19.0 | 18.3 | 0.1053 | 0.02 |
| ICAM1 | 18.1 | 17.6 | 0.1059 | 0.02 |
| SRF | 17.2 | 16.8 | 0.1097 | 0.02 |
| ABL1 | 19.5 | 18.9 | 0.1251 | 0.02 |
| APC | 18.3 | 18.0 | 0.1255 | 0.02 |
| SMAD4 | 17.7 | 17.4 | 0.1256 | 0.02 |

(continued)

| Gene | Dead Mean | Alive Mean | Likelihood Ratio p-val | Entropy R-sq |
|---|---|---|---|---|
| CCND2 | 18.3 | 17.4 | 0.1277 | 0.02 |
| SP1 | 16.3 | 15.9 | 0.1279 | 0.02 |
| SERPINE1 | 20.7 | 21.2 | 0.1282 | 0.02 |
| AKT1 | 16.1 | 15.7 | 0.1283 | 0.02 |
| GSK3B | 16.2 | 15.9 | 0.1331 | 0.02 |
| HSPA1A | 15.1 | 14.7 | 0.1333 | 0.02 |
| NCOA1 | 16.7 | 16.4 | 0.1363 | 0.02 |
| IQGAP1 | 14.3 | 14.0 | 0.1517 | 0.02 |
| NOTCH2 | 16.6 | 16.2 | 0.1520 | 0.02 |
| MTF1 | 18.1 | 17.7 | 0.1591 | 0.02 |
| E2F5 | 22.7 | 21.7 | 0.1630 | 0.02 |
| GADD45A | 19.1 | 19.6 | 0.1723 | 0.02 |
| HMGA1 | 16.5 | 16.0 | 0.1764 | 0.02 |
| ABCC1 | 17.3 | 16.7 | 0.1777 | 0.02 |
| PDGFA | 19.4 | 19.7 | 0.1809 | 0.02 |
| STAT3 | 14.4 | 14.1 | 0.1818 | 0.02 |
| GSTT1 | 22.4 | 22.0 | 0.1827 | 0.02 |
| NFKB1 | 17.5 | 17.0 | 0.1833 | 0.02 |
| ERBB2 | 23.7 | 23.0 | 0.1853 | 0.02 |
| CDKN2D | 14.9 | 15.1 | 0.1888 | 0.02 |
| CDH1 | 20.0 | 20.5 | 0.1909 | 0.02 |
| COL6A2 | 19.8 | 19.1 | 0.1925 | 0.02 |
| HRAS | 22.1 | 21.3 | 0.1936 | 0.02 |
| IFI16 | 14.8 | 14.5 | 0.1983 | 0.02 |
| GNB1 | 13.8 | 13.5 | 0.2027 | 0.02 |
| IGF2BP2 | 15.4 | 15.9 | 0.2046 | 0.02 |
| NAB2 | 21.4 | 20.7 | 0.2088 | 0.02 |
| RAF1 | 15.0 | 14.7 | 0.2106 | 0.02 |
| EP300 | 16.7 | 16.4 | 0.2135 | 0.02 |
| ZNF350 | 19.9 | 19.6 | 0.2189 | 0.02 |
| IL8 | 23.2 | 22.3 | 0.2215 | 0.02 |
| BRAF | 17.3 | 17.1 | 0.2239 | 0.02 |
| IGF1R | 16.2 | 15.9 | 0.2434 | 0.02 |
| NRP1 | 24.0 | 23.3 | 0.2451 | 0.02 |
| NAB1 | 17.4 | 17.1 | 0.2463 | 0.02 |
| VHL | 17.9 | 17.6 | 0.2585 | 0.02 |
| MTA1 | 20.3 | 19.8 | 0.2641 | 0.02 |

(continued)

| Gene | Dead Mean | Alive Mean | Likelihood Ratio p-val | Entropy R-sq |
|---|---|---|---|---|
| XRCC1 | 19.0 | 18.6 | 0.2698 | 0.02 |
| IGFBP3 | 23.1 | 22.4 | 0.2744 | 0.02 |
| THBS1 | 17.8 | 18.1 | 0.2777 | 0.02 |
| TNFRSF1A | 15.9 | 15.6 | 0.2796 | 0.02 |
| CDC25A | 23.2 | 23.6 | 0.2870 | 0.02 |
| NR4A2 | 21.8 | 21.5 | 0.2944 | 0.02 |
| PLXDC2 | 16.6 | 16.4 | 0.2951 | 0.02 |
| MAP2K1 | 16.6 | 16.2 | 0.2953 | 0.02 |
| EPAS1 | 20.8 | 20.5 | 0.2967 | 0.02 |
| IRF1 | 13.6 | 13.3 | 0.3023 | 0.02 |
| TP53 | 17.5 | 16.8 | 0.3081 | 0.02 |
| PLAU | 23.7 | 24.1 | 0.3197 | 0.01 |
| BRCA1 | 21.3 | 21.1 | 0.3249 | 0.01 |
| ETS2 | 17.3 | 17.0 | 0.3285 | 0.01 |
| CDK2 | 20.1 | 19.7 | 0.3349 | 0.01 |
| APAF1 | 17.5 | 17.2 | 0.3409 | 0.01 |
| TOPBP1 | 18.5 | 18.2 | 0.3466 | 0.01 |
| CASP9 | 18.8 | 18.5 | 0.3609 | 0.01 |
| VEGF | 23.2 | 22.8 | 0.3617 | 0.01 |
| PTGS2 | 17.6 | 17.2 | 0.3698 | 0.01 |
| SVIL | 17.2 | 16.9 | 0.3716 | 0.01 |
| MMP9 | 13.6 | 14.1 | 0.3760 | 0.01 |
| G1P3 | 15.8 | 15.9 | 0.3825 | 0.01 |
| SOX4 | 20.3 | 20.0 | 0.3910 | 0.01 |
| ALOX5 | 15.6 | 15.4 | 0.3948 | 0.01 |
| SRC | 19.3 | 18.9 | 0.3965 | 0.01 |
| CFLAR | 14.9 | 14.7 | 0.3989 | 0.01 |
| CTSD | 13.5 | 13.2 | 0.3998 | 0.01 |
| ZNF185 | 17.3 | 17.5 | 0.4033 | 0.01 |
| RB1 | 17.8 | 17.6 | 0.4146 | 0.01 |
| COVA1 | 19.9 | 19.4 | 0.4207 | 0.01 |
| SERPING1 | 18.7 | 18.5 | 0.4229 | 0.01 |
| CA4 | 18.5 | 18.9 | 0.4231 | 0.01 |
| FOS | 15.8 | 15.6 | 0.4494 | 0.01 |
| KLK3 | 25.5 | 25.6 | 0.4551 | 0.01 |
| ITGB1 | 15.3 | 15.2 | 0.4708 | 0.01 |
| PLEK2 | 18.2 | 18.6 | 0.4739 | 0.01 |

(continued)

| Gene | Dead Mean | Alive Mean | Likelihood Ratio p-val | Entropy R-sq |
|------|-----------|------------|------------------------|--------------|
| ANGPT1 | 20.7 | 20.5 | 0.4924 | 0.01 |
| POV1 | 18.5 | 18.6 | 0.5009 | 0.01 |
| CCL3 | 21.0 | 20.6 | 0.5062 | 0.01 |
| SKIL | 18.5 | 18.3 | 0.5097 | 0.01 |
| LGALS8 | 17.8 | 17.5 | 0.5100 | 0.01 |
| PTPRC | 12.4 | 12.2 | 0.5130 | 0.01 |
| IL1B | 16.6 | 16.3 | 0.5168 | 0.01 |
| ADAM17 | 18.3 | 18.1 | 0.5225 | 0.01 |
| CD44 | 14.6 | 14.2 | 0.5290 | 0.01 |
| MAPK14 | 15.3 | 15.2 | 0.5344 | 0.01 |
| EGR3 | 24.1 | 23.5 | 0.5486 | 0.01 |
| SORBS1 | 22.8 | 23.0 | 0.5626 | 0.01 |
| RHOC | 17.1 | 17.0 | 0.5763 | 0.01 |
| AOC3 | 20.1 | 19.7 | 0.5768 | 0.01 |
| ST14 | 18.7 | 18.6 | 0.5879 | 0.01 |
| TNF | 19.7 | 19.1 | 0.5907 | 0.01 |
| USP7 | 15.7 | 15.5 | 0.6188 | 0.01 |
| MYD88 | 15.0 | 14.9 | 0.6288 | 0.01 |
| TLR2 | 15.6 | 15.6 | 0.6402 | 0.01 |
| NRAS | 17.6 | 17.4 | 0.6418 | 0.01 |
| S100A6 | 15.6 | 15.3 | 0.6669 | 0.01 |
| NME4 | 17.6 | 17.6 | 0.7033 | 0.01 |
| TGFB1 | 13.3 | 13.2 | 0.7040 | 0.01 |
| CDKN2A | 22.0 | 21.6 | 0.7153 | 0.01 |
| IL18 | 21.5 | 21.6 | 0.7289 | 0.01 |
| IFITM1 | 8.8 | 8.9 | 0.7510 | 0.01 |
| RHOA | 12.2 | 12.0 | 0.7549 | 0.01 |
| NME1 | 20.8 | 20.6 | 0.7817 | 0.01 |
| CD97 | 13.5 | 13.3 | 0.8026 | 0.01 |
| DAD1 | 15.8 | 15.6 | 0.8070 | 0.01 |
| KRT5 | 25.6 | 25.6 | 0.8095 | 0.01 |
| EGR1 | 19.8 | 19.7 | 0.8105 | 0.01 |
| CEBPB | 14.8 | 14.8 | 0.8123 | 0.01 |
| ANLN | 21.8 | 22.0 | 0.8329 | 0.01 |
| FAS | 16.7 | 16.6 | 0.8365 | 0.01 |
| PTEN | 14.0 | 13.9 | 0.8448 | 0.01 |
| MAPK1 | 14.7 | 14.7 | 0.8479 | 0.01 |

(continued)

| Gene | Dead Mean | Alive Mean | Likelihood Ratio p-val | Entropy R-sq |
|---|---|---|---|---|
| CD48 | 16.0 | 15.8 | 0.8512 | 0.01 |
| G6PD | 15.8 | 15.8 | 0.8629 | 0.01 |
| TEGT | 12.7 | 12.5 | 0.8678 | 0.01 |
| CDK5 | 19.3 | 19.2 | 0.8709 | 0.01 |
| TXNRD1 | 17.2 | 17.1 | 0.8751 | 0.01 |
| ACPP | 17.9 | 17.8 | 0.8764 | 0.01 |
| MME | 15.3 | 15.3 | 0.8834 | 0.01 |
| ITGA1 | 21.7 | 21.4 | 0.8870 | 0.01 |
| CEACAM1 | 18.1 | 18.0 | 0.8907 | 0.01 |
| PYCARD | 15.5 | 15.4 | 0.9000 | 0.01 |
| UBE2C | 21.0 | 21.0 | 0.9000 | 0.01 |
| SERPINA1 | 12.7 | 12.6 | 0.9203 | 0.01 |
| S100A11 | 11.1 | 11.0 | 0.9207 | 0.01 |
| SMARCD3 | 17.4 | 17.3 | 0.9292 | 0.01 |
| CTNNA1 | 17.1 | 17.0 | 0.9367 | 0.01 |
| VIM | 11.9 | 11.8 | 0.9571 | 0.01 |
| MNDA | 12.9 | 12.8 | 0.9617 | 0.01 |
| CCL5 | 13.1 | 13.0 | 0.9652 | 0.01 |
| CCNE1 | 24.1 | 23.8 | 0.9840 | 0.01 |

**Table 7A:** Probability of being a long-term survivor based on the Zero-Inflated Poisson Surival Model-ABL2

| | | | | Long term survivor |
|---|---|---|---|---|
| | Entropy R-sq = 0.270 | | | |
| | | June 20 08 | #Weeks | |
| SourceID | ABL2 | Status | Exposed | Prob |
| 178930 | 20.13 | Alive | 387 | 1.00 |
| 187770 | 19.78 | Alive | 269 | 0.99 |
| 229664 | 19.84 | Alive | 241 | 0.99 |
| 334666 | 19.24 | Alive | 14 | 0.99 |
| 249044 | 19.82 | Alive | 191 | 0.99 |
| 322703 | 19.34 | Alive | 33 | 0.99 |
| 244769 | 19.8 | Alive | 118 | 0.98 |
| 103398 | 20.24 | Alive | 218 | 0.97 |
| 224210 | 19.83 | Alive | 108 | 0.97 |
| 229247 | 20.34 | Alive | 240 | 0.97 |
| 72 | 20.57 | Alive | 281 | 0.97 |
| 113 | 21.22 | Alive | 450 | 0.97 |

(continued)

| SourceID | ABL2 | June 20 08 Status | #Weeks Exposed | Long term survivor Prob |
|---|---|---|---|---|
| | | Entropy R-sq = 0.270 | | |
| 164406 | 19.98 | Alive | 136 | 0.97 |
| 196262 | 20.37 | Alive | 225 | 0.96 |
| 78 | 20.1 | Alive | 124 | 0.95 |
| 185401 | 20.17 | Alive | 139 | 0.95 |
| 155 | 20.27 | Alive | 156 | 0.95 |
| 233923 | 20.56 | Alive | 228 | 0.95 |
| 196141 | 21.06 | Alive | 320 | 0.93 |
| 303333 | 20.07 | Alive | 59 | 0.93 |
| 223748 | 20.86 | Alive | 249 | 0.92 |
| 152331 | 20.58 | Alive | 177 | 0.92 |
| 50796156 | 20.13 | Alive | 60 | 0.92 |
| 48 | 20.54 | Alive | 165 | 0.91 |
| 137633 | 20.49 | Alive | 152 | 0.91 |
| 50223520 | 20.23 | Alive | 78 | 0.91 |
| 322324 | 20.15 | Alive | 32 | 0.90 |
| 187129 | 20.67 | Alive | 186 | 0.90 |
| 330355 | 20.18 | Alive | 20 | 0.90 |
| 109722 | 21.33 | Alive | 346 | 0.89 |
| 208893 | 20.31 | Alive | 48 | 0.87 |
| 252906 | 20.52 | Alive | 116 | 0.87 |
| 279316 | 20.53 | Alive | 119 | 0.87 |
| 1 | 20.87 | Alive | 192 | 0.86 |
| 16 | 20.51 | Alive | 87 | 0.85 |
| 59 | 20.8 | Alive | 161 | 0.84 |
| 99 | 21.15 | Alive | 205 | 0.77 |
| 200871 | 21.01 | Alive | 156 | 0.76 |
| 261891 | 20.62 | Alive | 49 | 0.76 |
| 336476 | 20.63 | Alive | 6 | 0.74 |
| 279014 | 20.71 | Alive | 68 | 0.73 |
| 56 | 21.43 | Alive | 233 | 0.70 |
| 221617 | 20.9 | Alive | 97 | 0.69 |
| 295740 | 21.17 | Alive | 82 | 0.50 |
| 272956 | 21.42 | Alive | 92 | 0.38 |
| 50254384 | 21.38 | Alive | 59 | 0.34 |
| 26 | 21.53 | Alive | 87 | 0.32 |

(continued)

| SourceID | ABL2 | June 20 08 Status | #Weeks Exposed | Long term survivor Prob |
|---|---|---|---|---|
| | | Entropy R-sq = 0.270 | | |
| 6 | 22 | Dead | 110 | 0.00 |
| 9 | 21 07 | Dead | 49 | 0 00 |
| 31 | 21.65 | Dead | 87 | 0.00 |
| 32 | 21.89 | Dead | 104 | 0.00 |
| 44 | 21.71 | Dead | 97 | 0.00 |
| 46 | 20.46 | Dead | 99 | 0.00 |
| 57 | 21.64 | Dead | 77 | 0.00 |
| 63 | 22.05 | Dead | 202 | 0.00 |
| 88 | 20.97 | Dead | 115 | 0.00 |
| 124 | 20.94 | Dead | 77 | 0.00 |
| 219180 | 20.25 | Dead | 115 | 0.00 |
| 275979 | 20.77 | Dead | 68 | 0.00 |
| 290701 | 20.84 | Dead | 33 | 0.00 |
| 294238 | 21.21 | Dead | 64 | 0.00 |
| 323394 | 21.97 | Dead | 12 | 0.00 |

**Table 7B**: Probability of being a long-term survivor based on the Zero-Inflated Poisson Survival Model ABL2 and C1QA

| Entropy R-sq = .315 | | | | | Long term survivor |
| --- | --- | --- | --- | --- | --- |
| SourceID | ABL2 | C1QA | June 20 08 Status | #Weeks Exposed | Prob |
| 187770 | 19.78 | 21.6 | Alive | 269 | 1.00 |
| 224210 | 19.83 | 22.05 | Alive | 108 | 1.00 |
| 229664 | 19.84 | 21.51 | Alive | 241 | 1.00 |
| 322703 | 19.34 | 20.77 | Alive | 33 | 1.00 |
| 330355 | 20.18 | 22.46 | Alive | 20 | 1.00 |
| 249044 | 19.82 | 20.74 | Alive | 191 | 1.00 |
| 229247 | 20.34 | 21.35 | Alive | 240 | 1.00 |
| 244769 | 19.8 | 20.89 | Alive | 118 | 1.00 |
| 113 | 21.22 | 21.77 | Alive | 450 | 1.00 |
| 164406 | 19.98 | 21.02 | Alive | 136 | 1.00 |
| 196262 | 20.37 | 21.27 | Alive | 225 | 1.00 |
| 178930 | 20.13 | 19.44 | Alive | 387 | 1.00 |
| 99 | 21.15 | 22.72 | Alive | 205 | 1.00 |
| 233923 | 20.56 | 21.05 | Alive | 228 | 1.00 |
| 334666 | 19.24 | 19.28 | Alive | 14 | 1.00 |
| 223748 | 20.86 | 21.54 | Alive | 249 | 1.00 |
| 187129 | 20.67 | 21.52 | Alive | 186 | 1.00 |
| 252906 | 20.52 | 21.44 | Alive | 116 | 0.99 |
| 50796156 | 20.13 | 20.71 | Alive | 60 | 0.99 |
| 155 | 20.27 | 20.41 | Alive | 156 | 0.99 |
| 200871 | 21.01 | 21.91 | Alive | 156 | 0.99 |
| 303333 | 20.07 | 20.42 | Alive | 59 | 0.99 |
| 208893 | 20.31 | 20.95 | Alive | 48 | 0.99 |
| 78 | 20.1 | 20.05 | Alive | 124 | 0.99 |
| 137633 | 20.49 | 20.76 | Alive | 152 | 0.99 |
| 279014 | 20.71 | 21.72 | Alive | 68 | 0.99 |
| 152331 | 20.58 | 20.71 | Alive | 177 | 0.98 |
| 196141 | 21.06 | 20.58 | Alive | 320 | 0.97 |
| 109722 | 21.33 | 21.01 | Alive | 346 | 0.97 |
| 72 | 20.57 | 19.65 | Alive | 281 | 0.97 |
| 261891 | 20.62 | 21.07 | Alive | 49 | 0.96 |
| 50223520 | 20.23 | 20.04 | Alive | 78 | 0.96 |
| 322324 | 20.15 | 19.98 | Alive | 32 | 0.96 |
| 59 | 20.8 | 20.69 | Alive | 161 | 0.95 |
| 221617 | 20.9 | 21.21 | Alive | 97 | 0.94 |
| 336476 | 20.63 | 20.88 | Alive | 6 | 0.94 |
| 103398 | 20.24 | 18.97 | Alive | 218 | 0.94 |
| 48 | 20.54 | 19.81 | Alive | 165 | 0.92 |
| 1 | 20.87 | 20.28 | Alive | 192 | 0.91 |
| 185401 | 20.17 | 19.07 | Alive | 139 | 0.91 |
| 16 | 20.51 | 19.92 | Alive | 87 | 0.87 |
| 279316 | 20.53 | 19.76 | Alive | 119 | 0.86 |
| 26 | 21.53 | 21.86 | Alive | 87 | 0.80 |

| Entropy R-sq = .315 | | | | | Long |
| | | | | | term |
| | | | June 20 08 | #Weeks | survivor |
| SourceID | ABL2 | C1QA | Status | Exposed | Prob |
| 56 | 21.43 | 20.72 | Alive | 233 | 0.79 |
| 295740 | 21.17 | 21.02 | Alive | 82 | 0.76 |
| 272956 | 21.42 | 20.77 | Alive | 92 | 0.45 |
| 50254384 | 21.38 | 20.42 | Alive | 59 | 0.26 |
| | | | | | |
| 6 | 22 | 20.12 | Dead | 110 | 0.00 |
| 9 | 21.07 | 18.87 | Dead | 49 | 0.00 |
| 31 | 21.65 | 19.81 | Dead | 87 | 0.00 |
| 32 | 21.89 | 20.56 | Dead | 104 | 0.00 |
| 44 | 21.71 | 19.08 | Dead | 97 | 0.00 |
| 46 | 20.46 | 18.11 | Dead | 99 | 0.00 |
| 57 | 21.64 | 20.68 | Dead | 77 | 0.00 |
| 63 | 22.05 | 21.82 | Dead | 202 | 0.00 |
| 88 | 20.97 | 18.66 | Dead | 115 | 0.00 |
| 124 | 20.94 | 19.97 | Dead | 77 | 0.00 |
| 219180 | 20.25 | 18.44 | Dead | 115 | 0.00 |
| 275979 | 20.77 | 21.39 | Dead | 68 | 0.00 |
| 290701 | 20.84 | 19.46 | Dead | 33 | 0.00 |
| 294238 | 21.21 | 21.01 | Dead | 64 | 0.00 |
| 323394 | 21.97 | 19.88 | Dead | 12 | 0.00 |

**Table 7C**: Probability of being a long-term survivor based on the Zero-Inflated Poisson Survival Model SEMA4D and TIMP1

| Entropy R-sq = 0.285 | | | | | Long |
| | | | | | term |
| | | | June 20 08 | #Weeks | survivor |
| SourceID | SEMA4D | TIMP1 | Status | Exposed | Prob |
| 223748 | 14.9 | 16.16 | Alive | 249 | 1.00 |
| 99 | 14.59 | 15.73 | Alive | 205 | 1.00 |
| 113 | 15.03 | 15.58 | Alive | 450 | 1.00 |
| 233923 | 14.49 | 15.08 | Alive | 228 | 1.00 |
| 196141 | 15.02 | 15.47 | Alive | 320 | 1.00 |
| 322703 | 13.58 | 14.11 | Alive | 33 | 1.00 |
| 56 | 15.84 | 16.76 | Alive | 233 | 1.00 |
| 229247 | 14.17 | 14.28 | Alive | 240 | 1.00 |
| 152331 | 14.91 | 15.49 | Alive | 177 | 0.99 |
| 16 | 14.13 | 14.37 | Alive | 87 | 0.99 |
| 103398 | 14.56 | 14.56 | Alive | 218 | 0.98 |
| 336476 | 14.17 | 14.53 | Alive | 6 | 0.98 |
| 279316 | 14.33 | 14.53 | Alive | 119 | 0.98 |
| 155 | 14.55 | 14.67 | Alive | 156 | 0.98 |
| 1 | 15.28 | 15.59 | Alive | 192 | 0.98 |
| 249044 | 14.23 | 14.05 | Alive | 191 | 0.98 |
| 224210 | 14.43 | 14.59 | Alive | 108 | 0.98 |
| 178930 | 14.93 | 14.36 | Alive | 387 | 0.98 |
| 187770 | 14.46 | 14.05 | Alive | 269 | 0.97 |

| Entropy R-sq = 0.285 | | | | | Long |
| | | | | | term |
| | | | June 20 08 | #Weeks | survivor |
| SourceID | SEMA4D | TIMP1 | Status | Exposed | Prob |
| 72 | 14.11 | 13.41 | Alive | 281 | 0.97 |
| 229664 | 14.97 | 14.81 | Alive | 241 | 0.97 |
| 208893 | 14.67 | 14.87 | Alive | 48 | 0.95 |
| 187129 | 14.83 | 14.67 | Alive | 186 | 0.95 |
| 244769 | 14.12 | 13.81 | Alive | 118 | 0.94 |
| 279014 | 14.8 | 14.91 | Alive | 68 | 0.93 |
| 137633 | 15.04 | 14.98 | Alive | 152 | 0.93 |
| 59 | 14.74 | 14.47 | Alive | 161 | 0.93 |
| 50796156 | 14.75 | 14.8 | Alive | 60 | 0.92 |
| 261891 | 15.04 | 15.2 | Alive | 49 | 0.91 |
| 322324 | 14.41 | 14.28 | Alive | 32 | 0.91 |
| 109722 | 15.79 | 15.31 | Alive | 346 | 0.91 |
| 185401 | 14.59 | 14.16 | Alive | 139 | 0.89 |
| 295740 | 14.79 | 14.66 | Alive | 82 | 0.88 |
| 334666 | 14.18 | 13.8 | Alive | 14 | 0.86 |
| 164406 | 14.96 | 14.61 | Alive | 136 | 0.83 |
| 196262 | 14.72 | 13.94 | Alive | 225 | 0.83 |
| 303333 | 15.12 | 15.01 | Alive | 59 | 0.82 |
| 50254384 | 15.34 | 15.3 | Alive | 59 | 0.80 |
| 200871 | 15.06 | 14.55 | Alive | 156 | 0.79 |
| 252906 | 15.07 | 14.68 | Alive | 116 | 0.76 |
| 50223520 | 14.68 | 14.14 | Alive | 78 | 0.71 |
| 221617 | 15.24 | 14.86 | Alive | 97 | 0.68 |
| 48 | 14.62 | 13.66 | Alive | 165 | 0.64 |
| 78 | 14 44 | 13 32 | Alive | 124 | 0 49 |
| 330355 | 15.3 | 14.6 | Alive | 20 | 0.32 |
| 26 | 15.61 | 14.93 | Alive | 87 | 0.32 |
| 272956 | 15.19 | 13.9 | Alive | 92 | 0.12 |
| | | | | | |
| 6 | 15.81 | 14.51 | Dead | 110 | 0.00 |
| 9 | 14.88 | 14.04 | Dead | 49 | 0.00 |
| 31 | 15.35 | 14.74 | Dead | 87 | 0.00 |
| 32 | 15.59 | 14.94 | Dead | 104 | 0.00 |
| 44 | 15.52 | 14.37 | Dead | 97 | 0.00 |
| 46 | 14.71 | 13.98 | Dead | 99 | 0.00 |
| 57 | 15.38 | 14.37 | Dead | 77 | 0.00 |
| 63 | 15.19 | 14.44 | Dead | 202 | 0.00 |
| 88 | 15.31 | 13.88 | Dead | 115 | 0.00 |
| 124 | 15.03 | 14.21 | Dead | 77 | 0.00 |
| 219180 | 14.5 | 14.1 | Dead | 115 | 0.00 |
| 275979 | 15.11 | 13.74 | Dead | 68 | 0.00 |
| 290701 | 14.72 | 14.94 | Dead | 33 | 0.00 |
| 294238 | 15.5 | 14.55 | Dead | 64 | 0.00 |
| 323394 | 16.1 | 14.29 | Dead | 12 | 0.00 |

**Table 7D:** Probability of being a long-term survivor based on the Zero-Inflated Poisson Survival Model -Average of 4 genes ABL2 and SEMA4D and C1QA and TIMP1 SEMA4D

| Entropy R-sq = 0.323 | | | | | Long |
| 4-gene model | | | | | term |
| | | | June 20 08 | #Weeks | survivor |
| SourceID | Abl2Sema4d | C1qaTimp1 | Status | Exposed | Prob |
| 99 | 17.87 | 19.22 | Alive | 205 | 1.00 |
| 113 | 18.13 | 18.68 | Alive | 450 | 1.00 |
| 187770 | 17.12 | 17.82 | Alive | 269 | 1.00 |
| 223748 | 17.88 | 18.85 | Alive | 249 | 1.00 |
| 224210 | 17.13 | 18.32 | Alive | 108 | 1.00 |
| 229664 | 17.4 | 18.16 | Alive | 241 | 1.00 |
| 322703 | 16.46 | 17.44 | Alive | 33 | 1.00 |
| 229247 | 17.26 | 17.82 | Alive | 240 | 1.00 |
| 233923 | 17.52 | 18.06 | Alive | 228 | 1.00 |
| 249044 | 17.03 | 17.39 | Alive | 191 | 1.00 |
| 244769 | 16.96 | 17.35 | Alive | 118 | 1.00 |
| 330355 | 17.74 | 18.53 | Alive | 20 | 1.00 |
| 164406 | 17.47 | 17.81 | Alive | 136 | 1.00 |
| 152331 | 17.74 | 18.1 | Alive | 177 | 1.00 |
| 187129 | 17.75 | 18.09 | Alive | 186 | 1.00 |
| 155 | 17.41 | 17.54 | Alive | 156 | 1.00 |
| 196262 | 17.54 | 17.61 | Alive | 225 | 1.00 |
| 279014 | 17.76 | 18.32 | Alive | 68 | 1.00 |
| 208893 | 17.49 | 17.91 | Alive | 48 | 1.00 |
| 50796156 | 17.44 | 17.76 | Alive | 60 | 1.00 |
| 196141 | 18.04 | 18.03 | Alive | 320 | 1.00 |
| 336476 | 17.4 | 17.7 | Alive | 6 | 1.00 |
| 334666 | 16.71 | 16.54 | Alive | 14 | 1.00 |
| 252906 | 17.8 | 18.06 | Alive | 116 | 1.00 |
| 137633 | 17.77 | 17.87 | Alive | 152 | 0.99 |
| 178930 | 17.53 | 16.9 | Alive | 387 | 0.99 |
| 200871 | 18.04 | 18.23 | Alive | 156 | 0.99 |
| 261891 | 17.83 | 18.14 | Alive | 49 | 0.99 |
| 303333 | 17.6 | 17.72 | Alive | 59 | 0.99 |
| 16 | 17.32 | 17.14 | Alive | 87 | 0.99 |
| 322324 | 17.28 | 17.13 | Alive | 32 | 0.98 |
| 56 | 18.63 | 18.74 | Alive | 233 | 0.98 |
| 59 | 17.77 | 17.58 | Alive | 161 | 0.98 |
| 1 | 18.08 | 17.93 | Alive | 192 | 0.98 |

(continued)

| Entropy R-sq = 0.323 | | | | | Long |
|---|---|---|---|---|---|
| 4-gene model | | | | | term |
| | | | June 20 08 | #Weeks | survivor |
| SourceID | Abl2Sema4d | C1qaTimp1 | Status | Exposed | Prob |
| 279316 | 17.43 | 17.15 | Alive | 119 | 0.98 |
| 72 | 17.34 | 16.53 | Alive | 281 | 0.97 |
| 103398 | 17.4 | 16.76 | Alive | 218 | 0.97 |
| 221617 | 18.07 | 18.04 | Alive | 97 | 0.96 |
| 109722 | 18.56 | 18.16 | Alive | 346 | 0.95 |
| 50223520 | 17.46 | 17.09 | Alive | 78 | 0.94 |
| 78 | 17.27 | 16.68 | Alive | 124 | 0.94 |
| 295740 | 17.98 | 17.84 | Alive | 82 | 0.93 |
| 185401 | 17.38 | 16.62 | Alive | 139 | 0.87 |
| 48 | 17.58 | 16.73 | Alive | 165 | 0.77 |
| 26 | 18.57 | 18.39 | Alive | 87 | 0.72 |
| 50254384 | 18.36 | 17.86 | Alive | 59 | 0.40 |
| 272956 | 18.31 | 17.34 | Alive | 92 | 0.10 |
| | | | | | |
| 6 | 18.91 | 17.32 | Dead | 110 | 0.00 |
| 9 | 17.97 | 16.46 | Dead | 49 | 0.00 |
| 31 | 18.5 | 17.27 | Dead | 87 | 0.00 |
| 32 | 18.74 | 17.75 | Dead | 104 | 0.00 |
| 44 | 18.61 | 16.73 | Dead | 97 | 0.00 |
| 46 | 17.59 | 16.04 | Dead | 99 | 0.00 |
| 57 | 18.51 | 17.52 | Dead | 77 | 0.00 |
| 63 | 18.62 | 18.13 | Dead | 202 | 0.00 |
| 88 | 18.14 | 16.27 | Dead | 115 | 0.00 |
| 124 | 17.99 | 17.09 | Dead | 77 | 0.00 |
| 219180 | 17.38 | 16.27 | Dead | 115 | 0.00 |
| 275979 | 17.94 | 17.56 | Dead | 68 | 0.00 |
| 290701 | 17.78 | 17.2 | Dead | 33 | 0.00 |
| 294238 | 18.35 | 17.78 | Dead | 64 | 0.00 |
| 323394 | 19.04 | 17.09 | Dead | 12 | 0.00 |

**Table 8:**

Comparison of Zero-Inflated Poisson models with Different Genes

**Table 9**: Predicted Probability of Transition from Alive to Dead State based on the Markov Survival Model ABL2 and C1QA

| SourceID | ABL2 | C1QA | Predicted Hazard Rate | | | | | Risk Score =10+1.204*ABL2-1.455*C1QA ABL2=21.71; C1QA=19.08 | |
| | | | Period = | | | | | | June 20 2008 |
| SourceID | ABL2 | C1QA | 1 | 2-3 | 4 | 5 | 6+ | Risk Score | Status |
|---|---|---|---|---|---|---|---|---|---|
| 44 | 21.71 | 19.08 | 0.1872 | 0.1660 | 0.2979 | 0.3070 | 0.3801 | 8.39 | Dead |
| 46 | 20.46 | 18.11 | 0.1738 | 0.1538 | 0.2793 | 0.2880 | 0.3590 | 8.29 | Dead |
| 88 | 20.97 | 18.66 | 0.1477 | 0.1303 | 0.2420 | 0.2500 | 0.3157 | 8.11 | Dead |
| 9 | 21.07 | 18.87 | 0.1264 | 0.1112 | | | | 7.92 | Dead |
| 219180 | 20.25 | 18.44 | 0.0916 | 0.0802 | 0.1567 | 0.1624 | 0.2116 | 7.56 | Dead |
| 323394 | 21.97 | 19.88 | 0.0900 | | | | | 7.54 | Dead |
| 31 | 21.65 | 19.81 | 0.0691 | 0.0603 | 0.1204 | 0.1250 | 0.1651 | 7.25 | Dead |
| 6 | 22.00 | 20.12 | 0.0678 | 0.0591 | 0.1182 | 0.1227 | 0.1622 | 7.22 | Dead |
| 103398 | 20.24 | 18.97 | 0.0444 | 0.0386 | 0.0788 | 0.0820 | 0.1100 | 6.78 | Alive |
| 290701 | 20.84 | 19.46 | 0.0443 | 0.0385 | | | | 6.79 | Dead |
| 185401 | 20.17 | 19.07 | 0.0354 | 0.0308 | 0.0634 | 0.0660 | 0.0891 | 6.55 | Alive |
| 32 | 21.89 | 20.56 | 0.0321 | 0.0279 | 0.0576 | 0.0600 | 0.0812 | 6.45 | Dead |
| 72 | 20.57 | 19.65 | 0.0247 | 0.0214 | 0.0446 | 0.0465 | 0.0632 | 6.19 | Alive |
| 124 | 20.94 | 19.97 | 0.0245 | 0.0213 | 0.0443 | 0.0462 | | 6.17 | Dead |
| 50254384 | 21.38 | 20.42 | 0.0218 | 0.0189 | 0.0395 | | | 6.04 | Alive |
| 57 | 21.64 | 20.68 | 0.0204 | 0.0177 | 0.0369 | 0.0385 | | 5.98 | Dead |
| 279316 | 20.53 | 19.76 | 0.0203 | 0.0176 | 0.0367 | 0.0383 | 0.0522 | 5.98 | Alive |
| 178930 | 20.13 | 19.44 | 0.0200 | 0.0173 | 0.0362 | 0.0377 | 0.0514 | 5.96 | Alive |
| 48 | 20.54 | 19.81 | 0.0192 | 0.0166 | 0.0348 | 0.0363 | 0.0496 | 5.92 | Alive |
| 16 | 20.51 | 19.92 | 0.0159 | 0.0137 | 0.0288 | 0.0301 | 0.0411 | 5.72 | Alive |
| 56 | 21.43 | 20.72 | 0.0149 | 0.0129 | 0.0271 | 0.0283 | 0.0387 | 5.66 | Alive |
| 1 | 20.87 | 20.28 | 0.0145 | 0.0126 | 0.0264 | 0.0275 | 0.0377 | 5.63 | Alive |
| 272956 | 21.42 | 20.77 | 0.0137 | 0.0119 | 0.0250 | 0.0261 | 0.0357 | 5.58 | Alive |
| 196141 | 21.06 | 20.58 | 0.0118 | 0.0102 | 0.0215 | 0.0224 | 0.0308 | 5.42 | Alive |
| 50223520 | 20.23 | 20.04 | 0.0095 | 0.0082 | 0.0174 | 0.0182 | | 5.21 | Alive |
| 322324 | 20.15 | 19.98 | 0.0095 | 0.0082 | | | | 5.20 | Alive |
| 109722 | 21.33 | 21.01 | 0.0088 | 0.0076 | 0.0162 | 0.0169 | 0.0232 | 5.12 | Alive |
| 334666 | 19.24 | 19.28 | 0.0087 | | | | | 5.12 | Alive |
| 78 | 20.10 | 20.05 | 0.0081 | 0.0070 | 0.0149 | 0.0155 | 0.0213 | 5.04 | Alive |
| 294238 | 21.21 | 21.01 | 0.0075 | 0.0065 | 0.0137 | | | 4.98 | Dead |
| 59 | 20.80 | 20.69 | 0.0074 | 0.0064 | 0.0135 | 0.0141 | 0.0195 | 4.95 | Alive |
| 295740 | 21.17 | 21.02 | 0.0071 | 0.0061 | 0.0130 | 0.0136 | | 4.92 | Alive |
| 63 | 22.05 | 21.82 | 0.0064 | 0.0055 | 0.0117 | 0.0122 | 0.0168 | 4.81 | Dead |

| | | | Predicted Hazard Rate | | | | | Risk Score =10+1.204*ABL2-1.455*C1QA |
| | | | Period = | | | | | ABL2=21.71; C1QA=19.08 | |
| | | | | | | | | | June 20 2008 |
| SourceID | ABL2 | C1QA | 1 | 2-3 | 4 | 5 | 6+ | Risk Score | Status |
|---|---|---|---|---|---|---|---|---|---|
| 155 | 20.27 | 20.41 | 0.0059 | 0.0051 | 0.0107 | 0.0112 | 0.0155 | 4.72 | Alive |
| 152331 | 20.58 | 20.71 | 0.0055 | 0.0048 | 0.0101 | 0.0105 | 0.0145 | 4.66 | Alive |
| 336476 | 20.63 | 20.88 | 0.0046 | | | | | 4.47 | Alive |
| 137633 | 20.49 | 20.76 | 0.0046 | 0.0040 | 0.0085 | 0.0088 | 0.0122 | 4.47 | Alive |
| 303333 | 20.07 | 20.42 | 0.0046 | 0.0039 | 0.0084 | | | 4.46 | Alive |
| 221617 | 20.90 | 21.21 | 0.0039 | 0.0034 | 0.0072 | 0.0075 | 0.0103 | 4.31 | Alive |
| 261891 | 20.62 | 21.07 | 0.0034 | 0.0029 | | | | 4.18 | Alive |
| 26 | 21.53 | 21.86 | 0.0033 | 0.0028 | 0.0060 | 0.0063 | 0.0086 | 4.13 | Alive |
| 233923 | 20.56 | 21.05 | 0.0033 | 0.0028 | 0.0061 | 0.0063 | 0.0087 | 4.14 | Alive |
| 50796156 | 20.13 | 20.71 | 0.0032 | 0.0028 | 0.0059 | | | 4.11 | Alive |
| 208893 | 20.31 | 20.95 | 0.0028 | 0.0024 | | | | 3.98 | Alive |
| 275979 | 20.77 | 21.39 | 0.0026 | 0.0022 | 0.0048 | | | 3.90 | Dead |
| 113 | 21.22 | 21.77 | 0.0025 | 0.0022 | 0.0046 | 0.0049 | 0.0067 | 3.88 | Alive |
| 223748 | 20.86 | 21.54 | 0.0023 | 0.0020 | 0.0043 | 0.0044 | 0.0061 | 3.79 | Alive |
| 249044 | 19.82 | 20.74 | 0.0021 | 0.0018 | 0.0039 | 0.0041 | 0.0056 | 3.70 | Alive |
| 187129 | 20.67 | 21.52 | 0.0019 | 0.0016 | 0.0035 | 0.0036 | 0.0050 | 3.59 | Alive |
| 196262 | 20.37 | 21.27 | 0.0019 | 0.0016 | 0.0035 | 0.0036 | 0.0050 | 3.59 | Alive |
| 252906 | 20.52 | 21.44 | 0.0018 | 0.0015 | 0.0033 | 0.0034 | 0.0047 | 3.52 | Alive |
| 164406 | 19.98 | 21.02 | 0.0017 | 0.0015 | 0.0031 | 0.0033 | 0.0045 | 3.48 | Alive |
| 200871 | 21.01 | 21.91 | 0.0016 | 0.0014 | 0.0030 | 0.0031 | 0.0043 | 3.43 | Alive |
| 229247 | 20.34 | 21.35 | 0.0016 | 0.0014 | 0.0030 | 0.0031 | 0.0043 | 3.44 | Alive |
| 244769 | 19.80 | 20.89 | 0.0016 | 0.0014 | 0.0030 | 0.0032 | 0.0044 | 3.45 | Alive |
| 279014 | 20.71 | 21.72 | 0.0015 | 0.0013 | 0.0027 | | | 3.34 | Alive |
| 322703 | 19.34 | 20.77 | 0.0011 | 0.0010 | | | | 3.07 | Alive |
| 229664 | 19.84 | 21.51 | 0.0007 | 0.0006 | 0.0013 | 0.0014 | 0.0019 | 2.60 | Alive |
| 99 | 21.15 | 22.72 | 0.0006 | 0.0005 | 0.0011 | 0.0011 | 0.0016 | 2.42 | Alive |
| 187770 | 19.78 | 21.60 | 0.0006 | 0.0005 | 0.0011 | 0.0011 | 0.0015 | 2.40 | Alive |
| 224210 | 19.83 | 22.05 | 0.0003 | 0.0003 | 0.0006 | 0.0006 | 0.0008 | 1.80 | Alive |
| 330355 | 20.18 | 22.46 | 0.0003 | 0.0002 | | | | 1.63 | Alive |

Table 10:

Consistently high risk of death is associated with low expression (high delta c) of gene 1 and high expression of gene 2 in model

| Regression Coefficients – Cox Model | | | Entropy | Incorrect Predictions | | Correct Predictions | | Cox-type model | | | | Zero-inflated Model | | Markov Model | |
|---|---|---|---|---|---|---|---|---|---|---|---|---|---|---|---|
| | | | | | | | | Likelihood Ratio | | Wald Statistic | | Wald Statistic | | Wald Statistic | |
| gene 1 | top 25 2-gene models | gene 2 | R-sq | Alive | Dead | Alive | Dead | p-val 1 | p-val 2 | p-val 1 | p-val 2 | wald pval1 | wald pval2 | wald pval1 | wald pval2 |
| 2.3 ABL2 | C1QA | -1.3 | 0.21 | 3/47 | 2/15 | 94% | 87% | 3.0E-07 | 2.2E-06 | 1.7E-06 | 1.5E-05 | 0.0039 | 0.0065 | 2.8E-05 | 2.7E-06 |
| 3.4 SEMA4D | TIMP1 | -2.4 | 0.19 | 7/47 | 2/15 | 85% | 87% | 1.2E-07 | 2.3E-06 | 1.2E-06 | 8.1E-05 | 0.003 | 0.017 | 7.7E-05 | 1.7E-05 |
| 6.8 SEMA4D | MYD88 | -4.7 | 0.18 | | | | | 1.7E-06 | 1.6E-08 | 3.2E-05 | 2.1E-06 | | | | |
| 7.4 SEMA4D | SV1L | -4.2 | 0.17 | | | | | 5.3E-08 | 4.1E-06 | 2.2E-06 | 2.4E-05 | | | | |
| 2.3 ITGAL | CDKN1A | -2.8 | 0.17 | | | | | 2.9E-07 | 1.7E-06 | 7.7E-06 | 7.7E-05 | | | | |
| 1.6 ABL2 | C1QB | -1.8 | 0.17 | | | | | 4.4E-06 | 0.0001 | 0.0001 | 0.0004 | | | | |
| 3.1 ABL2 | PYCARD | -3.0 | 0.17 | | | | | 5.0E-08 | 0.0001 | 1.2E-06 | 0.0010 | | | | |
| 3.4 ABL2 | MNDA | -2.7 | 0.17 | | | | | 5.7E-08 | 0.0001 | 4.2E-06 | 0.0008 | | | | |
| | CDKN1A | SMAD3 | 0.16 | | | | | 3.7E-07 | 4.0E-05 | 3.4E-05 | 6.1E-05 | | | | |
| | ABL2 | CDKN1A | 0.16 | | | | | 4.2E-05 | 0.0002 | 7.6E-05 | 0.0026 | | | | |
| | S100A11 | SEMA4D | 0.16 | | | | | 1.3E-05 | 1.1E-07 | 6.3E-05 | 2.2E-06 | | | | |
| | CCL5 | CDKN1A | 0.16 | | | | | 4.8E-05 | 1.1E-07 | 0.0004 | 2.9E-05 | | | | |
| | ABL2 | ST14 | 0.16 | | | | | 1.6E-07 | 0.0003 | 7.4E-06 | 0.0042 | | | | |
| | C1QB | SEMA4D | 0.16 | | | | | 1.7E-05 | 0.0001 | 0.0002 | 0.0003 | | | | |
| | ABL2 | TIMP1 | 0.16 | | | | | 1.8E-06 | 0.0004 | 2.6E-06 | 0.0022 | | | | |
| | NFATC2 | RHOC | 0.16 | | | | | 2.1E-07 | 2.0E-06 | 2.7E-06 | 1.9E-05 | | | | |
| | CDKN1A | TGFB1 | 0.15 | | | | | 2.5E-07 | 9.7E-05 | 5.4E-06 | 0.0002 | | | | |
| | CDKN1A | NFATC2 | 0.15 | | | | | 2.7E-06 | 0.0001 | 0.0001 | 0.0003 | | | | |
| | MNDA | SEMA4D | 0.15 | | | | | 3.2E-05 | 2.7E-07 | 0.0008 | 6.9E-05 | | | | |
| | ABL2 | CDKN1A | 0.15 | | | | | 0.0001 | 9.4E-07 | 0.0002 | 3.5E-05 | | | | |
| | SEMA4D | TEGT | 0.15 | | | | | 3.0E-07 | 3.5E-05 | 5.9E-06 | 0.0002 | | | | |
| | BRCA1 | C1QB | 0.15 | | | | | 0.0003 | 4.9E-07 | 0.0014 | 1.2E-05 | | | | |
| | SEMA4D | SERPINA1 | 0.15 | | | | | 3.4E-07 | 4.1E-05 | 6.0E-06 | 9.3E-05 | | | | |
| | C1QB | SERPING2 | 0.15 | | | | | 5.9E-07 | 0.0004 | 8.2E-05 | 0.0022 | | | | |
| | RBM5 | TIMP1 | 0.15 | | | | | 4.8E-06 | 3.0E-06 | 6.2E-05 | 0.0001 | | | | |

**Table 11**: Summary of Wald p-values obtained from Cox-Type, Zero Inflated Poisson and Markov Survival Models

| 2-gene models and | | Entropy | Cox-type model | | Zero-Inflated Model | | Markov Model | |
|---|---|---|---|---|---|---|---|---|
| 1-gene models | | R-sq | wald pval1 | wald pval2 | wald pval1 | wald pval2 | wald pval1 | wald pval2 |
| ABL2 | C1QA | 0.21 | 1.7E-06 | 1.5E-05 | 0.0039 | 0.0065 | 2.8E-05 | 2.7E-06 |
| SEMA4D | TIMP1 | 0.19 | 1.2E-06 | 8.1E-05 | 0.003 | 0.017 | 7.7E-05 | 1.7E-05 |
| ABL2 | | 0.09 | 0.0001 | | 0.0041 | | 0.0005 | |

**Table 12**:

Model Comparisons: 2-gene model containing genes ABL2 & C1QA

Sorted by Exposure

Markov Risk Score = f(2.07*ABL2-1.08*C1QA)
Cox Risk Score =2.26*ABL2-1.31*C1QA
2P Risk Score = Prob of high risk of death

| | | | Model-based Risk Score | | | June 20 2008 | Exposure | |
|---|---|---|---|---|---|---|---|---|
| SourceID | ABL2 | C1QA | Markov | 2P | Cox | Status | CTC | # weeks |
| 336476 | 20.63 | 20.28 | 0.005 | 0.06 | 19.03 | Alive | 68 | 6 |
| 313334 | 21.57 | 19.88 | 0.177 | N/A | 23.61 | Dead | ? | 12 |
| 334686 | 19.24 | 18.28 | 0.001 | 0.00 | 18.23 | Alive | ? | 14 |
| 330355 | 20.18 | 21.86 | 0.005 | 0.00 | 16.58 | Alive | ? | 20 |
| 322324 | 20.15 | 19.98 | 0.004 | 0.05 | 19.37 | Alive | ? | 32 |
| 293703 | 20.84 | 19.46 | 0.031 | N/A | 21.61 | Dead | 8 | 33 |
| 322793 | 19.34 | 20.77 | 0.000 | 0.00 | 16.50 | Alive | ? | 33 |
| 298835 | 20.33 | 20.03 | 0.002 | 0.01 | 18.46 | Alive | ? | 46 |
| 9 | 21.07 | 18.87 | 0.090 | N/A | 22.90 | Dead | 4 | 49 |
| 261831 | 20.62 | 21.07 | 0.004 | 0.05 | 19.03 | Alive | 263 | 69 |
| 50254384 | 21.38 | 20.42 | 0.002 | 0.74 | 21.57 | Alive | 15 | 59 |
| 303333 | 20.07 | 19.42 | 0.004 | 0.01 | 18.61 | Alive | ? | 59 |
| 50796156 | 20.13 | 20.71 | 0.002 | 0.01 | 18.36 | Alive | 22 | 60 |
| 291238 | 21.13 | 21.01 | 0.013 | N/A | 20.41 | Dead | 80 | 64 |
| 273979 | 20.77 | 21.39 | 0.004 | N/A | 18.92 | Dead | 7 | 68 |
| 279084 | 20.71 | 21.72 | 0.002 | 0.02 | 18.35 | Alive | 5 | 68 |
| 57 | 21.64 | 20.68 | 0.044 | N/A | 21.82 | Dead | 132 | 77 |
| 124 | 20.94 | 18.97 | 0.023 | N/A | 21.36 | Dead | 13 | 77 |
| 50223529 | 20.23 | 20.04 | 0.005 | 0.05 | 19.47 | Alive | 0 | 78 |
| 293740 | 21.17 | 21.62 | 0.012 | 0.24 | 20.31 | Alive | 49 | 82 |
| 31 | 21.65 | 19.81 | 0.010 | N/A | 22.98 | Dead | 92 | 87 |
| 28 | 21.53 | 21.86 | 0.010 | 0.20 | 20.02 | Alive | 931 | 87 |
| 16 | 20.33 | 19.92 | 0.106 | 0.13 | 20.26 | Alive | 0 | 87 |
| 272366 | 21.42 | 29.77 | 0.026 | 0.55 | 21.20 | Alive | 44 | 92 |
| 66 | 21.71 | 19.08 | 0.228 | N/A | 24.07 | Dead | ? | 97 |
| 221617 | 20.90 | 21.21 | 0.006 | 0.06 | 19.45 | Alive | 1 | 97 |
| 46 | 20.46 | 18.11 | 0.060 | N/A | 22.52 | Dead | 114 | 99 |
| 32 | 21.89 | 29.56 | 0.079 | N/A | 22.54 | Dead | 60 | 108 |
| 224210 | 19.83 | 22.06 | 0.000 | 0.00 | 16.82 | Alive | 0 | 108 |
| 6 | 22.00 | 29.12 | 0.156 | N/A | 23.36 | Dead | 4 | 110 |
| 88 | 20.97 | 18.65 | 0.081 | N/A | 22.35 | Dead | 0 | 113 |
| 218380 | 20.25 | 18.44 | 0.028 | N/A | 21.63 | Dead | 1 | 115 |
| 252006 | 20.52 | 21.84 | 0.002 | 0.01 | 18.29 | Alive | 6 | 116 |
| 244769 | 19.80 | 19.83 | 0.001 | 0.00 | 17.38 | Alive | 15 | 118 |
| 279316 | 20.53 | 19.76 | 0.012 | 0.18 | 20.51 | Alive | 0 | 118 |
| 78 | 20.10 | 20.05 | 0.004 | 0.01 | 18.16 | Alive | 1 | 128 |
| 184406 | 18.96 | 21.02 | 0.001 | 0.00 | 17.61 | Alive | 60 | 138 |
| 185401 | 20.17 | 19.07 | 0.012 | 0.08 | 20.60 | Alive | 0 | 133 |
| 137633 | 20.49 | 20.76 | 0.004 | 0.01 | 18.11 | Alive | 0 | 152 |
| 293871 | 21.03 | 21.91 | 0.004 | 0.01 | 18.78 | Alive | 0 | 156 |
| 155 | 20.27 | 20.81 | 0.003 | 0.01 | 19.07 | Alive | 0 | 156 |
| 59 | 20.80 | 20.63 | 0.003 | 0.05 | 19.90 | Alive | 2 | 161 |
| 68 | 20.54 | 19.21 | 0.012 | 0.08 | 20.87 | Alive | 0 | 165 |
| 152531 | 20.58 | 20.71 | 0.005 | 0.02 | 18.38 | Alive | 0 | 177 |
| 187129 | 20.67 | 21.52 | 0.003 | 0.00 | 18.52 | Alive | 5 | 186 |
| 248044 | 19.82 | 29.74 | 0.001 | 0.00 | 17.62 | Alive | 66 | 191 |
| 1 | 20.87 | 20.28 | 0.014 | 0.09 | 20.60 | Alive | 3 | 192 |
| 83 | 22.05 | 21.82 | 0.030 | N/A | 21.25 | Dead | 2 | 202 |
| 39 | 21.15 | 21.22 | 0.002 | 0.00 | 18.04 | Alive | 0 | 205 |
| 103398 | 20.24 | 18.97 | 0.016 | 0.06 | 20.89 | Alive | 0 | 218 |
| 198262 | 20.37 | 21.27 | 0.002 | 0.00 | 18.17 | Alive | 3 | 225 |
| 233923 | 20.56 | 21.05 | 0.003 | 0.00 | 18.89 | Alive | 2 | 228 |
| 56 | 21.43 | 20.22 | 0.027 | 0.21 | 21.29 | Alive | 0 | 233 |
| 223247 | 20.34 | 21.35 | 0.002 | 0.03 | 18.00 | Alive | 0 | 240 |
| 209664 | 19.84 | 21.51 | 0.000 | 0.00 | 16.66 | Alive | 13 | 241 |
| 223748 | 20.86 | 21.54 | 0.004 | 0.03 | 18.93 | Alive | 1 | 243 |
| 187779 | 18.78 | 21.60 | 0.000 | 0.00 | 16.61 | Alive | 0 | 269 |
| 72 | 20.57 | 18.65 | 0.015 | 0.03 | 20.75 | Alive | 0 | 281 |
| 196161 | 21.06 | 20.58 | 0.015 | 0.03 | 20.64 | Alive | 61 | 320 |
| 108720 | 21.33 | 21.01 | 0.017 | 0.03 | 20.88 | Alive | 0 | 346 |
| 178989 | 20.13 | 19.84 | 0.008 | 0.00 | 20.05 | Alive | 0 | 387 |
| 113 | 21.22 | 21.77 | 0.006 | 0.03 | 18.44 | Alive | 3 | 490 |

**Table 13**:

Model Comparisons: 2-gene model containing genes ABL2 & C1QA

Sorted by Cox Risk Score within Status

Markov Risk Score = f(2.07*ABL2-1.98*C1QA)

Cox Risk Score = 2.26*ABL2-1.31*C1QA

2IP Risk Score = Prob of high risk of death

| | | | Model-based Risk Score | | | June 20 2008 | (B)-(A) | A | B | C |
|---|---|---|---|---|---|---|---|---|---|---|
| SourceID | ABL2 | C1QA | Markov | 2IP | Cox | Status | CTC # weeks | Exposure Cohort 4 | Date of Death(Censor) | Blood draw |

Table 14:

## Stable Risk Scores Obtained from Additional Blood Draw

| Patient ID# | Lab Date | ABL2 | C1QA | Risk Score Markov | Cox | ZIP |
|---|---|---|---|---|---|---|
| 2 Alive Patients | | | | | | |
| 072 (1st draw) | 1/11/2007 | 20.57 | 19.65 | 21.4 | 20.7 | 23.7 |
| 111457 (2nd draw) | 4/5/2007 | 20.90 | 20.55 | 21.1 | 20.3 | 23.5 |
| | | | | | | |
| 156 (1st draw) | 3/1/2007 | 21.27 | 22.70 | 19.5 | 18.3 | 22.3 |
| 279014 (2nd draw) | 4/12/2007 | 20.71 | 21.72 | 19.4 | 18.4 | 22.0 |
| | | | | | | |
| 1 Dead Patient | | | | | | |
| 124 (1st draw) | 1/22/2007 | 20.94 | 19.97 | 21.8 | 21.2 | 24.2 |
| 250157 (2nd draw) | 4/5/2007 | 21.38 | 20.84 | 21.8 | 21.0 | 24.2 |

Legend:
Shaded           Used in Model

Markov Risk Score = 2.07*ABL2 - 1.08*C1QA
Cox Risk Score    = 2.26*ABL2 - 1.31*C1QA
ZIP Risk Score    = 2.05*ABL2 - 0.94*C1QA

**Table 15:** Comparison of p-values for selected significant genes from Cox models estimated using different definitions of survival time.

Survival time measured from: cohort 4 status blood draw

| gene | p-val | p-Val |
|---|---|---|
| ABL2 | 8.1E-05 | 3.1E-04 |
| CAV2 | 7.9E-04 | 9.7E-04 |
| SEMA4D | 0.0011 | 0.0017 |
| C1QB | 0.0013 | 8.2E-04 |
| C1QA | 0.0028 | 0.0043 |
| NFATC2 | 0 0044 | 0.0026 |
| COKN1A | 0.0058 | 0.0040 |
| ITGAL | 0.0071 | 0.0098 |
| BCL2 | 0.0094 | 0.0036 |
| CREBBP | 0.0120 | 0 0262 |
| MYC | 0.0131 | 0.0146 |
| XK | 0.0142 | 0.0093 |
| FGF2 | 0.0151 | 0.029 |
| E2F1 | 0.0152 | 0.0266 |
| RBM5 | 0.0160 | 0 0095 |
| NUDT4 | 0.0201 | 0.0176 |
| BCAM | 0.0204 | 0.083 |
| SRF | 0.0262 | 0.0436 |
| PTCH1 | 0.0265 | 0.0074 |
| JUN | 0.0299 | 00271 |
| SMAD3 | 0.0317 | 0.0123 |
| ABL1 | 0.0358 | 0.0148 |

(continued)

Survival time measured from: cohort 4 status blood draw

| gene | p-val | p-Val |
|---|---|---|
| E2F5 | 0.0380 | 0.0048 |
| KAI1 | 0.0393 | 0.0092 |
| SMAD4 | 0.0400 | 0.0128 |
| SPARC | 0 0450 | 0.0168 |
| SIAH2 | 0.0453 | 0.0219 |
| ICAM1 | 0.0488 | 0.0370 |

**Table 16**: Comparison of effects in the best 2-gene models obtained under different definitions of survival time

| 2-gene Cox model with survival time measured from cohort 4 status | | | | | | | |
|---|---|---|---|---|---|---|---|
| | B | SE | Wald | df | Sig. | | |
| ABL2 | 2.09 | 0.487 | 18.3 | 1 | 1.85E-05 | | |
| C1QA | -1.08 | 0.297 | 13.1 | 1 | 0.000288 | | |
| | | | | | | | |
| 2-gene Cox model with survival time measured from blood draw | | | | | | | |
| | B | SE | Wald | df | Sig. | | |
| ABL2 | 1.91 | 0.451 | | 1 | 2.31E-05 | | |
| C1QA | -1.15 | 0.326 | 12.5 | 1 | 0.000417 | | |

**Table 17**: Comparison of effects in the second best 2-gene models obtained under different definitions of survival time.

| 2-gene Cox model with survival time measured from Cohort 4 statu | | | | | |
|---|---|---|---|---|---|
| | B | SE | Wald | df | Sig. |
| SEMA4D | 2.99 | 0.677 | 19.6 | 1 | 9.7E-06 |
| TIMP1 | -1.90 | 0.590 | 10.4 | 1 | 1.2E-03 |
| | | | | | |
| 2-gene Cox model with survival time measured from blood draw | | | | | |
| | B | SE | Wald | df | Sig. |
| SEMA4D | 2.70 | 0.609 | 19.7 | 1 | 9.2E-06 |
| TIMP1 | -1.90 | 0.613 | 9.6 | 1 | 2.0E-03 |

**Table 18**: Follow-up Validation Study Design

| time since start of hormone refractory disease | | | | | |
|---|---|---|---|---|---|
| | | high risk score expected | | | |
| | target sample | expected % | number | expected # deaths within lyr of bld draw | power |
| < 1 yr | 100 | 30% | 30 | 20 | 99% |
| 12-26 months | 100 | 40% | 40 | 40 | |
| >26 months | 50 | 5% | 2 | 1 | |
| | | | | 61 | |
| < 1 yr | 50 | 30% | 15 | 10 | 97% |
| 12-26 months | 50 | 40% | 20 | 20 | |
| >26 months | 25 | 5% | 1 | 0 | |

(continued)

| time since start of hormone refractory disease | | | | |
|---|---|---|---|---|
| | | high risk score expected | | |
| | target sample | expected % | number | expected # deaths within lyr of bld draw | power |
| | | | | 30 | |
| < 1 yr | 20 | 30% | 6 | 4 | 70% |
| 12-26 months | 20 | 40% | 8 | 8 | |
| >26 months | 10 | 5% | 0 | | |
| | | | | 12 | |

**Table 19**: Target gene mean differences

**DFCI Hormone-Refractory Cohort N=62**
**July 2008 Analysis**

| Source MDx Gene panel | | Dead Δct | Alive Δct | Cox p-value | mean diff dead vs Alive |
|---|---|---|---|---|---|
| cancer general | ABL2 | 21.29 | 20.50 | 1.22E-04 | -0.8 |
| Prostate Spec. | CAV2 | 22.85 | 24.09 | 2.53E-04 | 1.2 |
| cross cancer | C1QB | 20.14 | 21.24 | 2.94E-04 | 1.1 |
| cancer general | CDKN1A | 16.22 | 16.79 | 6.69E-04 | 0.6 |
| Inflammation | C1QA | 19.86 | 20.80 | 9.61E-04 | 0.9 |
| cancer general | SEMA4D | 15.25 | 14.76 | 2.45E-03 | -0.5 |
| cross cancer | XK | 16.53 | 17.70 | 1.05E-02 | 1.2 |
| Prostate Spec. | FGF2 | 23.43 | 24.44 | 1.19E-02 | 1.0 |
| Prostate Spec. | BCAM | 18.75 | 20.26 | 2.29E-02 | 1.5 |
| cross cancer | NUDT4 | 15.04 | 15.95 | 2.63E-02 | 0.9 |
| cancer general | BCL2 | 18.56 | 17.66 | 2.80E-02 | -0.9 |
| EGR1 | NFATC2 | 17.44 | 16.62 | 3.03E-02 | -0.8 |
| cancer general | TIMP1 | 14.34 | 14.67 | 3.12E-02 | 0.3 |
| EGR1 | CREBBP | 15.53 | 15.10 | 3.39E-02 | -0.4 |
| cancer general | E2F1 | 20.08 | 20.71 | 4.23E-02 | 0.6 |
| cancer general | MYC | 18.95 | 18.28 | 4.44E-02 | -0.7 |
| Prostate Spec. | NCOA4 | 11.25 | 11.88 | 4.60E-02 | 0.6 |
| Inflammation | ELA2 | 18.31 | 19.56 | 4.67E-02 | 1.2 |
| cross cancer | SPARC | 14.62 | 15.24 | 4.69E-02 | 0.6 |
| cross cancer | RP51077B9.4 | 16.57 | 16.75 | 4.89E-02 | 0.2 |
| cross cancer | ITGAL | 15.63 | 15.10 | 5.16E-02 | -0.5 |
| cross cancer | RBM5 | 16.34 | 15.91 | 5.31E-02 | -0.4 |
| cross cancer | SIAH2 | 12.86 | 13.78 | 5.99E-02 | 0.9 |
| cancer general | PTCH1 | 21.27 | 20.54 | 6.18E-02 | -0.7 |
| cancer general | SOCS1 | 17.29 | 17.01 | 6.28E-02 | -0.3 |

Best 6 Gene Model

**Table 20**: Proteins corresponding to genes differentially expressed in long term vs. short term prostate cancer survivors

| Gene Symbol | Protein | Accession Number |
|---|---|---|
| ABCC1 | Multidrug resistance-associated protein 1; ATP-binding cassette, sub-family C, member 1 isoform 6 | NP_063956, NP_063957, NP_004987, NP_063915, NP_063953, NP_063954, NP 063955 |
| ABL1 | Abelson murine leukemia viral (v-abl) oncogene homolog 1 | NP_005148, NP_009297 |
| ABL2 | v-abl Abelson murine leukemia viral oncogene homolog 2 | NP_001093578, NP_005149, NP_009298 |

(continued)

| Gene Symbol | Protein | Accession Number |
|---|---|---|
| BCAM | Lutheran blood group glycoprotein precursor, (basal cell adhesion molecule) | NP_001013275, NP_005572 |
| BCL2 | B-cell CLL/lymphoma 2 | NP 000624, NP 000648 |
| C1QA | complement component 1, q subcomponent, A chain | NP_057075 |
| C1QB | complement component 1, q subcomponent, B chain | NP_000482 |
| CAV2 | Caveolin 2 | NP 001224, NP 937855 |
| CDKN1A | Cvclin-dependent kinase inhibitor 1 | NP 000380, NP 510867 |
| CREBBP | CREB-binding protein | NP 001073315, NP 004371 |
| CTSD | Cathepsin D precursor | NP 001900 |
| E2F1 | Transcription factor E2F 1 | NP 005216 |
| ELA2 | Elastase 2, neutrophil preproprotein | NP 001963 |
| FGF2 | Fibroblast growth factor 2 | NP 001997 |
| ICAM1 | Intercellular adhesion molecule 1 precursor | NP 000192 |
| IL8 | Interleukin-8 precursor | NP 000575 |
| IRAK3 | interleukin-1 receptor-associated kinase 3 | NP 009130 |
| ITGAL | Integrin alpha-L precursor | NP 002200 |
| MYC | Myc proto-oncogene protein; transcription factor 64 | NP_002458 |
| NFATC2 | Nuclear factor of activated T-cells, cytoplasmic 2 | NP 036472, NP 775114 |
| NFKB1 | Nuclear factor NF-kappa-B p105 subunit | NP 003989 |
| NUDT4 | Diphosphoinositol polyphosphate phosphohydrolase 2; nudix (nucleoside diphosphate linked moiety X)-type motif 4 | NP_061967, NP_950241 |
| PLA2G7 | Platelet-activating factor acetylhydrolase precursor | NP_005075 |
| PTCH1 | Patched isoform L | NP_000255, NP_001077071, NP_ 001077072, NP_001077073, NP_ 001077074, NP 001077075, NP 001077076 |
| RBM5 | RNA-binding motif protein 5 | NP 005769 |
| SEMA4D | Semaphorin-4D | NP 006369 |
| SIAH2 | Seven in absentia homolog 2 | NP 005058 |
| SMAD3 | Mothers against decapentaplegic homolog 3 | NP 005893 |

(continued)

| Gene Symbol | Protein | Accession Number |
|---|---|---|
| SMAD4 | Mothers against decapentaplegic homolog 4 | NP 005350 |
| TIMP1 | Metalloproteinase inhibitor 1 precursor | NP 003245 |
| TP53 | Cellular tumor antigen p53 | NP 000537 |
| TXNRD1 | Thioredoxin reductase 1, cytoplasmic precursor | NP_001087240, NP_00332, NP_877393, NP 877419, NP 877420 |
| XK | Membrane transport protein XK: X-linked Kx blood group (McLeod syndrome) | NP_066569 |

**Table 21**: 18-gene Custom Precision Profile™ for Cell Fractionation Study

| Gene Symbol | Gene Name | Gene Description | Function / Process |
|---|---|---|---|
| ABL2 | v-abl Abelson murine leukemia viral oncogene homolog 2 (arg, Abelson-related gene) | Encodes a member of the Abelson family of nonreceptor tyrosine protein kinase. The protein is highly similar to the ABL1 protein, including the tyrosine kinase, SH2 and SH3 domains, and has a role in cytoskeletal rearrangements by its C-terminal F-actin- and microtubule-binding sequences. This gene is expressed in both normal and tumor cells, and is involved in translocation with the ETV6 gene in leukemia. | Protein tyrosine kinase activity, nucleotide binding / Cell adhesion, signal transduction, protein amino acid phosphorylation |
| C1QA | complement component 1, q subcomponent, A chain | This gene encodes a major constituent of the human complement subcomponent C1q. C1q associates with C1r and C1s in order to yield the first component of the serum complement system. Deficiency of C1q has been associated with lupus erythematosus and glomerulonephritis. C1q is composed of 18 polypeptide chains: six A-chains, six B-chains, and six C-chains. This gene encodes the A-chain polypeptide of human complement subcomponent C1q | Complement component C1 complex / Cell-cell signaling, inate immune response |

(continued)

| Gene Symbol | Gene Name | Gene Description | Function / Process |
|---|---|---|---|
| CDKN1A | cyclin-dependent kinase inhibitor 1A (p21, Cip1) | This gene encodes a potent cyclin-dependent kinase inhibitor. The encoded protein binds to and inhibits the activity of cyclin-CDK2 or -CDK4 complexes, and thus functions as a regulator of cell cycle progression at G1. The expression of this gene is tightly controlled by the tumor suppressor protein p53, through which this protein mediates the p53-dependent cell cycle G1 phase arrest in response to a variety of stress stimuli. This protein can interact with proliferating cell nuclear antigen (PCNA), a DNA polymerase accessory factor, and plays a regulatory role in S phase DNA replication and DNA damage repair. This protein was reported to be specifically cleaved by CASP3-like caspases, which thus leads to a dramatic activation of CDK2, and may be instrumental in the execution of apoptosis following caspase activation. | Protein kinase inhibitor activity / cellular response to external signals, negative regulation of cell cycle, apoptosis, cell growth and proliferation, cyclin-dependent protein kinase activity and response to DNA damage stimulus |
| ITGAL | integrin, alpha L (antigen CD11A (p180), lymphocyte function-associated antigen 1; alpha polypeptide) | ITGAL encodes the integrin alpha L chain. Integrins are heterodimeric integral membrane proteins composed of an alpha chain and a beta chain. Alpha integrin combines with the beta 2 chain (ITGB2) to form the integrin lymphocyte function-associated antigen-1 (LFA-1), which is expressed on all leukocytes LFA-1 plays a central role in leukocyte intercellular adhesion through interactions with its ligands, ICAMs 1-3 (intercellular adhesion molecules 1 through 3), and also functions in lymphocyte costimulatory signaling. | Cell adhesion molecule binding / Inflammatory response, T cell activation |

(continued)

| Gene Symbol | Gene Name | Gene Description | Function / Process |
|---|---|---|---|
| SEMA4D | sema domain, immunoglobulin domain (1g), transmembrane domain (TM) and short cytoplasmic domain, (semaphorin) 4D | First identified as a cell surface protein of resting T cells; previous studies had shown that it was involved in lymphocyte activation. SEMA4D is a member of the semaphorin family and the first semaphorin believed to be involved in the immune system. | Receptor activity / Cell adhesion, Anti-apoptosis, Immune reponse |
| TIMP1 | tissue inhibitor of metalloproteinase 1 | This gene belongs to the TIMP gene family. The proteins encoded by this gene family are natural inhibitors of the matrix metalloproteinases (MMPs), a group of peptidases involved in degradation of the extracellular matrix. It is also able to promote cell proliferation in a wide range of cell types, and may also have an anti-apoptotic function. Transcription of this gene is highly inducible in response to many cytokines and hormones. | Enzyme inhibitor / postive regulation of cell proliferation, negative regulation of membrane protein ectodomain proteolysis |
| CTSD | cathepsin D | This gene encodes a lysosomal aspartyl protease composed of a dimer of disulfide-linked heavy and light chains, both produced from a single protein precursor. This proteinase, which is a member of the peptidase C1 family, has a specificity similar to but narrower than that of pepsin A. Transcription of this gene is initiated from several sites, including one which is a start site for an estrogen-regulated transcript. Mutations in this gene are involved in the pathogenesis of several diseases, including breast cancer and possibly Alzheimer disease. | aspartic-type endopeptidase activity / peptidase activity / proteolysis |

(continued)

| Gene Symbol | Gene Name | Gene Description | Function / Process |
|---|---|---|---|
| IRAK3 | interleukin-1 receptor-associated kinase 3 | Is rapidly upregulated in human monocytes pre-exposed to tumor cells and could be involved in deactivation of tumor-infiltrating monocytes mediated by tumor cells. Human monocytes had enhanced expression of IRAK3 mRNA and protein in the presence of tumor cells, tumor cell supernatant, or hyaluronan. Blood monocytes from leukemia patients and patients with metastatic disease also overexpressed IRAK3. Monocyte deactivation by tumor cells involves IRAK3 upregulation and is mediated by hyaluronan engagement of CD44 and TLR4 | Protein serine/threonine kinase activity, nucleotide binding / cytokine-mediated signaling pathway, protein amino acid phosphorylation |
| PLA2G7 | phospholipase A2, group VII (platelet-activating factor acetylhydrolase, plasma) | The PLA2G7 gene encodes platelet-activating factor (PAF) acetylhydrolase (EC 3.1.1.47), a secreted enzyme that catalyzes the degradation of PAF to inactive products by hydrolysis of the acetyl group at the sn-2 position, producing the biologically inactive products LYSO-PAF and acetate. | Hydrolase activity / phospholipid binding / involved in the inflammatory and lipid catabolic processes |

(continued)

| Gene Symbol | Gene Name | Gene Description | Function / Process |
|---|---|---|---|
| TXNRD1 | thioredoxin reductase 1 | This gene encodes a member of the family of pyridine nucleotide oxidoreductases. This protein reduces thioredoxins as well as other substrates, and plays a role in selenium metabolism and protection against oxidative stress. The functional enzyme is thought to be a homodimer which uses FAD as a cofactor. Each subunit contains a selenocysteine (Sec) residue which is required for catalytic activity. The selenocysteine is encoded by the UGA codon that normally signals translation termination. The 3' UTR of selenocysteine-containing genes have a common stem-loop structure, the sec insertion sequence (SECIS), that is necessary for the recognition of UGA as a Sec codon rather than as a stop signal. Alternative splicing results in several transcript variants encoding the same or different isoforms. | Thioredoxin-disulfide reductase activity / cell redox homeostasis, signal transduction, transport |
| GAS1 | growth arrest-specific 1 | Growth arrest-specific 1 plays a role in growth suppression. GAS1 blocks entry to S phase and prevents cycling of normal and transformed cells. Gas1 is a putative tumor suppressor gene | Protein binding / regulation of apoptosis |

(continued)

| Gene Symbol | Gene Name | Gene Description | Function / Process |
|---|---|---|---|
| **HK1** | hexokinase 1 | Hexokinases phosphorylate glucose to produce glucose-6-phosphate, the first step in most glucose metabolism pathways. This gene encodes a ubiquitous form of hexokinase which localizes to the outer membrane of mitochondria. Mutations in this gene have been associated with hemolytic anemia due to hexokinase deficiency. Alternative splicing of this gene results in five transcript variants which encode different isoforms, some of which are tissue-specific. Each isoform has a distinct N-terminus; the remainder of the protein is identical among all the isoforms. | Hexokinase activity / glycolysis |
| **CD82** | CD82 molecule | This metastasis suppressor gene product is a membrane glycoprotein that is a member of the transmembrane 4 superfamily. Expression of this gene has been shown to be downregulated in tumor progression of human cancers and can be activated by p53 through a consensus binding sequence in the promoter. Its expression and that of p53 are strongly correlated, and the loss of expression of these two proteins is associated with poor survival for prostate cancer patients. | Protein binding |
| **CD14** | CD14 molecule | CD14 is a surface protein preferentially expressed on monocytes/macrophages. It binds lipopolysaccharide binding protein and recently has been shown to bind apoptotic cells | Protein binding / immune response, apoptosis |

(continued)

| Gene Symbol | Gene Name | Gene Description | Function / Process |
|---|---|---|---|
| **CD19** | CD19 molecule | Lymphocytes proliferate and differentiate in response to various concentrations of different antigens. The ability of the B cell to respond in a specific, yet sensitive manner to the various antigens is achieved with the use of low-affinity antigen receptors. This gene encodes a cell surface molecule which assembles with the antigen receptor of B lymphocytes in order to decrease the threshold for antigen receptor-dependent stimulation | Protein binding / B cell receptor signaling pathway |
| **NCAM1** | neural cell adhesion molecule 1 | NCAM is a membrane-bound glycoprotein that plays a role in cell-cell and cell-matrix adhesion through both its homophilic and heterophilic binding activity. NCAM shares many features with immunoglobulins and is considered a member of the immunoglobulin superfamily. | Protein binding / cell adhesion |
| **CD4** | CD4 molecule | CD4 is the official designation for T-cell antigen T4/leu3. CD4 binds to relatively invariant sites on class II major histocompatibility complex (MHC) molecules outside the peptide-binding groove, which interacts with the T-cell receptor (TCR). CD4 enhances T-cell sensitivity to antigen and binds to LCK (153390), which phosphorylates CD3Z. | MHC class II protein binding / immune response |
| **CD8A** | CD8A molecule | The CD8 antigen is a cell surface glycoprotein found on most cytotoxic T lymphocytes that mediates efficient cell-cell interactions within the immune system. The CD8 antigen acts as a corepressor with the T-cell receptor on the T lymphocyte to recognize antigens displayed by an antigen presenting cell (APC) in the context of class I MHC molecules | MHC class I protein binding / immune response |

Table 22: PRCA Cohort 4 Averaged Gene Expression Response Relative to PBMC's

| Cell Type | ABL2 | C1QA | CD14 | CD19 | CD4 | CD82 | CD8A | CDKN1A | CTSD |
|---|---|---|---|---|---|---|---|---|---|
| Enriched B Cells | 0.88 | 1.45 | 0.53 | 0.98 | 0.52 | 1.33 | 0.38 | 0.84 | 0.68 |
| Enriched Monocytes | 0.86 | 2.48 | 2.32 | 0.22 | 1.70 | 1.58 | 0.18 | 2.42 | 2.36 |
| Enriched NK Cells | 1.06 | 1.69 | 0.45 | 0.53 | 0.40 | 0.60 | 0.90 | 0.57 | 1.11 |
| Enriched T Cells | 1.31 | 0.35 | 0.11 | 0.21 | 0.67 | 0.63 | 1.54 | 0.21 | 0.26 |
| | | | | | | | | | |
| Depleted B Cells | 1.08 | 1.15 | 0.89 | 0.37 | 0.85 | 0.90 | 1.15 | 0.94 | 0.97 |
| DepletedMonocytes | 1.18 | 0.55 | 0.31 | 1.33 | 0.71 | 0.57 | 1.39 | 0.46 | 0.50 |
| Depleted NKCells | 0.87 | 0.90 | 0.96 | 0.93 | 0.98 | 0.98 | 1.13 | 1.07 | 0.95 |
| Depleted TCells | 0.79 | 1.83 | 1.62 | 1.48 | 1.16 | 1.16 | 0.56 | 1.63 | 1.65 |

| Cell Type | GAS1 | HK1 | IRAK3 | ITGAL | NCAM1 | PLA2G7 | SEMA4D | TIMP1 | TXNRD1 |
|---|---|---|---|---|---|---|---|---|---|
| Enriched B Cells | 0.47 | 1.01 | 0.53 | 0.38 | 0.26 | 0.53 | 0.50 | 0.76 | 0.48 |
| Enriched Monocytes | 0.94 | 1.52 | 2.72 | 0.91 | 0.30 | 3.11 | 0.90 | 2.21 | 1.82 |
| Enriched NK Cells | 2.18 | 1.28 | 0.64 | 1.94 | 6.25 | 0.36 | 1.01 | 0.75 | 0.92 |
| Enriched T Cells | 0.42 | 0.64 | 0.11 | 0.75 | 0.15 | 0.09 | 1.09 | 0.33 | 0.55 |
| | | | | | | | | | |
| Depleted B Cells | 1.27 | 0.84 | 0.92 | 1.09 | 1.01 | 0.94 | 0.94 | 0.95 | 1.07 |
| DepletedMonocytes | 0.68 | 0.91 | 0.39 | 1.08 | 1.16 | 0.39 | 1.08 | 0.54 | 0.80 |
| Depleted NKCells | 0.83 | 1.13 | 0.95 | 0.88 | 0.14 | 1.21 | 0.93 | 1.00 | 0.97 |
| Depleted TCells | 1.33 | 1.24 | 1.75 | 1.30 | 1.75 | 1.94 | 0.86 | 1.60 | 1.41 |

**Table 23**: MDNO Averaged Gene Expression Response Relative to PBMC's

| Cell Type | ABL2 | C1QA | CD14 | CD19 | CD4 | CD82 | CD8A | CDKN1A | CTSD |
|---|---|---|---|---|---|---|---|---|---|
| Enriched B Cells | 0.71 | 0.85 | 0.52 | 7.37 | 0.56 | 1.30 | 0.43 | 0.89 | 0.73 |
| Enriched Monocytes | 0.97 | 2.11 | 2.58 | 0.15 | 1.87 | 2.10 | 0.13 | 2.56 | 2.37 |
| Enriched NK Cells | 0.73 | 1.71 | 0.46 | 0.46 | 0.49 | 0.75 | 1.16 | 0.68 | 1.02 |
| Enriched T Cells | 0.93 | 0.27 | 0.06 | 0.10 | 0.49 | 0.30 | 1.17 | 0.13 | 0.17 |
| | | | | | | | | | |
| Depleted B Cells | 1.09 | 0.85 | 0.77 | 0.41 | 0.85 | 0.87 | 1.11 | 0.84 | 0.82 |
| Depleted Monocytes | 0.94 | 0.52 | 0.20 | 1.28 | 0.52 | 0.43 | 1.22 | 0.32 | 0.34 |
| Depleted NK Cells | 1.01 | 0.70 | 0.73 | 0.99 | 0.84 | 0.83 | 0.99 | 0.85 | 0.80 |
| Depleted T Cells | 1.05 | 1.41 | 1.62 | 2.14 | 1.21 | 1.56 | 0.78 | 1.64 | 1.52 |

| Cell Type | GAS1 | HK1 | IRAK3 | ITGAL | NCAM1 | PLA2G7 | SEMA4D | TIMP1 | TXNRD1 |
|---|---|---|---|---|---|---|---|---|---|
| Enriched B Cells | 0.59 | 0.78 | 0.59 | 0.36 | 0.31 | 0.65 | 0.56 | 0.97 | 0.61 |
| Enriched Monocytes | 0.95 | 1.73 | 2.70 | 0.83 | 0.27 | 3.12 | 0.94 | 2.93 | 2.06 |
| Enriched NK Cells | 3.94 | 1.29 | 0.75 | 1.91 | 6.74 | 0.38 | 0.91 | 0.99 | 0.87 |
| Enriched T Cells | 0.36 | 0.64 | 0.08 | 0.68 | 0.15 | 0.06 | 1.18 | 0.23 | 0.44 |
| | | | | | | | | | |
| Depleted B Cells | 0.99 | 1.05 | 0.99 | 1.07 | 1.15 | 1.06 | 1.09 | 0.97 | 1.05 |
| Depleted Monocytes | 0.86 | 0.83 | 0.30 | 0.96 | 1.03 | 0.26 | 1.02 | 0.39 | 0.62 |
| Depleted NK Cells | 1.30 | 1.26 | 0.85 | 0.85 | 0.21 | 1.12 | 1.07 | 0.95 | 0.92 |
| Depleted T Cells | 1.82 | 1.34 | 2.05 | 1.26 | 1.94 | 2.29 | 0.91 | 1.85 | 1.71 |

**Claims**

1. A method for predicting the survivability of a subject diagnosed with prostate cancer based on a sample from the subject, the sample providing a source of RNAs, comprising:

   a) determining a quantitative measure of a level of an RNA transcript of SPARC, or an expression product thereof,
   b) normalizing the level of the RNA transcript of SPARC, or an expression product thereof, and
   c) predicting the survivability of the subject based on the normalized level of the RNA transcript of SPARC, or an expression product thereof.

2. A method as claimed in claim 1, further comprising determining a normalized level of an RNA transcript, or an expression product thereof, of one or more of ABL2, CAV2, CIQB, CDKN1A, C1QA, SEMA4D, XK, FGF2, BCAM, NUDT4, BCL2, NFATC2, TIMP1, CREBBP, E2F1, MYC, NCOA4, ELA2, and RP51077B9.4.

3. A method as claimed in claim 1 or 2, wherein the normalized level of the RNA transcript of SPARC, or an expression product thereof, is negatively correlated with an increased likelihood of survival of the patient.

4. A method as claimed in any of claim 1, 2 or 3, wherein the normalized level of the RNA transcript, or an expression product thereof, is determined using a PCR-based method.

5. A method as claimed in any preceding claim, wherein the normalized level of the RNA transcript, or an expression product thereof, is normalized relative to the expression level of at least one reference gene.

6. A method as claimed in any preceding claim, further comprising providing an index based on the normalized level of the RNA transcript, or an expression product thereof, that is indicative of the predicted survivability or survival time of the subject.

7. A method as claimed in any preceding claim, wherein the sample is a blood sample.

8. A method as claimed in any preceding claim, wherein the sample is a tissue sample.

9. A method as claimed in claim 8, wherein the tissue sample is a biopsy sample.

Fig. 1

Fig. 2

SEMA4D = 5.46 + 0.66 * TIMP1

Note: Slope based on dead vs. alive logit model

Correct Classification

| % Alive | % Dead |
|---------|--------|
| 85.1% | 86.7% |

Fig. 3

Fig. 4

Abl2Sema4d = 6.16 + 0.68 * ClqaTimp1

Note: Slope based on dead vs. alive logit model

Correct Classification

| % Alive | % Dead |
|---------|--------|
| 95.7% | 93.3% |

Fig. 5

C1TiCd = C1QA + TIMP1 + CDKN1A

AbSelt = 2*ABL2 + SEMA4D + ITGAL

## Fig. 6

Fig. 7

Fig. 8

**Survival Functions**

Lower risk: 2.3*ABL2 - 1.3*C1QA < 21
Higher risk: 2.3*ABL2 - 1.3*C1QA > 21

P-value = 1.87E-05

## Fig. 9

**Survival Functions**

Lower risk: 2.3*ABL2 - 1.3*C1QA < 21
Higher risk: 2.3*ABL2 - 1.3*C1QA > 21

P-value = 4.32E-05

## Fig. 10

**Survival Functions**

Lower risk: 2*ABL1 + SEMA4D + ITGAL - C1QA - TIMP1 - CDKN1A < 21.21
Higher risk: 2*ABL1 + SEMA4D + ITGAL - C1QA - TIMP1 - CDKN1A > 21.21

P-value = 1.11E-04

Fig. 11

**Survival Functions**

Lower risk: 2*ABL1 + SEMA4D + ITGAL - C1QA - TIMP1 - CDKN1A < 21.21
Higher risk: 2*ABL1 + SEMA4D + ITGAL - C1QA - TIMP1 - CDKN1A > 21.21

P-value = 1.96E-04

## Fig. 12

Fig. 13

| Gene | Protein | Effect of Observed Changes | | | Discussion |
|---|---|---|---|---|---|
| | | Cellular Immunity | Humeral Immunity | Macro-φ | |
| C1QA | Complement C1q subunit A | ↓ | ↓ | ↑ | The up-regulation of these genes in patients with increased mortality is indicative of a process that is driving monocyte differentiation (CDKN1A) towards the production of tissue macrophages (C1Qa and TIMP1) and, by default, away from dendritic cells. This process will result in a relative down-regulation of cellular and humoral immunity in these patients.. |
| CDKN1A | Cyclin-dependent kinase inhibitor 1A | ↓ | ↓ | ↑ | |
| TIMP1 | Metalloproteinase inhibitor 1 | ↓ | ↓ | ↑ | |
| ABL2 | Tyrosine-protein kinase ABL2 | ↓ | ↓ | | The down-regulation of these genes in patients with increased mortality is indicative of a down-regulation of cellular and humoral immunity. ABL2, semaphorn-4D and LFA-1 are components of an integrated system required for T-cell motility and antigen surveillance and T-helper cell activity with respect to B-cell activation. |
| ITGAL | Integrin alpha-L (LFA-1) | ↓ | ↓ | | |
| SEMA4D | Semaphorin-4D | ↓ | ↓ | | |

<p style="text-align:center">Fig. 14</p>

PRCA Cht 4 Averaged Gene Expression Response for Enriched and Depleted Cell Types Relative to PBMC's
(* Denotes Cell-Specific Marker)

ENRICHED B CELLS
ENRICHED MONOCYTES
ENRICHED NK CELLS
ENRICHED T CELLS

DEPLETED B CELLS
DEPLETED MONOCYTES
DEPLETED NK CELLS
DEPLETED T CELLS

CTSD
CDKN1A
CD8A
CD82
CD4
CD19
CD14
C1QA
ABL2

GENE LOCI

RELATIVE mRNA EXPRESSION

10

1

0.1

Fig. 15A

PRCA Cht 4 Averaged Gene Expression Response for Enriched and Depleted Cell Types Relative to PBMC's
(* Denotes Cell-Specific Marker)

ENRICHED B CELLS
ENRICHED MONOCYTES
ENRICHED NK CELLS
ENRICHED T CELLS

DEPLETED B CELLS
DEPLETED MONOCYTES
DEPLETED NK CELLS
DEPLETED T CELLS

RELATIVE mRNA EXPRESSION

GAS1    HK1    IRAK3    ITGAL    NCAM1    PLA2G7    SEMA4D    TIMP1    TXNRD1

GENE LOCI

Fig. 15B

PRCA Cht 4 Gene Expression Response for Enriched B Cells Relative to PBMC's
(*Denotes Cell-Specific Marker)

■ PBMC
□ 120340 B CELL ENRICHED
□ 145974 B CELL ENRICHED
□ 156471 B CELL ENRICHED
□ 163876 B CELL ENRICHED
□ 263060 B CELL ENRICHED
□ 289370 B CELL ENRICHED
□ 300823 B CELL ENRICHED
□ 302187 B CELL ENRICHED
□ 306051 B CELL ENRICHED
□ 308535 B CELL ENRICHED
□ 325310 B CELL ENRICHED

Fig. 16A

PRCA Cht 4 Gene Expression Response for Depleted B Cells Relative to PBMC's
(*Denotes Cell-Specific Marker)

Fig. 16B

PRCA Cht 4 Gene Expression Response for Enriched Monocytes Relative to PBMC's
(*Denotes Cell-Specific Marker)

RELATIVE mRNA EXPRESSION

ABL2 C1QA CD14 CD19 CD4 CD82 CD8A CDKN1A CTSD GAS1 HK1 IRAK3 ITGAL NCAM1 PLA2G7 SEMA4D TIMP1 TXNRD1

GENE LOCI

Fig. 17A

■ PBMC
□ 120340 MONOCYTE ENRICHED
□ 145974 MONOCYTE ENRICHED
□ 156471 MONOCYTE ENRICHED
□ 163876 MONOCYTE ENRICHED
□ 263060 MONOCYTE ENRICHED
□ 289370 MONOCYTE ENRICHED
□ 300823 MONOCYTE ENRICHED
□ 302187 MONOCYTE ENRICHED
□ 306051 MONOCYTE ENRICHED
□ 308535 MONOCYTE ENRICHED
□ 325310 MONOCYTE ENRICHED

PRCA Cht 4 Gene Expression Response for Depleted Monocytes Relative to PBMC's
(*Denotes Cell-Specific Marker)

Fig. 17B

GENE LOCI

RELATIVE mRNA EXPRESSION

■ PBMC
□ 120340 MONOCYTE DEPLETED
□ 145974 MONOCYTE DEPLETED
□ 156471 MONOCYTE DEPLETED
□ 163876 MONOCYTE DEPLETED
□ 263060 MONOCYTE DEPLETED
□ 289370 MONOCYTE DEPLETED
□ 300823 MONOCYTE DEPLETED
□ 302187 MONOCYTE DEPLETED
□ 306051 MONOCYTE DEPLETED
□ 308535 MONOCYTE DEPLETED
□ 325310 MONOCYTE DEPLETED

ABL2  C1QA  CD14  CD19  CD4  CD82  CD8A  CDKN1A  CTSD  GAS1  HK1  IRAK3  ITGAL  NCAM1  PLA2G7  SEMA4D  TIMP1  TXNRD1

PRCA Cht 4 Gene Expression Response for Enriched NK Cells Relative to PBMC's
(*Denotes Cell-Specific Marker)

■ PBMC
□ 120340 NK CELL ENRICHED
□ 145974 NK CELL ENRICHED
□ 156471 NK CELL ENRICHED
□ 163876 NK CELL ENRICHED
□ 263060 NK CELL ENRICHED
□ 289370 NK CELL ENRICHED
□ 300823 NK CELL ENRICHED
□ 302187 NK CELL ENRICHED
□ 306051 NK CELL ENRICHED
□ 308535 NK CELL ENRICHED
□ 325310 NK CELL ENRICHED

TXNRD1
TIMP1
SEMA4D
PLA2G7
NCAM1
ITGAL
IRAK3
HK1
GAS1
CTSD
CDKN1A
CD8A
CD82
CD4
CD19
CD14
C1QA
ABL2

GENE LOCI

RELATIVE mRNA EXPRESSION

10
1
0.1

Fig. 18A

PRCA Cht 4 Gene Expression Response for Depleted NK Cells Relative to PBMC's
(*Denotes Cell-Specific Marker)

■ PBMC
□ 120340 NK CELL DEPLETED
□ 145974 NK CELL DEPLETED
□ 156471 NK CELL DEPLETED
□ 163876 NK CELL DEPLETED
□ 263060 NK CELL DEPLETED
□ 289370 NK CELL DEPLETED
□ 300823 NK CELL DEPLETED
□ 302187 NK CELL DEPLETED
□ 306051 NK CELL DEPLETED
□ 308535 NK CELL DEPLETED
□ 325310 NK CELL DEPLETED

RELATIVE mRNA EXPRESSION

GENE LOCI

ABL2  C1QA  CD14  CD19  CD4  CD82  CD8A  CDKN1A  CTSD  GAS1  HK1  IRAK3  ITGAL  NCAM1  PLA2G7  SEMA4D  TIMP1  TXNRD1

Fig. 18B

EP 2 405 022 A2

Fig. 19A

PRCA Cht 4 Gene Expression Response for Depleted T Cells Relative to PBMC's
(*Denotes Cell-Specific Marker)

GENE LOCI

Fig. 19B

■ PBMC
□ 120340 T CELL DEPLETED
□ 145974 T CELL DEPLETED
□ 156471 T CELL DEPLETED
□ 163876 T CELL DEPLETED
□ 263060 T CELL DEPLETED
□ 289370 T CELL DEPLETED
□ 300823 T CELL DEPLETED
□ 302187 T CELL DEPLETED
□ 306051 T CELL DEPLETED
□ 308535 T CELL DEPLETED
□ 325310 T CELL DEPLETED

MDNO Averaged Gene Expression Response for Enriched and Depleted Cell Types Relative to PBMC's
(* Denotes Cell-Specific Marker)

ENRICHED B CELLS
ENRICHED MONOCYTES
ENRICHED NK CELLS
ENRICHED T CELLS

DEPLETED B CELLS
DEPLETED MONOCYTES
DEPLETED NK CELLS
DEPLETED T CELLS

CTSD
CDKN1A
CD8A
CD82
CD4
CD19
CD14
C1QA
ABL2

GENE LOCI

RELATIVE mRNA EXPRESSION

10

1

0.1

Fig. 20A

128

MDNO Averaged Gene Expression Response for Enriched and Depleted Cell Types Relative to PBMC's
(* Denotes Cell-Specific Marker)

Fig. 20B

MDNO Gene Expression Response for Enriched B Cells Relative to PBMC's
(*Denotes Cell-Specific Marker)

■ PBMC
□ 201 B CELL ENRICHED
□ 203 B CELL ENRICHED
□ 207 B CELL ENRICHED
□ 209 B CELL ENRICHED
□ 211 B CELL ENRICHED
□ 217 B CELL ENRICHED
□ 218 B CELL ENRICHED

TXNRD1
TIMP1
SEMA4D
PLA2G7
NCAM1
ITGAL
IRAK3
HK1
GAS1
CTSD
CDKN1A
CD8A
CD82
CD4
CD19
CD14
C1QA
ABL2

GENE LOCI

RELATIVE mRNA EXPRESSION

10
1
0.1

Fig. 21A

MDNO Gene Expression Response for Depleted B Cells Relative to PBMC's
(*Denotes Cell-Specific Marker)

Fig. 21B

EP 2 405 022 A2

Fig. 22A

MDNO Gene Expression Response for Depleted Monocytes Relative to PBMC's
(*Denotes Cell-Specific Marker)

Legend:
- ■ PBMC
- □ 201 MONOCYTE DEPLETED
- □ 203 MONOCYTE DEPLETED
- □ 207 MONOCYTE DEPLETED
- □ 209 MONOCYTE DEPLETED
- □ 211 MONOCYTE DEPLETED
- □ 217 MONOCYTE DEPLETED
- □ 218 MONOCYTE DEPLETED

Gene Loci: TXNRD1, TIMP1, SEMA4D, PLA2G7, NCAM1, ITGAL, IRAK3, HK1, GAS1, CTSD, CDKN1A, CD8A, CD82, CD4, CD19, CD14, C1QA, ABL2

GENE LOCI

RELATIVE mRNA EXPRESSION

Fig. 22B

MDNO Gene Expression Response for Enriched NK Cells Relative to PBMC's (*Denotes Cell-Specific Marker)

Fig. 23A

MDNO Gene Expression Response for Depleted NK Cells Relative to PBMC's
(*Denotes Cell-Specific Marker)

Fig. 23B

EP 2 405 022 A2

MDNO Gene Expression Response for Enriched T Cells Relative to PBMC's
(*Denotes Cell-Specific Marker)

Fig. 24A

MDNO Gene Expression Response for Depleted T Cells Relative to PBMC's
(*Denotes Cell-Specific Marker)

■ PBMC
□ 201 T CELL DEPLETED
□ 203 T CELL DEPLETED
□ 207 T CELL DEPLETED
□ 209 T CELL DEPLETED
□ 211 T CELL DEPLETED
□ 217 T CELL DEPLETED
□ 218 T CELL DEPLETED

RELATIVE mRNA EXPRESSION

GENE LOCI

ABL2  C1QA  CD14  CD19  CD4  CD82  CD8A  CDKN1A  CTSD  GAS1  HK1  IRAK3  ITGAL  NCAM1  PLA2G7  SEMA4D  TIMP1  TXNRD1

Fig. 24B

EP 2 405 022 A2

## REFERENCES CITED IN THE DESCRIPTION

*This list of references cited by the applicant is for the reader's convenience only. It does not form part of the European patent document. Even though great care has been taken in compiling the references, errors or omissions cannot be excluded and the EPO disclaims all liability in this regard.*

### Patent documents cited in the description

- US 61134208 A
- US 61135007 A
- US 61191688 A
- WO 0125473 A
- US 2007023425 W
- WO 9824935 A
- US 4727022 A, Skold
- US 4659678 A, Forrest
- US 4376110 A, David
- US 4275149 A, Litman
- US 4233402 A, Maggio
- US 4230767 A, Boguslaski
- WO O125473 A
- US 5744305 A
- US 7326579 B
- EP 09790154 A

### Non-patent literature cited in the description

- **O'MARCAIGH AS ; JACOBSON RM.** Estimating the Predictive Value of a Diagnostic Test, How to Prevent Misleading or Confusing Results. *Clin. Ped.,* 1993, vol. 32 (8), 485-491
- **PEPE et al.** Limitations of the Odds Ratio in Gauging the Performance of a Diagnostic, Prognostic, or Screening Marker. *Am. J. Epidemiol,* 2004, vol. 159 (9), 882-890
- Clinical Interpretation of Laboratory Procedures. **SHULTZ.** Fundamentals of Clinical Chemistry. W.B. Saunders Company, 1996, 192-199
- **ZWEIG et al.** ROC Curve Analysis: An Example Showing the Relationships Among Serum Lipid and Apolipoprotein Concentrations in Identifying Subjects with Coronory Artery Disease. *Clin. Chem.,* 1992, vol. 38 (8), 1425-1428
- **COOK.** Use and Misuse of the Receiver Operating Characteristic Curve in Risk Prediction. *Circulation,* 2007, vol. 115, 928-935
- **HIRAYAMA et al.** *Blood,* 1998, vol. 92, 46-52
- RNA Isolation Strategies. RNA Methodologies, A laboratory guide for isolation and characterization. Academic Press, 1998, 55-104
- RNA Methodologies, A Laboratory Guide for Isolation and Characterization. Academic Press, 1998
- RNA Isolation and Characterization Protocols. Methods in Molecular Biology. Human Press, 1998, vol. 86, 143-151
- Statistical refinement of primer design parameters
- PCR Applications: protocols for functional genomics. Academic Press, 1999, 55-72
- Nucleic acid detection methods. Molecular Methods for Virus Detection. Academic Press, 1995, 1-24
- **Y.S. LIE ; C.J. PETROPOLUS.** Advances in Quantitative PCR Technology: 5' Nuclease Assays. *Current Opinion in Biotechnology,* 1998, vol. 9, 43-48
- Rapid Thermal Cycling and PCR Kinetics. PCR applications: protocols for functional genomics. Academic Press, 1999, 211-229
- **E. MAGGIO.** Enzyme-Immunoassay. CRC Press, Inc, 1980
- **MAGIDSON, J.** GOLDMineR User's Guide. Statistical Innovations Inc, 1998
- **VERMUNT ; MAGIDSON.** Latent GOLD 4.0 Technical Guide. Statistical Innovations, 2005
- **VERMUNT ; MAGIDSON.** LG-Syntax™ User's Guide: Manual for Latent GOLD® 4.5 Syntax Module. Statistical Innovations, 2007
- Latent Class Cluster Analysis. **VERMUNT J.K. ; J. MAGIDSON.** Applied Latent Class Analysis. Cambridge University Press, 2002, 89-106
- Maximum Likelihood Assessment of Clinical Trials Based on an Ordered Categorical Response. **MAGIDSON, J.** Drug Information Journal. Drug Information Association, 1996, vol. 30, 143-170